# EUROPEAN PATENT APPLICATION

(11) **EP 4 477 230 A2**
(43) Date of publication of application: **18.12.2024**
(21) Application number: 24193312.6
(22) Date of filing: 06.08.2020
(51) Int. Cl.: A61K 39/00

(54) **METHODS AND COMPOSITIONS FOR STABILIZED RECOMBINANT FLAVIVIRUS E PROTEIN DIMERS**

(30) Priority: 06.08.2019 US 201962883382 P
(62) Divisional of application: 20851176.6
(71) Applicant: The University of North Carolina at Chapel Hill, Chapel Hill, NC 27516 (US)
(72) Inventor: KUHLMAN, Brian, North Carolina, 27516 (US); KUDLACEK, Stephan T., Texas, 77493 (US); DE SILVA, Aravinda, North Carolina, 27517 (US); PAYNE, Alexander, New York, 10021 (US); PHAN, Thanh, North Carolina, 27510 (US); METZ, Stefan, North Carolina, 27713 (US)
(74) Representative: HGF

(57) **Abstract**

The present invention provides compositions and methods of use comprising a stabilized recombinant E glycoprotein comprising a flavivirus E glycoprotein backbone, which comprises amino acid substitutions that stabilize the E glycoprotein in dimer conformation under physiological conditions.

## Description

### RELATED APPLICATIONS

This application claims the benefit, under 35 U.S.C. § 119(e), of U.S. Provisional Application Serial No. 62/883,382, filed August 6, 2019, the entire contents of which are incorporated by reference herein.

### STATEMENT OF GOVERNMENT SUPPORT

This invention was made with government support under Grant Number W81XWH1820035 awarded by the Department of Defense. The government has certain rights in the invention.

### STATEMENT REGARDING ELECTRONIC FILING OF A SEQUENCE LISTING

A Sequence Listing in ASCII text format, submitted under 37 C.F.R. § 1.821, entitled 5470-844WO_ST25.txt, 534,075 bytes in size, generated on August 6, 2020 and filed via EFS-Web, is provided in lieu of a paper copy. This Sequence Listing is hereby incorporated herein by reference into the specification for its disclosures

### FIELD OF THE INVENTION

The present invention is directed to recombinant stabilized flavivirus E glycoproteins that induce neutralizing antibodies against quaternary epitopes and form dimers in physiological, e.g., vaccine, conditions.

### BACKGROUND OF THE INVENTION

Vaccine control of dengue virus (DENV), a member of the flavivirus family, has remained elusive. About 25% of the ~400 million annual DENV infections worldwide result in severe clinical manifestations, necessitating effective vaccine control of DENV. A substantial challenge in eliminating DENV infections is the prevalence of four DENV serotypes, each capable of eliciting both unique, serotype-specific and DENV cross-reactive immune responses upon infection. Some responses, such as the immune response following a first or "primary" infection with one DENV serotype, can lead to lifelong protection against that serotype. However, all four DENV serotypes co-circulate in the same regions and are transmitted by the same *A. aegypti* and *A. albopictus* mosquito vectors, which allows for infections of the same individual by a different DENV serotype. These secondary DENV infections can result in severe and sometimes lethal disease symptoms, such as dengue hemorrhagic fever and shock syndromes. A supported hypothesis for the cause of these severe dengue clinical manifestations is through increased viral burden caused by antibody-dependent enhancement (ADE), where a population of non-neutralizing cross-reactive antibodies elicited from the primary DENV infection are able to bind to the DENV serotype present in the second infection, allowing for Fc-receptor mediated endocytosis of the virus by macrophages thus enhancing the infection through increased replication of the virus. Despite these challenges, advancements have been made with multiple live-attenuated dengue vaccines including the only approved dengue vaccine, Dengvaxia (Rey et al., 2018 EMBO Rep. 19:206-224; Thomas and Yoon, 2019 Hum. Vaccines Immunother. 15:2295-2314), and several others in late stage clinical trials. Dengvaxia has proven capable of providing protection to people who have overcome a primary dengue infection against a secondary dengue infection. However, it is evidenced that vaccinating individuals who have had no prior dengue infections increases their risk of developing severe clinical manifestations of dengue, which is suggested to be caused by ADE. These observations highlight the difficulty of vaccine mediated control of dengue infection and the need for more targeted vaccination strategies to prevent DENV infections.

Characterization of antibodies elicited by DENV infected individuals reveal that the DENV envelope protein is the primary target of neutralizing antibodies, which has led to a large body of work in assessing the immunogenicity and efficacy of this protein as a subunit vaccine. While promising, E protein-based subunit vaccines have only had limited success in clinical trials. Recently, it has been appreciated that antibodies that target quaternary epitopes, present within a single E dimer and across multiple dimers in the E dimer lipid raft, are potently neutralizing and provide protection against DENV infection.

Previous work has shown that the DENV2 soluble E protein dimer has a homodimer Kd of 12µM at 37°C, which is too weak to be relevant under vaccination conditions and limits its current use as a vaccine antigen by reducing the presentation of critical quaternary epitopes. The present invention overcomes shortcomings in the art by providing flavivirus E glycoproteins that retain dimer conformation at physiological conditions and can be expressed without co-expression of pr, allowing for safer vaccines.

### SUMMARY OF THE INVENTION

In one aspect, the present invention provides a stabilized recombinant flavivirus E glycoprotein, comprising a flavivirus E glycoprotein backbone and an amino acid substitution selected from the group consisting of 2M, 6L, 8L, 9V, 13F, 14A, 15E, 27P, 29Y, 29K, 32V, 33V, 34L, 35M, 44M, 44L, 481, 106D, 107C, 131I, 144Y, 154L, 154M, 191Y, 198W, 204F, 205L, 206F, 209D, 244Q, 244F, 246I, 246Y, 251F, 255E, 256Y, 258A, 258E, 259V, 259W, 259C, 260L, 261L, 261F, 262H, 262R, 262Y, 263W, 263L, 266W, 270V, 277M, 279W, 280A, 280P, 289W, 299L, 313C, 316M, 330A, 359Y, 373D, 375L, 377V, 386I, 390Q, 392R,393R, and/or any combination thereof (e.g., amino acid substitutions shown in **Table 1** and/or **Table 2**), wherein the numbering is based on the reference amino acid sequence of an E glycoprotein of dengue virus serotype 2 (DENV2) identified as **SEQ ID NO:6.**

In another aspect, the present invention provides a flavivirus E glycoprotein dimer comprising two stabilized recombinant flavivirus E glycoproteins of the present invention.

Additionally provided herein is a dengue virus particle, a flavivirus particle and/or a virus like particle (VLP) comprising the E glycoprotein of this invention.

An isolated nucleic acid molecule encoding the E glycoprotein of this invention is also provided herein, as well as an isolated nucleic acid molecule encoding the dengue virus particle, flavivirus particle or VLP of this invention.

The present invention also provides a composition comprising the E glycoprotein of this invention in a pharmaceutically acceptable carrier and also provides a composition comprising the E glycoprotein dimer of this invention, nucleic acid molecule of this invention, the vector of this invention, the particle of this invention and/or the population of this invention, in a pharmaceutically acceptable carrier.

The present invention also provides a method of producing a virus like particle (VLP) comprising the E glycoprotein of the present invention, wherein the method does not comprise co-expression of pr.

The present invention further provides the E glycoprotein of this invention, the E glycoprotein dimer of this invention, the dengue virus particle of this invention, the flavivirus particle of this invention, the VLP of this invention, the nucleic acid molecule of this invention, the vector of this invention, the population of this invention and/or the composition of this invention, singly or in any combination, for use in the manufacture of a medicament for producing an immune response to a flavivirus in a subject, for treating a flavivirus infection in a subject in need thereof, for preventing a flavivirus infection in a subject and/or for protecting a subject from the effects of flavivirus infection, and/or for use in any of the methods as disclosed herein.

Also provided herein is the use of the E glycoprotein of this invention, the E glycoprotein dimer of this invention, the dengue virus particle of this invention, the flavivirus particle of this invention, the VLP of this invention, the nucleic acid molecule of this invention, the vector of this invention, the population of this invention and/or the composition of this invention, singly or in any combination, for use in producing an immune response to a flavivirus in a subject, in treating a flavivirus infection in a subject in need thereof, in preventing a flavivirus infection in a subject and/or in protecting a subject from the effects of flavivirus infection, and/or for use in any of the methods as disclosed herein.

Also provided herein is a method of producing an immune response to a flavivirus in a subject (e.g., a subject in need thereof), comprising administering to the subject an effective amount of the E glycoprotein of this invention, the E glycoprotein dimer of this invention, the flavivirus particle of this invention, the VLP of this invention, the nucleic acid molecule of this invention, the population of this invention, and/or the composition of this invention and any combination thereof.

Additionally provided herein is a method of treating a flavivirus infection in a subject (e.g., a subject in need thereof), comprising administering to the subject an effective amount of the E glycoprotein of this invention, the E glycoprotein dimer of this invention, the flavivirus particle of this invention, the VLP of this invention, the nucleic acid molecule of this invention, the population of this invention, and/or the composition of this invention and any combination thereof.

Further provided herein is a method of preventing a disorder associated with flavivirus infection in a subject (e.g., a subject in need thereof), comprising administering to the subject an effective amount of the E glycoprotein of this invention, the E glycoprotein dimer of this invention, the flavivirus particle of this invention, the VLP of this invention, the nucleic acid molecule of this invention, the population of this invention, and/or the composition of this invention and any combination thereof.

As an additional aspect, the present invention provides a method of protecting a subject from the effects of flavivirus infection, comprising administering to the subject an effective amount of the E glycoprotein of this invention, the E glycoprotein dimer of this invention, the flavivirus particle of this invention, the VLP of this invention, the nucleic acid molecule of this invention, the population of this invention, and/or the composition of this invention and any combination thereof.

In further aspects, the present invention provides methods of identifying the presence of a neutralizing antibody to a flavivirus in a biological sample from a subject, comprising: a) administering a composition comprising an E glycoprotein of the present invention or an E glycoprotein dimer of the present invention to the subject in an amount effective to induce an antibody response to the E glycoprotein; b) contacting a biological sample from the subject with flavivirus particles comprising the E glycoprotein of step (a) above under conditions whereby neutralization of the flavivirus particles can be detected; and c) detecting neutralization in step (b), thereby identifying the presence of a neutralizing antibody to the flavivirus in the biological sample from the subject.

The present invention additionally provides methods of identifying the presence of a neutralizing antibody to a flavivirus in a biological sample from a subject, comprising: a) contacting a biological sample from a subject that has been administered an E glycoprotein of the present invention or an E glycoprotein dimer of the present invention with flavivirus particles comprising the E glycoprotein under conditions whereby neutralization of the flavivirus particles can be detected; and b) detecting neutralization in step (a), thereby identifying the presence of a neutralizing antibody to the flavivirus in the biological sample from the subject.

In other embodiments, the present invention provides methods of identifying an immunogenic composition that induces a neutralizing antibody to a flavivirus in a subject, the method comprising: a) administering an immunogenic composition comprising an E glycoprotein of the present invention or an E glycoprotein dimer of the present invention to a subject in an amount effective to induce an antibody response to the E glycoprotein; b) contacting a biological sample from the subject with flavivirus particles comprising the E glycoprotein of step (a) under conditions whereby neutralization of the flavivirus particles can be detected; c) determining if the biological sample comprises an antibody that neutralizes flavivirus particles comprising the E glycoprotein of step (a); and d) identifying the immunogenic composition as inducing a neutralizing antibody to the flavivirus in the subject if the biological sample comprises an antibody that neutralizes flavivirus particles comprising the E glycoprotein of (a).

Further provided herein are methods of identifying an immunogenic composition that induces a neutralizing antibody to a flavivirus in a subject, the method comprising: a) contacting a biological sample from a subject that has been administered an immunogenic composition comprising an E glycoprotein of the present invention or an E glycoprotein dimer of the present invention with flavivirus particles comprising the E glycoprotein under conditions whereby neutralization of the flavivirus particles can be detected; b) determining if the biological sample comprises an antibody that neutralizes flavivirus particles comprising the E glycoprotein of step (a); and c) identifying the immunogenic composition as inducing a neutralizing antibody to the flavivirus in the subject if the biological sample comprises an antibody that neutralizes flavivirus particles comprising the E glycoprotein of (a).

The present invention also provides methods of detecting an antibody to a flavivirus in a sample, comprising: a) contacting the sample with an E glycoprotein of the present invention or an E glycoprotein dimer of the present invention under conditions whereby an antigen/antibody complex can form; and b) detecting formation of an antigen/antibody complex, thereby detecting an antibody to the flavivirus in the sample.

The present invention also provides methods of identifying an antibody to a flavivirus in a biological sample from a subject, comprising: a) administering a composition comprising an E glycoprotein of the present invention or an E glycoprotein dimer of the present invention to the subject in an amount effective to induce an antibody response to the E glycoprotein; b) contacting a biological sample from the subject with the E glycoprotein of (a) under conditions whereby an antigen/antibody complex can form; and c) detecting formation of an antigen/antibody complex, thereby identifying an antibody to the flavivirus in the biological sample from the subject.

A further embodiment of the present invention includes methods of identifying an antibody to a flavivirus in a biological sample from a subject, comprising: a) contacting a biological sample from a subject that has been administered an immunogenic composition comprising an E glycoprotein of the present invention or an E glycoprotein dimer of the present invention with the E glycoprotein under conditions whereby an antigen/antibody complex can form; and b) detecting formation of an antigen/antibody complex, thereby identifying an antibody to the flavivirus in the biological sample from the subject.

Another embodiment of the present invention include methods of identifying an immunogenic composition that induces an antibody to a flavivirus in a subject, the method comprising: a) contacting a biological sample from a subject that has been administered an immunogenic composition comprising an E glycoprotein of any of the present invention or an E glycoprotein dimer of the present invention with the E glycoprotein under conditions whereby an antigen/antibody complex can form; and b) detecting formation of an antigen/antibody complex, thereby identifying an immunogenic composition that induces an antibody to the flavivirus in the subject.

Further aspects of the present invention include methods of identifying an immunogenic composition that induces a neutralizing antibody to a flavivirus in a subject, comprising: a) administering an immunogenic composition comprising an E glycoprotein of the present invention or an E glycoprotein dimer of the present invention to a subject in an amount effective to induce an antibody response to the E glycoprotein; b) contacting a biological sample from the subject with the E glycoprotein of (a) under conditions whereby an antigen/antibody complex can form; and c) detecting formation an antigen/antibody complex, thereby identifying an immunogenic composition that induces a neutralizing antibody to the flavivirus in the subject.

Another aspect of the present invention provides methods of producing a stabilized recombinant E glycoprotein of the present invention, comprising: introducing one or more amino acid substitutions into a flavivirus E glycoprotein backbone, wherein the one or more amino acid substitutions are selected from the group consisting of 2M, 6L, 8L, 9V, 13F, 14A, 15E, 27P, 29Y, 29K, 32V, 33V, 34L, 35M, 44M, 44L, 48I, 106D, 107C, 131I, 144Y, 154L, 154M, 191Y, 198W, 204F, 205L, 206F, 209D, 244Q, 244F, 246I, 246Y, 251F, 255E, 256Y, 258A, 258E, 259V, 259W, 259C, 260L, 261L, 261F, 262H, 262R, 262Y, 263W, 263L, 266W, 270V, 277M, 279W, 280A, 280P, 289W, 299L, 313C, 316M, 330A, 359Y, 373D, 375L, 377V, 386I, 390Q, 392R,393R, and/or any combination thereof (e.g., amino acid substitutions shown in **Table 1** and/or **Table 2**), wherein the numbering is based on the reference amino acid sequence of an E glycoprotein of dengue virus serotype 2 (DENV2) identified as **SEQ ID NO:6,** thereby stabilizing the flavivirus E glycoprotein into dimer conformation.

### BRIEF DESCRIPTION OF THE DRAWINGS

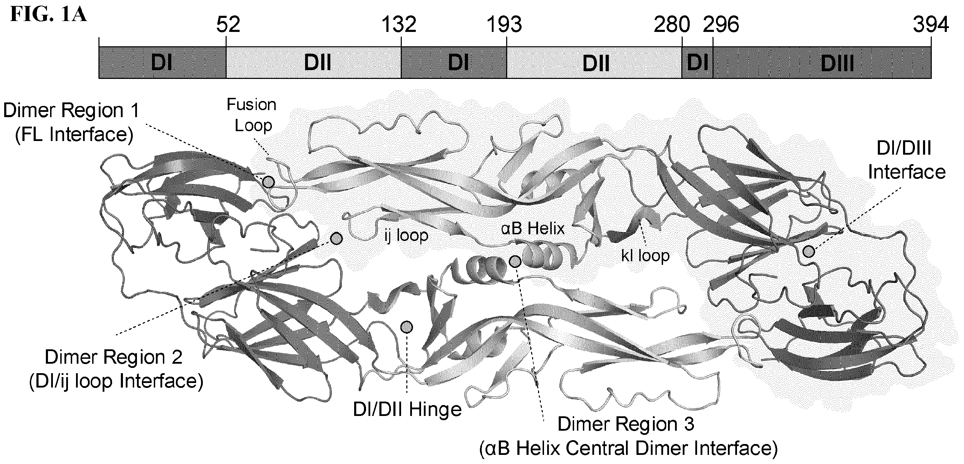

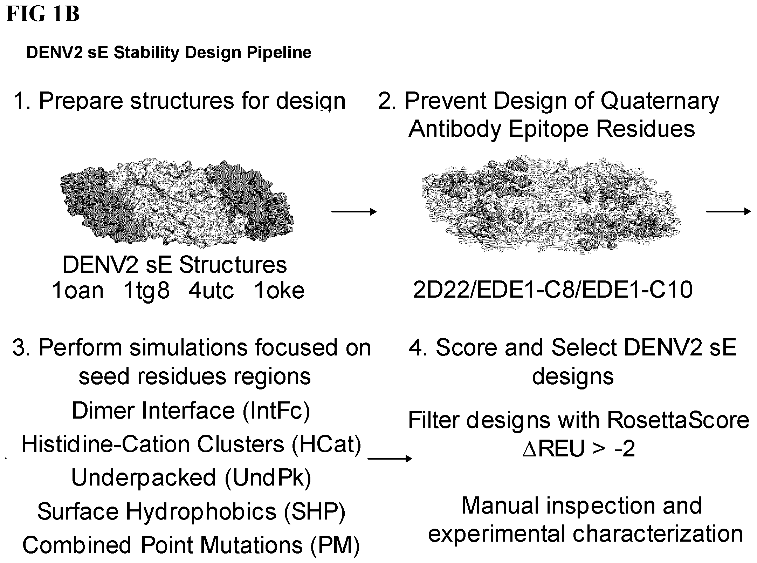

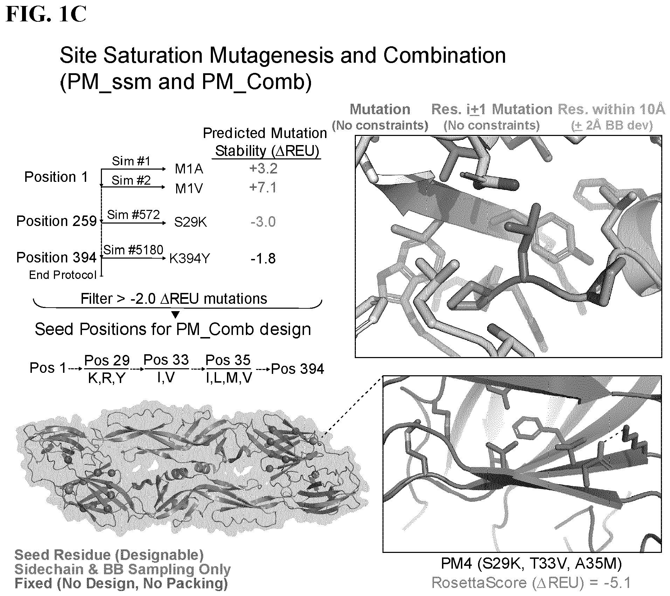

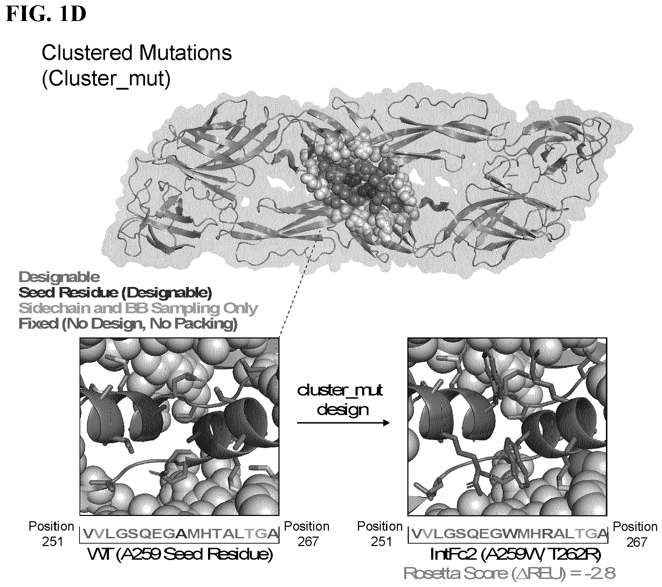

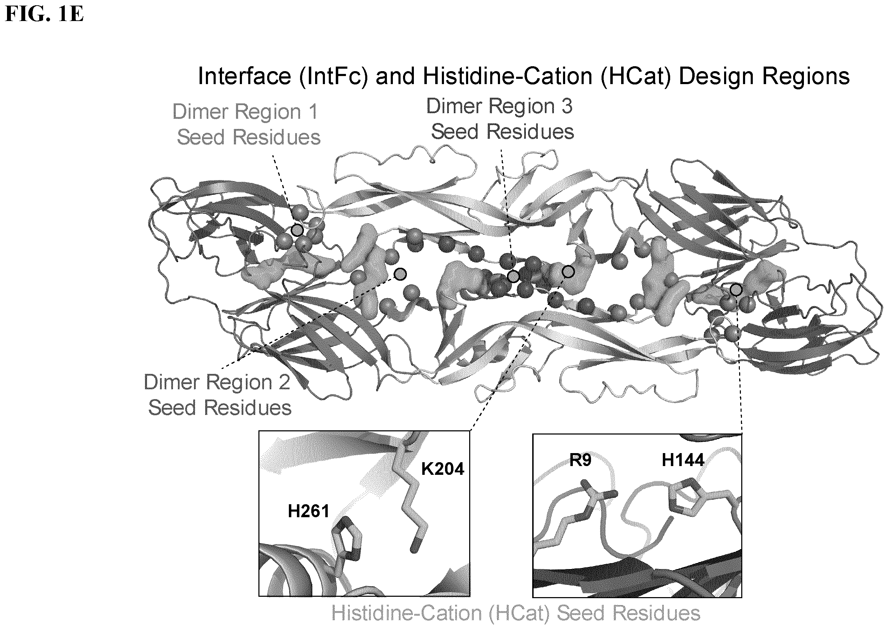

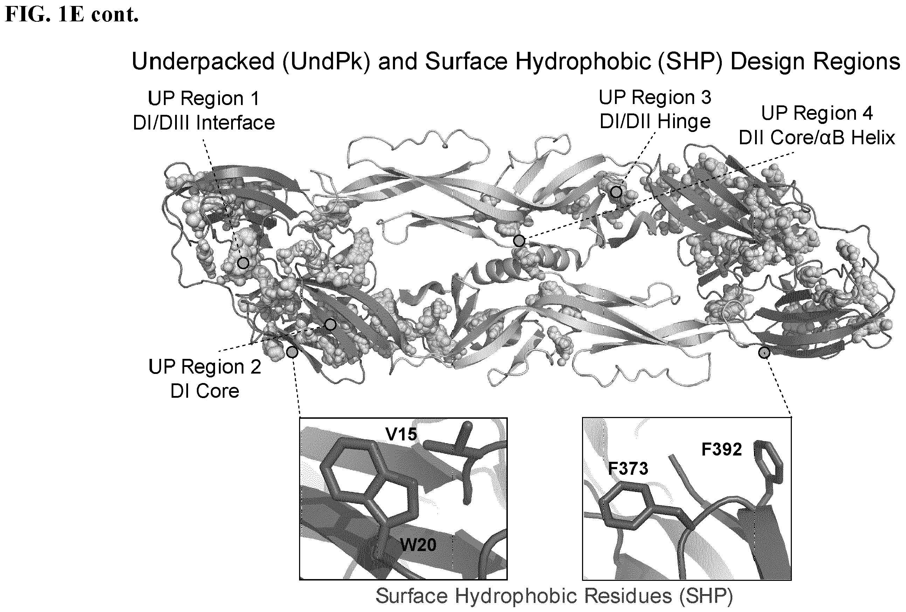

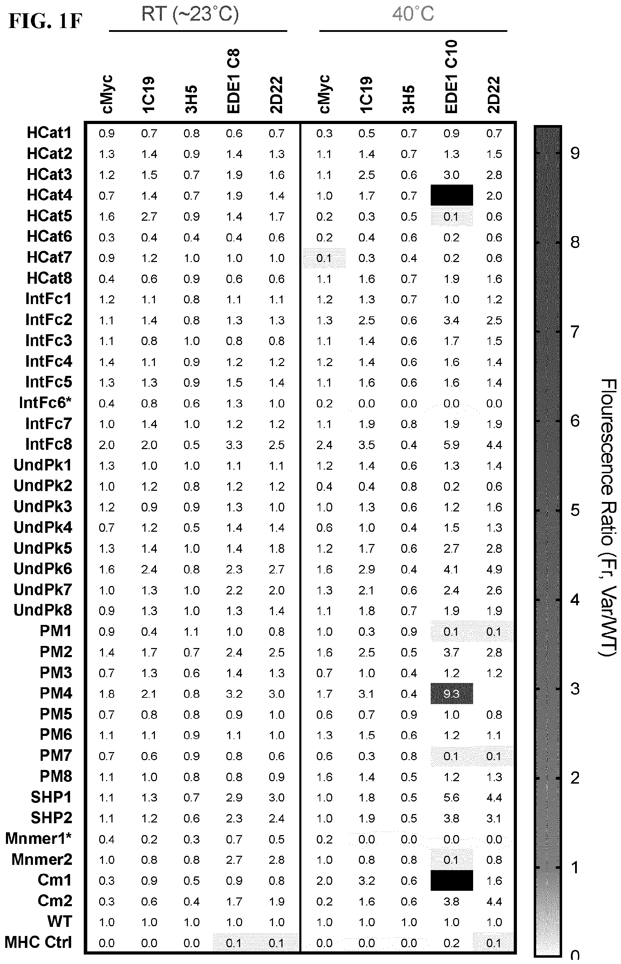

**FIGS. 1A-1F** show schematics of the computational design strategy for the identification of DENV2 sE dimer and monomer stabilizing mutations. **FIG. 1A** shows a schematic of the DI/DII hinge containing the structural kl loop motif, and DI/DIII linker regions, which together form the anti-parallel homodimer. The sE dimer interface can be described by three regions, Dimer Region 1, containing the fusion loop (FL) peptide (FL interface), Dimer Region 2, where the ij loop of one monomer contacts DI of the adjacent monomer, and Dimer Region 3, the central dimer interface where the αB helix joins to at the 2-fold axis of the dimer. **FIG. 1B** shows an overview of the *in silico* exhaustive site-saturation mutagenesis protocol (PM_ssm), which independently simulated mutating every residue to each of the 20 canonical amino acids (AA) at every position in the sE dimer. The protocol walked through every residue position in primary sequence and executed an independent simulation where the residue is mutated and the design model is scored using the Rosetta scorefunction. The rosetta total score of the mutated design was subtracted by the score of the simulation where the residue is mutated to the WT residue, representing the change in rosetta score relative to WT (ΔREU), predicting the stability change upon mutation. The position being mutated and the primary sequence neighbors, residue i+1 and i-1, had no backbone (BB) constraints, allowing freedom of the backbone to accommodate the new mutation. All residues within a 10Å sphere of these residues underwent constrained BB sampling (+ 2Å deviation) and sidechain repacking to alleviate clashes introduced by the mutation. All other residues were fixed and had no backbone or sidechain sampling. The mutations with ΔREU > -2 were rejected and the remaining mutations were used as seed residues to be combined in silico using the PM_Comb protocol into entire domains, domain interfaces, or at the dimer interface. **FIG. 1C** shows the IntFc, HCat and UndPk simulations that were performed using the cluster_mut protocol to identify clusters of stabilizing mutations that form new contacts to each other and to the WT residues **(****FIG. 1C****, right).** All residues within 7Å the seed residue (dark) were designed together (light) allowing side chain and constrained BB sampling of residues up to 10Å away from the seed residue, excluding the designable residues, were allowed to accommodate the mutations **(****FIG. 1C****, top and left).** **FIG. 1D** shows a schematic of regions in the DENV2 sE dimer selected to focus the Rosetta simulations for identification of stabilizing DENV2 sE dimer and monomer mutations. **FIG. 1D****, Top** shows the seed residues, represented as spheres, in the Dimer region 1, Dimer region 2, and Dimer Region 3 that were used in the IntFc simulations. Histidine-cation (HCat) clusters, involved in the low pH dissociation of the sE dimer to trimer, were targeted and used as seed residues for the HCat simulations. **FIG. 1D****, Bottom** shows RosettaHoles analysis used to identify regions throughout the DENV2 sE dimer predicted to contribute to both dimer and monomer instability. Residues within the DI/DIII interface (UP Region 1), DI core (UP Region 2), DI/DII Hinge (UP Region 3) and DII core and αB helix (UP region 4) were selected as seed residues for the UndPk simulations. Hydrophobic residues normally buried in the E dimer on the virus or at the E post-fusion trimer interface, surface exposed in the sE dimer, were selected as input for the SHP simulations to be redesigned to polar residues. Sequences shown are amino acid residues in position 251-267 of SEQ ID NO:6 (WT DENV2 sE) and of SEQ ID NO:# (IntFc2), wherein the numbering is based on the reference amino acid sequence of an E glycoprotein of dengue virus serotype 2 (DENV2) identified as SEQ ID NO:6. **FIG. 1E** shows an overview of the DENV2 sE stability design simulations pipeline. The four representative DENV2 sE crystal structures, PDB 1oan, 1tg8, 1oke and 4utc were minimized using the FastRelax Rosetta algorithm in preparation for the simulations (Step 1). Residues representing the contacts made by quaternary epitope antibodies 2D22, EDE1 C8 and EDE1 C10 (Step 2, spheres) are selected to prevent design during the simulations. The seed residue for a given simulation is passed to the cluster_mut protocol or multiple seed residues to the PM_Comb protocol using the logic shown in **FIG. 1B** **&** **FIG. 1D****,** where the designated residues are mutated to any of the 20 canonical AA, except cysteine (Step 3). After design was complete, each design was evaluated using the rosetta scorefunction and the process repeated until 100 design models per simulation were produced. After aggregating the results, the designs with ΔREU > -2 were discarded and designs were manually inspected and selected for further experimental characterization (Step 4). **FIG. 1F** shows a table of cytometry data of cells displaying DENV2 sE variant proteins bound to cMyc, 1C19, 3H5, 2D22, EDE1 C8 and EDE1 C10 antibodies, represented by geometric mean fluorescence intensity ratio (Fr) of DENV2 sE variant to DENV2 sE WT displayed cells. Fr data for cells stained at RT(23°C, left) or 40°C (right) are presented. Fr values > 1 indicate improved antibody binding to DENV2 sE variants, while Fr values ≈ 1 and <1 represent similar and reduced antibody binding, respectively. Black squares indicate antibody binding was not tested for those sE variants. Asterisks indicate DENV2 sE variants (IntFc6 and Mnmer2) were unfolded after incubation at 40°C evidenced by no observed binding of the DENV antibodies.

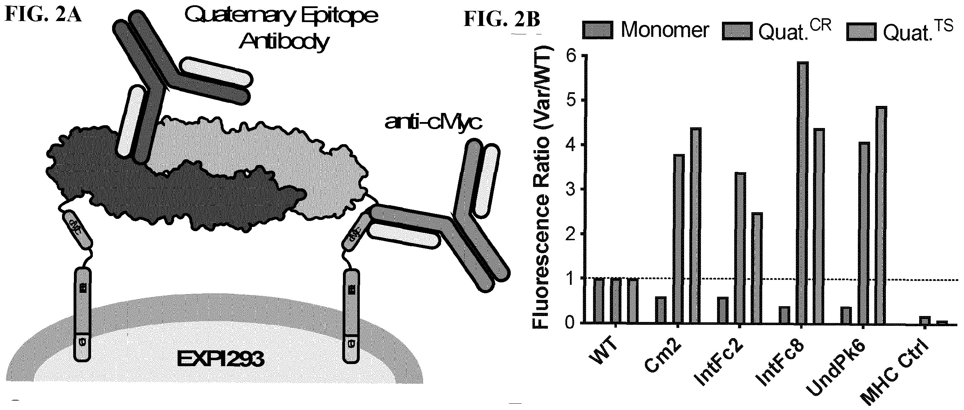

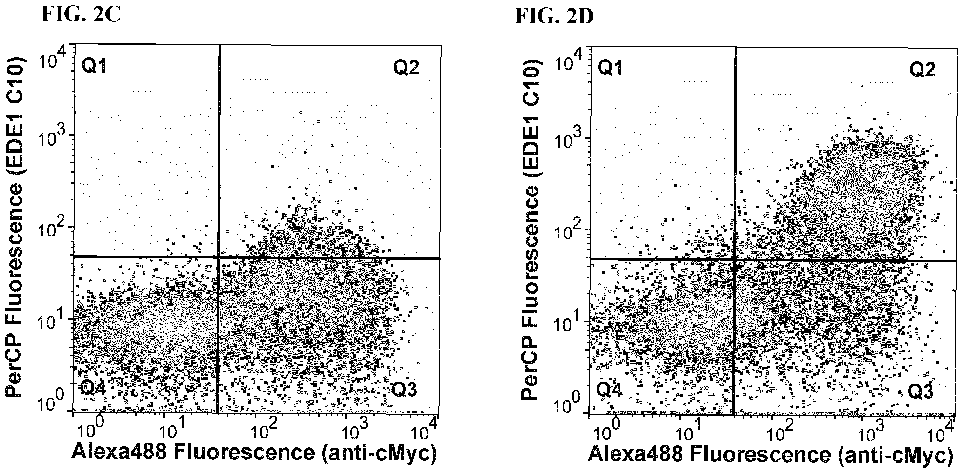

**FIGS. 2A-2D** show representative flow cytometry data of antibody binding to mammalian surface displayed DENV2 sE variants. Mammalian surface display was used to screen for improved DENV2 sE Rosetta variant quaternary epitope presentation at 37°C. **FIG. 2A** shows a diagram of DENV2 sE mammalian display platform. Secreted DENV2 sE are displayed on the surface of EXPI293F cells by genetically fusing the c-terminus to the MHC Iα cytoplasmic and transmembrane domains via a GS linker. In the linker, a cMyc epitope tag is inserted for detection of successful surface display by anti-cMyc antibody binding. Quaternary epitope presentation of the DENV2 sE variants are detected via binding of representative quaternary epitope antibodies. **FIG. 2B** shows a bar graph displaying flow cytometric analysis of monomer epitope antibody 3H5 ("monomer"; left bar), and cross-reactive EDE1 C10 ("Quat.^{CR}"; center bar) and type-specific 2D22 ("Quat.^{TS}"; right bar) quaternary epitope antibody binding to surface displayed DENV2 sE WT, DENV2 sE variants (Cm2, IntFc2, IntFc8 and UndPk6) and membrane anchor negative control (MHC ctrl). The geometric mean fluorescence ratio (Fr) of sE variant to sE WT surface displayed cells post antibody immunostaining are plotted. **FIGS. 2C** and **2D** show representative flow cytometry plots with the representative gating strategy for analysis of DENV anti-E monomer and quaternary epitope specific antibody binding to DENV2 sE proteins, represented double stained cells displaying DENV2 sE WT **(****FIG. 2C****)** or DENV2 sE UndPk6 **(FIC. 2D)** at 40°C. sE surface displayed cells were stained with chicken anti-cMyc (x-axis) and detected by secondary anti-chicken antibody (Alexa-488) to discriminate cells displaying sE (Q3) from cells with no sE display (Q4) and also stained with anti-DENV E antibody (represented by EDE1 C10) and detected by fluorescently-labeled goat anti-human IgG (PerCP), to differentiate between sE proteins presenting (Q2) or lacking epitope presentation measured by antibody binding. The PerCP geometric mean fluorescence intensity of gated cMyc positive cells is recorded (Q3 & Q4) and used for Fr analysis.

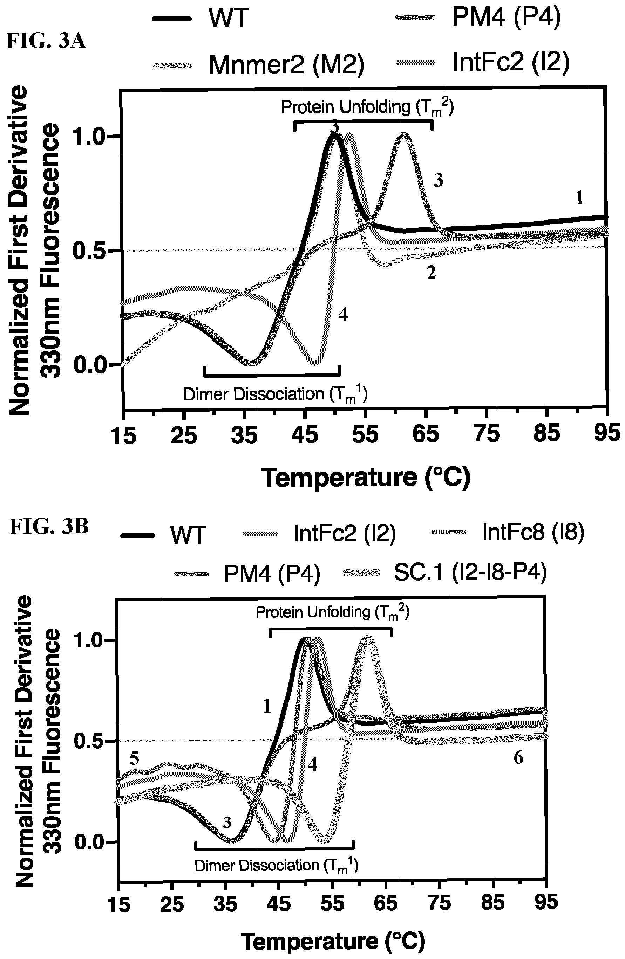

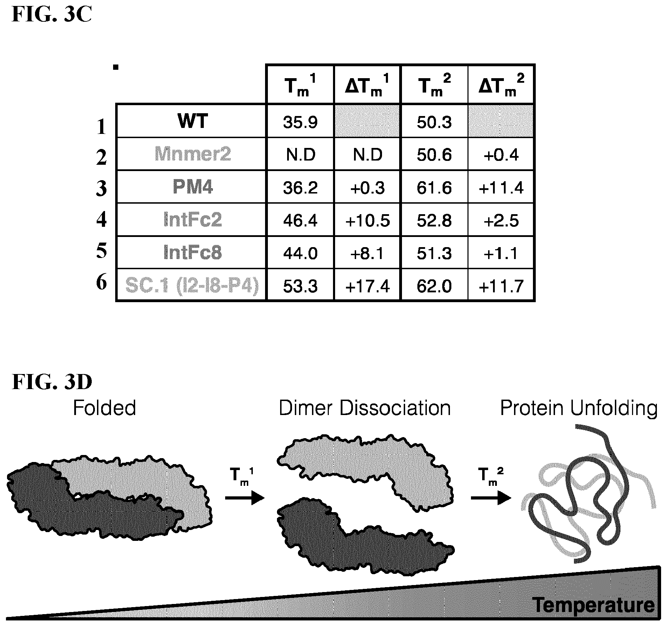

**FIGS. 3A-3D** show representative nanoDSF measured DENV2 sE protein nanoDSF thermal melt experiments at 4µM concentration, monitoring 330nm fluorescence from 15°C to 95°C. **FIG. 3A** shows first-derivative 330nm nanoDSF thermal melt plots of DENV2 sE WT (black; 1), Mnmer2 (pink; 2), PM4 (purple; 3) and IntFc2 (blue; 4). DENV2 sE protein thermal melts contain two melting transitions, with sE dimer dissociation corresponding in the first transition (Tm1, negative first derivative), followed by complete protein unfolding occurring during the second transition (Tm2, positive first derivative). Increases in dimer stability or monomer stability were observed with increases in Tm1 or Tm2 as observed for IntFc2 and PM4 respectively. Loss of the Tm 1 transition for Mnmer2 is indicative of no observed dimer dissociation. **FIG. 3B** thermal melt traces for DENV2 sE WT, IntFc2 (I2, blue; 4), IntFc8 (I8, green; 5) and PM4 (P4, purple; 3) are shown. The sE stable combination (SC) variant, SC.1 (orange; 6) containing I2, I8 and P4, had a nearly identical Tm2 as P4, and Tm1 close to the addition of I2 and I8's change in Tm1 (dimer stability) compared to WT. **FIG. 3C** shows a table of quantified absolute (Tm1 and Tm2) and relative to WT (ΔTm1 and ΔTm2) melting points for DENV2 sE proteins presented in **FIGS. 3A** and **3B****.** **FIG. 3D** shows a schematic of the DENV2 sE nanoDSF experiment model, describing the sE conformations as a function of temperature. At low temperature, dimer formation is favored, with increasing temperature inducing dimer dissociation observed in the first melting transition corresponding to Tm1, followed by complete protein unfolding and aggregation occurring during the second transition (Tm2).

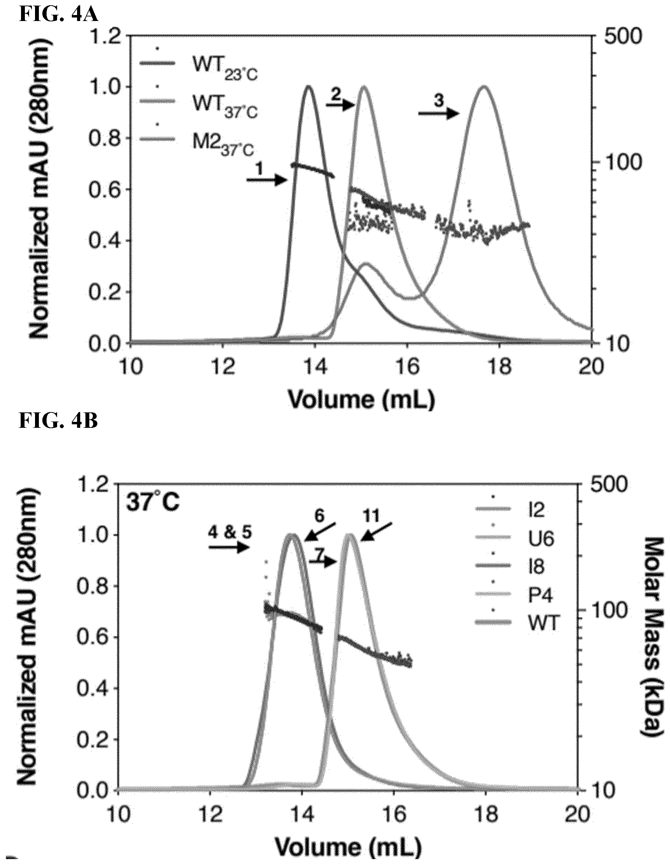

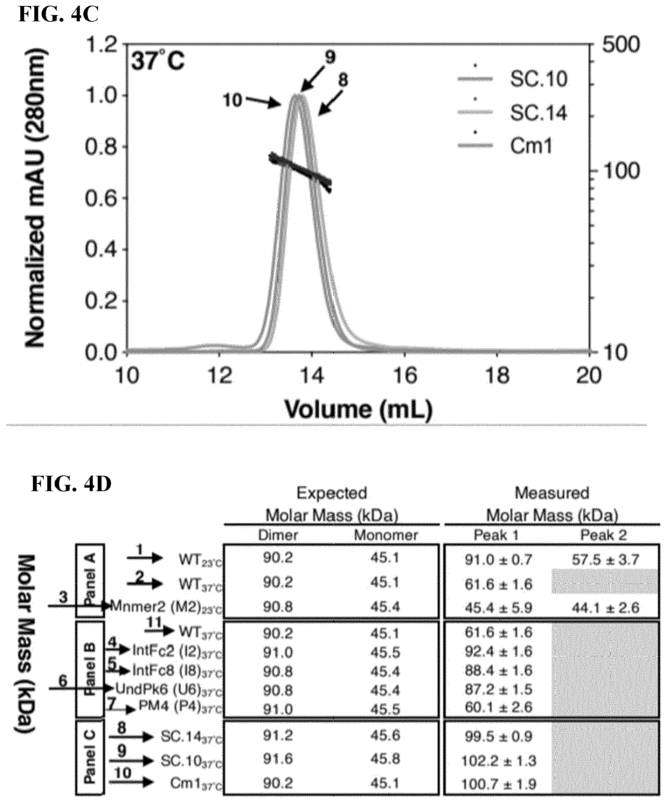

**FIGS. 4A-4D** show validation of nanoDSF DENV2 sE variant dimer stability using SEC-MALS. Size-exclusion coupled with multi-angle light scattering (SEC-MALS) experiments were used to validate the improved dimer stability of DENV2 sE variants by measuring their oligomeric state at RT and 37°C. **FIG. 4A** shows a SEC-MALS experiment using 250µg of DENV2 sE WT at 2.5mg/mL and 150µg DENV2 sE Mnmer2 at 1.5mg/mL, both independently loaded onto a GE Superdex 10/200 gL analytical size exclusion column for SEC-MALS at RT (WT and Mnmer2), or at 37°C (WT). Plotted are the protein elution volumes, on the x-axis, with the normalized absorbance at 280nm (lines) on the left x-axis and the protein molar mass (dots) calculated from MALS data on the right y-axis. **FIG. 4B** shows a SEC-MALS experiment using 250 µg of DENV2 sE WT, IntFc8 (I8), UndPk6 (U6), and PM4 (P4) at 2.5mg/mL and 160µg of DENV2 sE IntFc2 (I2) at 1.6 mg/mL (I2) as analyzed at 37°C. **FIG. 4C** shows 37°C SEC-MALS analysis of stable combination variants (SC) DENV2 sE SC. 10 and SC. 14 and disulfide stabilized DENV2 sE Cm1 loaded with 250µg at 2.5 mg/mL. **FIG. 4D** shows a table of theoretical (expected) and MALS measured molar masses of DENV2 sE proteins at RT or 37°C presented in **FIGS. 4A-4C****,** with the Peak 1 or 2 mean molar mass + SEM (%) of the fitted MALS data reported. The absorbance peaks at the earliest elution volume and the later elution volume corresponding to Peak 1 and 2, respectively.

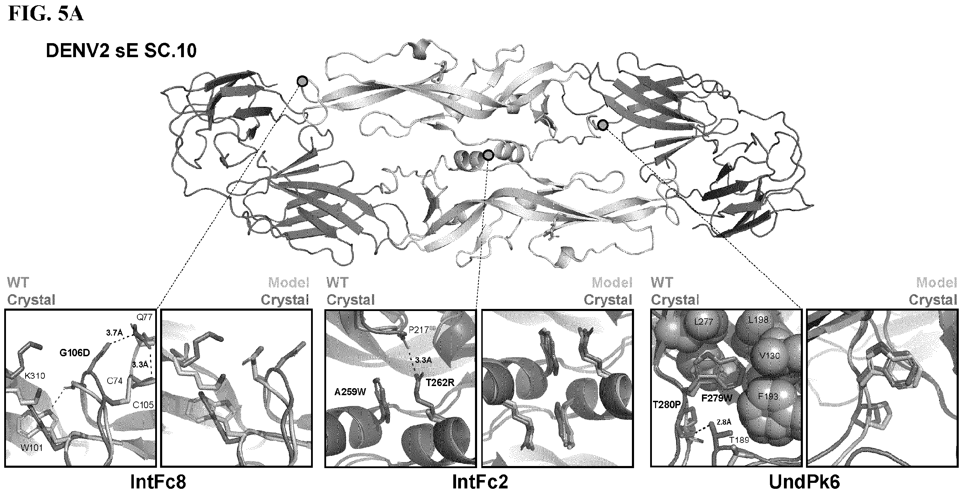

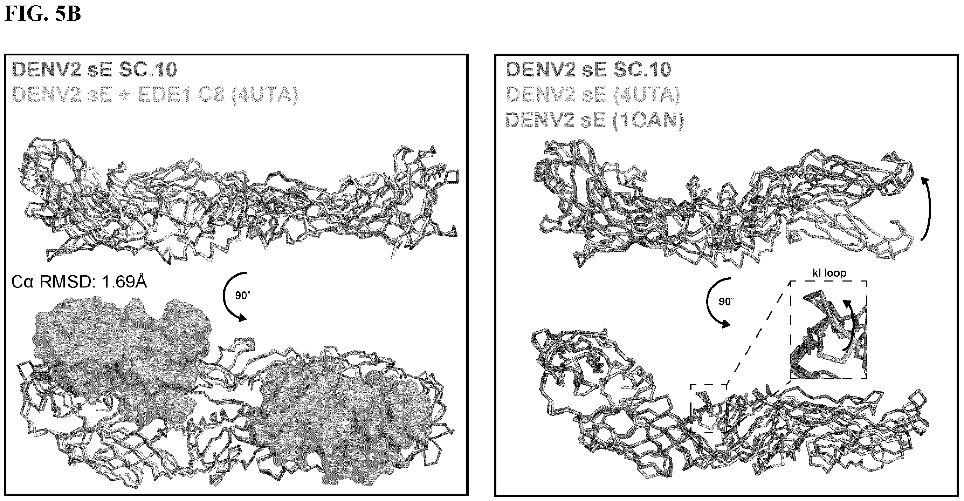

**FIGS. 5A-5B** show schematics of DENV2 sE SC. 10 crystal structure comparisons to Rosetta model and EDE1 C8/sE co-crystal structure. **FIG. 5A****, Top** shows DENV2 sE SC. 10 dimer conformation observed in the crystal structure, colored by domain (DI), (DII), (DII). The red dotted line represents the DI glycan loop residues, unmodeled due to insufficient density and disorder of the loop. **FIG. 5A****, Bottom** shows a comparison of IntFc8 (left) IntFc2 (middle) and UndPk6 (right) mutations observed in the crystal structure to DENV2 sE WT crystal structure (PDB 1oan), the structure used as input for design of these variants, and the Rosetta predicted model. Hydrogen bonds are represented by black dotted lines. **FIG. 5B****, Left** shows the root mean square deviation (RMSD) Cα alignment of the DENV2 sE SC. 10 dimer with the DENV2 sE WT dimer co-crystallized with EDE1 C8, a DENV broadly neutralizing quaternary epitope antibody, using PyMOL align algorithm, with surface representation of the EDE1 C8 Fabs. **FIG. 5B****, Right** shows the Cα alignment of the DI and DIII domains from DENV2 sE WT sE (PDB 1OAN and 4UTA) and DENV2 sE SC. 10. Inset shows a closer view of the kl loop conformation observed in each structure after DI and DIII alignment.

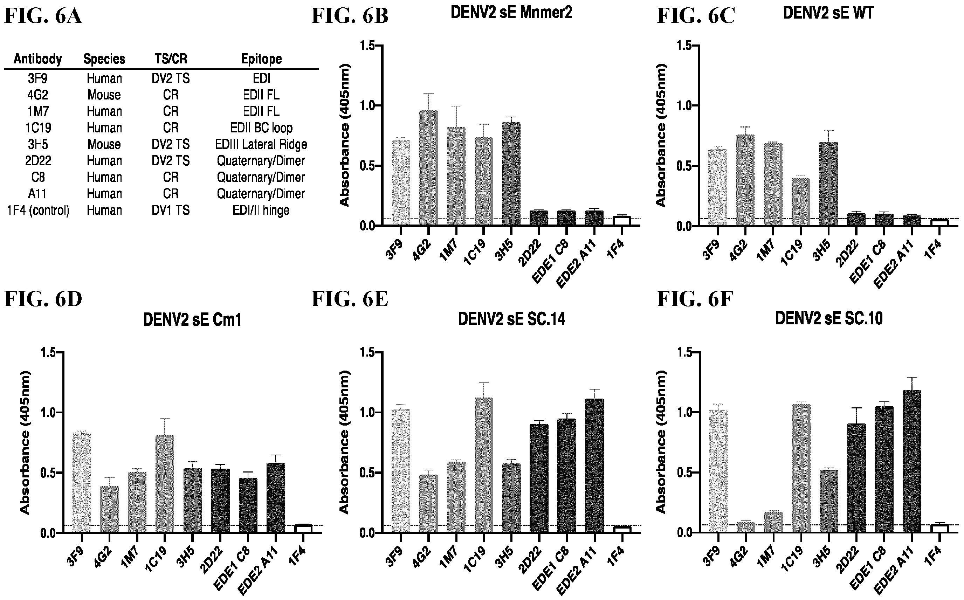

**FIGS. 6A-6F** show bar graphs depicting epitope presentation of DENV2 sE proteins assessed by binding of representative DENV E monomer and quaternary epitope antibodies. ELISA antibody binding analysis at 37°C of representative DENV E monomer and quaternary epitope antibodies **(****FIG. 6A****)** to DENV2 sE monomers, WT **(****FIG. 6B****)** Mnmer2 **(****FIG. 6C****),** and dimers, Cm1 **(****FIG. 6D****),** SC. 14 **(****FIG. 6E****),** and SC. 10 **(****FIG. 6F****).** 100ng of sE protein at 45nM was immobilized to the nickel coated ELISA plate, and incubated with 2µg/mL of each antibody. Antibody binding was detected using AP-conjugated anti-mouse or anti-human IgG and measuring the absorbance at 405nm of converted AP substrate. Antibody binding signals of antibodies targeting DI (3F9), DII (1C19), DIII (3H5) and fusion loop (FL, 4G2 and 1M7) monomer epitopes (grayscale) and quaternary antibody epitopes (2D22, EDE1 C8 and EDE2 A11) (black) are plotted, with DENV1 specific antibody, 1F4 (white), used to as a negative control. Experiment was performed in triplicate with error bars representing the mean 405nm signal + standard deviation of the mean (SD).

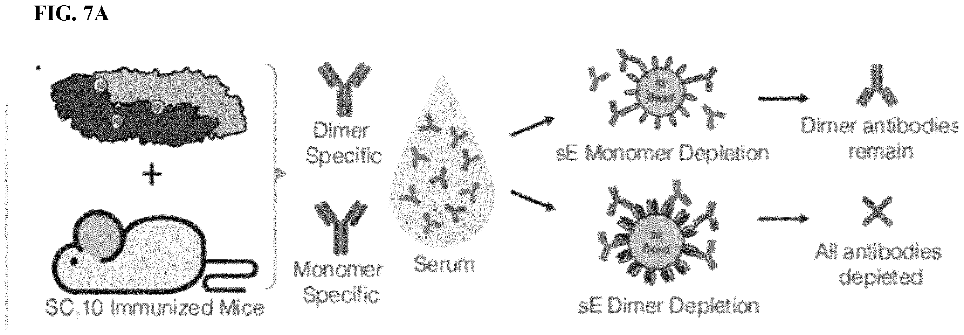

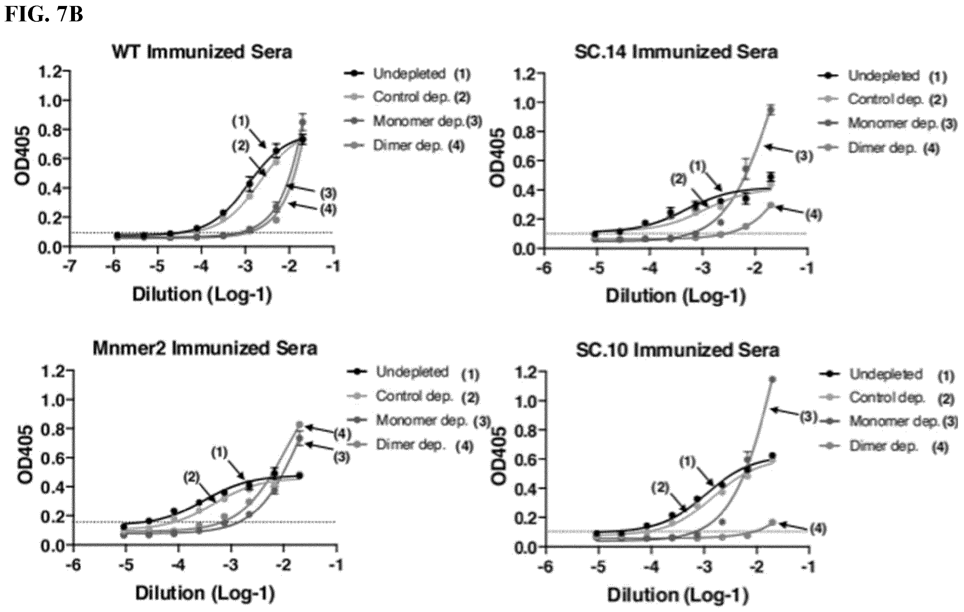

**FIGS. 7A-7C** show that DENV2 sE SC. 14 & SC. 10 stable dimers elicit DENV2 envelope dimer specific antibodies in mice. **FIG. 7A** shows a schematic representation of the experimental protocol. Serum of mice immunized 3 times with 5µg of DENV2 sE WT, Mnmer2, SC. 14 or SC. 10 protein was harvested 16 weeks post initial immunization. To identify if elicited antibodies target E monomer epitopes or dimer epitopes, sE monomer (DENV2 sE Mnmer2) or sE dimer (SC. 14) loaded Ni-NTA beads were incubated with the mice immunized sera 37°C. Antibodies specific to sE monomer were depleted by beads loaded with either sE monomer or dimer, as monomer epitopes are presented on both proteins. Dimer specific antibodies were only depleted by sE dimer, and remained in the sera post depletion with sE monomer loaded beads. Depleted sera were analyzed in a DENV2 capture ELISA to detect the remaining antibodies present post depletion with sE monomer or sE dimer loaded beads. **FIG. 7B** shows representative DENV2 ELISA serum antibody binding data of undepleted (1), His-tagged spycatcher negative control depleted (control depletion, 2), Mnmer2 depleted (monomer depleted, 3) and SC. 14 depleted (dimer depleted, 4) sera from mice immunized with DENV2 sE WT, Mnmer2, SC. 14 and SC. 10. Depleted sera was serially diluted 4-fold and tested for serum antibody binding to DENV2 in triplicate, with error bars reporting mean 405nm absorbance ± standard deviation of the mean (SD). C) ELISA analysis of antibody binding of serially diluted, Mnmer2 (monomer dep.) or SC. 14 (dimer dep.) depleted serum antibody binding to DENV2 (left), sE Mnmer2 (middle) or sE SC. 14 (right) of each mouse (n=5) immunized with either DENV2 sE WT (red), Mnmer2 (M2, black), SC. 14 (purple) or SC. 10 (blue) depleted with Mnmer2 (monomer depleted) or SC. 14. Each serum dilution was performed in triplicate with error bars representing the mean 405nm absorbance ± the standard deviation of the mean (SD), which was fitted to obtain the end point dilution titer (EPD). Data is represented as the ratio of end point dilution (EPD) of monomer depleted sera to dimer depleted sera.

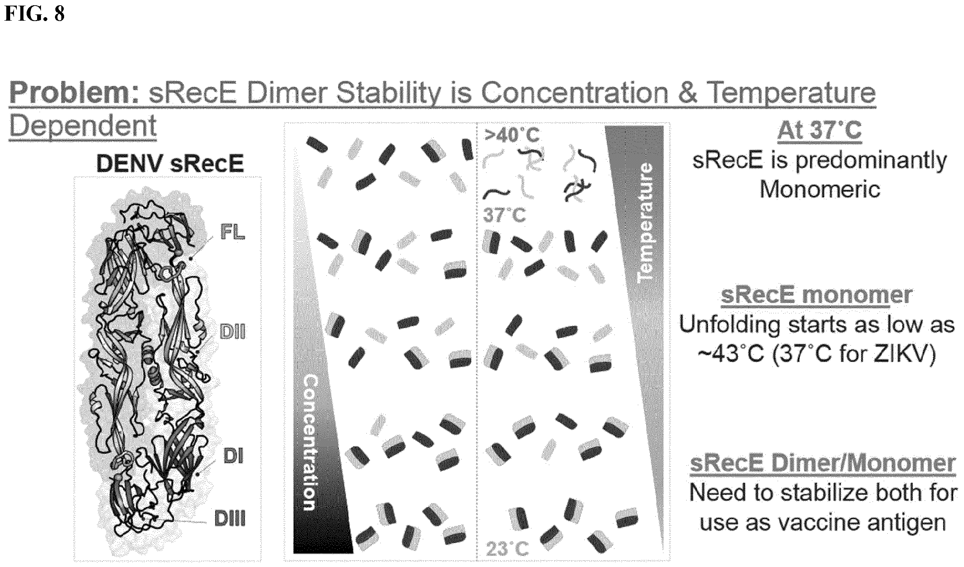

**FIG. 8** shows a schematic of sRecE dimer stability during changes in concentration and temperature.

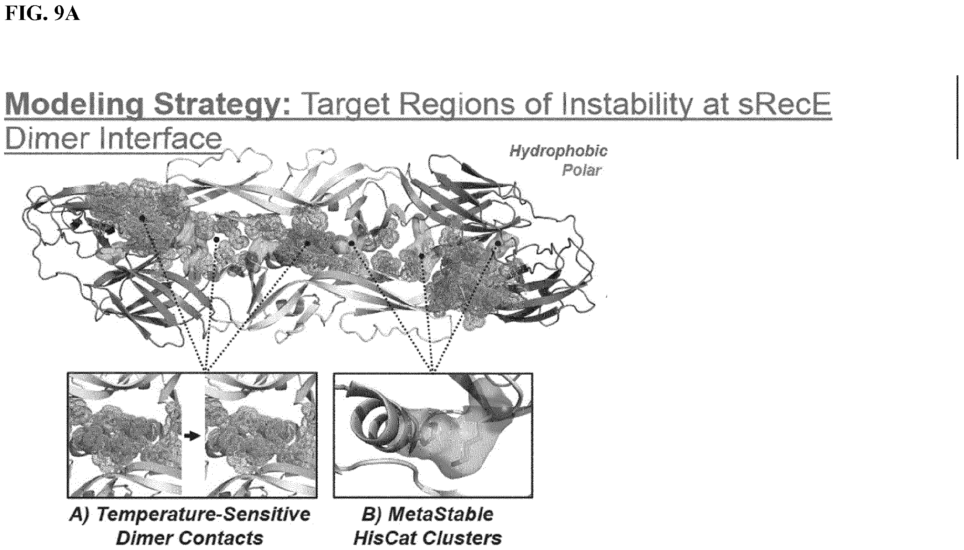

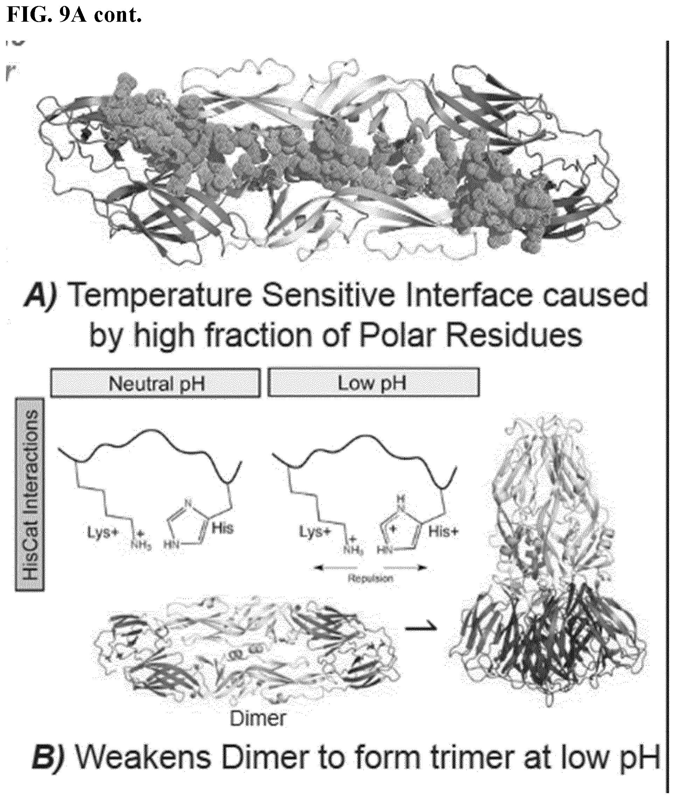

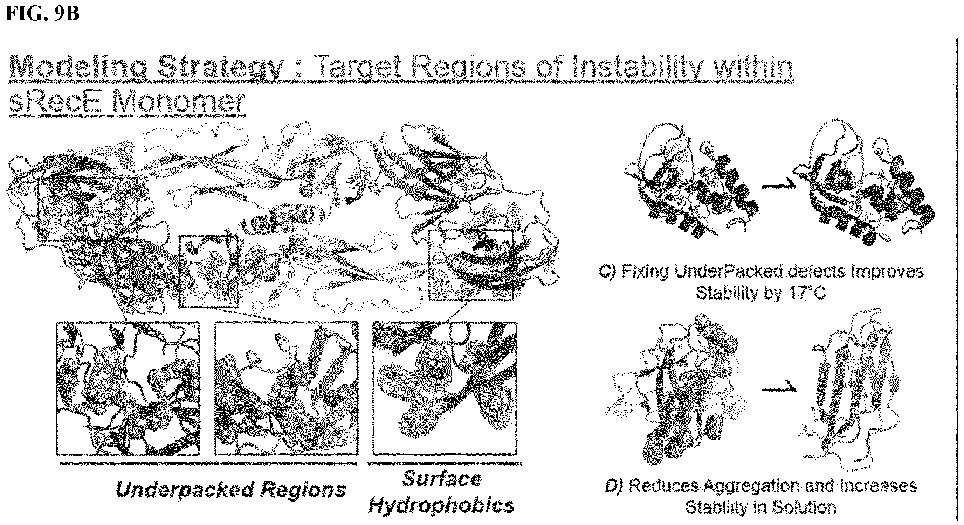

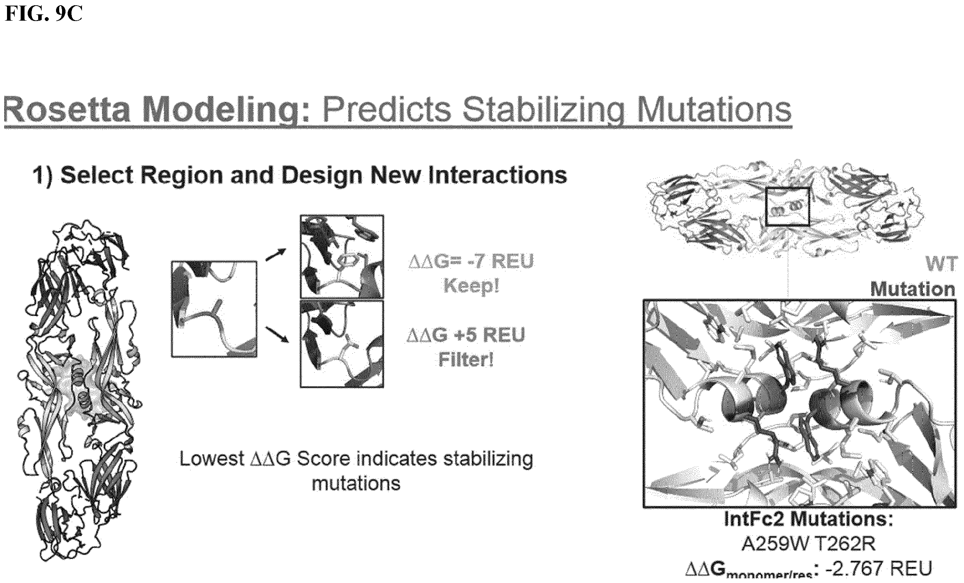

**FIGS. 9A-9C** show schematics of the modeling strategy. **FIG. 19A** shows depictions of temperature-sensitive dimer contacts and HisCat clusters. **FIG. 19B** shows depictions of Underpacked Regions and surface hydrophobics. **FIG. 19C** shows an example stabilized recombinant E glycoprotein (IntFc2) with two mutations, A259W and T262R, predicted as stabilizing by the modeling strategy described herein.

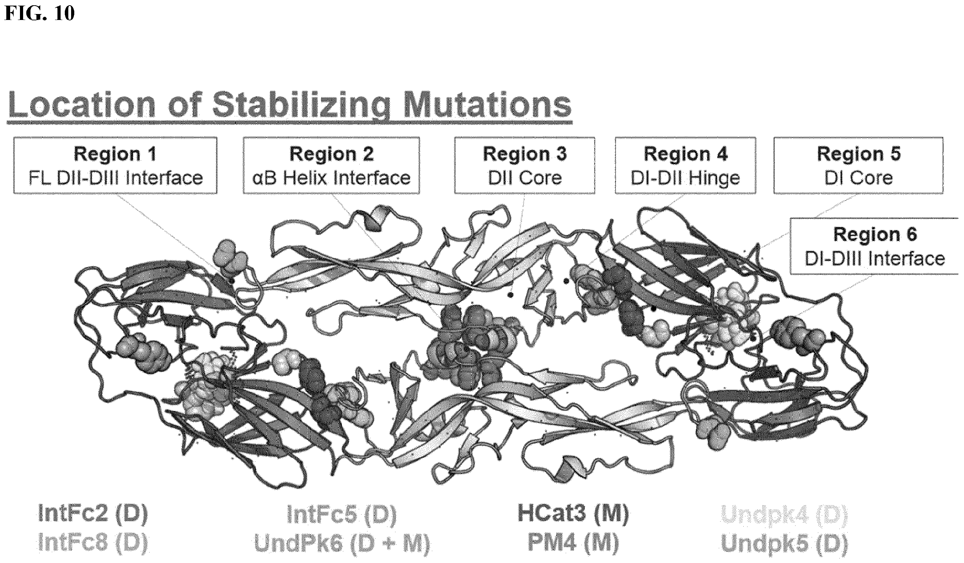

**FIG. 10** shows a schematic of the E glycoprotein structure with indicated regions targeted for mutations.

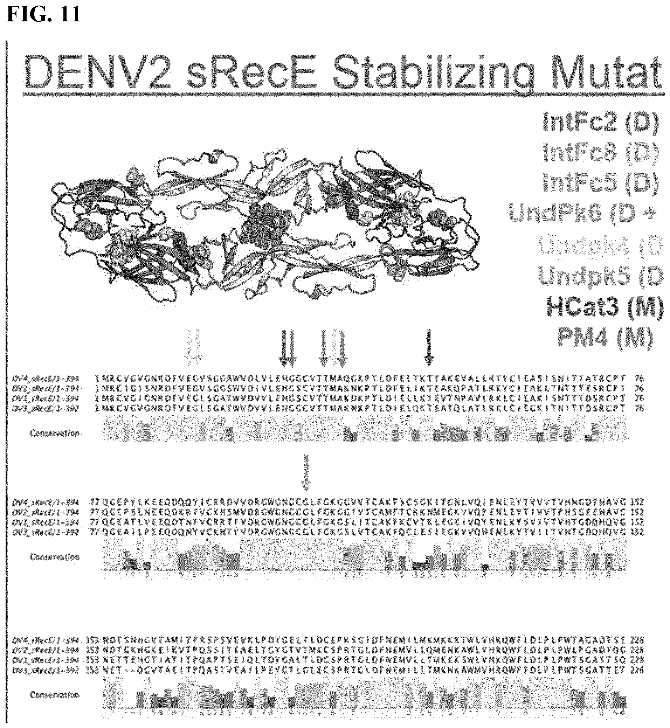

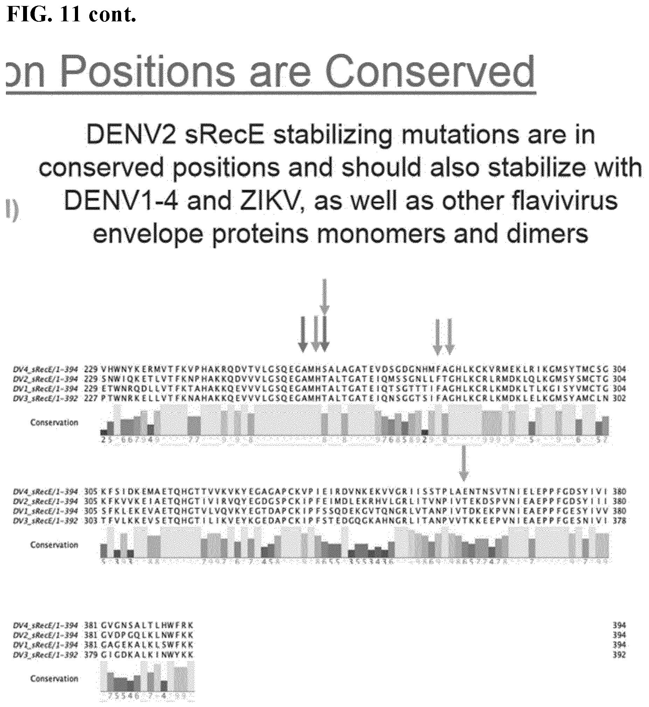

**FIG. 11** shows an alignment of the E glycoprotein soluble ectodomain amino acid sequence of DENV1 (SEQ ID NO: 147), DENV2 (SEQ ID NO:6), DENV3 (SEQ ID NO: 152), and DENV4 (SEQ ID NO: 169).

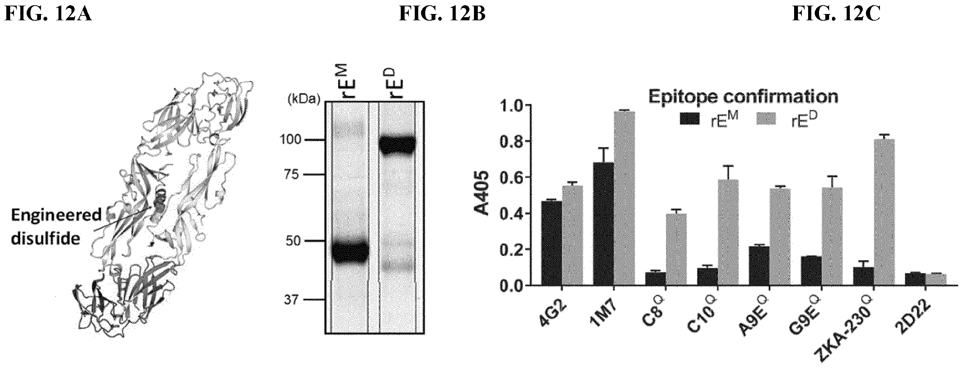

**FIGS. 12A-12C** show ZIKV mutation experiments. **FIG. 12A** shows a diagram of the ZIKV E glycoprotein structure with an engineered disulfide. **FIG. 12B** shows protein gel analysis between the recombinant monomer and the stabilized recombinant dimer. **FIG. 12C** shows a bar graph of neutralizing antibody binding to recombinant E glycoprotein monomer and stabilized recombinant dimer.

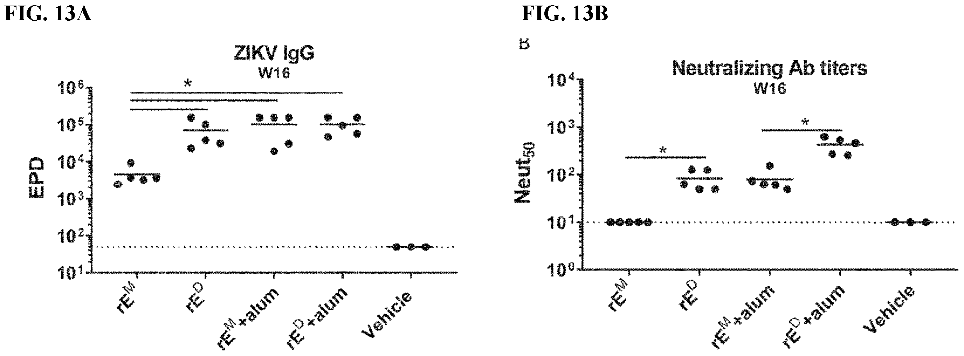

**FIGS. 13A-13B** show *in vivo* ZIKV experiments. **FIG. 13A** shows ZIKV IgG levels analyzed at week 16 post infection, wherein mice were primed at week 3 and boosted at week 9 with soluble rE monomer ("rEM") or rE dimer ("rED") with or without alum adjuvant, or vehicle control. **FIG. 13B** shows neutralizing antibody titers under the same conditions as in **FIG. 13A****.**

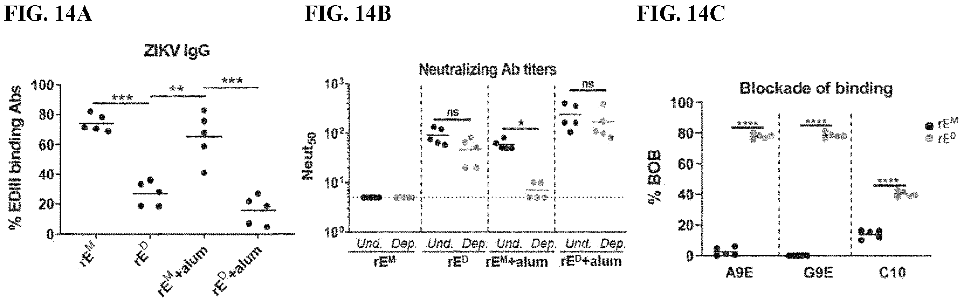

**FIGS. 14A-14C** show experimental results of EDIII serum depletion assays. **FIG. 14A** shows the percentage of antibodies that bound to EDIII. **FIG. 14B** shows neutralizing antibody titers in undepleted and depleted conditions. **FIG. 14C** shows % blockade of binding between recombinant E monomer and stabilized recombinant E dimer using the antibodies A9E, G9E, and C10.

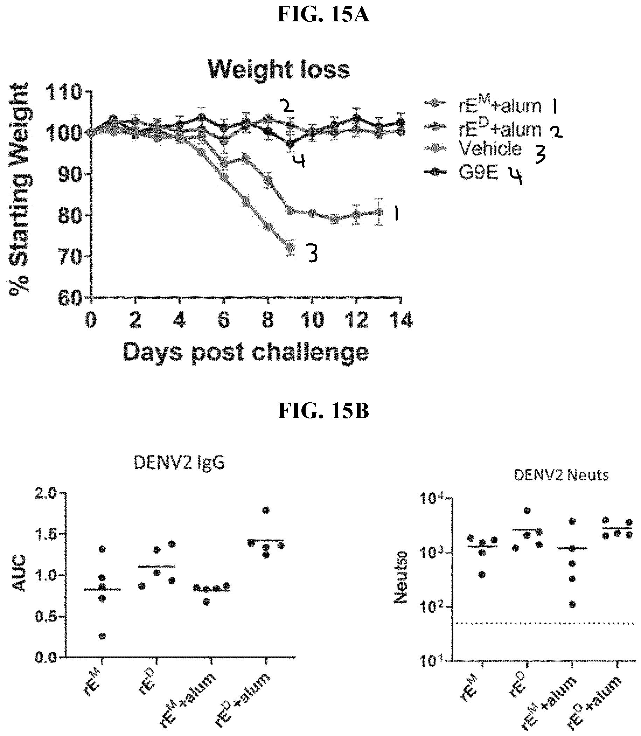

**FIGS. 15A-15B** show *in vivo* ZIKV and DENV2 infection experiments. **FIG. 15A** shows weight loss in mice permissive to ZIKV infection following serum transfer from mice that were immunized with monomer or dimer and then challenged with ZIKV. **FIG. 15B** shows DENV2 IgG levels (left) and neutralizing antibody levels (right) in DENV2-challenged mice immunized with recombinant E monomer ("rEM"), stabilized recombinant E dimer ("rED"), plus or minus alum adjuvant.

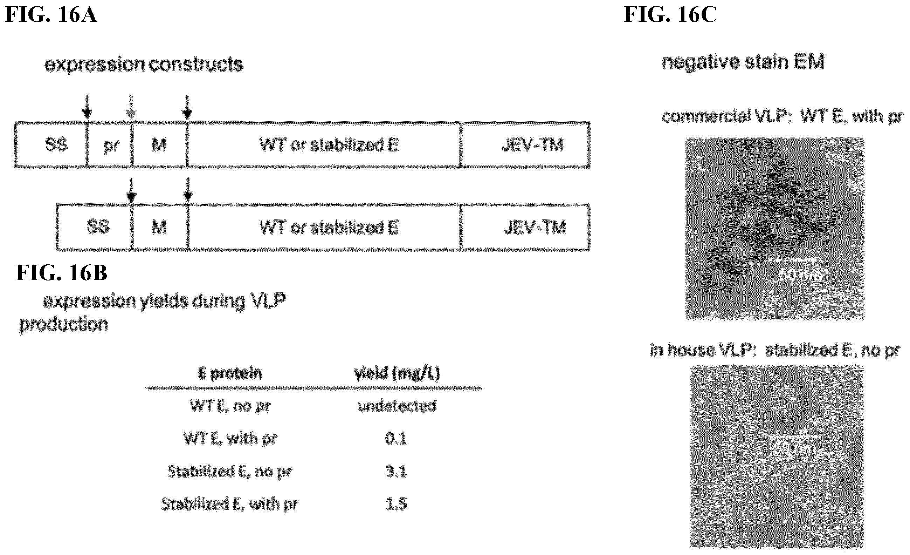

**FIGS. 16A-16C** show an overview and EM results of production of VLPs. FIG. 16A shows a schematic of WT or stabilized E proteins that were expressed with and without pr. Cleavage sites are marked with arrows, wherein the top SS:pr and M:E arrows, and bottom SS:M and M:E arrows represent cellular signalase sites; top middle arrow (pr:M) represents a furin site. Both constructs contain a signal sequence from human serum albumin (SS), the M protein, and replace the c-terminal transmembrane region of the DENV E protein with that from Japanese encephalitis (JEV-TM). **FIG. 16B** shows a table of expression yields measured with dot blot analysis and the E protein antibody 1M7. **FIG. 16C** shows EM images from our first VLP production trial; particles incorporating the stabilized recombinant E protein, U6-I8-PM4, expressed without the pr domain (bottom) compared to commercially prepared VLPs expressed with pr (top).

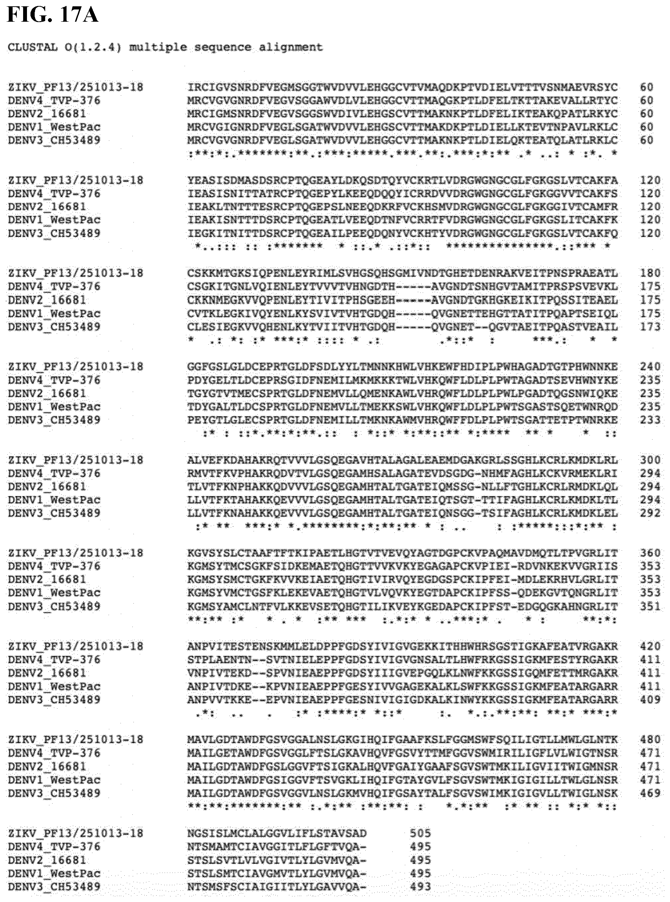

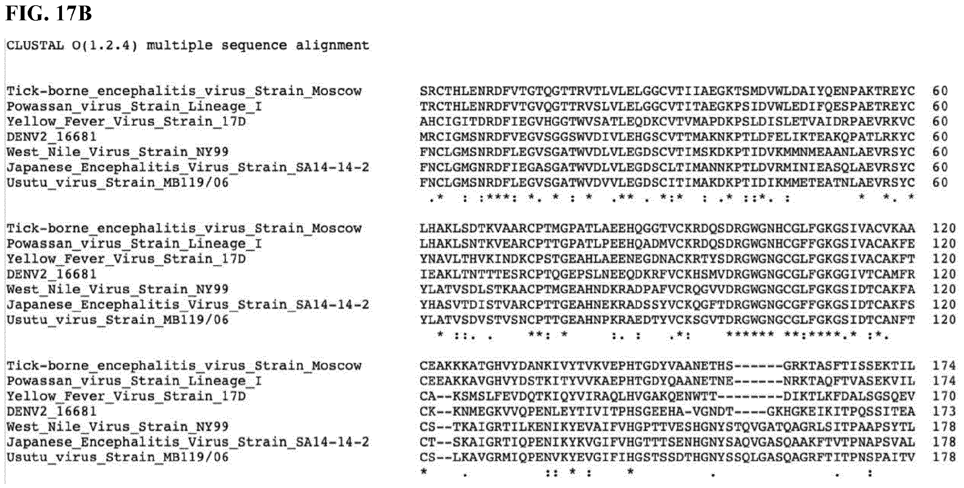

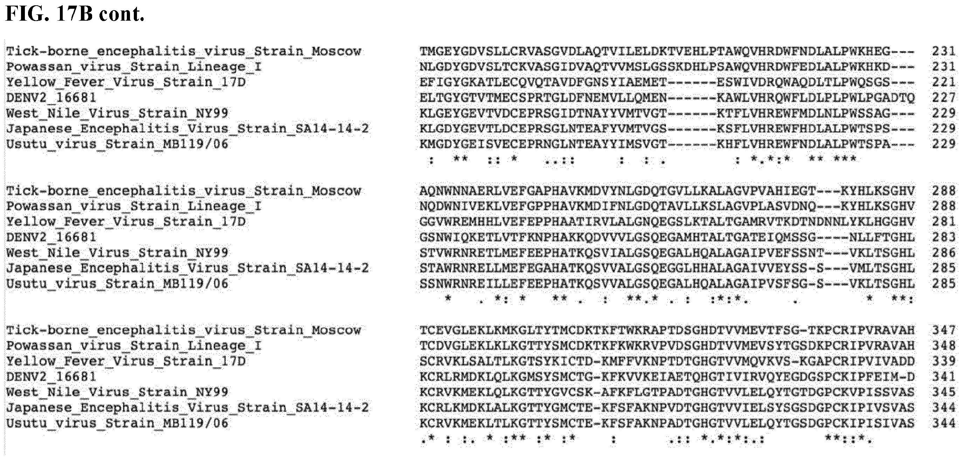

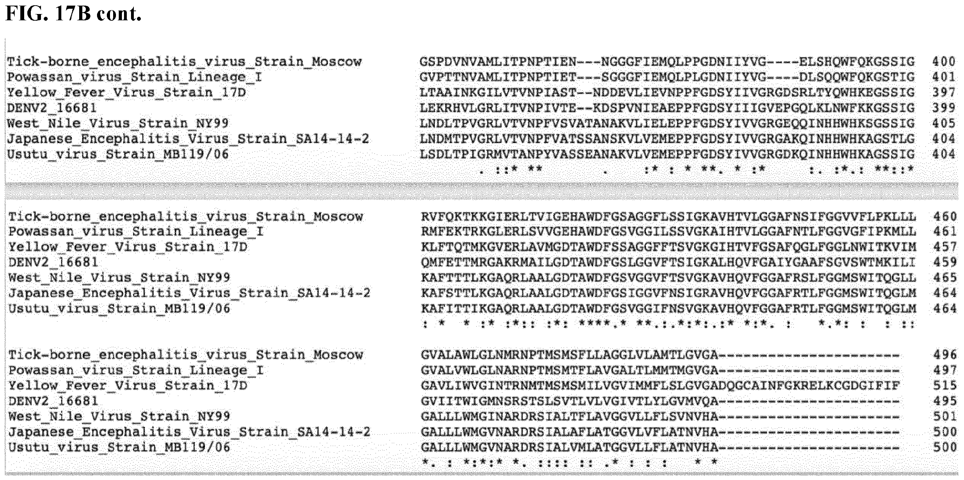

**FIGS. 17A-17B** show sequence alignments between the E glycoprotein sequences of wildtype strains of (FIG. 17A) DENV2 (SEQ ID NO: 182), DENV1 (SEQ ID NO: 183), DENV3 (SEQ ID NO: 184), DENV4 (SEQ ID NO: 185), and ZIKV (SEQ ID NO: 186); and (FIG. 17B) DENV2 (SEQ ID NO:182), YFV (SEQ ID NO:187), JEV (SEQ ID NO:188), WNV (SEQ ID NO: 189), TBEV (SEQ ID NO: 190), Usutu virus (SEQ ID NO: 191), and Powassan virus (SEQ ID NO: 192).

### DETAILED DESCRIPTION OF THE INVENTION

The present invention will now be described more fully hereinafter with reference to the accompanying drawings and specification, in which preferred embodiments of the invention are shown. This invention may, however, be embodied in different forms and should not be construed as limited to the embodiments set forth herein.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

All publications, patent applications, patents and other references cited herein are incorporated by reference in their entireties for the teachings relevant to the sentence and/or paragraph in which the reference is presented.

As used herein, "a," "an" or "the" can mean one or more than one. For example, "a" cell can mean a single cell or a multiplicity of cells.

Also as used herein, "and/or" refers to and encompasses any and all possible combinations of one or more of the associated listed items, as well as the lack of combinations when interpreted in the alternative ("or").

The term "about," as used herein when referring to a measurable value such as an amount of dose (e.g., an amount of a non-viral vector) and the like, is meant to encompass variations of ± 20%, ± 10%, ± 5%, ± 1%, ± 0.5%, or even ± 0.1% of the specified amount.

As used herein, the transitional phrase "consisting essentially of" (and grammatical variants) means that the scope of a claim is to be interpreted to encompass the specified materials or steps recited in the claim, "and those that do not materially affect the basic and novel characteristic(s)" of the claimed invention. Thus, the term "consisting essentially of" when used in a claim of this invention is not intended to be interpreted to be equivalent to "comprising."

As used herein, the term "nucleic acid" encompasses both RNA and DNA, including cDNA, genomic DNA, synthetic (e.g., chemically synthesized) DNA and chimeras of RNA and DNA. The nucleic acid may be double-stranded or single-stranded. The nucleic acid may be synthesized using nucleotide analogs or derivatives (e.g., inosine or phosphorothioate nucleotides). Such nucleotides can be used, for example, to prepare nucleic acids that have altered base-pairing abilities or increased resistance to nucleases.

As used herein, the term "polypeptide" encompasses both peptides and proteins (including fusion proteins), unless indicated otherwise.

In embodiments of the invention, an "immunogenically active fragment" of a flavivirus polypeptide (e.g., the E protein) comprises, consists essentially of or consists of at least about 6, 8, 10, 12, 15, 20, 30, 50, 75, 100, 125, 150, 200, 250, 300, 350, 400, 450 or more amino acids, optionally contiguous amino acids, and/or less than about 495, 475, 450, 425, 400, 350, 300, 250, 200, 150, 100, 75 or 50 amino acids, optionally contiguous amino acids, including any combination of the foregoing as long as the lower limit is less than the upper limit, and the "immunogenically active fragment" induces an immune response (e.g., IgG and/or IgA that react with the native antigen), optionally a protective immune response, against dengue virus in a host and induces the production of antibodies that specifically bind to the quaternary dengue virus epitope newly identified by the inventors.

The term "epitope" as used herein means a specific amino acid sequence that, when present in the proper conformation, provides a reactive site for an antibody (e.g., B cell epitope) or T cell receptor (e.g., T cell epitope).

Portions of a given polypeptide that include a B-cell epitope can be identified using any number of epitope mapping techniques that are known in the art. (*See,* e.g., Epitope Mapping Protocols in Methods in Molecular Biology, Vol. 66, Glenn E. Morris, Ed., 1996, Humana Press, Totowa, N.J.). For example, linear epitopes can be determined by, e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Pat. No. 4,708,871; Geysen et al. (1984) Proc. Natl. Acad. Sci. USA 81:3998-4002; Geysen et al. (1986) Molec. Immunol. 23:709-715.

Similarly, conformational epitopes can be readily identified by determining spatial conformation of amino acids such as by, e.g., x-ray crystallography and 2-dimensional nuclear magnetic resonance. Antigenic regions of proteins can also be identified using standard antigenicity and hydropathy plots, such as those calculated using, e.g., the Omiga version 1.0 software program available from the Oxford Molecular Group. This computer program employs the Hopp/Woods method (Hopp et al. Proc. Natl. Acad. Sci USA (1981) 78:3824-3828) for determining antigenicity profiles and the Kyte-Doolittle technique (Kyte et al. J. Mol. Biol. (1982) 157: 105-132) for hydropathy plots.

Generally, T-cell epitopes that are involved in stimulating the cellular arm of a subject's immune system are short peptides of about 8-25 amino acids. A common way to identify T-cell epitopes is to use overlapping synthetic peptides and analyze pools of these peptides, or the individual ones, that are recognized by T cells from animals that are immune to the antigen of interest, using, for example, an enzyme-linked immunospot assay (ELISPOT). These overlapping peptides can also be used in other assays such as the stimulation of cytokine release or secretion, or evaluated by constructing major histocompatibility (MHC) tetramers containing the peptide. Such immunogenically active fragments can also be identified based on their ability to stimulate lymphocyte proliferation in response to stimulation by various fragments from the antigen of interest.

A "recombinant" nucleic acid, polynucleotide or nucleotide sequence is one produced by genetic engineering techniques.

A "recombinant" polypeptide is produced from a recombinant nucleic acid, polypeptide or nucleotide sequence.

As used herein, an "isolated" polynucleotide (e.g., an "isolated nucleic acid" or an "isolated nucleotide sequence") means a polynucleotide at least partially separated from at least some of the other components of the naturally occurring organism or virus, for example, the cell or viral structural components or other polypeptides or nucleic acids commonly found associated with the polynucleotide. Optionally, but not necessarily, the "isolated" polynucleotide is present at a greater concentration (i.e., is enriched) as compared with the starting material (e.g., at least about a two-fold, three-fold, four-fold, ten-fold, twenty-fold, fifty-fold, one-hundred-fold, five-hundred-fold, one thousand-fold, ten thousand-fold or greater concentration). In representative embodiments, the isolated polynucleotide is at least about 1%, 5%, 10%, 20%, 30%, 40%, 50%, 60%, 70%, 80%, 90%, 95% or more pure.

An "isolated" polypeptide means a polypeptide that is at least partially separated from at least some of the other components of the naturally occurring organism or virus, for example, the cell or viral structural components or other polypeptides or nucleic acids commonly found associated with the polypeptide. Optionally, but not necessarily, the "isolated" polypeptide is present at a greater concentration (i.e., is enriched) as compared with the starting material (e.g., at least about a two-fold, three-fold, four-fold, ten-fold, twenty-fold, fifty-fold, one-hundred-fold, five-hundred-fold, one thousand-fold, ten thousand-fold or greater concentration). In representative embodiments, the isolated polypeptide is at least about 1%, 5%, 10%, 20%, 30%, 40%, 50%, 60%, 70%, 80%, 90%, 95% or more pure.

Furthermore, an "isolated" cell is a cell that has been partially or completely separated from other components with which it is normally associated in nature. For example, an isolated cell can be a cell in culture medium and/or a cell in a pharmaceutically acceptable carrier.

The terms "immunogen" and "antigen" are used interchangeably herein and mean any compound (including polypeptides) to which a cellular and/or humoral immune response can be directed. In particular embodiments, an immunogen or antigen can induce a protective immune response against the effects of dengue virus infection.

"Effective amount" as used herein refers to an amount of a vector, nucleic acid, epitope, polypeptide, cell, particle, VLP, composition or formulation of the invention that is sufficient to produce a desired effect, which can be a therapeutic and/or beneficial effect. The effective amount will vary with the age, general condition of the subject, the severity of the condition being treated, the particular agent administered, the duration of the treatment, the nature of any concurrent treatment, the pharmaceutically acceptable carrier used, and like factors within the knowledge and expertise of those skilled in the art. As appropriate, an "effective amount" in any individual case can be determined by one of ordinary skill in the art by reference to the pertinent texts and literature and/or by using routine experimentation.

The term "immunogenic amount" or "effective immunizing dose," as used herein, unless otherwise indicated, means an amount or dose sufficient to induce an immune response (which can optionally be a protective response) in the treated subject that is greater than the inherent immunity of non-immunized subjects. An immunogenic amount or effective immunizing dose in any particular context can be routinely determined using methods known in the art.

The terms "vaccine," "vaccination" and "immunization" are well-understood in the art, and are used interchangeably herein. For example, the terms vaccine, vaccination or immunization can be understood to be a process or composition that increases a subject's immune reaction to an immunogen (e.g., by providing an active immune response), and therefore its ability to resist, overcome and/or recover from infection (i.e., a protective immune response).

By the terms "treat," "treating" or "treatment of" (and grammatical variations thereof) it is meant that the severity of the subject's condition is reduced, at least partially improved or ameliorated and/or that some alleviation, mitigation or decrease in at least one clinical symptom is achieved and/or there is a delay in the progression of the disease or disorder. In representative embodiments, the terms "treat," "treating" or "treatment of" (and grammatical variations thereof) refer to a reduction in the severity of viremia and/or a delay in the progression of viremia, with or without other signs of clinical disease.

A "treatment effective" amount as used herein is an amount that is sufficient to treat (as defined herein) the subject. Those skilled in the art will appreciate that the therapeutic effects need not be complete or curative, as long as some benefit is provided to the subject.

The term "prevent," "preventing" or "prevention of" (and grammatical variations thereof) refer to prevention and/or delay of the onset and/or progression of a disease, disorder and/or a clinical symptom(s) in a subject and/or a reduction in the severity of the onset and/or progression of the disease, disorder and/or clinical symptom(s) relative to what would occur in the absence of the methods of the invention. In representative embodiments, the terms "prevent," "preventing" or "prevention of" (and grammatical variations thereof) refer to prevention and/or delay of the onset and/or progression of viremia in the subject, with or without other signs of clinical disease. The prevention can be complete, e.g., the total absence of the disease, disorder and/or clinical symptom(s). The prevention can also be partial, such that the occurrence of the disease, disorder and/or clinical symptom(s) in the subject and/or the severity of onset and/or the progression is less than what would occur in the absence of the present invention.

A "prevention effective" amount as used herein is an amount that is sufficient to prevent (as defined herein) the disease, disorder and/or clinical symptom in the subject. Those skilled in the art will appreciate that the level of prevention need not be complete, as long as some benefit is provided to the subject.

The efficacy of treating and/or preventing dengue virus infection by the methods of the present invention can be determined by detecting a clinical improvement as indicated by a change in the subject's symptoms and/or clinical parameters (e.g., viremia), as would be well known to one of skill in the art.

Unless indicated otherwise, the terms "protect," "protecting," "protection" and "protective" (and grammatical variations thereof) encompass both methods of preventing and treating dengue virus infection in a subject, whether against one or multiple strains, genotypes or serotypes of dengue virus.

The terms "protective" immune response or "protective" immunity as used herein indicates that the immune response confers some benefit to the subject in that it prevents or reduces the incidence and/or severity and/or duration of disease or any other manifestation of infection. For example, in representative embodiments, a protective immune response or protective immunity results in reduced viremia, whether or not accompanied by clinical disease. Alternatively, a protective immune response or protective immunity may be useful in the therapeutic treatment of existing disease.

An "active immune response" or "active immunity" is characterized by "participation of host tissues and cells after an encounter with the immunogen. It involves differentiation and proliferation of immunocompetent cells in lymphoreticular tissues, which lead to synthesis of antibody or the development of cell-mediated reactivity, or both." Herbert B. Herscowitz, Immunophysiology: Cell Function and Cellular Interactions in Antibody Formation, in IMMUNOLOGY: BASIC PROCESSES 117 (Joseph A. Bellanti ed., 1985). Alternatively stated, an active immune response is mounted by the host after exposure to immunogens by infection or by vaccination. Active immunity can be contrasted with passive immunity, which is acquired through the "transfer of preformed substances (antibody, transfer factor, thymic graft, interleukin-2) from an actively immunized host to a non-immune host." *Id.*

A "subject" of the invention includes any animal susceptible to dengue virus infection. Such a subject is generally a mammalian subject (e.g., a laboratory animal such as a rat, mouse, guinea pig, rabbit, primates, etc.), a farm or commercial animal (e.g., a cow, horse, goat, donkey, sheep, etc.), or a domestic animal (e.g., cat, dog, ferret, etc.). In particular embodiments, the subject is a primate subject, a non-human primate subject (e.g., a chimpanzee, baboon, monkey, gorilla, etc.) or a human. Subjects of the invention can be a subject known or believed to be at risk of infection by a flavivirus (e.g., dengue virus and/or Zika virus). Alternatively, a subject according to the invention can also include a subject not previously known or suspected to be infected by a flavivirus or in need of treatment for flavivirus infection.

Subjects may be treated for any purpose, such as for eliciting a protective immune response or for eliciting the production of antibodies in that subject, which antibodies can be collected and used for other purposes such as research or diagnostic purposes or for administering to other subjects to produce passive immunity therein, etc.

Subjects include males and/or females of any age, including neonates, juvenile, mature and geriatric subjects. With respect to human subjects, in representative embodiments, the subject can be an infant (e.g., less than about 12 months, 10 months, 9 months, 8 months, 7 months, 6 months, or younger), a toddler (e.g., at least about 12, 18 or 24 months and/or less than about 36, 30 or 24 months), or a child (e.g., at least about 1, 2, 3, 4 or 5 years of age and/or less than about 14, 12, 10, 8, 7, 6, 5, or 4 years of age). In embodiments of the invention, the subject is a human subject that is from about 0 to 3, 4, 5, 6, 9, 12, 15, 18, 24, 30, 36, 48 or 60 months of age, from about 3 to 6, 9, 12, 15, 18, 24, 30, 36, 48 or 60 months of age, from about 6 to 9, 12, 15, 18, 24, 30, 36, 48 or 60 months of age, from about 9 to 12, 15, 18, 24, 30, 36, 48 or 60 months of age, from about 12 to 18, 24, 36, 48 or 60 months of age, from about 18 to 24, 30, 36, 48 or 60 months of age, or from about 24 to 30, 36, 48 or 60 months of age.

A "subject in need" of the methods of the invention can be a subject known to be, or suspected of being, infected with, or at risk of being infected with, a flavivirus (e.g., dengue virus and/or Zika virus).

The present invention is based, in part on the unexpected discovery that particular amino acid residues can be incorporated into a flavivirus E glycoprotein to stabilize the dimer conformation at physiological, e.g., vaccine, conditions. These particular amino acid residues can be transferred into the backbone amino acid sequence of multiple flavivirus E glycoproteins to create stabilized recombinant E glycoproteins, e.g., E glycoproteins which would be stabilized as dimers in physiological conditions (e.g., vaccine conditions, e.g., in low protein concentration conditions and/or at a temperature of about 37 °C).

The enveloped DENV is an enveloped virus that contains 180 copies of the envelope (E) glycoprotein on the viral surface. The E glycoprotein is the only protein presented on the viral surface, and is arranged in a herringbone pattern as anti-parallel homodimers that pack laterally into a lipid raft containing three dimers. The E protein monomer contains three predominantly β-sheet domains referred to as DI, DII, and DIII. These domains are connected via linker regions to form the monomer, which is connected to a transmembrane domain by an amphipathic helix stem peptide. The E protein exists in multiple conformations consistent with the different stages the virus undergoes in the infection cycle, including a pre-fusion dimer at neutral pH and a post-fusion trimer that forms as the virus enters the low pH endosome. While not wishing to be bound to theory, it is believed that this requirement that the E protein transitions between different conformational states provides selective pressure during viral evolution that favors E protein sequences that are adept at transitioning between different structures and are not overly stable in a single conformation such as the pre-fusion dimer. As used herein, the term "dimer conformation" refers to the physical structure (e.g., tertiary and/or quaternary structure) of an E glycoprotein that is capable of forming dimers (e.g., homodimers). Similarly, the term "trimer conformation" refers to the physical structure (e.g., tertiary and/or quaternary structure) of an E glycoprotein that is capable of forming trimers (e.g., homotrimers), and the term "monomer conformation" refers to the physical structure (e.g., tertiary and/or quaternary structure) of an E glycoprotein that is not capable of forming higher order multimers. The present invention provides modifications, e.g., substitutions, that may stabilize an E glycoprotein into a particular preferred conformation, such as the monomer conformation, dimer conformation, and/or trimer conformation.

A soluble recombinant E protein (sE, also referred to herein as sRecE), comprising the soluble ectodomain (i.e., E glycoprotein DI, DII and DIII), can form dimers at high concentrations and under crystallization conditions. These sE dimers resemble the anti-parallel E homodimer observed on the virion surface, which presents epitopes targeted by DENV neutralizing antibodies **(****FIG. 1A****).** Structural studies have revealed the epitope of potently neutralizing quaternary epitope antibodies, such as 2D22 and the E dimer epitope (EDE) antibodies, are displayed within a single sE dimer **(****FIG. 1D****).** Dimeric sE antigens induce higher neutralizing antibody titers, thus it was hypothesized by the inventors of the present invention that dimeric sE protein could be used as an antigen to present quaternary epitopes. For example, Zika virus and dengue virus have at least one conserved quaternary epitope, recognized by the EDE neutralizing antibody. Antibodies that bind to quaternary E glycoprotein epitopes protect against dengue virus and Zika virus infection, such as EDE (a pan DENV1-4 antibody), 5J7 (DENV3), 2D22 (DENV2), and HM14c10 (DENV1). Thus, in some embodiments, the stabilized recombinant flavivirus E glycoprotein of the present invention may comprise at least one quaternary epitope, e.g., a quaternary epitope recognized by antibodies including but not limited to 5J7, 2D22, EDE1 C8, EDE1 C10, EDE2 A11, EDE2 B7, 3F9, 1L12, DV3-290, DV3-144, DV3-415, DV3-066, D4-126, D4-131, A9E, G9E, ZKA-230, and/or ZKA-230. Recognition of the quaternary epitope may depend on the E glycoprotein being in dimer form, e.g., a recombinant flavivirus E glycoprotein stabilized in dimer conformation, e.g., stabilized in a conformation such that the E glycoprotein forms homodimers *in vitro* and/or *in vivo* such as under physiological and/or vaccine conditions. Non-limiting examples of antibodies that recognize quaternary epitopes include but are not limited to 5J7, 2D22, EDE1 C8, EDE1 C10, EDE2 A11, EDE2 B7, 3F9, 1L12, DV3-290, DV3-144, DV3-415, DV3-066, D4-126, D4-131, A9E, G9E, ZKA-230, and/or ZKA-230.

However, the DENV2 sE protein is also sensitive to environmental stresses, such as pH and temperature, similar to the native E protein. In addition, the DENV2 sE protein dimer has a homodimer Kd of 12µM at 37°C, which is too weak to be relevant under vaccination conditions and limits its current use as a vaccine antigen by reducing the presentation of critical quaternary epitopes. Recombinant soluble E glycoprotein dimer stability is concentration and temperature dependent, wherein at physiological temperatures (e.g., 37°C), sE is predominantly monomeric. The monomer has limited presentation of broadly neutralizing epitopes (e.g., quaternary epitopes) that are important for effective immune responses. Unfolding of the sE monomer protein may start as low as about 43 °C (about 37°C for ZIKV). Dimer formation may occur at high concentration of protein, and/or at low temperature (e.g., about 23°C). Therefore, in order to use sE as a vaccine antigen, it may be beneficial to stabilize both the dimer and monomer conformation. Stabilized monomers may be useful, e.g., in the application of diagnostics, wherein the stabilized monomer may be used as control and/or depleting less broadly-neutralizing antibodies (e.g., antibodies binding non-quaternary epitopes).

Thus, in one embodiment, the present invention provides a stabilized recombinant flavivirus E glycoprotein, comprising a flavivirus E glycoprotein backbone; and one or more (e.g., one or more, two or more, three or more, four or more, etc., e.g., at least one, at least two, at least three, at least four, or more) amino acid substitution(s) selected from the group consisting of 2M, 6L, 8L, 9V, 13F, 14A, 15E, 27P, 29Y, 29K, 32V, 33V, 34L, 35M, 44M, 44L, 48I, 106D, 107C, 131I, 144Y, 154L, 154M, 191Y, 198W, 204F, 205L, 206F, 209D, 244Q, 244F, 246I, 246Y, 251F, 255E, 256Y, 258A, 258E, 259V, 259W, 259C, 260L, 261L, 261F, 262H, 262R, 262Y, 263W, 263L, 266W, 270V, 277M, 279W, 280A, 280P, 289W, 299L, 313C, 316M, 330A, 359Y, 373D, 375L, 377V, 386I, 390Q, 392R, 393R, and/or any combination thereof (e.g., amino acid substitutions shown in **Table 1** and/or **Table 2**), wherein the numbering is based on the reference amino acid sequence of an E glycoprotein of dengue virus serotype 2 (DENV2) identified as **SEQ ID NO:6.**

In some embodiments, the present invention provides a stabilized recombinant flavivirus E glycoprotein, comprising a flavivirus E glycoprotein backbone; and one or more (e.g., one or more, two or more, three or more, four or more, etc., e.g., at least one, at least two, at least three, at least four, or more) amino acid substitution(s) in the flavivirus E glycoprotein backbone DI/DII hinge region, αB helix central interface, and/or fusion loop (FL) dimer interface. In some embodiments, the present invention provides a stabilized recombinant flavivirus E glycoprotein, comprising a flavivirus E glycoprotein backbone; and one or more (e.g., one or more, two or more, three or more, four or more, etc., e.g., at least one, at least two, at least three, at least four, or more) amino acid substitution(s) in the flavivirus E glycoprotein backbone DI/DII hinge region, e.g., outside of the dimer interface. Non-limiting examples of amino acid substitutions in the E glycoprotein DI/DII hinge region, e.g., outside of the E glycoprotein dimer interface, include 279W and 280P.

In some embodiments, the flavivirus E glycoprotein backbone comprises a backbone from a dengue virus (DENV, Zika virus (ZIKV), yellow fever virus (YFV), Japanese encephalitis virus (JEV), West Nile virus (WNV), tick-borne Encephalitis virus (TBEV), Powassan virus, or any combination thereof.

In some embodiments, the flavivirus E glycoprotein backbone comprises a backbone of a dengue virus (DENV), e.g., dengue virus serotype 1 (DENV1), dengue virus serotype 2 (DENV2), dengue virus serotype 3 (DENV3), and/or dengue virus serotype 4 (DENV4). A DENV backbone may comprise the amino acid sequence of any DENV genotype and/or strain and/or isolate currently known or as yet identified and/or isolated. Non-limiting examples of DENV1 genotypes, strains, and/or isolates include Genotypes I, II, III, IV, and V, and strains Western Pacific 1974 (SEQ ID NO: 183). Non-limiting examples of DENV2 genotypes, strains, and/or isolates include DENV2 strain 16681 (SEQ ID NO: 182). Non-limiting examples of DENV3 genotypes, strains, and/or isolates include Genotype I, II, III, IV, and strains such as Sri Lanka 1989, Indonesia 1982, Thailand 1995, Cuba 2002, and Puerto Rico 1977, and DENV3 strain CH53489 (SEQ ID NO: 184). Non-limiting examples of DENV4 genotypes, strains, and/or isolates include DENV4 strain TVP-376 (SEQ ID NO: 185). In some embodiments, the flavivirus E glycoprotein backbone comprises a backbone of a chimeric virus, e.g., a chimeric dengue virus E glycoprotein such as, but not limited to, DENV1/2, DENV 1/3, DENV 1/4, DENV 2/1, DENV 2/3, DENV 2/4, DENV 3/1, DENV 3/2, DENV 3/4, DENV 4/1, DENV 4/2, DENV 4/3.

In some embodiments, the flavivirus E glycoprotein backbone comprises a backbone of a Zika virus (ZIKV), e.g., ZIKV strain PF13/251013-18 (SEQ ID NO:186).

In some embodiments, the flavivirus E glycoprotein backbone comprises a backbone of a Yellow Fever virus (YFV), e.g., YFV strain 17D (SEQ ID NO:187).

In some embodiments, the flavivirus E glycoprotein backbone comprises a backbone of a Japanese Encephalitis virus (JEV), e.g., JEV strain SA14-14-2 (SEQ ID NO:188).

In some embodiments, the flavivirus E glycoprotein backbone comprises a backbone of a West Nile virus (WNV), e.g., WNV strain NY99 (SEQ ID NO:189).

In some embodiments, the flavivirus E glycoprotein backbone comprises a backbone of a Tick-borne Encephalitis virus (TBEV), e.g., TBEV strain Moscow B-4 (SEQ ID NO:190).

In some embodiments, the flavivirus E glycoprotein backbone comprises a backbone of an Usutu virus, e.g., Usutu virus strain MB 119/06 (SEQ ID NO:191).

In some embodiments, the flavivirus E glycoprotein backbone comprises a backbone of a Powassan virus, e.g., Powassan virus Lineage I (SEQ ID NO:192).

In some embodiments, the E glycoprotein backbone comprises a full-length E glycoprotein. In some embodiments, the E glycoprotein backbone comprises a soluble ectodomain formed from E glycoprotein domains DI, DII, and DIII, e.g., soluble recombinant E protein ("sE," also referred to herein as "sRecE").

In some embodiments, a stabilized recombinant flavivirus E glycoprotein of the present invention may comprise one or more (e.g., one or more, two or more, three or more, four or more, etc., e.g., at least one, at least two, at least three, at least four, or more) of the substitutions as shown in the tables of **Table 1** and/or **Table 2,** in any combination, wherein the numbering is based on the reference amino acid sequence of an E glycoprotein (e.g., a soluble recombinant glycoprotein) of dengue virus serotype 2 (DENV2) identified as SEQ ID NO:6.

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: R2M and E44M.

In some embodiments, a stabilized recombinant flavivirus E glycoprotein of the present invention may comprise, consist essentially of, or consist of the amino acid sequence:

MMCIGMSNRDFVEGVSGGSWVDIVLEHGSCVTTMAKNKPTLDFMLIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 HCat2"; SEQ ID NO:10)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: H27P and T48I.

In some embodiments, a stabilized recombinant flavivirus E glycoprotein of the present invention may comprise, consist essentially of, or consist of the amino acid sequence:

SMRCIGMSNRDFVEGVSGGSWVDIVLEPGSCVTTMAKNKPTLDFELIKIEAKQPATLRKYCI
EAKLTNTTTESRCPTQGEPSLNEEQDKRFVCKHSMVDRGWGNGCGLFGKGGIVTCAMFRCKK
NMEGKVVQPENLEYTIVITPHSGEEHAVGNDTGKHGKEIKITPQSSITEAELTGYGTVTMEC
SPRTGLDFNEMVLLQMENKAWLVHRQWFLDLPLPWLPGADTQGSNWIQKETLVTFKNPHAKK
QDVVVLGSQEGAMHTALTGATEIQMSSGNLLFTGHLKCRLRMDKLQLKGMSYSMCTGKFKVV
KEIAETQHGTIVIRVQYEGDGSPCKIPFEIMDLEKRHVLGRLITVNPIVTEKDSPVNIEAEP
PFGDSYIIIGVEPGQLKLNWFKK ("DENV2 HCat3; SEQ ID NO:12)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: R2M, E44L, D154L, and K246Y.

In some embodiments, a stabilized recombinant flavivirus E glycoprotein of the present invention may comprise, consist essentially of, or consist of the amino acid sequence:

SMMCIGMSNRDFVEGVSGGSWVDIVLEHGSCVTTMAKNKPTLDFLLIKTEAKQPATLRKYCI
EAKLTNTTTESRCPTQGEPSLNEEQDKRFVCKHSMVDRGWGNGCGLFGKGGIVTCAMFRCKK
NMEGKVVQPENLEYTIVITPHSGEEHAVGNLTGKHGKEIKITPQSSITEAELTGYGTVTMEC
SPRTGLDFNEMVLLQMENKAWLVHRQWFLDLPLPWLPGADTQGSNWIQKETLVTFKNPHAIK
QDVVVLGSQEGAMHTALTGATEIQMSSGNLLFTGHLKCRLRMDKLQLKGMSYSMCTGKFKVV
KEIAETQHGTIVIRVQYEGDGSPCKIPFEIMDLEKRHVLGRLITVNPIVTEKDSPVNIEAEP
PFGDSYIIIGVEPGQLKLNWFKK ("DENV2 HCat4"; SEQ ID NO:14)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: K204F and H261L.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENFAWLVHRQWFLDLPLPWLPGADTQGSNWIQKETLVTFKNPHAKKQ
DVVVLGSQEGAMLTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 HCat8"; SEQ ID NO:22)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: A259V and T262H.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGVMHHALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 IntFc1"; SEQ ID NO:24)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: A259W and T262R.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 IntFc2"; SEQ ID NO:26)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: A259W, T262Y, and A263L.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGVMHRWLTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 IntFc3"; SEQ ID NO:28)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: T262R and A263W .

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHRWLTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 IntFc5"; SEQ ID NO:32)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: N8L, S29Y, and H244F.

In some embodiments, a stabilized recombinant flavivirus E glycoprotein of the present invention may comprise, consist essentially of, or consist of the amino acid sequence:

MRCIGMSLRDFVEGVSGGSWVDIVLEHGYCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPFAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 IntFc6"; SEQ ID NO:34)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: T262R.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 IntFc7"; SEQ ID NO:36)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: G106D.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 IntFc8"; SEQ ID NO:38)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: M6L and Q316M.

In some embodiments, a stabilized recombinant flavivirus E glycoprotein of the present invention may comprise, consist essentially of, or consist of the amino acid sequence:

MRCIGLSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETMHGTIVIRVQYEGDGSPCKIPFEIMDLEKRHVLGRLITVNPIVTEKDSPVNIEAEPP
FGDSYIIIGVEPGQLKLNWFKK ("DENV2 PM1"; SEQ ID NO:44)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: Q131I and Y299L.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVIPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSLSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 PM2"; SEQ ID NO:46)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: A35M and M289W.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRWDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 PM3"; SEQ ID NO:48)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, and A35M.

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 PM4"; SEQ ID NO:50)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: A35M.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 PM4.1"; SEQ ID NO:52)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: T33V and A35M.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTVMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 PM4.2"; SEQ ID:54)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K.

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 PM4.3"; SEQ ID NO:56)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: H209D and G266W.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVDRQWFLDLPLPWLPGADTQGSNWIQKETLVTFKNPHAKKQ
DVVVLGSQEGAMHTALTWATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 PM5"; SEQ ID NO:58)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: V15E, F373D, and F392R.

In some embodiments, a stabilized recombinant flavivirus E glycoprotein of the present invention may comprise, consist essentially of, or consist of the amino acid sequence:

MRCIGMSNRDFVEGESGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
DGDSYIIIGVEPGQLKLNWRKK ("DENV2 SHP1"; SEQ ID NO:66)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: E13F, G14A, and M34L.

In some embodiments, a stabilized recombinant flavivirus E glycoprotein of the present invention may comprise, consist essentially of, or consist of the amino acid sequence:

MRCIGMSNRDFVFAVSGGSWVDIVLEHGSCVTTLAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 UndPk4"; SEQ ID NO:76)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: T359Y.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK ("DENV2 UndPk5"; SEQ ID NO:78)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: F279W and T280P.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 UndPk6"; SEQ ID NO:80)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: T280P.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 UndPk6.1"; SEQ ID NO:82)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: T48I and L277M.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKIEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNMLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 UndPk7"; SEQ ID NO:84)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: Q256Y, G258A, A259W, and M260L.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSYEAWLHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 UndPk8"; SEQ ID NO:86)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, A35M, G106D, A259W, and T262R.

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.1 (I2-I8-P4)"; SEQ ID NO:92)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: H27P, S29K, T33V, A35M, T48I, G106D, A259W, and T262R.

In some embodiments, a stabilized recombinant flavivirus E glycoprotein of the present invention may comprise, consist essentially of, or consist of the amino acid sequence:

MRCIGMSNRDFVEGVSGGSWVDIVLEPGKCVTVMMKNKPTLDFELIKIEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.2 (I2-I8-P4-H3)"; SEQ ID NO:94)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, A35M, G106D, H209D, A259W, T262R, G266W, F279W, F280P, and T359Y.

In some embodiments, a stabilized recombinant flavivirus E glycoprotein of the present invention may comprise, consist essentially of, or consist of the amino acid sequence:

MRCIGMSNRDFVFAVSGGSWVDIVLEHGSCVTTLAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVDRQWFLDLPLPWLPGADTQGSNWIQKETLVTFKNPHAKKQ
DVVVLGSQEGWMHRALTWATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK ("DENV2 SC.3 (I2-I8-U4-U5-U6-P5)"; SEQ ID NO:96)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: E13F, G14A, H27P, S29K, T33V, M34L, A35M, T48I, A259W, T262R, F279W, T280P, and T359Y.

In some embodiments, a stabilized recombinant flavivirus E glycoprotein of the present invention may comprise, consist essentially of, or consist of the amino acid sequence:

MRCIGMSNRDFVFAVSGGSWVDIVLEPGKCVTVLMKNKPTLDFELIKIEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVDRQWFLDLPLPWLPGADTQGSNWIQKETLVTFKNPHAKKQ
DVVVLGSQEGWMHRALTWATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK
("DENV2 SC.4 (I2-I8-U4-U5-U6-P4-H3)"; SEQ ID NO:98")

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: E13F, G14A, M34L, G106D, A259W, T262R, F279W, T280P.

In some embodiments, a stabilized recombinant flavivirus E glycoprotein of the present invention may comprise, consist essentially of, or consist of the amino acid sequence:

MRCIGMSNRDFVFAVSGGSWVDIVLEHGSCVTTLAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.5 (I2-I8-U4-U6)"; SEQ ID NO:100)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, A35M, G106D, A259W, T262R, and T359Y.

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK ("DENV2 SC.6 (I2-I8-U5-P4)"; SEQ ID NO:102)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: G106D, A259W, T262R, F279W, T280P, and T359Y.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK ("DENV2 SC.7 (I2-I8-U5-U6)"; SEQ ID NO:104)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, A35M, G106D, A259W, T262R, F279W, T280P, and T359Y.

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK ("DENV2 SC.8 (I2-I8-U5-U6-P4)"; SEQ ID NO:106)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: V15E, S29K, T33V, A35M, G106D, A259W, T262R, F279W, T280P, T359Y, F373D, and F392R.

In some embodiments, a stabilized recombinant flavivirus E glycoprotein of the present invention may comprise, consist essentially of, or consist of the amino acid sequence:

MRCIGMSNRDFVEGESGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
DGDSYIIIGVEPGQLKLNWRKK ("DENV2 SC.9 (I2-I8-U5-U6-P4-S1)"; SEQ ID NO:108)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: G106D, A259W, T262R, F279W, and T280P.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.10 (I2-I8-U6)"; SEQ ID NO:110)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: H27P, T48I, G106D, A259W, T262R, F279W, and T280P.

In some embodiments, a stabilized recombinant flavivirus E glycoprotein of the present invention may comprise, consist essentially of, or consist of the amino acid sequence:

MRCIGMSNRDFVEGVSGGSWVDIVLEPGSCVTTMAKNKPTLDFELIKIEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.11 (I2-I8-U6-H3)"; SEQ ID NO:112)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, A35M, G106D, A259W, T262R, F279W, and T280P.

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.12 (I2-I8-U6-P4)"; SEQ ID NO:114)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, A35M, A259W, T262R, F279W, T280P, and T359Y.

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK ("DENV2 SC.13 (I2-U5-U6-P4)"; SEQ ID NO:116)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: A259W, T262R, F279W, and T280P.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.14 (I2-U6)"; SEQ ID NO:118)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: G106D, F279W, and T280P.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.15 (I8-U6)"; SEQ ID NO:120)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: H27P, T48I, A259W, T262R, F279W, and T280P .

In some embodiments, a stabilized recombinant flavivirus E glycoprotein of the present invention may comprise, consist essentially of, or consist of the amino acid sequence:

MRCIGMSNRDFVEGVSGGSWVDIVLEPGSCVTTMAKNKPTLDFELIKIEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.16 (I2-U6-H3)"; SEQ ID NO:122)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, A35M, A259W, T262R, F279W, and T280P.

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.17 (I2-U6-P4)"; SEQ ID NO:124)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: E13F, G14A, S29K, T33V, M34L, A35M, G106D, A259W, T262R, F279W, and T280P.

In some embodiments, a stabilized recombinant flavivirus E glycoprotein of the present invention may comprise, consist essentially of, or consist of the amino acid sequence:

MRCIGMSNRDFVFAVSGGSWVDIVLEHGKCVTVLMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.18 (I2-I8-U4-U6-P4)"; SEQ ID NO:126)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, A35M, G106D, F279W, T280P, and T359Y.

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK ("DENV2 SC.19 (I8-U5-U6-P4)"; SEQ ID NO:128)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: E13F, G14A, S29K, T33V, M34L, and A35M.

In some embodiments, a stabilized recombinant flavivirus E glycoprotein of the present invention may comprise, consist essentially of, or consist of the amino acid sequence:

MRCIGMSNRDFVFAVSGGSWVDIVLEHGKCVTVLMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.20 (P4-U4)"; SEQ ID NO:130)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: E13F, G14A, M34L, F279W, T280P, and T359Y .

In some embodiments, a stabilized recombinant flavivirus E glycoprotein of the present invention may comprise, consist essentially of, or consist of the amino acid sequence:

MRCIGMSNRDFVFAVSGGSWVDIVLEHGSCVTTLAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK ("DENV2 SC.21 (U4-U5-U6)"; SEQ ID NO:132)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: E13F, G14A, M34L, H209D, G266W, F279W, T280P, and T359Y.

In some embodiments, a stabilized recombinant flavivirus E glycoprotein of the present invention may comprise, consist essentially of, or consist of the amino acid sequence:

MRCIGMSNRDFVFAVSGGSWVDIVLEHGSCVTTLAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVDRQWFLDLPLPWLPGADTQGSNWIQKETLVTFKNPHAKKQ
DVVVLGSQEGAMHTALTWATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK ("DENV2 SC.22 (U4-U5-U6-P5)"; SEQ ID NO:134)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: G106D, A259W, and T262R .

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.23 (I2-I8)"; SEQ ID NO:136)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: H27P, T48I, and G258E.

In some embodiments, a stabilized recombinant flavivirus E glycoprotein of the present invention may comprise, consist essentially of, or consist of the amino acid sequence:

MRCIGMSNRDFVEGVSGGSWVDIVLEPGSCVTTMAKNKPTLDFELIKIEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEEAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.24 (M2-H3)"; SEQ ID NO:138)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, A35M, and G258E.

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEEAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.25 (M2-P4)"; SEQ ID NO:140)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: G106D, A259V, T262R, A263W, F279W, and T280P.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGVMHRWLTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.26 (I3-I8-U6)"; SEQ ID NO:142)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, A35M, G106D, A259V, T262R, A263W, F279W, and T280P .

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGVMHRWLTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.27 (I3-I8-U6-P4)"; SEQ ID NO:144)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S29K, T33V, A35M, L107C, and A313C.

In some embodiments, a stabilized recombinant flavivirus E glycoprotein of the present invention may comprise, consist essentially of, or consist of the amino acid sequence:

MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCGCFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EICETQHGTIVIRVQYEGDGSPCKIPFEIMDLEKRHVLGRLITVNPIVTEKDSPVNIEAEPP
FGDSYIIIGVEPGQLKLNWFKK ("DENV2 SC.28 (P4-Cm2)"; SEQ ID NO:146)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: R9V, T32V, H144Y, and E368I.

In some embodiments, a stabilized recombinant flavivirus E glycoprotein of the present invention may comprise, consist essentially of, or consist of the amino acid sequence:

MRCIGMSNVDFVEGVSGGSWVDIVLEHGSCVVTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPYSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIIAEPP
FGDSYIIIGVEPGQLKLNWFKK ("DENV2 HCat1"; SEQ ID NO:8)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: R2M, H27P, E44M, T48I, D 154M, H244Q, and K246Y.

In some embodiments, a stabilized recombinant flavivirus E glycoprotein of the present invention may comprise, consist essentially of, or consist of the amino acid sequence:

MMCIGMSNRDFVEGVSGGSWVDIVLEPGSCVTTMAKNKPTLDFMLIKIEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNMTGKHGKEIKITPQSSITEAELTGYGTVTMECS
PRTGLDFNEMVLLQMENKAWLVHRQWFLDLPLPWLPGADTQGSNWIQKETLVTFKNPQAYKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 HCat5"; SEQ ID NO:16)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: K204F, W206F, and H261L.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENFAFLVHRQWFLDLPLPWLPGADTQGSNWIQKETLVTFKNPHAKKQ
DVVVLGSQEGAMLTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 HCat6"; SEQ ID NO:18)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: K204F, W251F, and H261F.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENFAWLVHRQWFLDLPLPWLPGADTQGSNWIQKETLVTFKNPHAKKQ
DVFVLGSQEGAMFTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 HCat7"; SEQ ID NO:20)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: A259W, T262Y, and A263L.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHYLLTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 IntFc4"; SEQ ID NO:30)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: D375L and N390Q.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGLSYIIIGVEPGQLKLQWFKK ("DENV2 PM6"; SEQ ID NO:60)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: G330A.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDASPCKIPFEIMDLEKRHVLGRLITVNPIVTEKDSPVNIEAEPP
FGDSYIIIGVEPGQLKLNWFKK ("DENV2 PM7"; SEQ ID NO:62)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: Y377V and K393R.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSVIIIGVEPGQLKLNWFRK ("DENV2 PM8"; SEQ ID NO:64)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: I270V and T280A.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEVQMSSGNLLFAGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 UndPk1"; SEQ ID NO:70)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: L198W.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVWLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 UndPk2"; SEQ ID NO:72)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: L191Y and H209D.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGYDFNEMVLLQMENKAWLVDRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("DENV2 UndPk3"; SEQ ID NO:74)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: V15E, W20H, F373D, and F392R.

In some embodiments, a stabilized recombinant flavivirus E glycoprotein of the present invention may comprise, consist essentially of, or consist of the amino acid sequence:

MRCIGMSNRDFVEGESGGSHVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
DGDSYIIIGVEPGQLKLNWRKK ("DENV2 SHP2"; SEQ ID NO:68)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV1 identified as SEQ ID NO: 147: S29K, T33V, and A35M.

In some embodiments, a stabilized recombinant flavivirus E glycoprotein of the present invention may comprise, consist essentially of, or consist of the amino acid sequence:

MRCVGIGNRDFVEGLSGATWVDVVLEHGKCVTVMMKDKPTLDIELLKTEVTNPAV
LRKLCIEAKISNTTTDSRCPTQGEATLVEEQDTNFVCRRTFVDRGWGNGCGLFGKGS
LITCAKFKCVTKLEGKIVQYENLKYSVIVTVHTGDQHQVGNETTEHGTTATITPQAPT
SEIQLTDYGALTLDCSPRTGLDFNEMVLLTMEKKSWLVHKQWFLDLPLPWTSGAST
SQETWNRQDLLVTFKTAHAKKQEVVVLGSQEGAMHTALTGATEIQTSGTTTIFAGH
LKCRLKMDKLTLKGMSYVMCTGSFKLEKEVAETQHGTVLVQVKYEGTDAPCKIPFS
SQDEKGVTQNGRLITANPIVTDKEKPVNIEAEPPFGESYIVVGAGEKALKLSWFKK
("DENV1 PM4"; SEQ ID NO:148)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV1 identified as SEQ ID NO: 147: A259W and S262R.

In some embodiments, a stabilized recombinant flavivirus E glycoprotein of the present invention may comprise, consist essentially of, or consist of the amino acid sequence:

MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCGLFGKGSLI
TCAKFKCVTKLEGKIVQYENLKYSVIVTVHTGDQHQVGNETTEHGTTATITPQAPTS
EIQLTDYGALTLDCSPRTGLDFNEMVLLTMEKKSWLVHKQWFLDLPLPWTSGASTS
QETWNRQDLLVTFKTAHAKKQEVVVLGSQEGWMHRALTGATEIQTSGTTTIFAGHL
KCRLKMDKLTLKGMSYVMCTGSFKLEKEVAETQHGTVLVQVKYEGTDAPCKIPFSS
QDEKGVTQNGRLITANPIVTDKEKPVNIEAEPPFGESYIVVGAGEKALKLSWFKK
("DENV1 IntFc2"; SEQ ID NO:149)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV1 identified as SEQ ID NO: 147: G106D.

In some embodiments, a stabilized recombinant flavivirus E glycoprotein of the present invention may comprise, consist essentially of, or consist of the amino acid sequence:

MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCDLFGKGSLI
TCAKFKCVTKLEGKIVQYENLKYSVIVTVHTGDQHQVGNETTEHGTTATITPQAPTS
EIQLTDYGALTLDCSPRTGLDFNEMVLLTMEKKSWLVHKQWFLDLPLPWTSGASTS
QETWNRQDLLVTFKTAHAKKQEVVVLGSQEGAMHTALTGATEIQTSGTTTIFAGHL
KCRLKMDKLTLKGMSYVMCTGSFKLEKEVAETQHGTVLVQVKYEGTDAPCKIPFSS
QDEKGVTQNGRLITANPIVTDKEKPVNIEAEPPFGESYIVVGAGEKALKLSWFKK
("DENV1 IntFc8"; SEQ ID NO:150)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV1 identified as SEQ ID NO: 147: F279W and A280P.

In some embodiments, a stabilized recombinant flavivirus E glycoprotein of the present invention may comprise, consist essentially of, or consist of the amino acid sequence:

MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCGLFGKGSLI
TCAKFKCVTKLEGKIVQYENLKYSVIVTVHTGDQHQVGNETTEHGTTATITPQAPTS
EIQLTDYGALTLDCSPRTGLDFNEMVLLTMEKKSWLVHKQWFLDLPLPWTSGASTS
QETWNRQDLLVTFKTAHAKKQEVVVLGSQEGAMHTALTGATEIQTSGTTTIWPGHL
KCRLKMDKLTLKGMSYVMCTGSFKLEKEVAETQHGTVLVQVKYEGTDAPCKIPFSS
QDEKGVTQNGRLITANPIVTDKEKPVNIEAEPPFGESYIVVGAGEKALKLSWFKK
("DENV1 UndPk6"; SEQ ID NO:151)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: G29K, T33V, and A35M.

In some embodiments, a stabilized recombinant flavivirus E glycoprotein of the present invention may comprise, consist essentially of, or consist of the amino acid sequence:

MRCVGVGNRDFVEGLSGATWVDVVLEHGKCVTVMMKNKPTLDIELQKTEATQLA
TLRKLCIEGKITNITTDSRCPTQGEAILPEEQDQNYVCKHTYVDRGWGNGCGLFGKG
SLVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
("DENV3 PM4"; SEQ ID NO:153)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: A257W and T260R.

In some embodiments, a stabilized recombinant flavivirus E glycoprotein of the present invention may comprise, consist essentially of, or consist of the amino acid sequence:

MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
("DENV3 IntFc2"; SEQ ID NO:154)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: G106D.

In some embodiments, a stabilized recombinant flavivirus E glycoprotein of the present invention may comprise, consist essentially of, or consist of the amino acid sequence:

MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
("DENV3 IntFc8"; SEQ ID NO:155)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: F277W and A278P.

In some embodiments, a stabilized recombinant flavivirus E glycoprotein of the present invention may comprise, consist essentially of, or consist of the amino acid sequence:

MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIWPGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
("DENV3 UndPk6"; SEQ ID NO:156)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: F277W, A278P, S275L, and M205L.

In some embodiments, a stabilized recombinant flavivirus E glycoprotein of the present invention may comprise, consist essentially of, or consist of the amino acid sequence:

MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWLVHRQWFFDLPLPWTSGATTET
PTWNRKELLVTFKNAHAKKQEVVVLGSQEGAMHTALTGATEIQNSGGTLIWPGHLK
CRLKMDKLELKGMSYAMCLNTFVLKKEVSETQHGTILIKVEYKGEDAPCKIPFSTED
GQGKAHNGRLITANPVVTKKEEPVNIEAEPPFGESNIVIGIGDKALKINWYKK
("DENV3 UndPk6 DV2HIngemut"; SEQ ID NO:157)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: G106D, A257W, T260R, F277W, and A278P.

In some embodiments, a stabilized recombinant flavivirus E glycoprotein of the present invention may comprise, consist essentially of, or consist of the amino acid sequence:

MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIWPGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
("DENV3 SC.10 (I2-I8-U6)"; SEQ ID NO:158)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: A257W, T260R, F277W, and A278P.

In some embodiments, a stabilized recombinant flavivirus E glycoprotein of the present invention may comprise, consist essentially of, or consist of the amino acid sequence:

MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIWPGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
("DENV3 SC.14 (I2-U6)"; SEQ ID NO:159)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: A278P.

In some embodiments, a stabilized recombinant flavivirus E glycoprotein of the present invention may comprise, consist essentially of, or consist of the amino acid sequence:

MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIFPGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
("DENV3 UndPk6.1 (A278P)"; SEQ ID NO:160)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: H27P and T48I.

In some embodiments, a stabilized recombinant flavivirus E glycoprotein of the present invention may comprise, consist essentially of, or consist of the amino acid sequence:

MRCVGVGNRDFVEGLSGATWVDVVLEPGGCVTTMAKNKPTLDIELQKIEATQLATL
RKLCIEGKITNITTDSRCPTQGEAILPEEQDQNYVCKHTYVDRGWGNGCGLFGKGSL
VTCAKFQCLESIEGKVVQHENLKYTVIITVHTGDQHQVGNETQGVTAEITPQASTVE
AILPEYGTLGLECSPRTGLDFNEMILLTMKNKAWMVHRQWFFDLPLPWTSGATTETP
TWNRKELLVTFKNAHAKKQEVVVLGSQEGAMHTALTGATEIQNSGGTSIFAGHLKC
RLKMDKLELKGMSYAMCLNTFVLKKEVSETQHGTILIKVEYKGEDAPCKIPFSTEDG
QGKAHNGRLITANPVVTKKEEPVNIEAEPPFGESNIVIGIGDKALKINWYKK
("DENV3 HCat3"; SEQ ID NO:161)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: G29K, T33V, A35M, G106D, A257W, T260R, F277W, and A278P.

In some embodiments, a stabilized recombinant flavivirus E glycoprotein of the present invention may comprise, consist essentially of, or consist of the amino acid sequence:

MRCVGVGNRDFVEGLSGATWVDVVLEHGKCVTVMMKNKPTLDIELQKTEATQLA
TLRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIWPGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
("DENV3 SC.12 (I2-I8-U6-P4)"; SEQ ID NO:162)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: G29K, T33V, A35M, G106D, F277W, and A278P.

In some embodiments, a stabilized recombinant flavivirus E glycoprotein of the present invention may comprise, consist essentially of, or consist of the amino acid sequence:

MRCVGVGNRDFVEGLSGATWVDVVLEHGKCVTVMMKNKPTLDIELQKTEATQLA
TLRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIWPGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
("DENV3 SC.29 (I8-U6-P4)"; SEQ ID NO:163)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: H27P, T48I, A257W, T260R, F277W, and A278P.

In some embodiments, a stabilized recombinant flavivirus E glycoprotein of the present invention may comprise, consist essentially of, or consist of the amino acid sequence:

MRCVGVGNRDFVEGLSGATWVDVVLEPGGCVTTMAKNKPTLDIELQKIEATQLATL
RKLCIEGKITNITTDSRCPTQGEAILPEEQDQNYVCKHTYVDRGWGNGCGLFGKGSL
VTCAKFQCLESIEGKVVQHENLKYTVIITVHTGDQHQVGNETQGVTAEITPQASTVE
AILPEYGTLGLECSPRTGLDFNEMILLTMKNKAWMVHRQWFFDLPLPWTSGATTETP
TWNRKELLVTFKNAHAKKQEVVVLGSQEGWMHRALTGATEIQNSGGTSIWPGHLK
CRLKMDKLELKGMSYAMCLNTFVLKKEVSETQHGTILIKVEYKGEDAPCKIPFSTED
GQGKAHNGRLITANPVVTKKEEPVNIEAEPPFGESNIVIGIGDKALKINWYKK
("DENV3 SC.16 (I2-U6-H3)"; SEQ ID NO:164)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: H27P, T48I, G106D, A257W, T260R, F277W, and A278P.

In some embodiments, a stabilized recombinant flavivirus E glycoprotein of the present invention may comprise, consist essentially of, or consist of the amino acid sequence:

MRCVGVGNRDFVEGLSGATWVDVVLEPGGCVTTMAKNKPTLDIELQKIEATQLATL
RKLCIEGKITNITTDSRCPTQGEAILPEEQDQNYVCKHTYVDRGWGNGCDLFGKGSL
VTCAKFQCLESIEGKVVQHENLKYTVIITVHTGDQHQVGNETQGVTAEITPQASTVE
AILPEYGTLGLECSPRTGLDFNEMILLTMKNKAWMVHRQWFFDLPLPWTSGATTETP
TWNRKELLVTFKNAHAKKQEVVVLGSQEGWMHRALTGATEIQNSGGTSIWPGHLK
CRLKMDKLELKGMSYAMCLNTFVLKKEVSETQHGTILIKVEYKGEDAPCKIPFSTED
GQGKAHNGRLITANPVVTKKEEPVNIEAEPPFGESNIVIGIGDKALKINWYKK
("DENV3 SC.11 (I2-I8-U6-H3)"; SEQ ID NO:165)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: G106D, F277W, and A278P.

In some embodiments, a stabilized recombinant flavivirus E glycoprotein of the present invention may comprise, consist essentially of, or consist of the amino acid sequence:

MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIWPGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
("DENV3 SC.15 (I8-U6)"; SEQ ID NO:166)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: G106D, A257W, and T260R.

In some embodiments, a stabilized recombinant flavivirus E glycoprotein of the present invention may comprise, consist essentially of, or consist of the amino acid sequence:

MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
("DENV3 SC.23 (I2-I8)"; SEQ ID NO:167)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV3 identified as SEQ ID NO: 152: G29K, T33V, A35M, G106D, A257W, and T260R.

In some embodiments, a stabilized recombinant flavivirus E glycoprotein of the present invention may comprise, consist essentially of, or consist of the amino acid sequence:

MRCVGVGNRDFVEGLSGATWVDVVLEHGKCVTVMMKNKPTLDIELQKTEATQLA
TLRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
("DENV3 SC.1 (I2-I8-P4)"; SEQ ID NO:168)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV4 identified as SEQ ID NO: 169: G29K, T33V, and A35M.

In some embodiments, a stabilized recombinant flavivirus E glycoprotein of the present invention may comprise, consist essentially of, or consist of the amino acid sequence:

MRCVGVGNRDFVEGVSGGAWVDLVLEHGKCVTVMMQGKPTLDFELTKTTAKEVA
LLRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MFAGHLKCKVRMEKLRIKGMSYTMCSGKFSIDKEMAETQHGTTVVKVKYEGAGAP
CKVPIEIRDVNKEKVVGRIISSTPLAENTNSVTNIELEPPFGDSYIVIGVGNSALTLHWF
RK ("DENV4 PM4"; SEQ ID NO:170)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV4 identified as SEQ ID NO: 169: A259W and S262R.

In some embodiments, a stabilized recombinant flavivirus E glycoprotein of the present invention may comprise, consist essentially of, or consist of the amino acid sequence:

MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGWMHRALAGATEVDSGDGN
HMFAGHLKCKVRMEKLRIKGMSYTMCSGKFSIDKEMAETQHGTTVVKVKYEGAGA
PCKVPIEIRDVNKEKVVGRIISSTPLAENTNSVTNIELEPPFGDSYIVIGVGNSALTLHW
FRK ("DENV4 IntFc2"; SEQ ID NO:171)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV4 identified as SEQ ID NO: 169: G106D.

In some embodiments, a stabilized recombinant flavivirus E glycoprotein of the present invention may comprise, consist essentially of, or consist of the amino acid sequence:

MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCDLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MFAGHLKCKVRMEKLRIKGMSYTMCSGKFSIDKEMAETQHGTTVVKVKYEGAGAP
CKVPIEIRDVNKEKVVGRIISSTPLAENTNSVTNIELEPPFGDSYIVIGVGNSALTLHWF
RK ("DENV4 IntFc8"; SEQ ID NO:172)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV4 identified as SEQ ID NO: 169: F279W and A280P.

In some embodiments, a stabilized recombinant flavivirus E glycoprotein of the present invention may comprise, consist essentially of, or consist of the amino acid sequence:

MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MWPAGHLKCKVRMEKLRIKGMSYTMCSGKFSIDKEMAETQHGTTVVKVKYEGAG
APCKVPIEIRDVNKEKVVGRIISSTPLAENTNSVTNIELEPPFGDSYIVIGVGNSALTLH
WFRK ("DENV4 UndPk6"; SEQ ID NO:173)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV4 identified as SEQ ID NO: 169: G29K, T33V, A35M, G106D, A259W, S262R, F279W, and A280P.

In some embodiments, a stabilized recombinant flavivirus E glycoprotein of the present invention may comprise, consist essentially of, or consist of the amino acid sequence:

MRCVGVGNRDFVEGVSGGAWVDLVLEHGKCVTVMMQGKPTLDFELTKTTAKEVA
LLRTYCIEASISNITTATRCPTQGEPYLKEEQDQQYICRRDVVDRGWGNGCDLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGWMHRALAGATEVDSGDGN
HMWPAGHLKCKVRMEKLRIKGMSYTMCSGKFSIDKEMAETQHGTTVVKVKYEGA
GAPCKVPIEIRDVNKEKVVGRIISSTPLAENTNSVTNIELEPPFGDSYIVIGVGNSALTL
HWFRK ("DENV4 SC.12 (I2-I8-U6-P4)"; SEQ ID NO:174)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV4 identified as SEQ ID NO: 169: A280P.

In some embodiments, a stabilized recombinant flavivirus E glycoprotein of the present invention may comprise, consist essentially of, or consist of the amino acid sequence:

MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MFPGHLKCKVRMEKLRIKGMSYTMCSGKFSIDKEMAETQHGTTVVKVKYEGAGAP
CKVPIEIRDVNKEKVVGRIISSTPLAENTNSVTNIELEPPFGDSYIVIGVGNSALTLHWF
RK ("DENV4 UndPk6.1 (A280P)"; SEQ ID NO:175)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV4 identified as SEQ ID NO: 169: H27P and T48I.

In some embodiments, a stabilized recombinant flavivirus E glycoprotein of the present invention may comprise, consist essentially of, or consist of the amino acid sequence:

MRCVGVGNRDFVEGVSGGAWVDLVLEPGGCVTTMAQGKPTLDFELTKITAKEVAL
LRTYCIEASISNITTATRCPTQGEPYLKEEQDQQYICRRDVVDRGWGNGCGLFGKGG
VVTCAKFSCSGKITGNLVQIENLEYTVVVTVHNGDTHAVGNDTSNHGVTAMITPRSP
SVEVKLPDYGELTLDCEPRSGIDFNEMILMKMKKKTWLVHKQWFLDLPLPWTAGA
DTSEVHWNYKERMVTFKVPHAKRQDVTVLGSQEGAMHSALAGATEVDSGDGNHM
FAGHLKCKVRMEKLRIKGMSYTMCSGKFSIDKEMAETQHGTTVVKVKYEGAGAPC
KVPIEIRDVNKEKVVGRIISSTPLAENTNSVTNIELEPPFGDSYIVIGVGNSALTLHWFR
K ("DENV4 HCat3"; SEQ ID NO:176)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV4 identified as SEQ ID NO: 169: G29K, T33V, A35M, G106D, A257W, and S262R.

In some embodiments, a stabilized recombinant flavivirus E glycoprotein of the present invention may comprise, consist essentially of, or consist of the amino acid sequence:

MRCVGVGNRDFVEGVSGGAWVDLVLEHGKCVTVMMQGKPTLDFELTKTTAKEVA
LLRTYCIEASISNITTATRCPTQGEPYLKEEQDQQYICRRDVVDRGWGNGCDLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGWMHRALAGATEVDSGDGN
HMFAGHLKCKVRMEKLRIKGMSYTMCSGKFSIDKEMAETQHGTTVVKVKYEGAGA
PCKVPIEIRDVNKEKVVGRIISSTPLAENTNSVTNIELEPPFGDSYIVIGVGNSALTLHW
FRK ("DENV4 SC.1 (I2-I8-P4)"; SEQ ID NO:177)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of ZIKV identified as SEQ ID NO: 178: S29K, V33, and A35M.

In some embodiments, a stabilized recombinant flavivirus E glycoprotein of the present invention may comprise, consist essentially of, or consist of the amino acid sequence:

IRCIGVSNRDFVEGMSGGTWVDIVLEHGKCVTVMMQDKPTVDIELVTTTVSNMAEV
RSYCYEASISDMASDSRCPTQGEAYLDKQSDTQYVCKRTLVDRGWGNGCGLFGKGS
LVTCAKFACSKKMTGKSIQPENLEYRIMLSVHGSQHSGMIVNDTGHETDENRAKVEI
TPNSPRAEATLGGFGSLGLDCEPRTGLDFSDLYYLTMNNKHWLVHKEWFHDIPLPW
HAGADTGTPHWNNKEALVEFKDAHAKRQTVVVLGSQEGAVHTALAGALEAEMDG
AKGRLSSGHLKCRLKMDKLRLKGVSYSLCTAAFTFTKIPAETLHGTVTVEVQYAGT
DGPCKVPAQMAVDMQTLTPVGRLITANPVITESTENSKMMLELDPPFGDSYIVIGVG
EKKITHHWHRSG ("ZIKV PM4"; SEQ ID NO:179)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of ZIKV identified as SEQ ID NO: 178: A264W and T267R.

In some embodiments, a stabilized recombinant flavivirus E glycoprotein of the present invention may comprise, consist essentially of, or consist of the amino acid sequence:

IRCIGVSNRDFVEGMSGGTWVDIVLEHGGCVTVMAQDKPTVDIELVTTTVSNMAEV
RSYCYEASISDMASDSRCPTQGEAYLDKQSDTQYVCKRTLVDRGWGNGCGLFGKGS
LVTCAKFACSKKMTGKSIQPENLEYRIMLSVHGSQHSGMIVNDTGHETDENRAKVEI
TPNSPRAEATLGGFGSLGLDCEPRTGLDFSDLYYLTMNNKHWLVHKEWFHDIPLPW
HAGADTGTPHWNNKEALVEFKDAHAKRQTVVVLGSQEGWVHRALAGALEAEMD
GAKGRLSSGHLKCRLKMDKLRLKGVSYSLCTAAFTFTKIPAETLHGTVTVEVQYAG
TDGPCKVPAQMAVDMQTLTPVGRLITANPVITESTENSKMMLELDPPFGDSYIVIGV
GEKKITHHWHRSG ("ZIKV IntFc2"; SEQ ID NO:180)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of ZIKV identified as SEQ ID NO: 178: G106D.

In some embodiments, a stabilized recombinant flavivirus E glycoprotein of the present invention may comprise, consist essentially of, or consist of the amino acid sequence:

IRCIGVSNRDFVEGMSGGTWVDIVLEHGGCVTVMAQDKPTVDIELVTTTVSNMAEV
RSYCYEASISDMASDSRCPTQGEAYLDKQSDTQYVCKRTLVDRGWGNGCDLFGKGS
LVTCAKFACSKKMTGKSIQPENLEYRIMLSVHGSQHSGMIVNDTGHETDENRAKVEI
TPNSPRAEATLGGFGSLGLDCEPRTGLDFSDLYYLTMNNKHWLVHKEWFHDIPLPW
HAGADTGTPHWNNKEALVEFKDAHAKRQTVVVLGSQEGAVHTALAGALEAEMDG
AKGRLSSGHLKCRLKMDKLRLKGVSYSLCTAAFTFTKIPAETLHGTVTVEVQYAGT
DGPCKVPAQMAVDMQTLTPVGRLITANPVITESTENSKMMLELDPPFGDSYIVIGVG
EKKITHHWHRSG ("ZIKV IntFc8"; SEQ ID NO:181)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: S255E.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGEQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("Mnmer1"; SEQ ID NO:40)

In some embodiments, a stabilized recombinant flavivirus E glycoprotein of the present invention may comprise the following amino acid substitutions wherein the numbering is based on the reference amino acid sequence of an E glycoprotein (e.g., a soluble recombinant glycoprotein) of DENV2 identified as SEQ ID NO:6: G258E.

In some embodiments, a stabilized recombinant flavivirus E glycoprotein of the present invention may comprise, consist essentially of, or consist of the amino acid sequence:

MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEEAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK ("Mnmer2"; SEQ ID NO:42)

Additional stabilized recombinant flavivirus E glycoprotein variants may include a stabilized recombinant flavivirus E glycoprotein comprising any of the amino acid substitutions as described in **Tables 1** and **2.** The present invention provides additional non limiting examples of stabilized recombinant flavivirus E glycoprotein of this invention that can be used in the compositions and methods described herein in the SEQUENCES section provided herein. Thus, further stabilized recombinant flavivirus E glycoprotein variants may also include any stabilized recombinant flavivirus E glycoprotein comprising, consisting essentially of, or consisting of any of the amino acid sequences and/or any stabilized recombinant flavivirus E glycoprotein encoded by any of the nucleic acid sequences described herein in the SEQUENCES section provided herein.

Additional stabilized recombinant flavivirus E glycoprotein variants can be made by introducing one or more (e.g., 2, 3, 4, 5, 6, 7, 8, 9, 10, 11, 12, 13, 14, 15, 16, 17, 18, 19, 20, etc.) substituted amino acid residues in the E glycoprotein domain sequence at any position and in any combination. Variants of this invention can also be made by insertion of amino acid residues and/or deletion of amino acid residues, with or without substitution of original amino acids residues. Amino acid residues that can be substituted include naturally occurring amino acid residues such as those shown in **Table 3,** as well as any modified amino acid residues such as those shown in **Table 4.**

In some embodiments, a stabilized recombinant flavivirus E glycoprotein of the present invention comprises substitutions which maintain a conformation of E glycoprotein that inhibits homodimer and/or trimer formation, e.g., monomer conformation, under physiological conditions, e.g., 37°C and/or low protein concentrations, e.g., vaccine conditions. In some embodiments, a stabilized recombinant flavivirus E glycoprotein of the present invention comprises substitutions which maintain a conformation of E glycoprotein that allows homodimer formation, e.g., dimer conformation, under physiological conditions, e.g., 37°C and/or low protein concentrations, e.g., vaccine conditions. Thus, the present invention also provides a stabilized recombinant flavivirus E glycoprotein dimer comprising two flavivirus E glycoproteins of the present invention.

In some embodiments, the dimer has a Tm¹ melting point of about 37°C or higher, as measured at a E glycoprotein concentration of above 1 micromolar (e.g., 4 micromolar, 8 micromolar), e.g., about 37°C to about 60°C, e.g., about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, or about 60°C or higher, as measured at a E glycoprotein concentration of above 1 micromolar. For example, some embodiments, the dimer has a Tm¹ melting point of about 37°C to about 45°C , about 45°C to about 65°C , about 37.5°C to about 50.75°C, or about 37.5°C to about 60.9°C, as measured at a E glycoprotein concentration of above 1 micromolar. In some embodiments, the dimer has a Tm1 melting point that is equal to its tm2 melting point, e.g., wherein the dimer does not dissociate until such temperature that it also unfolds (e.g., until such temperature that the monomeric form unfolds).

In some embodiments, a stabilized recombinant flavivirus E glycoprotein dimer of the present invention dissociates to monomer (a Tm¹ melting point) at a temperature that is higher (e.g., about 0.5 °C to about 45 °C higher, or any value or range therein) than the temperature at which a dimer of a corresponding wildtype flavivirus E glycoprotein dissociates to monomer. In some embodiments, a stabilized recombinant E glycoprotein dimer of the present invention has a Tm¹ melting point that is higher than the Tm¹ melting point of a wildtype (e.g., non-stabilized) sE glycoprotein dimer. In some embodiments, a stabilized recombinant E glycoprotein dimer of the present invention has a Tm¹ melting point that is about 0.5, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, or 45 or more degrees Celsius higher than the Tm¹ melting point of a wildtype sE glycoprotein (e.g., of the corresponding backbone, e.g., of a wildtype DENV1, 2, 3, or 4 sE and/or a wildtype ZIKV sE). For example, in some embodiments, a stabilized recombinant E glycoprotein dimer of the present invention has a Tm¹ melting point that is about 2°C higher than the Tm¹ melting point of a wildtype ZIKV sE, about 8°C higher than the Tm¹ melting point of a wildtype ZIKV sE, about 11°C higher than the Tm¹ melting point of a wildtype ZIKV sE, about 1.5°C higher than the Tm¹ melting point of a wildtype DENV2 sE, about 7°C higher than the Tm¹ melting point of a wildtype DENV2 sE, about 11°C higher than the Tm¹ melting point of a wildtype DENV2 sE, about 15.5°C higher than the Tm¹ melting point of a wildtype DENV2 sE, about 10°C higher than the Tm¹ melting point of a wildtype DENV2 sE, about 22°C higher than the Tm¹ melting point of a wildtype DENV2 sE, about 16°C higher than the Tm¹ melting point of a wildtype DENV3 sE, about 20°C higher than the Tm¹ melting point of a wildtype DENV3 sE, about 21.5°C higher than the Tm¹ melting point of a wildtype DENV3 sE, about 38°C higher than the Tm¹ melting point of a wildtype DENV3 sE, about 42.5°C higher than the Tm¹ melting point of a wildtype DENV3 sE, about 40°C higher than the Tm¹ melting point of a wildtype DENV4 sE, or any value or range therein, e.g., about 4 to about 11°C, about 6 to about 10°C, or about 2 to about 45°C higher than the Tm¹ melting point of a wildtype ZIKV sE, e.g., about 2 to about 20°C, about 4 to about 25°C, or about 6 to about 18.5°C higher than the Tm¹ melting point of a wildtype DENV2 sE, e.g., about 2 to about 45°C, about 4 to about 25°C, or about 6 to about 18.5°C higher than the Tm¹ melting point of a wildtype DENV3 sE.

In some embodiments, a stabilized recombinant E glycoprotein dimer of the present invention has a dimer affinity (K_{d}) of about 20 µM or lower under physiological conditions (e.g., vaccine conditions, e.g., at human body temperature, e.g., as measured at 37°C). In some embodiments, a stabilized recombinant E glycoprotein dimer of the present invention has a dimer affinity of about 18 µM or lower, about 15 µM or lower, about 10 µM or lower, about 3 µM or lower, about 1 µM or lower, about 100 nM or lower, about 10 nM or lower, about 1 nM or lower, about 0.1 nM or lower, or any value or range therein, as measured at 37 °C. For example, in some embodiments, a stabilized recombinant E glycoprotein dimer of the present invention has a dimer affinity of about 1 picomolar to about 20 µM, about 100 picomolar to about 18 µM, about 1 nM to about 3 µM, or about 1 picomolar, about 10 picomolar, about 100 picomolar, about 1 nM, about 10 nM, about 100 nM, about 1 µM, about 2 µM, about 3 µM, about 4 µM, about 5 µM, about 6 µM, about 7 µM, about 8 µM, about 9 µM, about 10 µM, about 11 µM, about 12 µM, about 13 µM, about 14 µM, about 15 µM, about 16 µM, about 17 µM, about 18 µM, about 19 µM, about 20 µM, or any value or range therein.

The present invention also provides a flavivirus particle and a virus like particle (VLP) comprising the stabilized recombinant E glycoprotein of this invention. The flavivirus E glycoprotein of the invention can be present in an intact virus particle (e.g., a killed or live attenuated virus particle or a recombinant flavivirus vector (e.g., a recombinant dengue virus vector)) or a virus-like particle (VLP), which may optionally be an intact dengue virus particle or dengue virus VLP.

Also provided is an isolated nucleic acid molecule encoding the E glycoprotein of this invention, an isolated nucleic acid molecule encoding the flavivirus particle or the VLP of this invention, a vector comprising the nucleic acid molecule of this invention and a population of flavivirus particles comprising the flavivirus particle this invention.

Further provided herein is a composition comprising the E glycoprotein of this invention in a pharmaceutically acceptable carrier, a composition comprising the E glycoprotein dimer of this invention in a pharmaceutically acceptable carrier, a composition comprising the nucleic acid molecule of this invention in a pharmaceutically acceptable carrier, a composition comprising the virus particle of this invention, a composition comprising the population of this invention in a pharmaceutically acceptable carrier and a composition comprising the VLP of this invention in a pharmaceutically acceptable carrier.

In some embodiments, a stabilized recombinant E glycoprotein or stabilized recombinant E glycoprotein dimer of the present invention may be used in combination (e.g., in scaffold(s) and/or conjugated with) other molecules such as, but not limited to, Fc fragments, Fc-fusion proteins, and/or nanoparticles. Fc molecules may be used, e.g., to increase serum half-life and/or serum immunogenicity. Nanoparticles may be used, e.g., as delivery devices.

Types of nanoparticles of this invention for use as delivery devices include, but are not limited to, polymer nanoparticles such as PLGA-based, PLA-based, polysaccharide-based (dextran, cyclodextrin, chitosan, heparin), dendrimer, hydrogel; lipid-based nanoparticles such as lipid nanoparticles, lipid hybrid nanoparticles, liposomes, micelles; inorganics-based nanoparticles such as superparamagnetic iron oxide nanoparticles, metal nanoparticles, platin nanoparticles, calcium phosphate nanoparticles, quantum dots; carbon-based nanoparticles such as fullerenes, carbon nanotubes; and protein-based complexes with nanoscales. Types of microparticles of this invention include but are not limited to particles with sizes at micrometer scale that are polymer microparticles including but not limited to, PLGA-based, PLA-based, polysaccharide-based (dextran, cyclodextrin, chitosan, heparin), dendrimer, hydrogel; lipid-based microparticles such as lipid microparticles, micelles; inorganics-based microparticles such as superparamagnetic iron oxide microparticles, platin microparticles and the like as are known in the art. These particles may be generated and/or have materials be absorbed, encapsulated, or chemically bound through known mechanisms in the art.

Production of the stabilized recombinant E glycoproteins of this invention can be carried out by introducing some (e.g., 1, 2, 3, 4, 5, 6, 7, 8, 9, 10, 11, 12, 13, 14, 15, etc.) or all of the amino acid substitutions identified as stabilizing a flavivirus quaternary epitope and/or flavivirus E glycoprotein dimer conformation (e.g., such as in Table 1 and 2) into a flavivirus E glycoprotein backbone. Not every amino acid identified as stabilizing a flavivirus quaternary epitope and/or flavivirus E glycoprotein dimer conformation is required to be substituted to produce a stabilized recombinant protein of this invention. For example, in some embodiments further substitutions and/or omission of substitutions of about 1, 2, 3, 4 or 5 amino acids at either end of the contiguous amino acid sequences identified as stabilizing a flavivirus quaternary epitope and/or flavivirus E glycoprotein dimer conformation can be included in the production of a stabilized recombinant E glycoprotein of this invention. The number of substitutions necessary to produce the desired conformational epitope or dimer structure can be readily determined by one of ordinary skill in the art according to the teachings herein and according to protocols well known in the art. The amino acid residue numbering provided in the amino acid sequences set forth here is based on the unmodified (e.g., wild type) E glycoprotein amino acid sequence of the soluble recombinant DENV2 E glycoprotein (SEQ ID NO:6). However it would be readily understood by one of ordinary skill in the art that the equivalent amino acid positions in other flavivirus E glycoprotein amino acid sequences (e.g., DENV and/or ZIKV) can be readily identified, e.g., such as through alignments as shown in **FIGS. 17A-17B****,** and employed in the production of the stabilized recombinant E glycoproteins of this invention..

Thus, in some embodiments the present invention provides a method of producing a stabilized recombinant E glycoprotein of the present invention, comprising: introducing one or more amino acid substitutions into a flavivirus E glycoprotein backbone, wherein the one or more amino acid substitutions are selected from the group consisting of 2M, 6L, 8L, 9V, 13F, 14A, 15E, 27P, 29Y, 29K, 32V, 33V, 34L, 35M, 44M, 44L, 48I, 106D, 107C, 131I, 144Y, 154L, 154M, 191Y, 198W, 204F, 205L, 206F, 209D, 244Q, 244F, 246I, 246Y, 251F, 255E, 256Y, 258A, 258E, 259V, 259W, 259C, 260L, 261L, 261F, 262H, 262R, 262Y, 263W, 263L, 266W, 270V, 277M, 279W, 280A, 280P, 289W, 299L, 313C, 316M, 330A, 359Y, 373D, 375L, 377V, 386I, 390Q, 392R, 393R, and/or any combination thereof (e.g., amino acid substitutions shown in **Table 1** and/or **Table 2),** wherein the numbering is based on the reference amino acid sequence of an E glycoprotein of dengue virus serotype 2 (DENV2) identified as **SEQ ID NO:6,** thereby stabilizing the flavivirus E glycoprotein into dimer conformation.

In some embodiments, the present invention provides a method of producing a stabilized recombinant E glycoprotein of the present invention, comprising introducing one or more amino acid substitutions into a flavivirus E glycoprotein backbone DI/DII hinge region, αB helix central interface, and/or fusion loop (FL) dimer interface, thereby stabilizing the flavivirus E glycoprotein into dimer conformation. In some embodiments, the present invention provides a method of producing a stabilized recombinant E glycoprotein of the present invention, comprising introducing one or more amino acid substitutions into a flavivirus E glycoprotein backbone DI/DII hinge region, thereby stabilizing the flavivirus E glycoprotein into dimer conformation. Non-limiting examples of amino acid substitutions in the E glycoprotein DI/DII hinge region, e.g., outside of the E glycoprotein dimer interface, include 279W and 280P. While not wishing to be bound to theory, substitutions in the E glycoprotein DI/DII hinge region outside of the dimer interface may increase dimer conformation stability through hydrophobic packing in the hinge region and/or stabilization of the kl loop. Thus, in some embodiments, the present invention provides a method of producing a stabilized recombinant E glycoprotein of the present invention, comprising introducing one or more amino acid substitutions into a flavivirus E glycoprotein backbone DI/DII hinge region, wherein the one or more amino acid substitutions hydrophobically pack the hinge region and/or to stabilize the kl loop, thereby stabilizing the flavivirus E glycoprotein into dimer conformation.

In some embodiments, a method of the present invention may comprise introducing one or more amino acid substitutions into a flavivirus E glycoprotein backbone, thereby stabilizing the flavivirus E glycoprotein into monomer conformation. Non-limiting examples of substitutions which may be introduced for monomer conformation stabilization include amino acid substitutions shown in **Table 1** and **Table 2** such as one or more substitutions comprised in the example variants Mnmer1, Mnmer2, and/or PM4, wherein the numbering is based on the reference amino acid sequence of an E glycoprotein of dengue virus serotype 2 (DENV2) identified as **SEQ ID NO:6.**

There is considerable interest in VLPs as a type of subunit vaccine for flaviviruses as the multivalent display of antigen on a particle can boost both B cell and T cell responses. To produce VLPs with the WT DENV E protein, it is necessary to co-express E with the full length prM protein. prM is one of three structural proteins in DENV, along with the E protein and capsid, and performs several functional roles during viral maturation. During secretion from the cell the pr domain binds to the fusion loop on domain DII of the E protein and prevents it from inserting into the membrane of the host cell as it passes through the ER and the trans-Golgi network. This "chaperone" activity by the pr domain is important as the virus transports through the low-pH environment of the trans-Golgi. The E protein is dynamic and has evolved to expose the fusion loop at low pH when infecting cells. During native viral maturation, when the virus leaves the host cell pr is cleaved from M and released from the virion. However, when producing VLPs using recombinant protein the cleavage of prM is very inefficient and a large amount of pr remains attached to the particles. This presents a problem for using DENV VLPs as a vaccine because the pr domain may occlude important epitopes on the surface of the particle and pr is a dominant epitope that elicits non-neutralizing antibodies that can contribute to antibody enhancement (ADE) and severe disease symptoms. Current flavivirus subunit vaccines and live attenuated virus vaccines, e.g., dengue virus and Zika virus, use co-expression of prM and E glycoprotein to improve expression and folding of the E and sRecE protein. The present invention stabilizes the E protein dimer at neutral and low pH, thereby sequestering the fusion loop at the dimer interface and disfavoring its insertion into the host membrane during secretion, removing the need for pr during VLP production.

Therefore, in some embodiments, the present invention provides a stabilized recombinant E glycoprotein that may interact with a prM protein. In some embodiments, the present invention provides a stabilized recombinant E glycoprotein that may not require interaction with a prM protein for appropriate expression and secretion. In some embodiments, a virus like particle (VLP) comprising the E glycoprotein of the present invention is produced via a method which does not comprise co-expression of pr. The sE stabilizing mutations of the present invention do not need and are not expressed with prM and have improved expression yields. This is an advantage for use, e.g., as a vaccine, diagnostics, and/or research tool applications.

The stabilizing mutations of the present invention are in conserved positions and should also stabilize with DENV1, 2, 3, and/or 4 and ZIKV, as well as other flavivirus envelope protein monomers and dimers, as shown in **FIGS. 11** and **17A****-17B.** Applications of the stabilizing mutations of the present invention include, for example, use of a stabilized recombinant E glycoprotein of the present invention in a subunit vaccine, improved DENV virus-like particle (VLP) production and stability, attenuating mutations (e.g., attenuation by stabilizing the E glycoprotein dimer conformation and reducing poorly neutralizing fusion loop (FL) epitope accessibility to antibodies *in vivo*), and/or research applications such as flavivirus (e.g., DENV, e.g., ZIKV) diagnostics and antibody epitope mapping.

It is contemplated that in some embodiments, the stabilized recombinant E glycoproteins and/or nucleic acid molecules of this invention can be used as immunogens and/or in a vaccine formulation. The stabilized recombinant E glycoproteins and/or nucleic acid molecules can be included in a pharmaceutical formulation, in any combination and/or ratio relative to one another.

Thus, the present invention further provides a method of producing an immune response to a flavivirus in a subject, comprising administering to the subject an effective amount of a stabilized recombinant E glycoprotein, a stabilized recombinant E glycoprotein dimer, a flavivirus particle or VLP, a nucleic acid molecule, a population, a composition, or any combination thereof of the present invention.

In some embodiments, the present invention provides a method of treating a flavivirus infection in a subject, comprising administering to the subject an effective amount of a stabilized recombinant E glycoprotein, a stabilized recombinant E glycoprotein dimer, a flavivirus particle or VLP, a nucleic acid molecule, a population, a composition, or any combination thereof of the present invention.

In some embodiments, the present invention provides a method of preventing a disorder associated with a flavivirus infection in a subject, comprising administering to the subject an effective amount of a stabilized recombinant E glycoprotein, a stabilized recombinant E glycoprotein dimer, a flavivirus particle or VLP, a nucleic acid molecule, a population, a composition, or any combination thereof of the present invention.

In some embodiments, the present invention provides a method of protecting a subject from the effects of a flavivirus infection, comprising administering to the subject an effective amount of a stabilized recombinant E glycoprotein, a stabilized recombinant E glycoprotein dimer, a flavivirus particle or VLP, a nucleic acid molecule, a population, a composition, or any combination thereof of the present invention.

In some embodiments, the present invention provides a method of identifying the presence of a neutralizing antibody to a flavivirus in a biological sample from a subject, comprising: a) administering a composition comprising a stabilized recombinant E glycoprotein or E glycoprotein dimer of the present invention to the subject in an amount effective to induce an antibody response to the E glycoprotein; b) contacting a biological sample from the subject with flavivirus particles comprising the E glycoprotein of step (a) above under conditions whereby neutralization of the flavivirus particles can be detected; and c) detecting neutralization in step (b), thereby identifying the presence of a neutralizing antibody to the flavivirus in the biological sample from the subject.

In some embodiments, the present invention provides a method of identifying the presence of a neutralizing antibody to a flavivirus in a biological sample from a subject, comprising: a) contacting a biological sample from a subject that has been administered a stabilized recombinant E glycoprotein or E glycoprotein dimer of the present invention with flavivirus particles comprising the E glycoprotein under conditions whereby neutralization of the flavivirus particles can be detected; and b) detecting neutralization in step (a), thereby identifying the presence of a neutralizing antibody to the flavivirus in the biological sample from the subject.

In some embodiments, the present invention provides a method of identifying an immunogenic composition that induces a neutralizing antibody to a flavivirus in a subject, the method comprising: a) administering an immunogenic composition comprising a stabilized recombinant E glycoprotein or E glycoprotein dimer of the present invention to a subject in an amount effective to induce an antibody response to the E glycoprotein; b) contacting a biological sample from the subject with flavivirus particles comprising the E glycoprotein of step (a) under conditions whereby neutralization of the flavivirus particles can be detected; c) determining if the biological sample comprises an antibody that neutralizes flavivirus particles comprising the E glycoprotein of step (a); and d) identifying the immunogenic composition as inducing a neutralizing antibody to the flavivirus in the subject if the biological sample comprises an antibody that neutralizes flavivirus particles comprising the E glycoprotein of (a).

In some embodiments, the present invention provides a method of identifying an immunogenic composition that induces a neutralizing antibody to a flavivirus in a subject, the method comprising: a) contacting a biological sample from a subject that has been administered an immunogenic composition comprising a stabilized recombinant E glycoprotein or E glycoprotein dimer of the present invention with flavivirus particles comprising the E glycoprotein under conditions whereby neutralization of the flavivirus particles can be detected; b) determining if the biological sample comprises an antibody that neutralizes flavivirus particles comprising the E glycoprotein of step (a); and c) identifying the immunogenic composition as inducing a neutralizing antibody to the flavivirus in the subject if the biological sample comprises an antibody that neutralizes flavivirus particles comprising the E glycoprotein of (a).

In some embodiments, the present invention provides a method of detecting an antibody to a flavivirus in a sample, comprising: a) contacting the sample with a stabilized recombinant E glycoprotein or E glycoprotein dimer of the present invention under conditions whereby an antigen/antibody complex can form; and b) detecting formation of an antigen/antibody complex, thereby detecting an antibody to the flavivirus in the sample.

In some embodiments, the present invention provides a method of identifying an antibody to a flavivirus in a biological sample from a subject, comprising: a) administering a composition a stabilized recombinant E glycoprotein or E glycoprotein dimer of the present invention to the subject in an amount effective to induce an antibody response to the E glycoprotein; b) contacting a biological sample from the subject with the E glycoprotein of (a) under conditions whereby an antigen/antibody complex can form; and c) detecting formation of an antigen/antibody complex, thereby identifying an antibody to the flavivirus in the biological sample from the subject.

In some embodiments, the present invention provides a method of identifying an antibody to a flavivirus in a biological sample from a subject, comprising: a) contacting a biological sample from a subject that has been administered an immunogenic composition comprising a stabilized recombinant E glycoprotein or E glycoprotein dimer of the present invention with the E glycoprotein under conditions whereby an antigen/antibody complex can form; and b) detecting formation of an antigen/antibody complex, thereby identifying an antibody to the flavivirus in the biological sample from the subject.

In some embodiments, the present invention provides a method of identifying an immunogenic composition that induces an antibody to a flavivirus in a subject, the method comprising: a) contacting a biological sample from a subject that has been administered an immunogenic composition comprising a stabilized recombinant E glycoprotein or E glycoprotein dimer of the present invention with the E glycoprotein under conditions whereby an antigen/antibody complex can form; and b) detecting formation of an antigen/antibody complex, thereby identifying an immunogenic composition that induces an antibody to the flavivirus in the subject.

In some embodiments, the present invention provides a method of identifying an immunogenic composition that induces a neutralizing antibody to a flavivirus in a subject, comprising: a) administering an immunogenic composition comprising a stabilized recombinant E glycoprotein or E glycoprotein dimer of the present invention to a subject in an amount effective to induce an antibody response to the E glycoprotein; b) contacting a biological sample from the subject with the E glycoprotein of (a) under conditions whereby an antigen/antibody complex can form; and c) detecting formation an antigen/antibody complex, thereby identifying an immunogenic composition that induces a neutralizing antibody to the flavivirus in the subject.

The present invention can be practiced for prophylactic, therapeutic and/or diagnostic purposes. In addition, the invention can be practiced to produce antibodies for any purpose, such as diagnostic or research purposes, or for passive immunization by transfer to another subj ect.

The present invention further provides a kit comprising one or more compositions of this invention. It would be well understood by one of ordinary skill in the art that the kit of this invention can comprise one or more containers and/or receptacles to hold the reagents (e.g., antibodies, antigens, nucleic acids) of the kit, along with appropriate buffers and/or diluents and/or other solutions and directions for using the kit, as would be well known in the art. Such kits can further comprise adjuvants and/or other immunostimulatory or immunomodulating agents, as are well known in the art.

The compositions and kits of the present invention can also include other medicinal agents, pharmaceutical agents, carriers, diluents, immunostimulatory cytokines, etc. Actual methods of preparing such dosage forms are known, or will be apparent, to those skilled in this art.

Administration to a subject can be by any route known in the art. As non-limiting examples, the route of administration can be by inhalation (e.g., oral and/or nasal inhalation), oral, buccal (e.g., sublingual), rectal, vaginal, topical (including administration to the airways), intraocular, transdermal, by parenteral (e.g., intramuscular [e.g., administration to skeletal muscle], intravenous, intra-arterial, intraperitoneal and the like), subcutaneous (including administration into the footpad), intradermal, intrapleural, intracerebral, and/or intrathecal routes.

The epitopes, polypeptides, VLPs and viral vectors of the invention can be delivered *per se* or by delivering a nucleic acid (e.g., DNA) that encodes the same.

Immunomodulatory compounds, such as immunomodulatory chemokines and cytokines (preferably, CTL inductive cytokines) can be administered concurrently to a subj ect.

Cytokines may be administered by any method known in the art. Exogenous cytokines may be administered to the subject, or alternatively, a nucleic acid encoding a cytokine may be delivered to the subject using a suitable vector, and the cytokine produced *in vivo.* In particular embodiments, a viral adjuvant expresses the cytokine.

In embodiments of the invention, multiple dosages (e.g., two, three or more) of a composition of the invention can be administered without detectable pathogenicity (e.g., Dengue Shock Syndrome/Dengue Hemorrhagic Fever).

Pharmaceutical formulations (e.g., immunogenic formulation) comprising the dengue virus epitopes, polypeptides, chimeric flavivirus VLPs or chimeric flavivirus particles, nucleic acids, vectors, cells or compositions of the invention and a pharmaceutically acceptable carrier are also provided, and can be formulated for administration in a pharmaceutical carrier in accordance with known techniques. *See,* e.g., Remington, *The Science and Practice of Pharmacy* (latest edition). In the manufacture of a pharmaceutical composition according to embodiments of the present invention, the composition of the invention is typically admixed with, *inter alia,* a pharmaceutically acceptable carrier. By "pharmaceutically acceptable carrier" is meant a carrier that is compatible with other ingredients in the pharmaceutical composition and that is not harmful or deleterious to the subject. The carrier may be a solid or a liquid, or both, and is preferably formulated with the composition of the invention as a unit-dose formulation, for example, a tablet, which may contain from about 0.01 or 0.5% to about 95% or 99% by weight of the composition. The pharmaceutical compositions are prepared by any of the well-known techniques of pharmacy including, but not limited to, admixing the components, optionally including one or more accessory ingredients. In certain embodiments, the pharmaceutically acceptable carrier is sterile and would be deemed suitable for administration into human subjects according to regulatory guidelines for pharmaceutical compositions comprising the carrier.

Furthermore, a "pharmaceutically acceptable" component such as a salt, carrier, excipient or diluent of a composition according to the present invention is a component that (i) is compatible with the other ingredients of the composition in that it can be combined with the compositions of the present invention without rendering the composition unsuitable for its intended purpose, and (ii) is suitable for use with subjects as provided herein without undue adverse side effects (such as toxicity, irritation, and allergic response). Side effects are "undue" when their risk outweighs the benefit provided by the composition. Non-limiting examples of pharmaceutically acceptable components include any of the standard pharmaceutical carriers such as phosphate buffered saline solutions, water, emulsions such as oil/water emulsion, microemulsions and various types of wetting agents.

In some embodiments, the compositions of the invention can further comprise one or more than one adjuvant. The adjuvants of the present invention can be in the form of an amino acid sequence, and/or in the form or a nucleic acid encoding an adjuvant. When in the form of a nucleic acid, the adjuvant can be a component of a nucleic acid encoding the polypeptide(s) or fragment(s) or epitope(s) and/or a separate component of the composition comprising the nucleic acid encoding the polypeptide(s) or fragment(s) or epitope(s) of the invention. According to the present invention, the adjuvant can also be an amino acid sequence that is a peptide, a protein fragment or a whole protein that functions as an adjuvant, and/or the adjuvant can be a nucleic acid encoding a peptide, protein fragment or whole protein that functions as an adjuvant. As used herein, "adjuvant" describes a substance, which can be any immunomodulating substance capable of being combined with a composition of the invention to enhance, improve or otherwise modulate an immune response in a subject.

In further embodiments, the adjuvant can be, but is not limited to, an immunostimulatory cytokine (including, but not limited to, GM/CSF, interleukin-2, interleukin-12, interferon-gamma, interleukin-4, tumor necrosis factor-alpha, interleukin-1, hematopoietic factor flt3L, CD40L, B7.1 co-stimulatory molecules and B7.2 co-stimulatory molecules), SYNTEX adjuvant formulation 1 (SAF-1) composed of 5 percent (wt/vol) squalene (DASF, Parsippany, N.J.), 2.5 percent Pluronic, L121 polymer (Aldrich Chemical, Milwaukee), and 0.2 percent polysorbate (Tween 80, Sigma) in phosphate-buffered saline. Suitable adjuvants also include an aluminum salt such as aluminum hydroxide gel (alum), aluminum phosphate, or algannmulin, but may also be a salt of calcium, iron or zinc, or may be an insoluble suspension of acylated tyrosine, or acylated sugars, cationically or anionically derivatized polysaccharides, or polyphosphazenes.

Other adjuvants are well known in the art and include without limitation MF 59, LT-K63, LT-R72 (Pal et al. Vaccine 24(6):766-75 (2005)), QS-21, Freund's adjuvant (complete and incomplete), aluminum hydroxide, N-acetyl-muramyl-L-threonyl-D-isoglutamine (thr-MDP), N-acetyl-normuramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn - glycero-3-hydroxyphosphoryloxy)-ethylamine (CGP 19835A, referred to as MTP-PE) and RIBI, which contains three components extracted from bacteria, monophosphoryl lipid A, trealose dimycolate and cell wall skeleton (MPL+TDM+CWS) in 2% squalene/Tween 80 emulsion.

Additional adjuvants can include, for example, a combination of monophosphoryl lipid A, preferably 3-de-O-acylated monophosphoryl. lipid A (3D-MPL) together with an aluminum salt. An enhanced adjuvant system involves the combination of a monophosphoryl lipid A and a saponin derivative, particularly the combination of QS21 and 3D-MPL as disclosed in PCT publication number WO 94/00153, or a less reactogenic composition where the QS21 is quenched with cholesterol as disclosed in PCT publication number WO 96/33739. A particularly potent adjuvant formulation involving QS21 3D-MPL & tocopherol in an oil in water emulsion is described in PCT publication number WO 95/17210. In addition, the nucleic acid compositions of the invention can include an adjuvant by comprising a nucleotide sequence encoding the antigen and a nucleotide sequence that provides an adjuvant function, such as CpG sequences. Such CpG sequences, or motifs, are well known in the art.

An adjuvant for use with the present invention, such as, for example, an immunostimulatory cytokine, can be administered before, concurrent with, and/or within a few hours, several hours, and/or 1, 2, 3, 4, 5, 6, 7, 8, 9, and/or 10 days before and/or after the administration of a composition of the invention to a subject.

Furthermore, any combination of adjuvants, such as immunostimulatory cytokines, can be co-administered to the subject before, after and/or concurrent with the administration of an immunogenic composition of the invention. For example, combinations of immunostimulatory cytokines, can consist of two or more immunostimulatory cytokines, such as GM/CSF, interleukin-2, interleukin-12, interferon-gamma, interleukin-4, tumor necrosis factor-alpha, interleukin-1, hematopoietic factor flt3L, CD40L, B7.1 co-stimulatory molecules and B7.2 co-stimulatory molecules. The effectiveness of an adjuvant or combination of adjuvants can be determined by measuring the immune response produced in response to administration of a composition of this invention to a subject with and without the adjuvant or combination of adjuvants, using standard procedures, as described herein and as known in the art.

In embodiments of the invention, the adjuvant comprises an alphavirus adjuvant as described, for example in U.S. 7,862,829.

Boosting dosages can further be administered over a time course of days, weeks, months or years. In chronic infection, initial high doses followed by boosting doses may be advantageous.

The pharmaceutical formulations of the invention can optionally comprise other medicinal agents, pharmaceutical agents, stabilizing agents, buffers, carriers, diluents, salts, tonicity adjusting agents, wetting agents, and the like, for example, sodium acetate, sodium lactate, sodium chloride, potassium chloride, calcium chloride, sorbitan monolaurate, triethanolamine oleate, etc.

For injection, the carrier will typically be a liquid. For other methods of administration, the carrier may be either solid or liquid. For inhalation administration, the carrier will be respirable, and is typically in a solid or liquid particulate form.

The compositions of the invention can be formulated for administration in a pharmaceutical carrier in accordance with known techniques. See, e.g., Remington, The Science and Practice of Pharmacy (9th Ed. 1995). In the manufacture of a pharmaceutical composition according to the invention, the VLPs are typically admixed with, inter alia, an acceptable carrier. The carrier can be a solid or a liquid, or both, and is optionally formulated with the compound as a unit-dose formulation, for example, a tablet. A variety of pharmaceutically acceptable aqueous carriers can be used, e.g., water, buffered water, 0.9% saline, 0.3% glycine, hyaluronic acid, pyrogen-free water, pyrogen-free phosphate-buffered saline solution, bacteriostatic water, or Cremophor EL[R] (BASF, Parsippany, N.J.), and the like. These compositions can be sterilized by conventional techniques. The formulations of the invention can be prepared by any of the well-known techniques of pharmacy.

The pharmaceutical formulations can be packaged for use as is, or lyophilized, the lyophilized preparation generally being combined with a sterile aqueous solution prior to administration. The compositions can further be packaged in unit/dose or multi-dose containers, for example, in sealed ampoules and vials.

The pharmaceutical formulations can be formulated for administration by any method known in the art according to conventional techniques of pharmacy. For example, the compositions can be formulated to be administered intranasally, by inhalation (e.g., oral inhalation), orally, buccally (e.g., sublingually), rectally, vaginally, topically, intrathecally, intraocularly, transdermally, by parenteral administration (e.g., intramuscular [e.g., skeletal muscle], intravenous, subcutaneous, intradermal, intrapleural, intracerebral and intra-arterial, intrathecal), or topically (e.g., to both skin and mucosal surfaces, including airway surfaces).

For intranasal or inhalation administration, the pharmaceutical formulation can be formulated as an aerosol (this term including both liquid and dry powder aerosols). For example, the pharmaceutical formulation can be provided in a finely divided form along with a surfactant and propellant. Typical percentages of the composition are 0.01-20% by weight, preferably 1-10%. The surfactant is generally nontoxic and soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1-20% by weight of the composition, preferably 0.25-5%. The balance of the composition is ordinarily propellant. A carrier can also be included, if desired, as with lecithin for intranasal delivery. Aerosols of liquid particles can be produced by any suitable means, such as with a pressure-driven aerosol nebulizer or an ultrasonic nebulizer, as is known to those of skill in the art. *See,* e.g., U.S. Patent No. 4,501,729. Aerosols of solid particles can likewise be produced with any solid particulate medicament aerosol generator, by techniques known in the pharmaceutical art. Intranasal administration can also be by droplet administration to a nasal surface.

Injectable formulations can be prepared in conventional forms, either as liquid solutions or suspensions, solid forms suitable for solution or suspension in liquid prior to injection, or as emulsions. Alternatively, one can administer the pharmaceutical formulations in a local rather than systemic manner, for example, in a depot or sustained-release formulation.

Extemporaneous injection solutions and suspensions can be prepared from sterile powders, granules and tablets of the kind previously described. For example, an injectable, stable, sterile formulation of the invention in a unit dosage form in a sealed container can be provided. The formulation can be provided in the form of a lyophilizate, which can be reconstituted with a suitable pharmaceutically acceptable carrier to form a liquid composition suitable for injection into a subject. The unit dosage form can be from about 1 µg to about 10 grams of the formulation. When the formulation is substantially water-insoluble, a sufficient amount of emulsifying agent, which is pharmaceutically acceptable, can be included in sufficient quantity to emulsify the formulation in an aqueous carrier. One such useful emulsifying agent is phosphatidyl choline.

Pharmaceutical formulations suitable for oral administration can be presented in discrete units, such as capsules, cachets, lozenges, or tables, as a powder or granules; as a solution or a suspension in an aqueous or non-aqueous liquid; or as an oil-in-water or water-in-oil emulsion. Oral delivery can be performed by complexing a compound(s) of the present invention to a carrier capable of withstanding degradation by digestive enzymes in the gut of an animal. Examples of such carriers include plastic capsules or tablets, as known in the art. Such formulations are prepared by any suitable method of pharmacy, which includes the step of bringing into association the protein(s) and a suitable carrier (which may contain one or more accessory ingredients as noted above). In general, the pharmaceutical formulations are prepared by uniformly and intimately admixing the compound(s) with a liquid or finely divided solid carrier, or both, and then, if necessary, shaping the resulting mixture. For example, a tablet can be prepared by compressing or molding a powder or granules, optionally with one or more accessory ingredients. Compressed tablets are prepared by compressing, in a suitable machine, the formulation in a free-flowing form, such as a powder or granules optionally mixed with a binder, lubricant, inert diluent, and/or surface active/dispersing agent(s). Molded tablets are made by molding, in a suitable machine, the powdered protein moistened with an inert liquid binder.

Pharmaceutical formulations suitable for buccal (sub-lingual) administration include lozenges comprising the compound(s) in a flavored base, usually sucrose and acacia or tragacanth; and pastilles in an inert base such as gelatin and glycerin or sucrose and acacia.

Pharmaceutical formulations suitable for parenteral administration can comprise sterile aqueous and non-aqueous injection solutions, which preparations are preferably isotonic with the blood of the intended recipient. These preparations can contain antioxidants, buffers, bacteriostats and solutes, which render the composition isotonic with the blood of the intended recipient. Aqueous and non-aqueous sterile suspensions, solutions and emulsions can include suspending agents and thickening agents. Examples of nonaqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Pharmaceutical formulations suitable for rectal administration are optionally presented as unit dose suppositories. These can be prepared by admixing the active agent with one or more conventional solid carriers, such as for example, cocoa butter and then shaping the resulting mixture.

Pharmaceutical formulations suitable for topical application to the skin preferably take the form of an ointment, cream, lotion, paste, gel, spray, aerosol, or oil. Carriers that can be used include, but are not limited to, petroleum jelly, lanoline, polyethylene glycols, alcohols, transdermal enhancers, and combinations of two or more thereof. In some embodiments, for example, topical delivery can be performed by mixing a pharmaceutical formulation of the present invention with a lipophilic reagent (e.g., DMSO) that is capable of passing into the skin.

Pharmaceutical formulations suitable for transdermal administration can be in the form of discrete patches adapted to remain in intimate contact with the epidermis of the subject for a prolonged period of time. Formulations suitable for transdermal administration can also be delivered by iontophoresis (*see,* for example, Pharmaceutical Research 3:318 (1986)) and typically take the form of a buffered aqueous solution of the compound(s). Suitable formulations can comprise citrate or bis\tris buffer (pH 6) or ethanol/water and can contain from 0.1 to 0.2M active ingredient.

In embodiments of the invention, the dosage of a virus particle of this invention can be in a range of about 10⁴ to about 10⁷ plaque forming units (PFUs). In embodiments of this invention, the dosage of a VLP of this invention can be in a range of about 500 micrograms to about 5 milligrams. In embodiments of this invention, the dosage of a protein of this invention can be in a range of about 10⁰ to about 10⁴ micrograms +/- adjuvant.

Further, the composition can be formulated as a liposomal formulation. The lipid layer employed can be of any conventional composition and can either contain cholesterol or can be cholesterol-free. The liposomes that are produced can be reduced in size, for example, through the use of standard sonication and homogenization techniques.

The liposomal formulations can be lyophilized to produce a lyophilizate which can be reconstituted with a pharmaceutically acceptable carrier, such as water, to regenerate a liposomal suspension.

The immunogenic formulations of the invention can optionally be sterile, and can further be provided in a closed pathogen-impermeable container.

### EXAMPLES

### EXAMPLE 1: Computational design identifies DENV2 sE dimer and monomer stabilizing mutations

Four crystal structures of the DENV2 sE dimer, each similar while containing differences that represent the conformations available to the DENV2 sE dimer., were used as input for design simulations. The structures were prepared for the design simulations by energy minimizing them with the FastRelax algorithm in Rosetta . Notably, this protocol only makes small structural perturbations (RMSD < 0.5Å), but lowers the calculated energy of the model as specific contacts are optimized according to the Rosetta energy function.

Two different computational protocols were used to identify DENV2 sE stabilizing mutations: PM_ssm and cluster_mut. PM_ssm is an exhaustive site-saturation mutagenesis protocol which identifies point mutations (PMs) predicted to stabilize the sE dimer and/or monomer. The protocol starts at the first residue in the protein and walks through the entire primary sequence, one position at a time, mutating the WT AA to each of the 20 AA in independent simulations. Since the sE homodimer was being modeled, mutations were simultaneously placed on both chains. After mutating a residue, the conformation of the mutated residue and surrounding residues were energy optimized with iterative rounds of rotamer-based side chain sampling and gradient-based minimization of side chain and backbone torsion angles. To minimize noise in the energy optimization simulations, residues distant from the site of mutation were constrained to remain in their initial conformation while residues immediately adjacent to the site of the mutation were energy optimized without constraints . The calculated energy of mutating to the same amino acid (AA) as wildtype (WT) was used as reference to calculate the change in rosetta energy (ΔREU, rosetta energy units) upon mutation, a ΔREU > -2 cutoff was used to select the best scoring mutations **(****FIG. 1B****).** The PM_ssm protocol produced predictions for 7880 mutations at the 394 residues in DENV2 sE, with 879 mutations (-11%) remaining after filtering mutations based on the ΔREU cutoff. Next, Rosetta was used to create designs containing combinations of the predicted PMs. These PM_comb simulations used the same selection logic from the PM_ssm simulations but allowed multiple residues to mutate in an entire sE protein domain, domain-domain interface, or dimer interface.

The Cluster_mut protocol was used to identify small numbers of favorable mutations (about 2 to about 4) in close proximity with each other. The protocol incorporated a "design sphere" in which all residues within 7Å of, and including, a seed residue were allowed to mutate to any AA except cysteine **(****FIG. 1C****).** Surrounding the design sphere was a 3Å layer in which side chains could adopt new conformations during the simulation, but their AA identities were fixed. Outside of the two spheres side chains were fixed. The design simulation was performed using the FastDesign protocol in Rosetta which iterates between rotamer-based sequence design and gradient-based optimization of backbone and side chain torsion angles. Only torsion angles within the design and repacking sphere were allowed to adjust during the minimization. To favor small numbers of mutations and only include mutations predicted to have a large impact on stability, a bonus was placed on WT amino acid during the simulations.

To focus the cluster_mut design simulations, seed residues in regions within the DENV2 sE protein were identified that were hypothesized to contribute to protein dimer and/or monomer instability. First, regions at the DENV2 sE dimer interface were selected to increase dimer stability. Three regions were identified with residues making contacts at the sE dimer interface at the central "αB helix" interface, the DI/ij-loop interface and the fusion loop (FL) interface **(****FIG. 1D****).** In addition to targeting interface residues, multiple reports indicate that conserved histidine residues in the flavivirus E protein are "pH sensing" residues that mediate the E conformational change from pre-fusion dimer to post-fusion trimer. These histidine residues are close in proximity with cationic residues such as arginine and lysine and have been implicated in contributing to E dimer destabilization. These histidine-cation (herein referred to as "HisCat") interactions are energetically weak at neutral pH and highly repulsive at low pH, resulting in protonation of the histidine side chain and repulsion from the neighboring cationic residue. The sE dimer was analyzed for residue clusters containing histidines near at least one cationic residue in local space. Three main clusters were identified: 1) at the DI/DIII domain interface, 2) near the DI/ij loop dimer interface and 3) the central αB helix dimer interface. The residues involved in the sE dimer interface contacts and the histidine-cation cluster residues were used as seed residues for the IntFc and HCat cluster_mut simulations, respectively.

We also sought to stabilize the dimer by stabilizing the sE monomer. The DENV has evolved the E protein to have conformational instability to adopt the multiple conformations necessary for the virus life cycle. While not wishing to be bound to theory, the dimer and monomer instability of sE may be linked to the inherent flexibility and dynamic structure of the E glycoprotein, and that stabilizing the E monomer in the conformation observed in the dimer could improve dimer affinity. The DENV2 sE dimer was analyzed using the RosettaHoles protocol to identify underpacked regions present in the core, domain-domain interface or dimer interface. Underpacked regions contain small voids and have been shown to contribute to protein instability. RosettaHoles identified four main underpacked regions: 1) the DI/DIII domain interface known as the DI/DIII linker; 2) the DI/DII domain interface, also known as the hinge region; 3) the core of DI; and 4) at the central αB helix and FL dimer interfaces **(****FIG. 1D****).** Three of the underpacked regions, the DI/DIII linker, DI/DII hinge region and DI, all change conformation to allow for transition of the E pre-fusion dimer conformation to the post-fusion trimer conformation. The residues defining these underpacked regions were used as seed residues input for the "UndPk" cluster_mut simulations.

Assessment of the DENV2 sE protein also revealed a large number of surface exposed hydrophobic residues that are buried either on the viral surface or at the post-fusion trimer interface. These hydrophobic residues were manually selected for input for the surface hydrophobic to polar (SHP) simulations, using a modified PM_comb protocol, where only polar amino acids were allowed to be assessed during the design at these positions.

After performing the various design simulations, eight designs were selected from each of the protocols: PM_Comb (PM), UndPk, HCat and IntFc, and two designs from the SHP simulations for experimental studies. Recent studies have also demonstrated that the sE protein dimer can be stabilized recombinant by introducing disulfides across the dimer interface. Two of these variants, Cm1 (A259C) and Cm2 (L107C, A313C), were used in these studies. For negative control experiments, two mutants (Mnmer1 and Mnmer2) from the PM_ssm results were used that were predicted to destabilize the dimer with the goal of creating sE protein that would remain monomer even at higher concentrations and low temperature.

Mammalian surface display screen identifies Rosetta sE designs with improved quaternary antibody epitope presentation at 37°C. To ensure the mutations allowed for sE dimer formation and to compare the ability of the variants to present quaternary epitopes at 37°C relative to WT sE, it was investigated if known quaternary epitope antibodies can bind to the designed Rosetta sE variants. To do this the transmembrane and cytoplasmic domains of MHC-I alpha chain (SEQ ID NO:3) were fused to the c-terminus of the DENV2 sE protein using a GS linker (SEQ ID NO:2), allowing for surface display of the protein on EXPI293 cells. The construct was further modified by a cMyc epitope tag inserted within the GS linker for independent detection of sE surface display **(****FIG. 2A****)** and an n-terminal human serum albumin signal peptide (SEQ ID NO:1), instead of co-expression of prM, was used to export the protein to the cell surface. After transfection of the plasmid containing the DENV2 sE/MHC-Iα fusion, the cells were enhanced to increase protein expression for 24 hours and harvested for immunostaining. DENV2 sE variant epitope presentation was assessed by measuring DENV2 type-specific (2D22) and cross reactive (EDE1 C8 and C10) neutralizing quaternary epitope antibody binding (PerCP positive fluorescence) to cells displaying sE protein, detected through cMyc antibody binding (Alexa488 fluorescence) **(****FIGS. 2A****,** **2C, 2D****).** We also probed for binding of simple-domain epitope antibodies including 3H5 (DIII) and 1C19 (DII/bc-loop) as a readout for proper folding of DENV2 sE variants **(****FIG. 2B****).** Consistent with previously observed dimerization of the DENV2 sE WT protein at lower temperatures, binding of both 2D22 and EDE1 C8 to WT sE was observed at room temperature (~23°C). Surprisingly, all antibodies tested were able to bind to all DENV2 sE Rosetta variants, albeit at different amounts relative to WT, indicating that all the variants expressed were folded properly, and able to form dimers similar to WT on the cell surface at ~23°C.

Retention of quaternary epitopes at >37°C was tested by staining the surface-displayed sE Rosetta variants with 2D22 or EDE1 C10 at 40°C. In contrast to the 23°C screen, minimal binding of 2D22 and EDE1 C10 to DENV2 sE WT was observed **(****FIG. 1F** and **FIG. 2C****),** while increased binding of the quaternary antibodies to both Cm1 and Cm2 was observed, consistent with stable dimer formation even at the increased temperature. Out of the 34 variants, two variants, IntFc6 and Mnmer1, showed complete unfolding as indicated by complete loss of 3H5 and 1C19 binding, and seven variants showed reduced binding of the quaternary antibodies, including Mnmer2, designed as a negative control for the experiment. Improvements (Fr > 2) in quaternary antibody binding to 12/34 of the sE Rosetta variants were observed. Similar to the Cm2 control, eight Rosetta sE variants, (HCat3, IntFc2, IntFc8, UndPk6, PM2, PM4, SHP1 and SHP2) had greater than 3-fold higher Fr values for either 2D22 or EDE1 C10 and > 2-fold higher Fr values for both antibodies, indicating these variants stably present of quaternary antibody epitope at >37°C **(****FIGS. 2B** **&** **2D****).**

DENV2 sE Rosetta variants increase expression and improve both sE dimer and monomer stability: Direct measuring of the stability of the DENV2 sE Rosetta variants was performed using nanoscale Differential Scanning Fluorimetry (nanoDSF). 22 selected variants and Mnmer2, Cm1, and Cm2 were cloned into a mammalian expression plasmid without prM containing a c-terminal His₈ₓ tag (SEQ ID NO:4) for Ni-affinity purification and n-terminal human serum albumin (HSA) signal peptide (SEQ ID NO:1) for extracellular secretion. It has been indicated that prM co-expression is not necessary for sE expression and folding, therefore the expression construct was designed to eliminate potential problems with co-expressing prM with variants containing mutations near the pr/E binding site and to prevent additional purification steps needed to ensure removal of pr, important for preventing unwanted anti-prM antibody elicitation in future vaccine experiments. The constructs were transiently transfected into EXPI293F cells at a 60-120mL scale, after which the His-tag proteins secreted in the media were directly affinity purified using a penta-Ni resin (Marvelgent). Despite the low yields observed with DENV2 sE WT (-0.2mg/L), this protocol yielded high purity directly from the nickel resin, eliminating the further loss of protein through multiple purification steps and allowing direct performance of nanoDSF measurements with no additional preparation. Out of the 22 variants tested, one variant (IntFc6) did not express, consistent with 40°C surface display data, five variants had lower than WT expression, eight variants had WT expression level, as well as Mnmer2, and eight had yields greater than 2-fold with drastic improvements in expression observed for DENV2 sE Rosetta variants PM4 (10-fold), IntFc8 (19.1-fold) and UndPk6 (22.5-fold) **(Table 1).** While Mnmer2 had WT expression yields, Cm1 and Cm2 both had below detectable yields when expressed at the same scale.

Dimer and monomer stability of these variants was assessed using nanoDSF. DENV2 sE dimer dissociation (Tₘ¹) and protein unfolding (Tₘ²) can be detected in a single thermal melt experiment **(****FIGS. 3A****,** **3D****).** Performing nanoDSF thermal melts with increasing protein concentrations results in dimer dissociation occurring at higher melting transitions (Tₘ¹), which can be used to quantify the homodimer affinity of the DENV2 sE dimer at 37°C. Single concentration nanoDSF thermal melts were performed and compared the DENV2 sE Rosetta variant dimer and monomer stabilities to WT **(****FIG. 3A** and **Table 1).** Analysis of DENV2 sE WT at 4µM revealed two thermal melt transitions, the first at 34.2°C (Tₘ¹) corresponding to the dissociation of sE dimer to monomer, and the second at 51.0°C corresponding to the unfolding of the sE monomer. Out the 20 selected variants that expressed with sufficient yield for analysis, five variants had lower Tₘ¹ values than WT, including the Mnmer2 reference variant, which showed no transition at Tₘ¹. Taken with the reduction of EDE1 C10 and 2D22 binding to Mnmer2 in the surface display experiment at 40°C, this suggested that the protein is a monomer in solution. Among the four variants that had dimer stabilities similar to WT, three of them had improved monomer stabilities of > +3.5°C, including HCat3 **(Table 1).** One of these variants, PM4, which contains three mutations S29K, T33V, and A35M within the core of DI, had no improvement in dimer stability (Tₘ¹), but had a striking +11.4°C improvement in monomer stability (Tₘ²) **(****FIGS. 3A** and **3C****).** DENV2 sE containing the point mutants A35M (PM4.1), S29K (PM4.3), and a double mutant containing T33V and A35M (PM4.2) all improved sE monomer stability with the addition of their respectively measured Tₘ² matching the measured Tₘ² for PM4 (T33V/A35M = +6.9°C, S29K = +4.6°C, Total = +11.5°C, PM4 = +11.4°C) **(Table 1).** Testing A35M yielded a Tₘ² of +1.5°C, which suggested that T33V provides a +5.4°C improvement. S29 and T33 make important contacts in the post-fusion trimer, suggesting that the sub-optimal contributions to stability of these residues may be related to functional selective pressure. The remaining 11 designs all had improved dimer stability, with Tₘ¹ transitions greater than 2.0°C over WT, with three variants (IntFc2, IntFc8 and UndPk6) all having a > 8.0°C improvements in dimer stability. The most stabilizing variant, IntFc2, had a +11.4°C improvement in Tₘ¹. IntFc2 was predicted to create a new pi cation interaction across the αB helix central dimer interface, where the M protein natively interfaces with the E protein on the virus surface **(****FIG. 1C** and **FIG. 5A****).** IntFc8 has a single point mutation in the fusion loop (G106D) which was predicted to have more favorable torsion energies and to create a salt-bridge with K310, stabilizing a critical interaction with W101 at the FL dimer interface **(****FIG. 5A****).** UndPk6, which stabilized the dimer and monomer simultaneously (+8.7 Tₘ¹ and +5.9 Tₘ²), contains 2 mutations buried in the DI/DII interface known as the hinge region. The model predicted increased hydrophobic packing in the hinge region with the F279W mutation and mutated the T280 residue at the base of the kl loop to proline **(****FIG. 5A****).** As prolines have been shown to stabilize loop conformations, similar to PM4, the point mutation T280P (UndPk6.1) was tested and showed a Tₘ¹ of +6.4 and Tₘ² of +2.6, indicating T280P accounts for the majority of the increased dimer stability and about half of the added monomer stability, likely due to stabilizing the kl loop.

The homodimer affinity of the most stable dimer variants was quantified by performing nanoDSF at concentrations ranging from 2-16µM, allowing measurement of variant sE dimer affinity (Kd) using a Van't Hoff Analysis. All variants and WT showed increasing Tₘ¹ values with increasing concentration, with a measured Kd of 12.9µM for DENV2 sE WT at 37°C **(Table 1).** Consistent with the improvements in Tₘ¹, IntFc2 had an extrapolated Kd of 8nM at 37°C, a >1000 fold increase in dimer affinity over WT. Similarly, IntFc8 and UndPk6 showed an improved extrapolated Kd at 37°C of 219 nM and 238nM, respectively (a ~50 fold improvement over WT). To further validate the improved dimer stability of these variants, exclusion chromatography coupled with multi-angle light scattering (SEC-MALS) was used to measure these variants oligomeric state at 37°C. Consistent with the previous characterization at RT, WT eluted with two peaks, the first at ~14mL with a molar mass of 91 kDa, and the second at ~15mL with a molar mass (MM) of 58 kDa, consistent with the previous characterization that the WT is in a monomer-dimer equilibrium, favoring the dimer conformation at lower temperature and high concentration **(****FIG. 4A****).** However, at 37°C, WT almost entirely eluted at ~15mL with a MALS measured MM of 61 kDa, consistent with the protein eluting predominantly as a monomer **(****FIGS. 4A****,** **4D****).** The 37°C MALS measured WT MM at the beginning of the peak was higher than the middle suggesting a minor fraction of dimers may be present. Consistent with the absence of a Tₘ¹ transition during nanoDSF analysis, Mnmer2 eluted as two peaks both with measured MM nearly identical to the theoretical monomer Mnmer2 MM. Monomeric DENV3 and DENV4 sE have also been observed to elute as two fractions in SEC, and may be due to the exposed FL peptide interacting with the size exclusion matrix. PM4, which had no improvement in dimer stability relative to WT, eluted at the same volume and had a nearly identical measured MM as sE WT at 37°C, while IntFc2, IntFc8 and UndPk6 eluted at the volume and with MALS-measured MM consistent with the WT sE dimer observed at RT, consistent with the improved Tₘ¹ **(****FIGS. 4B****,** **4D****).** The MALS MM for IntFc8 and UndPk6 were slightly lower than IntFc2, suggesting that while stabilized, partial dimer dissociation of the IntFc8 and UndPk6 protein variants may occur at 37°C which is consistent with the Kds measured by nanoDSF **(****FIG. 4D****).** The nanoDSF and SEC-MALS experiments indicated that both computational protocols, PM_Comb and Cluster_mut, were successful at identifying dimer and monomer stabilizing mutations, some of which had greatly improved dimer stability (e.g., IntFc2) and monomer stability (e.g., PM4).

Combining stabilizing mutations further increases DENV2 sE dimer stability and expression levels: The SEC-MALS analysis suggested that, while the initial designs had improved dimer stability at 37°C, they may not have sufficient stability to remain dimers under vaccination conditions (e.g., 37°C and low protein concentrations). To find combinations of mutations that would boost dimer stability even further, 28 stable combination (SC) variants were tested that were built from the most stabilizing designs: IntFc2, IntFc8, UndPk6, PM4 and HCat3 **(Table 1).** The SC variants were expressed, purified, and tested for dimer and monomer stability using nanoDSF thermal melts. All SC variants had improved expression levels compared to WT, with some increasing yield >70 fold **(Table 1).** Except for SC.20, all the SC variants containing dimer stabilizing mutations had at least an 8°C increase in Tₘ¹. The most stable dimer variant, SC.4, which contained 13 mutations, had a Tm¹ of 56.7°C, which is a 22.5 °C increase over wild type **(****FIGS. 3B****,** **3C** and **Table 1).** All SC variants had ΔTm² values ranging from +4°C (SC.23, 55.4°C) to +18.2°C (SC.9, 69.3°C). SC.24 and SC.25, which incorporated Mnmer2 and either HCat3 or PM4, respectively, both had nearly identical Tm² improvements as the HCat3 and PM4 variants without Mnmer2, however, both had no measurable Tm¹, similar to Mnmer2 alone. Likewise, the combination of PM4 with the disulfide mutant, Cm2 resulted in a +11.4°C increase in Tm² and successful disulfide formation, indicated by the protein migrating as a dimer in a non-reducing SDS-PAGE gel **(Table 1).** It was not possible to recover Cm2 post purification at the 120mL scale, however, addition of PM4 with Cm2 resulted in a 3.2-fold improvement in expression over WT with an ~1mg/L expression yield **(Table 1).** Taken together, these results indicate the utility of combining these mutations to tune the sE protein to the desired expression yield and dimer and monomer stabilities.

The homodimer Kd at 37°C of the SC variants was measured by performing nanoDSF at varying protein concentrations, measuring Tm¹ and using a Van't Hoff analysis to estimate binding affinity at 37°C. Consistent with the large improvements in Tm¹, all SC variants containing dimer stabilizing mutations had Kds at 37°C with nM affinity and 16 out of 28 SC variants had extrapolated Kds <100pM. One variant SC. 14 was able to raise Tm¹ by 16.1°C and lowered the Kd at 37°C to 1nM with only four mutations from two of the best stabilizing dimer mutation sets, IntFc2 and UndPk6. Just adding one additional mutation, G106D (IntFc8), to SC.14 to make SC.10 raised Tm¹ 19.8°C and lowered the homodimer Kd further to < 100 pM **(Table 1).** While not wishing to be bound to theory, the exceptional stability of the SC.10 dimer may be likely due to simultaneously stabilizing the central dimer interface (IntFc2), the hinge (U6) and the FL dimer interface (IntFc8), all critical regions that regulate dimer stability. Consistent with the nanoDSF data, both SC.14 and SC.10 eluted as a single peak upon analysis with SEC-MALS at 37°C with MM of 99.5 and 102.2 kDa, respectively, similar to the disulfide stabilized sE dimer, Cm1 **(****FIGS. 4C** and **4D****).** Taken together, combining the Rosetta variant stabilizing mutations resulted in large increases in monomer stability and dimer stability and revealed that the dimer stability is dependent on interactions in the DI/DII hinge region and at the αB helix central and FL dimer interfaces.

DENV2 sE SC.10 adopts the same dimer conformation as the EDE1 C8 bound sE dimer: The crystal structure of SC.10 was solved in order to determine if SC.10 maintained a dimer structure similar to WT sE and check the accuracy of the Rosetta design models. SC.10 was concentrated to 3.25mg/mL, and buffer exchanged into a low salt Tris buffer prior to crystallization. SC.10 hexagonal crystals formed after three days using the sitting drop method at 21°C in 15% PEG 3350 pH 4.4, 0.1M sodium acetate and 0.1M sodium iodide, that diffracted to a final resolution of 3.42Å. The diffraction data was processed and the phases were obtained by molecular replacement (MR) by using chain A of PDB 1OAN, the DENV2 sE crystal structure used for the design of IntFc2, IntFc8 and UndPk6. Successful MR required input of two models from the sE monomer chain of 1OAN, one model containing DI/DII and the second containing DIII. The model obtained from MR was refined to a Rfree/Rwork of 33.7/31.8, with the asymmetric unit containing a single sE monomer. Despite the low pH crystal condition, SC.10 adopted the biological DENV2 sE dimer conformation **(****FIG. 5A****).** This in contrast to the DENV1 sE protein crystallizing as the post-fusion trimer in a similar crystal condition and low pH (pH 4.5) and is consistent with the increased dimer stability observed by adding the IntFc2, IntFc8 and UndPk6 mutations (Table 1). The SC.10 dimer had an overall Cα RMSD of 3.5Å in comparison to 1OAN, the DENV2 sE dimer crystal structure solved at pH 9.0, and is consistent with the failed MR when using the entire monomer chain from 1OAN. Despite using domains from unliganded WT DENV2 (!OAN) for MR, the SC.10 dimer conformation closely matched the DENV2 sE dimer conformation when co-crystallized with EDE1 C8 (PDB 4UTA), a DENV broadly neutralizing quaternary epitope antibody, with a Cα RMSD of 1.69Å **(****FIG. 5B****).** With this observation, MR was successful when repeated using the PDB 4UTA full-length chain A as input.

Analysis of multiple DENV2 sE crystal structures has revealed that the DI/DII hinge angle is variable, consistent with the intrinsic dynamics and multiple conformations the E protein can adopt. To see if the changes in the SC. 10 dimer conformation were dependent on the hinge angle, the DI and DIII of the sE monomer from 1OAN and 4UTA were aligned to the DI and DIII of the SC. 10 monomer. Both had nearly identical DI/DIII conformations when aligned to DI and DIII of SC. 10, with a Cα RMSD of only 1.0Å (1OAN) and 0.8Å (4UTA). In contrast there was a larger difference in the conformation of DII relative to DI and DIII for the sE monomer in 4UTA and SC. 10 compared to 1OAN **(****FIG. 5B****).The** kl loop in the DI/DII hinge region was nearly identical for 4UTA and SC. 10, with the loop facing away from the hinge and towards the adjacent monomer in the dimer. However, in 1OAN the kl loop conformation was packed within the DI/DII hinge, suggesting the kl loop conformation may play a role in the regulating the hinge angle. Also, the glycan loop in both the SC. 10 and 4UTA sE structures was disordered, unlike the previously published apo DENV2 sE dimer structures, suggesting that stabilizing the sE dimer in this conformation through mutation or antibody binding may destabilize this loop.

Electron density was observed for all of the mutations, IntFc2, IntFc8, and UndPk6, in the SC. 10 crystal structure. Both UndPk6 mutations are present on the kl loop. F279W fills an underpacked region in the hinge. T280P is at the base of the kl loop, and with the mutation the loop was shifted so that a backbone-side chain hydrogen bond is formed between the P280 carbonyl to the T189 sidechain hydroxyl in the adjacent loop. This hydrogen bond was not observed in either 1OAN or 4UTA. In the SC. 10 structure, the density for the kl loop was well defined, suggesting that adding P280 at the base of the kl loop stabilized the observed kl conformation, which is similarly stabilized by interactions between the EDE1 C8 Fab and the kl loop when it is bound to sE in 4UTA. This was consistent with the observation that loop conformations can be stabilized with the insertion of proline at the loop ends, and that the T280P mutation contributed the most to dimer stability increase in UndPk6 **(Table 1).** On the underside of the central dimer interface, both IntFc2 mutations are present on the αB helix with T262R making two new dimer contacts: a pi-cation interaction directly with A259W and a new side chain hydrogen bond to the backbone carbonyl of P218. These are both present on adjacent monomer in the sE dimer and both observed in the IntFc2 Rosetta model **(****FIG. 5A****).** Aligning the UndPk6 and IntFc2 mutations in the Rosetta models to the SC. 10 crystal structure revealed that both the Rosetta model and the crystal structure were in high agreement. However, IntFc8 deviated from the predicted salt bridge with K310 in the Rosetta model and formed a hydrogen bond with the side chain of Q77, which changes sidechain conformation and forms a hydrogen bond with the backbone amide of C74 **(****FIG. 5A****).** Surprisingly, while the density is weaker, a change was also observed in the W101 and K310 rotamers **(****FIG. 5A****).** This may be a consequence of the low pH crystallization, as the ij loop and the n-terminus observed in the SC.10 crystal structure had conformational changes near the FL that matched more closely to the DENV sE structures also solved at low pH. Also, while not wishing to be bound to theory, the low pH condition would likely favor protonation of the Asp106, (typical pKa of 4.0) and hydrogen bonding to Q77 over formation of the salt bridge with K101. However, a similar W101 rotamer observed in the SC.10 crystal structure is also observed in the crystal structure of ZIKV sE when bound to a neutralizing antibody at the DI/DIII linker, indicating this rotamer can be formed at neutral pH (PDB 6NIU, chain A). Overall the SC.10 crystal structure retains the native sE dimer conformation, even under low pH conditions crystal conditions.

### DENV2 sE SC.14 & SC.10 elicit DENV2 envelope dimer specific antibodies in mice:

The data indicated that SC.14 and SC.10 are stable dimers at 37°C and efficiently present quaternary antibody epitopes at low concentrations. In order to determine if these stable dimers could elicit E protein dimer-specific antibodies in mice, female Balb/c mice (n=5) were immunized on day 0 with 5µg of DENV2 sE Mnmer2, WT, SC.14, and SC.10 formulated in isotonic sucrose/H₂O and boosted with 5µg of each protein on days 21 and 56, with blood collected on day 112 to harvest serum for analysis. The study was designed without adjuvant to measure the innate immune response to each protein antigen and eliminate adjuvant bias. To test total anti-DENV2 IgG titers sera was diluted 1:50 and a 6-fold serial dilution performed for each immunization group's five mice and tested for serum IgG binding against DENV2 in a capture ELISA. All of the groups immunized with DENV2 sE Mnmer2, WT, SC.14, and SC.10 produced similar levels of antibodies that bind to DENV2, with SC.10 having a slightly lower, but not statistically significant, mean titer.

Depletion experiments were used to assess if dimer specific antibodies were being elicited by mice immunized with DENV2 sE monomers (Mnmer2 and WT) or DENV2 sE dimers (SC.14 & S C.10). Two sets of magnetic beads were generated, one loaded with DENV2 sE monomers, using DENV2 sE Mnmer2 which remains monomeric in solution, and the other set of beads loaded with DENV2 sE dimers, using the stable dimer DENV2 sE SC.14, onto Ni-NTA magnetic beads via both protein's c-terminal 8x Histidine-tag **(****FIG. 7A****).** It was hypothesized that antibodies targeting E monomer epitopes would equally bind and be depleted by both the sE monomer loaded beads and the sE dimer loaded beads, as E monomer epitopes will be present on both sE monomer and dimers, as observed with the monomer epitope antibodies via direct ELISA analysis **(****FIG. 6B, 6E****).** Elicited antibodies that recognize E protein dimer-specific quaternary epitopes only bind to sE dimer loaded beads and are depleted from the sera, and similar to the direct ELISA experiment, these quaternary epitope antibodies would not bind to sE monomer and thus remain in the sera after depletion with sE monomer loaded beads **(****FIG. 7A****).** The relative change in signal in the DENV2 capture ELISA between the sE monomer depleted sera and the sE dimer depleted sera indicates whether mice, immunized with a particular DENV2 sE protein, elicited E dimer specific antibodies.

To validate the depletion protocol, sE monomer loaded beads or sE dimer loaded beads were incubated with 3H5, a DIII epitope antibody that binds monomer, and 2D22, a dimer-specific quaternary epitope antibody, to see if sE monomer or sE dimer beads could deplete these antibodies. Undepleted 3H5 bound to both sE monomer and sE dimer in a direct ELISA coated with either Mnmer2 or SC. 14. In contrast, and similar to the antibody binding direct ELISA experiments, only sE dimer beads were able to deplete 2D22, confirming that the depletion strategy can differentiate between E monomer and E dimer-specific quaternary epitope antibodies.

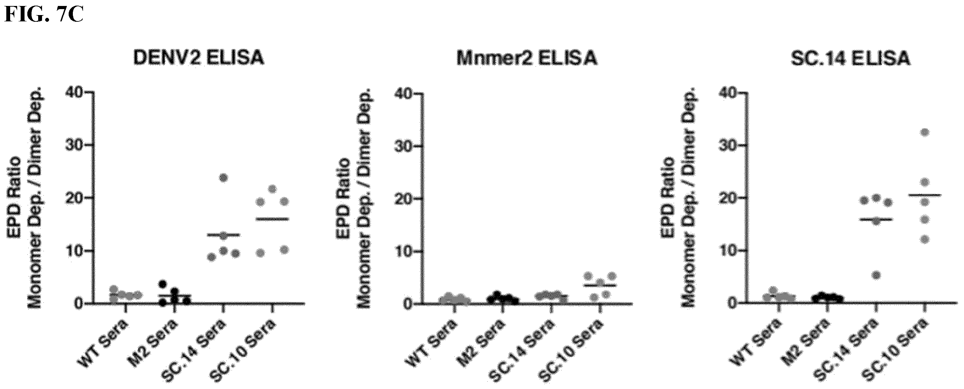

The sera from all four DENV2 sE protein mouse immunized groups (Mnmer2, WT, SC. 10, and SC. 14) 1:50 were diluted and depleted for 2-3 rounds with either sE monomer loaded magnetic beads (monomer depleted) or sE dimer loaded magnetic beads (dimer depleted) for 1hr at 37°C, with the beads magnetically removed after each round and the sera supplemented with new sE loaded beads for each subsequent round of depletion. As a negative control, sera were depleted with beads loaded with His-tagged Spycatcher protein (control depleted) to account for non-specific antibody depletion. Undepleted and depleted sera were titrated against DENV2 in a capture ELISA to assess binding of serum antibodies post-depletion. As expected, both undeleted and control depleted sera gave similar ELISA signals against DENV2 **(****FIG. 7B****).** For the Mnmer2 and WT immunized mice sera, both the sE monomer and dimer were able to equally deplete the mice sera from both of these groups, producing nearly identical signals against DENV2 **(****FIG. 7B** **&** **7C****).** For SC.14 and SC.10, sE monomer was capable of partially depleting both sera, indicating that E monomer epitope antibodies are elicited by these proteins. However, unlike WT and Mnmer2 sE dimer depleted sera, sE dimer was capable of depleting both SC.14 and SC.10 mice immunized sera, reducing DENV2 ELISA signal to near background signal, particularly for SC.10 **(****FIG. 7B****).** Taking the ratio of the end point dilution (EPD) for the monomer and dimer depleted sera, about 13 and about 19-fold mean improvement was observed for SC. 14 and SC. 10 respectively, suggesting that both SC. 14 and SC. 10, but not Mnmer2 or WT, elicit dimer specific antibodies **(****FIG. 7C****).** To confirm this result, the monomer and dimer depleted sera from all four DENV2 sE protein mouse immunized groups were tested in a direct ELISA coated with either DENV2 sE Mnmer2 (sE monomer) or DENV2 sE SC.14 (sE dimer) and the difference in the monomer depleted sera to the dimer depleted sera was compared using the EPD ratio. Similar to the DENV2 ELISA, Mnmer2, WT, SC.14 and SC. 10 depleted sera had low EPD ratios of 0.7, 0.9, 1.5, and 3.5, respectively, when tested against Mnmer2, indicating similar levels of monomer specific antibodies remained in both monomer and dimer depleted sera for all four groups **(****FIG. 7C****).** Similar to the DENV2 ELISA, when tested against SC.14, both Mnmer2 and WT had EPD ratios close to 1, while SC.14 and SC. 10 had EPD ratios of 15.9 and 20.5, confirming the DENV2 ELISA that mice immunized with these proteins elicit dimer specific antibodies.

In the DENV2 ELISA analysis of both SC. 14 and SC. 10, the signal for the monomer-depleted sera at the lowest dilutions was higher than the undepleted sera **(****FIG. 7B****).** This result can be explained if there were a mixture of low affinity (LA) and high affinity (HA) antibodies elicited during the immunization. In the undepleted sera, if the LA antibodies were more abundant compared to the HA antibodies, then the LA antibodies were able to outcompete the lower abundant HA antibody during initial binding to virus on the ELISA plate. However, during the subsequent wash and incubation steps the LA antibodies are more likely to release from the virus and lower the ELISA signal. In contrast, if in the monomer depleted serum there was a higher ratio of HA to LA antibodies, then more of the HA antibodies would bind to the virus and remain bound during the wash steps, thus leading to a higher ELISA signal. It was also predicted that this scenario would result in an increase of ELISA signal at the low dilutions that rapidly decreases, as the concentration of the HA antibodies would be low, thus binding would only be observed at the highest serum concentrations (e.g., low dilutions). To support this hypothesis, a competitive binding model was created in Matlab that simulated LA (Kd = 10nM) and HA (Kd = 1nM) antibody populations binding to virus and then unbinding during subsequent wash and incubation steps. Both populations of antibodies were given the same association rate constants typically observed with antibody-antigen binding, but the HA antibody population was assigned an off-rate that was 10-fold slower. When incorporating into the model association times (T_{A}) and dissociation times (T_{D}) that match the incubation times and wash times used in the ELISA protocol, the model was able to reproduce the trends observed in the DENV2 ELISA. This suggested that the E dimer specific antibodies elicited by SC.14 and SC. 10, although lower in abundance, may be higher affinity antibodies, and compete for binding to similar regions on the virus as the more abundant LA E monomer specific antibodies. Overall, the data indicated that the stabilized DENV2 sE SC. 14 and DENV2 sE SC. 10 dimers are capable of eliciting E dimer-specific quaternary epitope antibodies.

This study was able to computationally predict multiple DENV2 sE dimer and monomer stabilizing mutations. The most stable variants have homodimer Kds less than 100 pM at 37°C, compared to 12 µM for WT, and overall protein thermostability was raised by more than 18°C. Strikingly, adding only 1-2 mutations increased expression by up to ~20-fold and by combining these mutations, >70-fold. This increase in expression was also observed without co-expression of prM, which reduced additional purification steps and testing required to ensure no prM is present in the final sE vaccine formulation prior to immunization, as prM presents non-neutralizing epitopes that can elicit antibodies that cause ADE. Taken together, these stability and expression improvements aid in the practical consideration of producing these proteins at the scale that would be needed to create subunit vaccines.

Furthermore, this study demonstrated that alternate combinations of the mutations can be used to select for desired stability, expression and oligomerization properties. For example, the affinity of the DENV2 sE dimer was tunable by combining the dimer stabilizing mutations IntFc2, IntFc8 and UndPk6 at different regions of the sE protein to create the SC. 10 stable dimer. Combining Mnmer2 with PM4 (SC.25) retained the protein as a monomer while increasing expression and improving protein stability **(Table 1).** These mutations can also be combined with the previously published cysteine mutations, to increase the stability and expression of the covalently stabilized dimers **(Table 1,** SC.28). While this study focused on DENV2 sE, the computationally targeted DENV2 sE regions are largely conserved in other DENV serotype and flavivirus E proteins, including Zika, suggesting that this strategy can be applied to improve the stability and expression of other flavivirus E protein.

The most stabilizing mutation sets (IntFc2, IntFc8, UndPk6 and PM4) all targeted regions of the E protein that are important for the DENV infection cycle. IntFc2 creates new contacts on the "underside" of the E dimer that would natively be interacting with the M protein on the DENV envelope. UndPk6 stabilized the DI/DII hinge, a region where mutations have been shown to affect the pH threshold for viral infection. UndPk6 stabilized the sE dimer without any new contacts at the dimer interface (FIG. 5A). Structural analysis suggested that these mutations stabilize the sE dimer by reducing the entropic cost of forming the sE dimer by stabilizing the monomer in a dimer-favored monomer conformation, as this region has been shown to be inherently flexible in the sE WT protein. Additionally, mutating T280 to proline eliminated a native E contact with the M protein on the virus, which is lost in the context of sE. Comparison of the hinge angle in the SC. 10, EDE1 C8 bound sE and apo sE crystal structures suggested that the kl loop conformation affects the E dimer stability **(****FIG. 5B****).** This provides insight into the structural regulation of E protein conformational changes during virus infection. On the virus, the M protein makes contacts with T280 on the kl loop in E, which may stabilize the prefusion E dimer conformation. Loss of this E-M contact during virus endocytosis would destabilize the kl loop, thus favoring the new hinge loop conformation observed in the post-fusion trimer. The UndPk6 mutations also stabilized the kl loop through increased hydrophobic packing in the hinge and mutation to proline at the position 280 at the base of the kl loop, which restricts the conformational space of the kl loop by forming a new hydrogen bond with T189 on the adjacent hinge loop **(****FIG. 5A****).** It was also observed that a mutation L277M, present in the DI/DII hinge, destabilized the dimer that also was observed to allow for more infection at higher pH, lending support to the hypothesis that the mutation destabilized the dimer through changes in the DI/DII hinge region, allowing for trimer formation at a less stringent pH.

Rosetta predicted only a small number of unique mutations in near the FL that would still preserve native sE dimer formation that would be favorable, one being G106D, the IntFc8 variant, suggesting that the FL dimer interface is already largely optimized with favorable contacts. IntFc8 stabilizes the sE dimer, reduces FLE presentation, and drastically improves expression **(****FIG. 3A-3C****, Table 1** and **FIG. 6F****).** Furthermore, quaternary epitope antibody binding appeared unaffected by the mutation **(****FIG. 6F****).** Another mutation set, PM4, contains three mutations in the DI core. The analysis of underpacked regions implicated the DI core as a large underpacked region, consistent with the changes within DI required for the E dimer to transition to the post-fusion trimer. PM4 mutates the suboptimal buried polar residues, S29 and T33, that form contacts to DIII in the low pH postfusion trimer conformation. Mutating both residues drastically improved the stability of the protein, by +4.6°C and by the additive property +5.4°C, respectively, suggesting the WT amino acids have been selected to stabilize the E protein post fusion trimer conformation as opposed to maximizing the thermostability of DI.

To validate the analysis that these mutations affect E regions important for the virus life cycle, the PM4, IntFc8 and UndPk6 mutations were cloned into the E gene of the DENV2 16681 RNA genome and transfected the RNA into C6/36 cells. Only propagation of DENV2 16681 WT was observed and no propagation of the mutated viruses, indicating that these mutations either prevent virus production or render the virus non-infectious. The same results were observed when transfecting the RNA genome of DENV2 16681 WT and DENV2 16681 PM4 virus into Vero-81, confirming the lack of mutated virus propagation was not cell line dependent. This data was also consistent with the DENV2 sE SC. 10 stable dimer, which contains IntFc2, IntFc8 and Undpk6, crystallizing as the biological sE dimer under low pH conditions that typically favor the E trimer, supporting the long-standing notion that stabilizing the dimer, whether with antibodies or, in this case, dimer stabilizing mutations, renders the virus non-infectious. These results suggest that the virus has evolved the E protein to contain these local instability regions for functional purposes, allowing for adoption of multiple E conformations required for infection.

The improvements in the dimer stability for sE variants also provided improved quaternary epitope presentation at 37°C. Enhanced binding of both type-specific (2D22) and cross-reactive (EDE1 C8 & EDE2 A11) known neutralizing quaternary epitope antibodies to both DENV2 sE SC. 14 & SC. 10 variants are consistent with the improved dimer stability and stable epitope presentation at 37°C. Additionally, SC. 10 crystallized in the same sE dimer conformation that WT E adopts when bound by EDE1 C8, consistent with the improved binding of EDE1 C8 to SC. 10 observed in the ELISA experiment **(****FIG. 5B** and **6F****).** While the focus of this work was to improve sE quaternary epitope presentation, SC. 14 and SC. 10 also efficiently present the 3F9 DI epitope, an epitope known to be targeted by neutralizing antibodies elicited in natural DENV2 infected individuals. This indicates that in addition to presenting quaternary epitope antibodies, SC. 14 and SC. 10 also present other important neutralizing antibody epitopes observed to be elicited by the human immune response during natural infection.

A primary purpose of designing stabilized DENV2 sE dimers was to assess if these stabilized dimers can elicit quaternary epitope antibodies that target the E dimer. This study provides the first evidence that stabilized DENV2 sE dimers can elicit E dimer-specific antibodies *in vivo.* It was observed that these antibodies are low in abundance and in competition for similar monomer specific antibody epitope regions. This has also been observed during natural infection, as the monomer-specific FLE and dimer-specific EDE antibodies elicited compete for similar epitope regions on the DENV E protein. Furthermore, is has been shown that the EDE antibodies have a higher affinity to DENV and are more potently neutralizing than the FLE antibodies. A similar trend was observed in this study, with the E dimer-specific antibodies having increased binding to DENV2 after depletion of the mouse serum monomer-specific antibodies with Mnmer2.

While quaternary epitope antibodies were observed in SC. 10 and SC. 14 mouse immunized sera, a substantial fraction of elicited IgG was able to be depleted by the DENV2 sE Mnmer2 protein, suggesting both of these proteins still also antibodies that target epitopes within the DENV2 sE monomer. These monomer epitope antibodies may represent both potently neutralizing antibodies, such as 3H5 (DIII) and 3F9 (DI) for DENV2, and poorly neutralizing antibody epitopes including FLE antibodies such as 4G2 and 1M7, which have been shown to play a role in causing ADE.

Cell Lines and Viruses: EXPI293F (ThermoFisher, Cat: A14527) cells were maintained in EXPI293 expression media at 37°C, 8% CO₂ at 250rpm without antibiotics, until passage 25 to improve batch-to-batch reproducibility. Insect C6/36 (CRL-1660) cells were grown at 32° C with 5% CO₂ in minimal essential medium (MEM) media supplemented with 5% fetal bovine serum (FBS) and 100 U/mL penicillin, 100 mg/mL streptomycin. Vero-81 (CCL-81) cells were obtained from ATCC. Vero cells were grown at 37° C with 5% CO₂ in Dulbecco's Modified Eagle's Medium (DMEM) 5% fetal bovine serum (FBS), 100 U/mL penicillin, 100 mg/mL streptomycin, 1% GlutaMax, 1% Sodium Bicarbonate, 1% non-essential amino acids. The RNA genome of DENV2 strain 16681 was used for cloning of stabilizing mutations for transfection into C6/36 for virus induction.

DENV2 sE protein construct design, cloning and plasmid preparation for cell surface display and soluble expression: DNA encoding the DENV2 (strain 16681) soluble envelope protein, sE (res1-394), lacking the stem and transmembrane domains, was cloned into the modified PαH mammalian expression vector, originally derived from pHLSec vector by substitution of an alternative multiple cloning site containing a CAG promoter and human serum albumin signal peptide, replacing the original PTPα signal sequence. DENV2 sE was expressed for both mammalian cell surface display and soluble expression using the same pαH vector backbone and plasmids were renamed to differentiate function. For mammalian surface display, sE genes were cloned into the pD2sE_Dsp display vector, which genetically fuses a GS₆ₓ-cMyc-GS₇ₓ linker and the MHC Iα transmembrane/cytoplasmic domains to the c-terminus of sE; for membrane anchoring, a cMyc epitope tag was added for independent detection of sE surface display; for soluble expression, sE genes were cloned into the pD2sE_EV8 expression vector containing a c-terminal GS₆ₓ-His₈ₓ tag for nickel affinity purification. All DENV2 sE Rosetta variant and SC variant cloning was performed by Twist Biosciences. DENV2 sE cloned plasmids were heat-shock transformed into E. *coli* DH5α cells for amplification, miniprepped or midiprepped using DNA endotoxin-free kits and stored in endotoxin-free H₂O at -20°C.

Mouse immunizations: Female Balb/c mice were inoculated at 6 weeks of age. Each mouse was immunized subcutaneously with 5 µg of either DENV2 rE monomer (n=5), rE wild type (n=5), rE disulfide dimer (n=5), rE Rosetta dimer (n=5), rE Rosetta dimer FLmut (n=5), all formulated in isotonic 9.25% sucrose/H2O, or vehicle alone (n=3). All groups received 3 immunizations (day 0, 21 and 56) and serum samples were collected on day 21, 28, 63 and 112 by submandibular bleed.

Cloning, transfection and immunostaining of DENV2 16681 containing stabilizing mutations: Dengue virus 2 (DENV2) infectious clones were designed by subdividing the DENV2 genome into a plasmid system. Wild type and mutant DENV2 pre membrane and envelope sequences were synthetically derived and introduced into a four-plasmid infectious clone system utilizing the DENV2 16681 strain. Plasmids were transformed, miniprepped, digested, and analyzed by gel electrophoresis for accuracy. Digested DENV2 fragments were then ligated together and purified by a chloroform extraction. DENV2 full-length genomic RNA was then generated using T7 RNA polymerase. Infectious genome-length DENV2 RNA transcripts were electroporated into C6/36 (CRL-1660) cells. Four days later, cell supernatants were harvested and cells were immunostained to confirm virus recovery. The recovered viruses were sequenced to confirm sequence identity. Four days after viral RNA electroporation, cells were washed with 1X PBS and fixed with 10% Formalin for 20 min at room temperature. Fixed cells were then permeabilized by washing twice with 5ml of 1X Permeabilization buffer (ThermoFisher). Fixed and permeabilized cells were then blocked for 10 minutes at room temperature with blocking buffer (1X Permeabilization buffer containing 5% non-fat dry milk). Fixed and permeabilized cells were stained with anti-prM hybridoma, 2H2, and anti-E hybridoma, 4G2, at 1:1000 diluted in blocking buffer for 30 min at 37° C. Fixed and permeabilized cells were then washed three times with 1X PBS and were stained with an anti-mouse horseradish peroxidase (HRP)-labeled secondary antibody for 30 min at 37° C. After washing three times with 1X PBS, foci were visualized with TrueBlue substrate compared to cells electroporated with 1X PBS.

### EXAMPLE 2: Mutations stabilizing ZIKV E glycoprotein dimer

Using computational modeling of the DENV2 soluble recombinant envelope protein (sRecE), amino acid mutations were identified that were capable of (1) stabilizing the DENV2 sRecE dimer conformation at human body temperature (e.g., 37 °C) while retaining human DENV neutralizing antibody epitopes; (2) improving total protein stability by up to 18 °C (to a temperature of about 69.3 °C) compared to the wildtype parent protein; and (3) improving expression levels and final production yield by up to 72.9 fold compared to the wildtype parent protein. These mutations stabilized the dimer by targeting distinct instability regions throughout the sRecE protein.

A modeling strategy was implemented that targeted regions of instability at the sRecE dimer interface, as shown in **FIGS. 9A-9C****.** Targets included temperature sensitive interface regions caused by a high fraction of polar residues, HisCat clusters that weaken the dimer to form trimer at low pH, fixing UnderPacked defects to improve stability, and surface hydrophobics which can reduce aggregation and increase stability in solution. For example, the mutant IntFc2, comprising A259W and T262R mutations, had a ΔΔG_{monomer/res} score of - 2.767 REU (methods as described in Example 1). Lower ΔΔG Scores indicated stabilizing mutations. Identified mutants, the mutations comprised therein, and the dimer affinity (nM K_{D}), protein unfolding (Tₘ), expression data (mg/L yield) and location of mutations are shown in **Table 2** and **FIG. 10****.**

The Fusion Peptide (also referred to as the Fusion Loop) is an immunodominant epitope. Antibodies that target this region are poorly neutralizing and may lead to enhancement of disease. The fusion loop epitope is buried in the sRecE dimer, thus a stabilized dimer (e.g., comprising stabilizing mutations of the present invention) may prevent exposure of the fusion loop epitope, thereby not eliciting disease enhancing fusion loop antibodies during vaccination. For example, a stabilized E glycoprotein of the present invention, IntFc8, is stabilized in dimer conformation as compared to the WT sRecE and comprises a G106D mutation in the fusion loop. Mutating this position prevents binding of fusion loop binding antibodies, and this study shows that this mutation also helps with dimer stability, suggesting that this mutation may prevent elicitation of these disease enhancing antibodies in a vaccine application.

The quaternary epitopes as described in Example 1 are conserved in other flaviviruses such as ZIKV, as shown in **FIGS. 11** and **17A****-17B.** To engineer disulfide interactions for the expression of stable ZIKV rE dimers, an A264C mutation was introduced into the EDII dimer interphase **(****FIG. 12A****).** Expression of stable dimers was confirmed by protein gel analysis showing a protein band twice the size of the monomer **(****FIG. 12B****).** On ELISA, the stabilized dimer was shown to be bound by highly neutralizing antibodies that recognize epitopes of quaternary structure, including C8, C10, A9E, G9E, ZKA-230, and ZKA-230 **(****FIG. 12C****).**

To test *in vivo* efficacy, mice were primed and boosted on week 3 and week 9 with soluble rE monomer ("rEM") and rE dimer ("rED"), and ZIKV IgG levels and neutralizing antibody levels were analyzed at week 16 **(****FIGS. 13A** and **13B****).** ZIKV rE dimers induced higher levels of IgG antibodies and the serum also had higher levels of neutralizing antibodies. rEM did not induce any neutralizing antibodies, while rED did, suggesting that the oligomeric state of the protein is important for the induction of neutralizing antibodies.

The percentage of antibodies that bind to ED III was evaluated by performing EDIII serum depletion assays, as shown in **FIG. 14A****.** ZIKV monomer induced a very EDIII-targeted response, while ZIKV dimer had a low percentage of EDIII binding antibodies. The dimer induced neutralizing activity of the mouse serum did not change when EDIII antibodies were depleted, in contrast to monomers plus alum, where the EDIII antibodies were mainly responsible for neutralization **(****FIG. 14B****).** A blockade of binding assay was used to show that dimer induced immune sera was able to block binding of ZIKV specific antibodies with a quaternary footprint, where monomers were unable to do so **(****FIG. 14C****).**

Sera from mice that were immunized with monomers or dimers were transferred to mice that were permissive to ZIKV infection. Mice that received monomer sera lost significant amounts of body weight and were not protected against ZIKV viral challenge, while mice that were inoculated with dimer induced mouse sera were able to protect **(****FIG. 15A****).** Similar studies performed with DENV2 also show that dimers induced higher levels of IgG and neutralizing antibodies than monomers **(****FIG. 15B****).**

### EXAMPLE 3: Production of DENV VLPs without co-expression of pr, an antigen implicated in antibody dependent enhancement.

Current approaches for producing flavivirus VLPs require co-expression of full length prM to promote secretion from the cell and boost production yields. A downside of this approach is that pr is inefficiently cleaved from the particles and remains as a dominant epitope that elicits non-neutralizing antibodies implicated in antibody dependent enhancement. As shown in Example 1, by stabilizing the E dimer with the stabilizing mutations it was possible to create DENV VLPs without co-expression of pr. This strategy may result in particles that more closely resemble mature DENV and are safer to use as vaccines.

To establish the feasibility of producing DENV VLPs without pr, expression experiments were performed with four DENV2 constructs: (1) prM along with WT E, (2) M along with WT E, (3) prM along with the E protein stabilized with the mutations sets I8-U6-PM4, and (4) M along with the E protein stabilized with I8-U6-PM4 **(****FIGS. 16A-16B****).** The genes for the constructs were cloned into a mammalian expression vector (PaH) and transiently transfected into Expi293 cells. Following expression, low speed centrifugation was used to clear the culture media of the cells and the amount of E protein in the media was quantified with dot blot analysis and the DENV2 antibody 1M7. A dramatic boost in expression was observed with both VLP constructs containing the stabilizing mutations, while with WT E we were not able to detect any E protein in the case where pr was not included. To verify that the proteins were forming VLPs, sucrose gradient centrifugation was used to separate soluble protein from particles. Similar analysis was performed with the soluble E protein dimer for comparison. Dot blot analysis of the fractions collected from the sucrose gradient showed that while soluble E remained at the top of the sucrose gradient, the stabilized E VLP construct without pr was present in similar sucrose fractions as commercial VLPs (native antigen company) that was purchased for control experiments. The commercial VLPs were produced with the co-expression of pr. Negative stain EM confirmed that particles were being formed, which showed they are larger on average (about 35-40 nm versus about 25-30 nm) than the commercial VLPs produced using pr and WT E **(****FIG. 16C****).** Western blot analysis was used to characterize the amount of uncleaved prM on the commercial VLPs and found that it many cases more than 50% of the prM was uncleaved.

The foregoing is illustrative of the present invention, and is not to be construed as limiting thereof. The invention is defined by the following claims, with equivalents of the claims to be included therein.

All publications, patent applications, patents, nucleotide sequences, amino acid sequences, GenBank accession numbers and other references cited herein are incorporated by reference in their entireties for the teachings relevant to the sentence and/or paragraph in which the reference is presented.

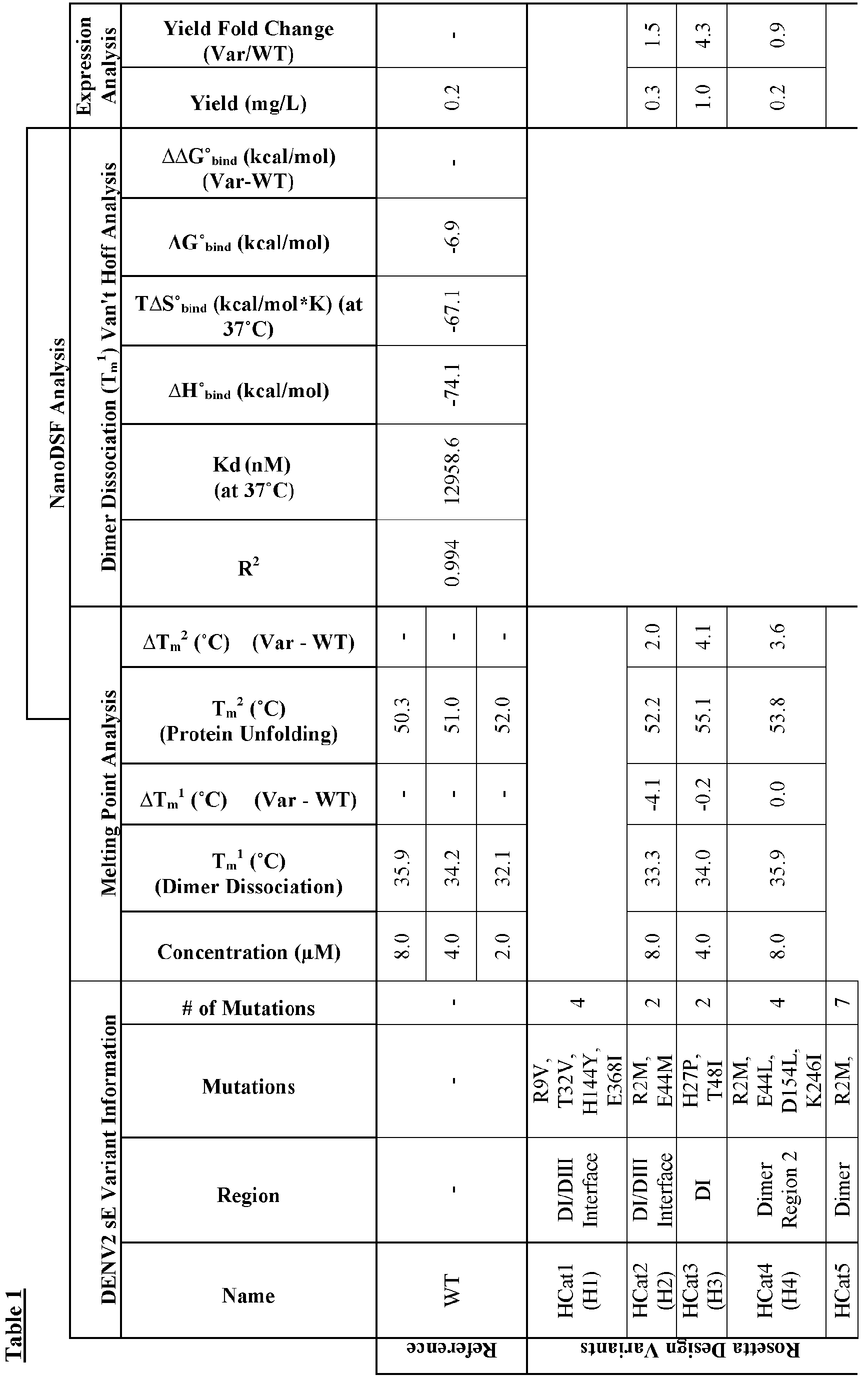

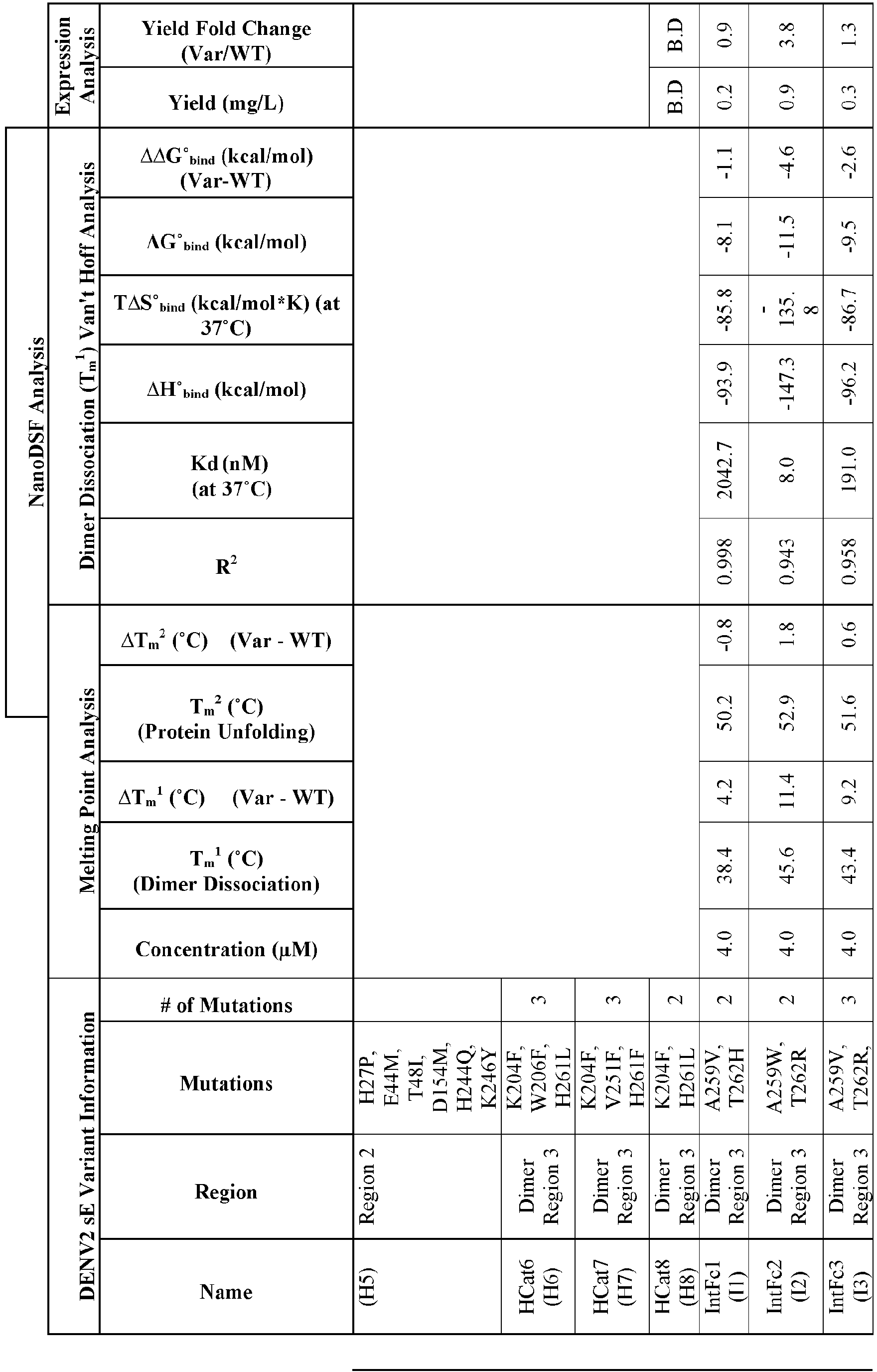

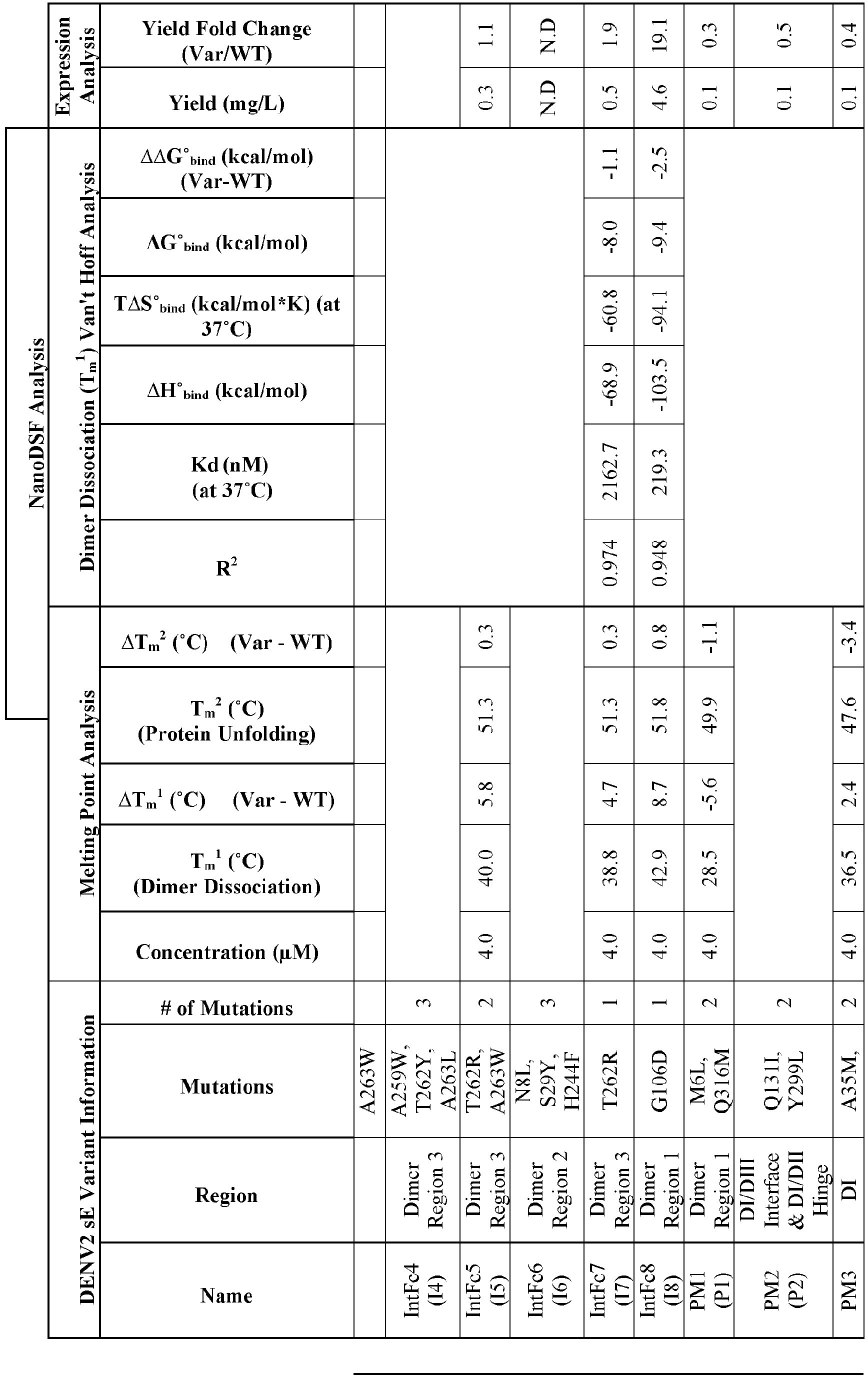

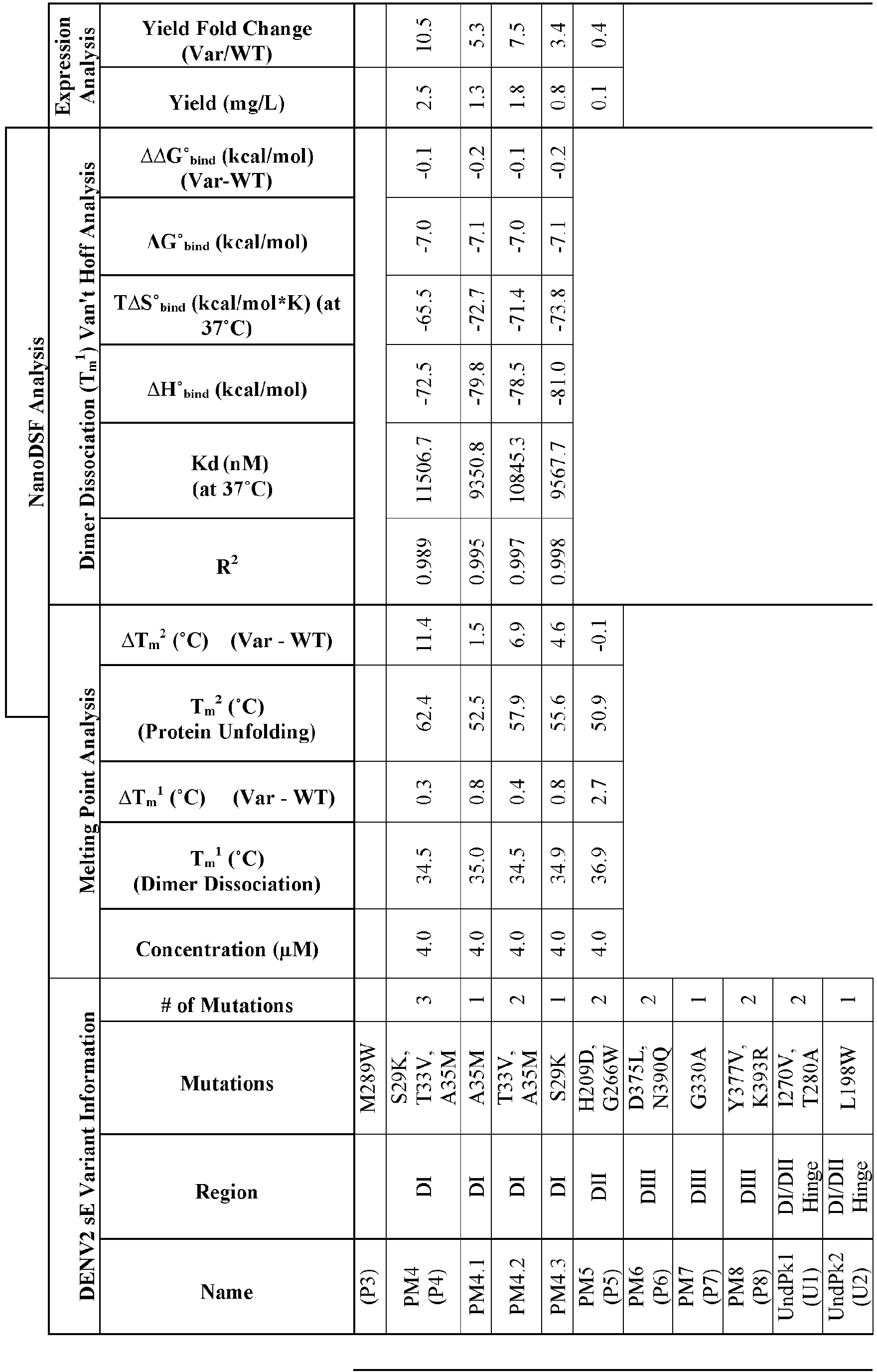

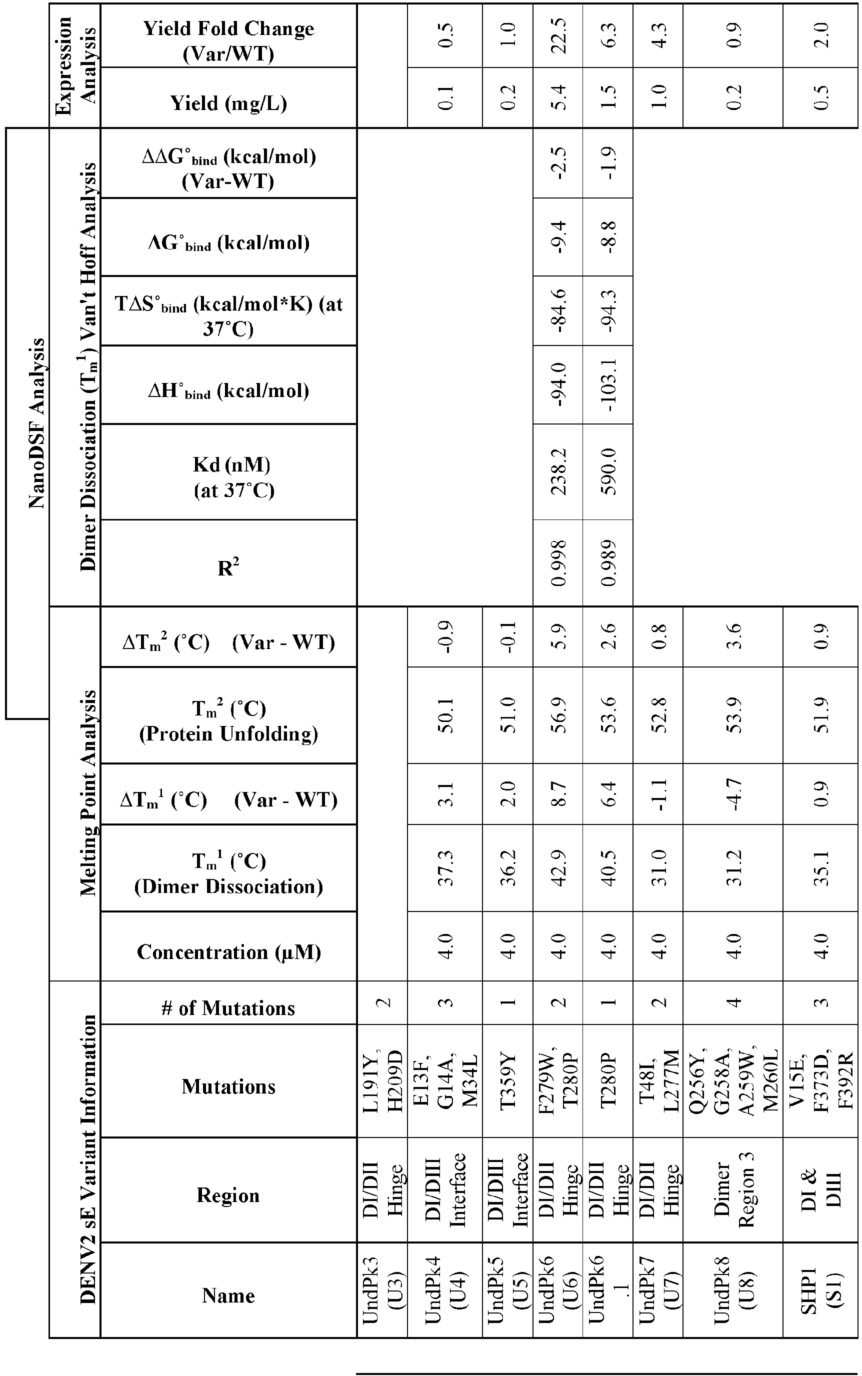

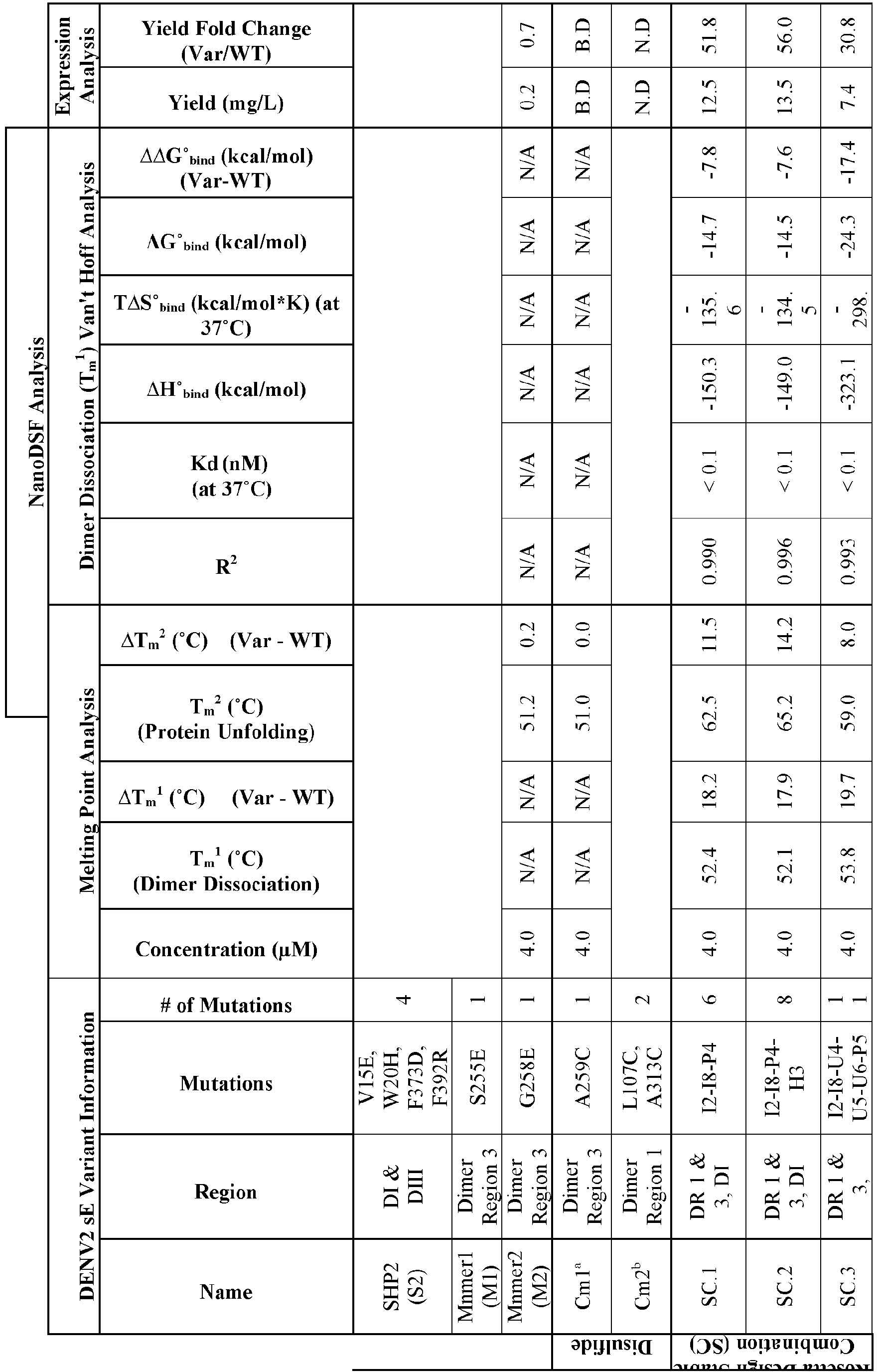

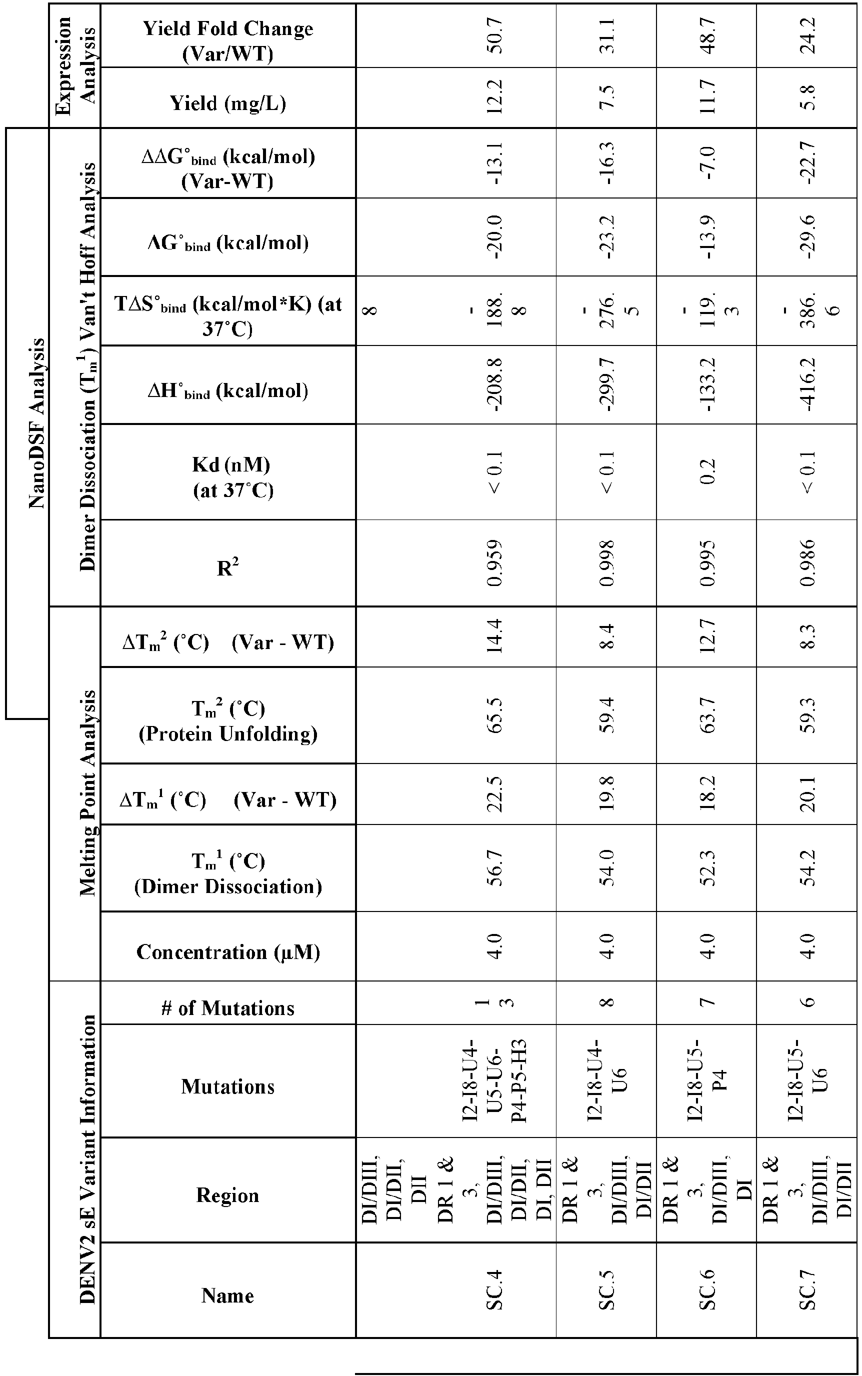

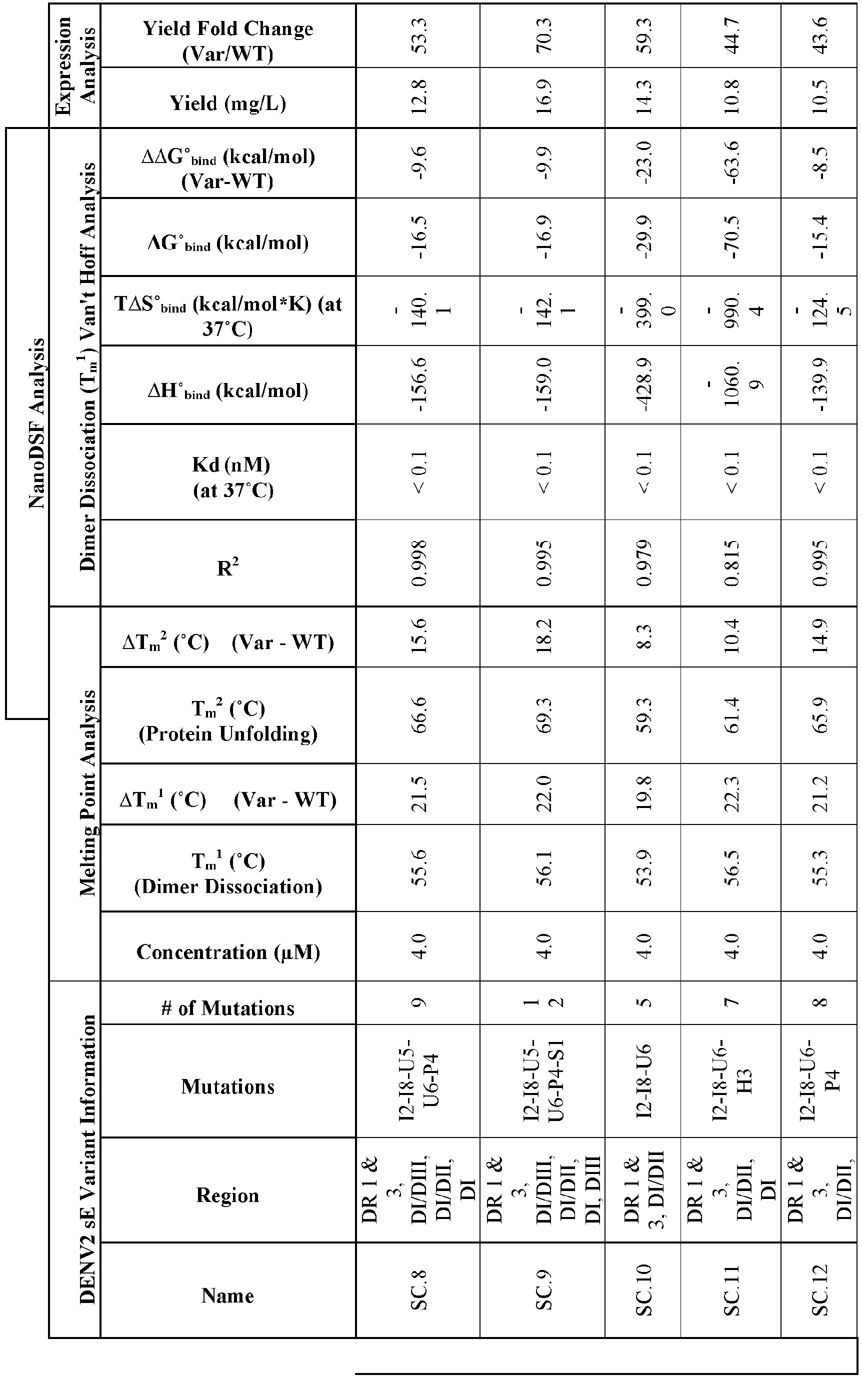

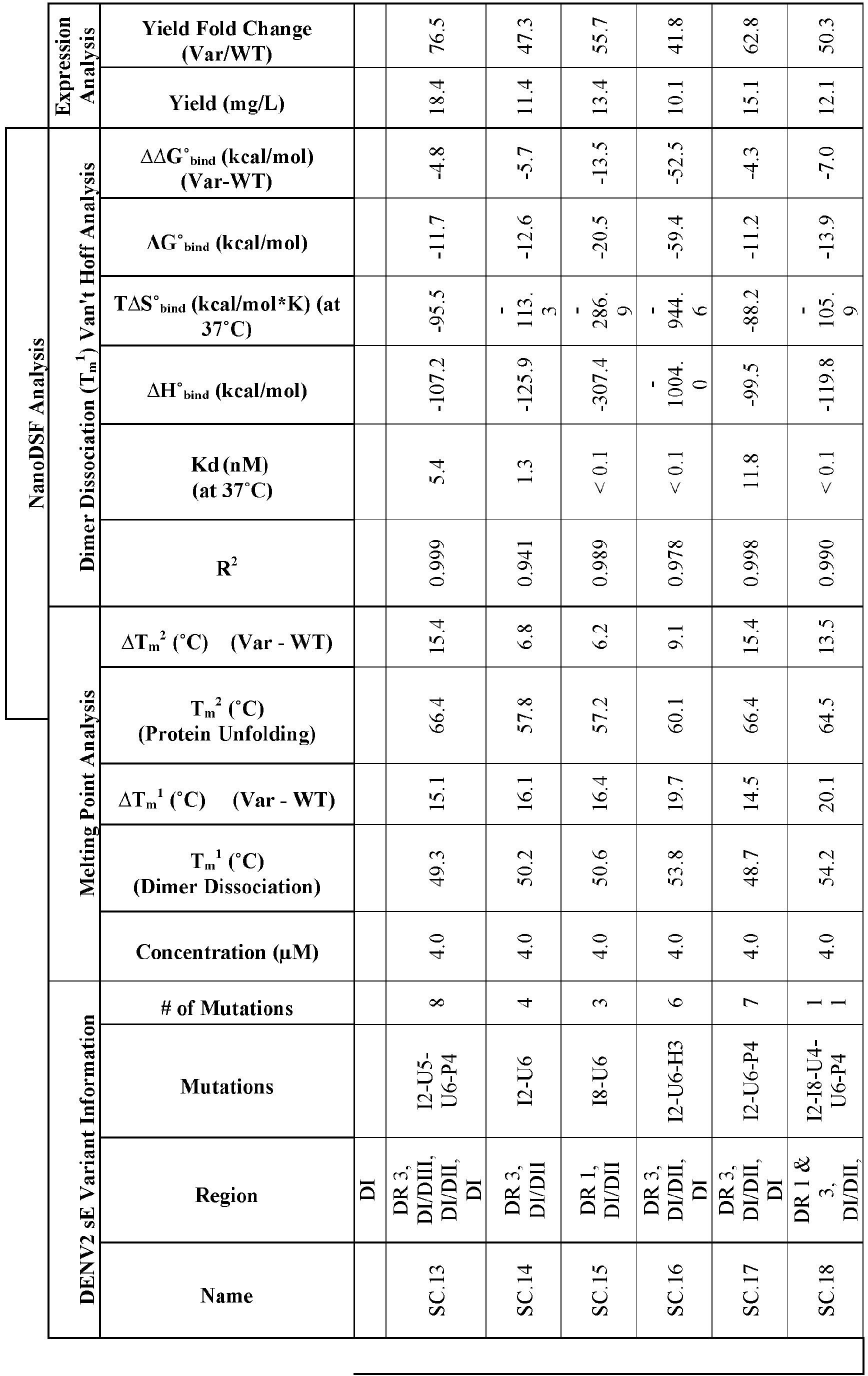

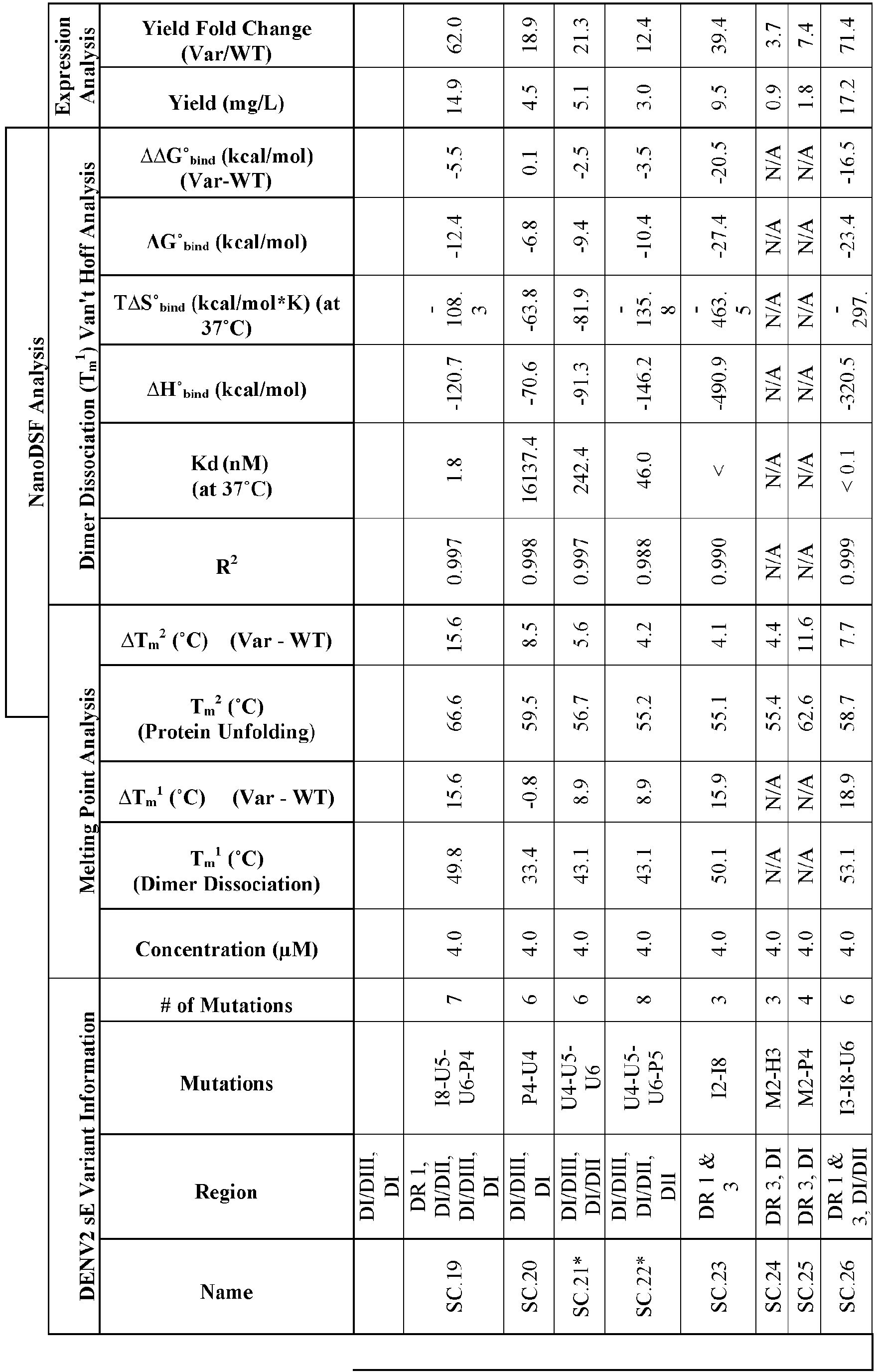

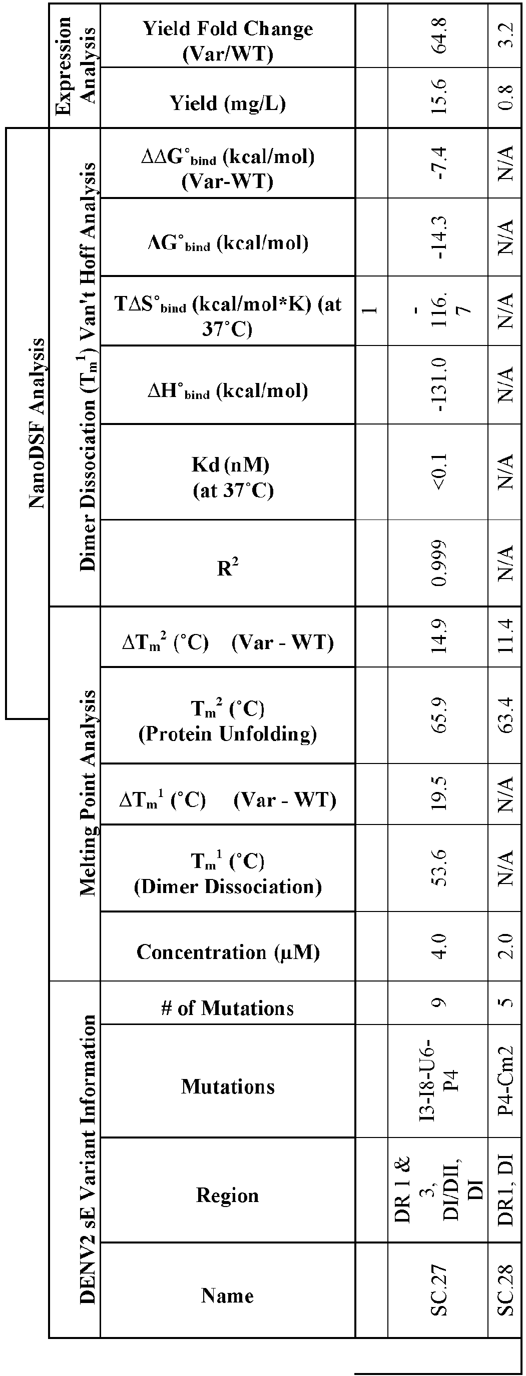

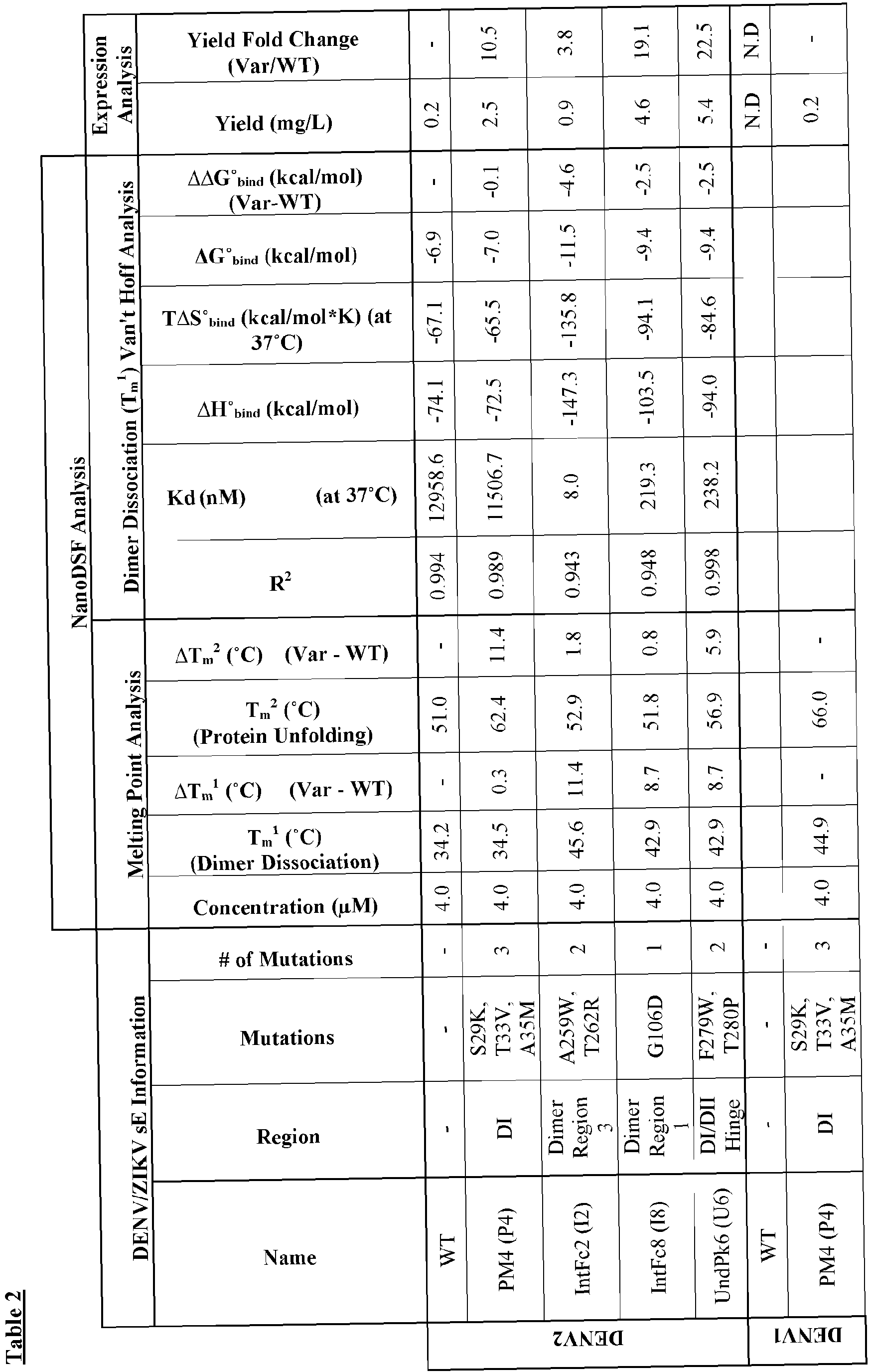

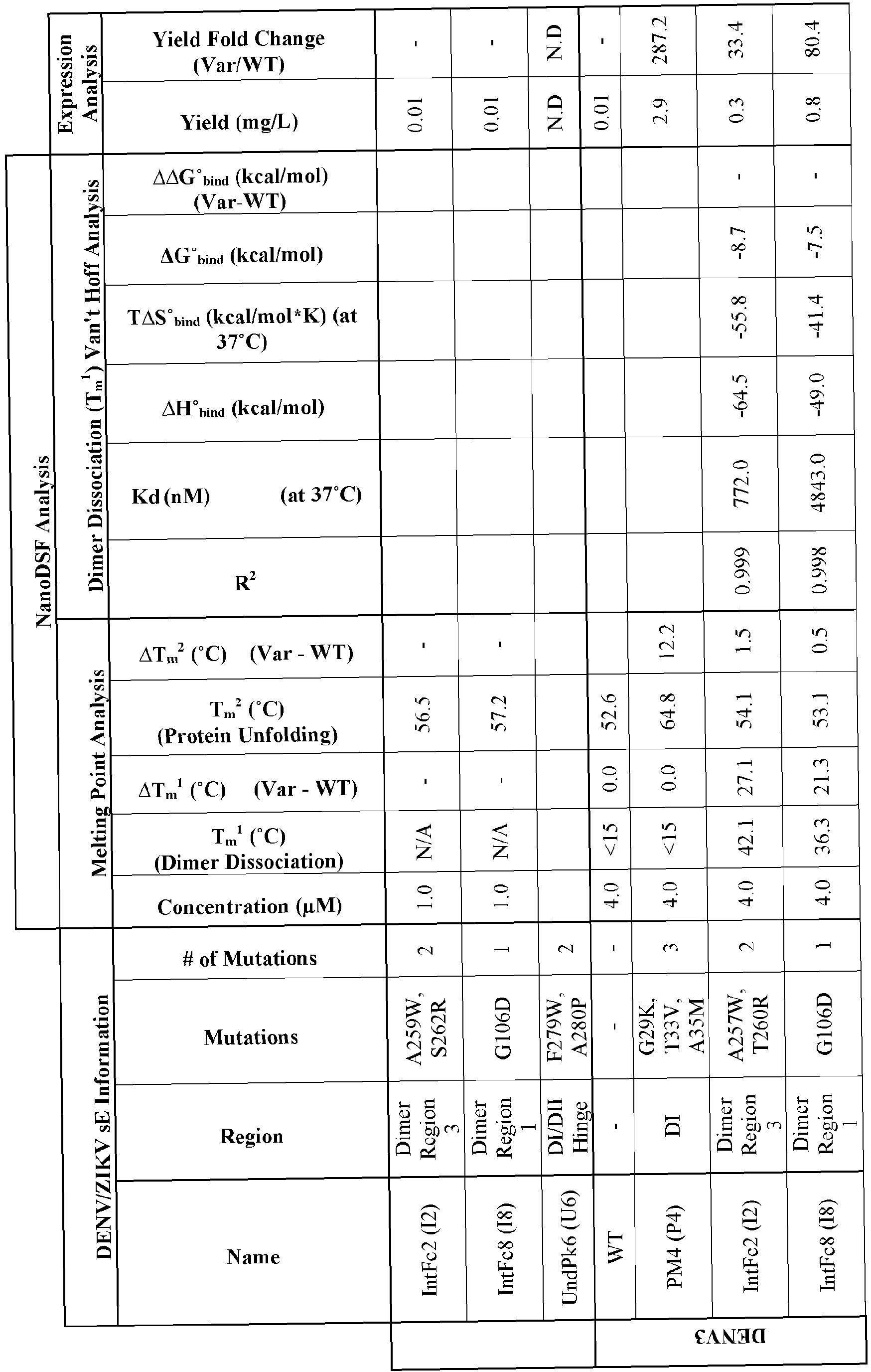

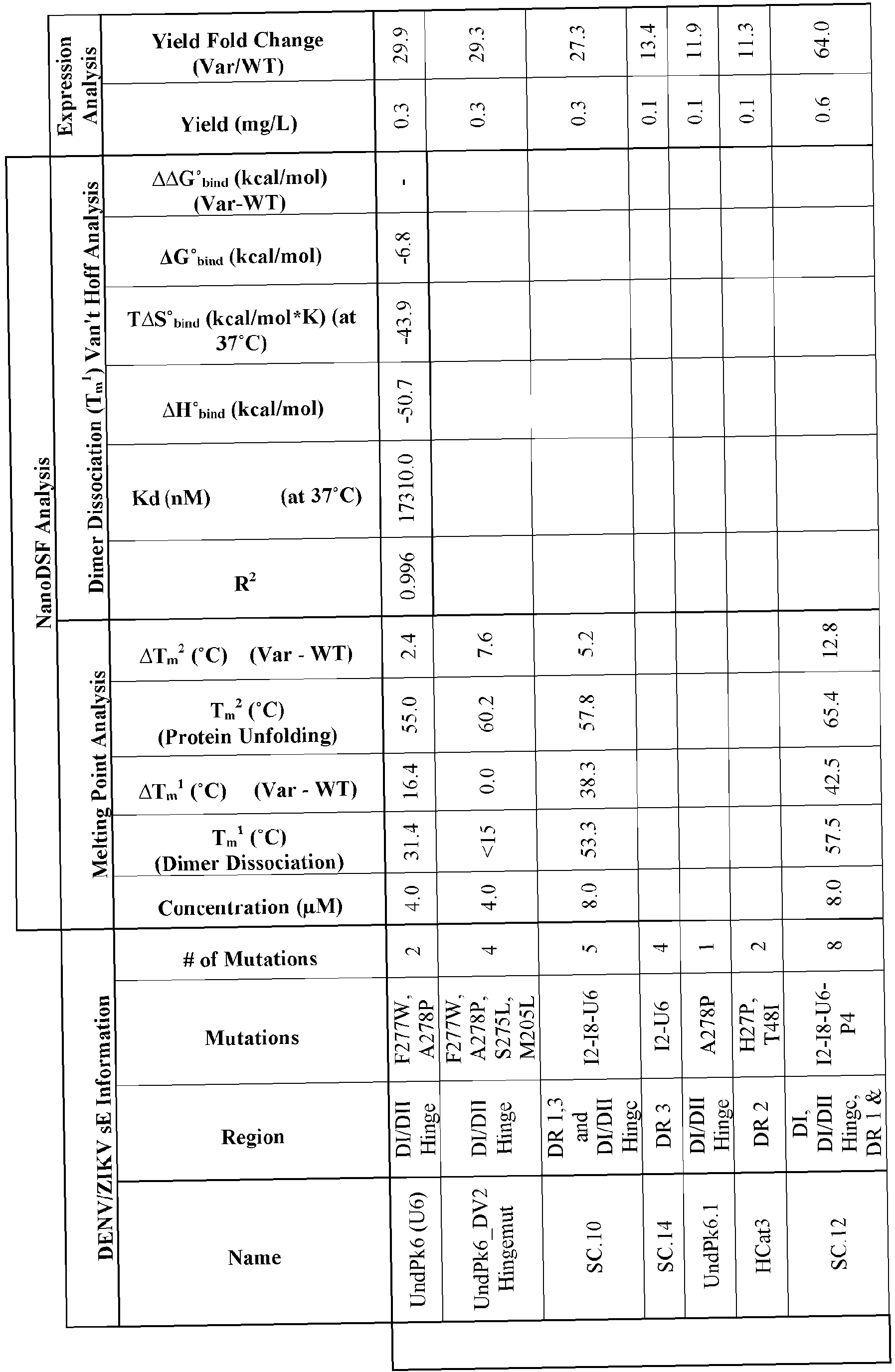

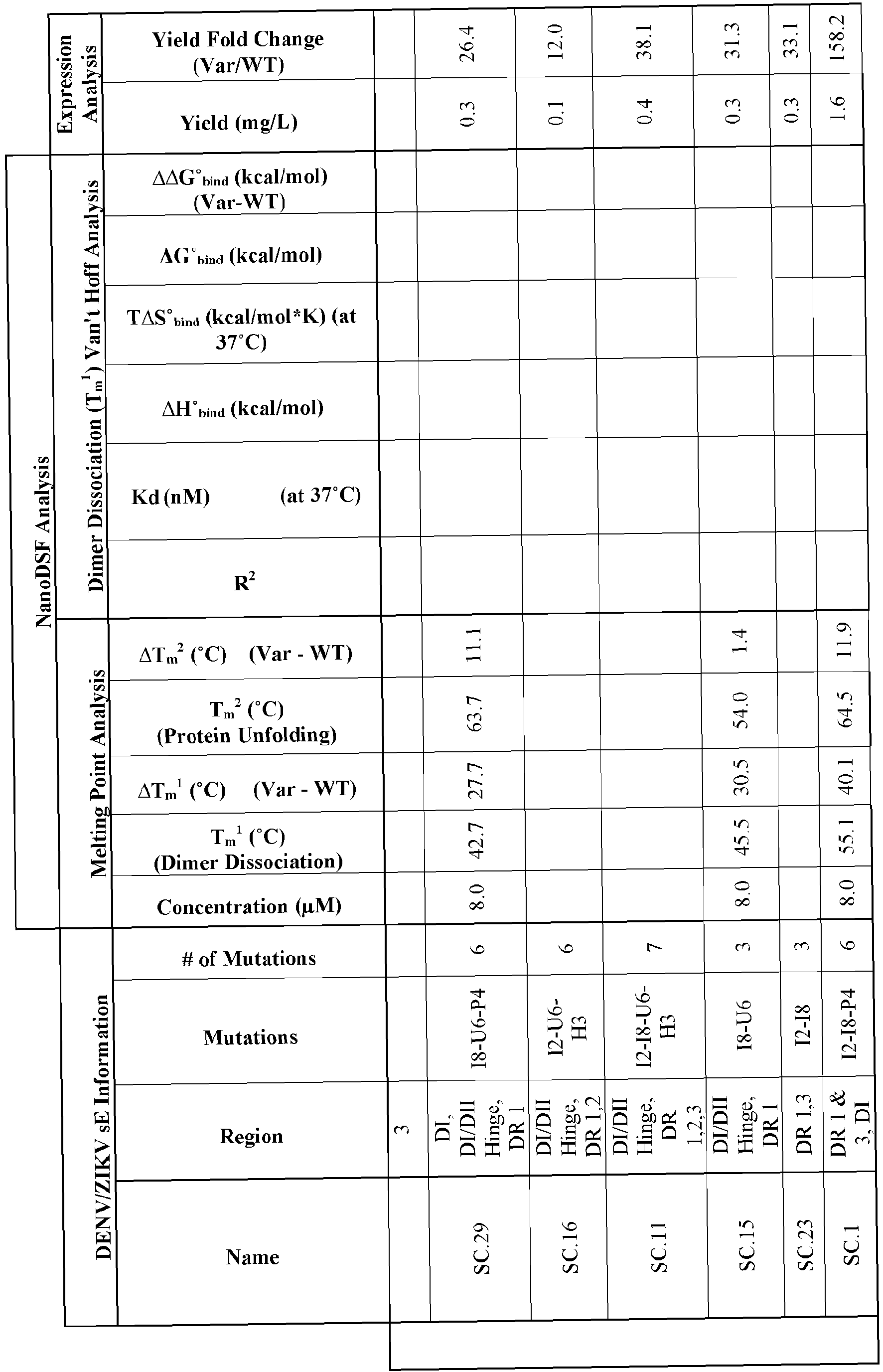

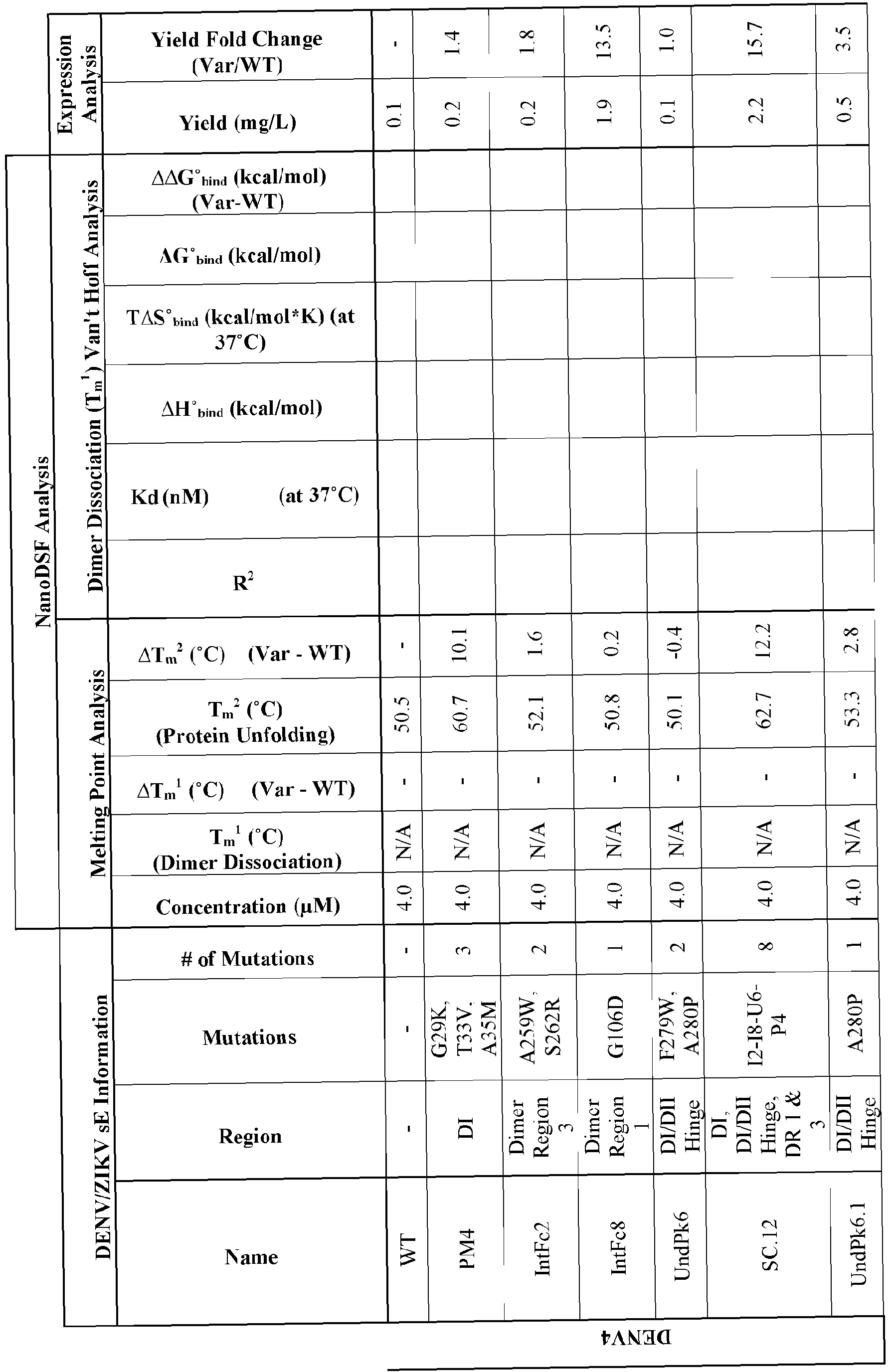

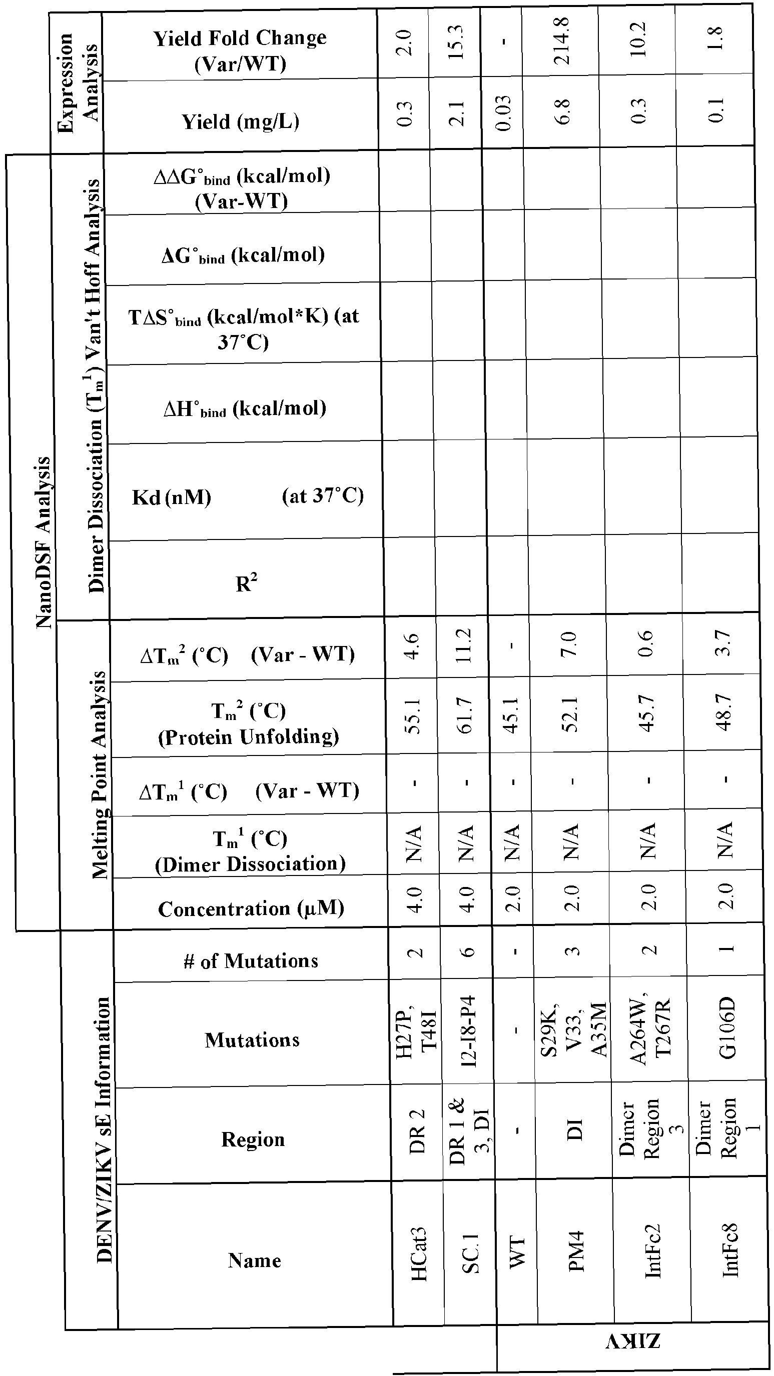

### Key for Table 1 and Table 2:

N/A: Not applicable; no melting transition observed and no Van't Hoff fit obtained
B.D.: Below detection; insufficient 280nm absorbance to quantify concentration, protein band observed via SDS-PAGE analysis post purification at given scale
N.D.: No detection; unable to observe protein band via SDS-PAGE analysis post purification at given scale
Gray: not tested
DR 1: Dimer Region 1, FL Dimer Interface
DR 2: Dimer Region 2, DI and ij loop Dimer Interface
DR 3: Dimer Region 3, αβ Helix Central Dimer Interface

**Table 3**

| Amino Acid Residue | Abbreviation | |
|---|---|---|
| | Three-Letter Code | One-Letter Code (can be upper or lower case) |
| Alanine | Ala | A |
| Arginine | Arg | R |
| Asparagine | Asn | N |
| Aspartic acid (Aspartate) | Asp | D |
| Cysteine | Cys | C |
| Glutamine | Gln | Q |
| Glutamic acid (Glutamate) | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

**Table 4**

| Modified Amino Acid Residue | Abbreviation |
|---|---|
| Amino Acid Residue Derivatives | |
| 2-Aminoadipic acid | Aad |
| 3-Aminoadipic acid | bAad |
| beta-Alanine, beta-Aminoproprionic acid | bAla |
| 2-Aminobutyric acid | Abu |
| 4-Aminobutyric acid, Piperidinic acid | 4Abu |
| 6-Aminocaproic acid | Acp |
| 2-Aminoheptanoic acid | Ahe |
| 2-Aminoisobutyric acid | Aib |
| 3-Aminoisobutyric acid | bAib |
| 2-Aminopimelic acid | Apm |
| t-butylalanine | t-BuA |
| Citrulline | Cit |
| Cyclohexylalanine | Cha |
| 2,4-Diaminobutyric acid | Dbu |
| Desmosine | Des |
| 2,2'-Diaminopimelic acid | Dpm |
| 2,3-Diaminoproprionic acid | Dpr |
| N-Ethylglycine | EtGly |
| N-Ethylasparagine | EtAsn |
| Homoarginine | hArg |
| Homocysteine | hCys |
| Homoserine | hSer |
| Hydroxylysine | Hyl |
| Allo-Hydroxylysine | aHyl |
| 3-Hydroxyproline | 3Hyp |
| 4-Hydroxyproline | 4Hyp |
| Isodesmosine | Ide |
| allo-Isoleucine | aIle |
| Methionine sulfoxide | MSO |
| N-Methylglycine, sarcosine | MeGly |
| N-Methylisoleucine | Melle |
| 6-N-Methyllysine | MeLys |
| N-Methylvaline | MeVal |
| 2-Naphthylalanine | 2-Nal |
| Norvaline | Nva |
| Norleucine | Nle |
| Ornithine | Orn |
| 4-Chlorophenylalanine | Phe(4-Cl) |
| 2-Fluorophenylalanine | Phe(2-F) |
| 3 -Fluorophenylalanine | Phe(3-F) |
| 4-Fluorophenylalanine | Phe(4-F) |
| Phenylglycine | Phg |
| Beta-2-thienylalanine | Thi |

### SEQUENCES

Human Serum Albumin Signal Sequence
   (SEQ ID NO:1)
   ATGAAGTGGGTAACCTTTATTTCCCTTCTTTTTCTCTTTAGCTCGGCTTATTCC
Glycine-Serine Linker
   (SEQ ID NO:2)
   GGCAGCAGCGGCGGCAGC
MHC I-alpha Cytoplasmic and Transmembrane Domains 
(SEQ ID NO:3)
GTAGGAATCATTGCGGGACTTGTTCTGCTCGGGGCCGTAATAACTGGAGCGGTCGTG
GCGGCTGTGATGTGGCGACGGAAAAGCAGTGATCGGAAGGGTGGTTCATATACCCA
AGCCGCTTCTAGCGATAGCGCACAAGGATCAGATGTTTCTCTTACCGCATGTAAAGT
A
His Tag
   (SEQ ID NO:4)
   CATCACCACCATCATCACCATCAT
DENV2 WT sE
   SEQ ID NO:5
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:6
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 HCat1
   SEQ ID NO:7
      DNA 
ATGAGGTGTATCGGCATGTCCAACGTGGACTTTGTGGAGGGAGTGAGCGGCGGC
AGCTGGGTGGACATTGTGCTGGAGCATGGAAGCTGCGTGGTGACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCTACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCATTGCCGAGCCTCCCTTTGGCGACTCCTACATCATCATTGGCGTGGAGC
CCGGACAGCTCAAGCTGAACTGGTTCAAGAAG
   SEQ ID NO:8
      Protein 
MRCIGMSNVDFVEGVSGGSWVDIVLEHGSCVVTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPYSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIIAEPPFGDSYIIIGVEPGQLKLNWFKK
DENV2 HCat2
   SEQ ID NO:9
      DNA 
ATGATGTGTATCGGCATGTCCAACAGGGACTTTGTGGAGGGAGTGAGCGGCGGCA
GCTGGGTGGACATTGTGCTGGAGCATGGAAGCTGCGTGACCACGATGGCGAAAA
ACAAGCCCACCCTGGACTTCATGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:10
      Protein 
MMCIGMSNRDFVEGVSGGSWVDIVLEHGSCVTTMAKNKPTLDFMLIKTEAKQPATLR
KYCIEAKLTNTTTESRCPTQGEPSLNEEQDKRFVCKHSMVDRGWGNGCGLFGKGGIV
TCAMFRCKKNMEGKVVQPENLEYTIVITPHSGEEHAVGNDTGKHGKEIKITPQSSITEA
ELTGYGTVTMECSPRTGLDFNEMVLLQMENKAWLVHRQWFLDLPLPWLPGADTQGS
NWIQKETLVTFKNPHAKKQDVVVLGSQEGAMHTALTGATEIQMSSGNLLFTGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 HCat3
   SEQ ID NO:11
      DNA 
ATGAGGTGTATCGGCATGTCCAACAGGGACTTTGTGGAGGGAGTGAGCGGCGGC
AGCTGGGTGGACATTGTGCTGGAGCCCGGAAGCTGCGTGACCACGATGGCGAAA
AACAAGCCCACCCTGGACTTCGAGCTCATCAAGATCGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:12
      Protein 
MRCIGMSNRDFVEGVSGGSWVDIVLEPGSCVTTMAKNKPTLDFELIKIEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 HCat4
   SEQ ID NO:13
      DNA 
ATGATGTGTATCGGCATGTCCAACAGGGACTTTGTGGAGGGAGTGAGCGGCGGCA
GCTGGGTGGACATTGTGCTGGAGCATGGAAGCTGCGTGACCACGATGGCGAAAA
ACAAGCCCACCCTGGACTTCCTGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATCTGACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTATCAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:14
      Protein 
MMCIGMSNRDFVEGVSGGSWVDIVLEHGSCVTTMAKNKPTLDFLLIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNLTGKHGKEIKITPQSSITEAE
LTGYGTVTMECSPRTGLDFNEMVLLQMENKAWLVHRQWFLDLPLPWLPGADTQGSN
WIQKETLVTFKNPHAIKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 HCat5
   SEQ ID NO:15
      DNA 
ATGATGTGTATCGGCATGTCCAACAGGGACTTTGTGGAGGGAGTGAGCGGCGGCA
GCTGGGTGGACATTGTGCTGGAGCCCGGAAGCTGCGTGACCACGATGGCGAAAA
ACAAGCCCACCCTGGACTTCATGCTCATCAAGATCGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATATGACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCAGGCTTACAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:16
      Protein 
MMCIGMSNRDFVEGVSGGSWVDIVLEPGSCVTTMAKNKPTLDFMLIKIEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNMTGKHGKEIKITPQSSITEAE
LTGYGTVTMECSPRTGLDFNEMVLLQMENKAWLVHRQWFLDLPLPWLPGADTQGSN
WIQKETLVTFKNPQAYKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 HCat6
   SEQ ID NO:17
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACTTCGCCTTTCTCGTGCACAGGCAATGGTTTCTGGATCTGCCTCTGCC
TTGGCTGCCTGGCGCCGATACACAGGGCTCCAACTGGATACAGAAAGAGACCCTC
GTCACCTTCAAGAATCCCCATGCTAAGAAGCAGGACGTGGTGGTGCTGGGCAGCC
AAGAAGGCGCCATGCTGACAGCCCTGACCGGAGCTACCGAGATCCAGATGAGCT
CCGGCAACCTGCTGTTCACCGGCCATCTGAAATGTAGGCTGAGGATGGATAAGCT
GCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:18
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENFAFLVHRQWFLDLPLPWLPGADTQGSN
WIQKETLVTFKNPHAKKQDVVVLGSQEGAMLTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 HCat7
   SEQ ID NO:19
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACTTCGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGTTCGTGCTGGGCAGC
CAAGAAGGCGCCATGTTCACAGCCCTGACCGGAGCTACCGAGATCCAGATGAGCT
CCGGCAACCTGCTGTTCACCGGCCATCTGAAATGTAGGCTGAGGATGGATAAGCT
GCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:20
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENFAWLVHRQWFLDLPLPWLPGADTQGSN
WIQKETLVTFKNPHAKKQDVFVLGSQEGAMFTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 HCat8
   SEQ ID NO:21
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACTTCGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCTGACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:22
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENFAWLVHRQWFLDLPLPWLPGADTQGSN
WIQKETLVTFKNPHAKKQDVVVLGSQEGAMLTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 IntFc1
   SEQ ID NO:23
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGTGATGCACCACGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:24
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGVMHHALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 IntFc2
   SEQ ID NO:25
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCTGGATGCACCGGGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:26
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLFTGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 IntFc3
   SEQ ID NO:27
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGTGATGCACCGGTGGCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:28
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGVMHRWLTGATEIQMSSGNLLFTGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 IntFc4
   SEQ ID NO:29
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCTGGATGCACTACCTCCTGACCGGAGCTACCGAGATCCAGATGAGCT
CCGGCAACCTGCTGTTCACCGGCCATCTGAAATGTAGGCTGAGGATGGATAAGCT
GCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:30
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHYLLTGATEIQMSSGNLLFTGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 IntFc5
   SEQ ID NO:31
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACCGGTGGCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:32
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHRWLTGATEIQMSSGNLLFTGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 IntFc6
   SEQ ID NO:33
      DNA 
ATGAGGTGTATCGGCATGTCCCTGAGGGACTTTGTGGAGGGAGTGAGCGGCGGC
AGCTGGGTGGACATTGTGCTGGAGCATGGATACTGCGTGACCACGATGGCGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCTTCGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:34
      Protein 
MRCIGMSLRDFVEGVSGGSWVDIVLEHGYCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPFAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 IntFc7
   SEQ ID NO:35
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACCGGGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:36
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHRALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 IntFc8
   SEQ ID NO:37
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGACCTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:38
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 Mnmer1
   SEQ ID NO:39
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCGAA
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:40
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGEQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 Mnmer2
   SEQ ID NO:41
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGAGGCCATGCACACAGCCCTGACCGGAGCTACCGAGATCCAGATGAGCT
CCGGCAACCTGCTGTTCACCGGCCATCTGAAATGTAGGCTGAGGATGGATAAGCT
GCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:42
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEEAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 PM1
   SEQ ID NO:43
      DNA 
ATGAGGTGTATCGGCCTGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACAATGCACGGCACCATCGTGATCAGGGTGCAGTATGAGGGCG
ACGGCTCCCCCTGTAAGATCCCCTTCGAAATCATGGACCTGGAAAAGAGGCACGT
GCTGGGCAGGCTCATCACCGTGAACCCCATTGTCACAGAGAAAGACTCCCCCGTG
AACATCGAGGCCGAGCCTCCCTTTGGCGACTCCTACATCATCATTGGCGTGGAGC
CCGGACAGCTCAAGCTGAACTGGTTCAAGAAG
   SEQ ID NO:44
      Protein 
MRCIGLSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETMHGTIVIRVQYEGDGSPCKIPFEIMDLEKRHV
LGRLITVNPIVTEKDSPVNIEAEPPFGDSYIIIGVEPGQLKLNWFKK
DENV2 PM2
   SEQ ID NO:45
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGATCCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCCTGTCCATGTGCACCGGAAAGTTCAAGGTGGTGAA
AGAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:46 Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVIPENLEYTIVITPHSGEEHAVGNDTGKHGKEIKITPQSSITEAEL
TGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSLSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 PM3
   SEQ ID NO:47
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGTGGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:48
      Protein 
MRCIGMSNRDFVEGVSGGSWVDIVLEHGSCVTTMMKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
WDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 PM4
   SEQ ID NO:49
      DNA 
ATGAGGTGTATCGGCATGTCCAACAGGGACTTTGTGGAGGGAGTGAGCGGCGGC
AGCTGGGTGGACATTGTGCTGGAGCATGGAAAGTGCGTGACCGTGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:50
      Protein 
MRCIGMSNRDFVEGVSGGSWVDIVLEHGKCVTVMMKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 PM4.1
   SEQ ID NO:51
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:52
      Protein 
MRCIGMSNRDFVEGVSGGSWVDIVLEHGSCVTTMMKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 PM4.2
   SEQ ID NO:53
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCGTGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:54
      Protein 
MRCIGMSNRDFVEGVSGGSWVDIVLEHGSCVTVMMKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 PM4.3
   SEQ ID NO:55
      DNA 
ATGAGGTGTATCGGCATGTCCAACAGGGACTTTGTGGAGGGAGTGAGCGGCGGC
AGCTGGGTGGACATTGTGCTGGAGCATGGAAAGTGCGTGACCACGATGGCGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:56
      Protein 
MRCIGMSNRDFVEGVSGGSWVDIVLEHGKCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 PM5
   SEQ ID NO:57
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGGACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCTGGGCTACCGAGATCCAGATGAGCT
CCGGCAACCTGCTGTTCACCGGCCATCTGAAATGTAGGCTGAGGATGGATAAGCT
GCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:58
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVDRQWFLDLPLPWLPGADTQGSN
WIQKETLVTFKNPHAKKQDVVVLGSQEGAMHTALTWATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 PM6
   SEQ ID NO:59
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCCTGTCCTACATCATCATTGGCGTGGAG
CCCGGACAGCTCAAGCTGCAGTGGTTCAAGAAG
   SEQ ID NO:60
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGLSYIIIGVEPGQLKLQWFKK
DENV2 PM7
   SEQ ID NO:61
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGCCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:62
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDASPCKIPFEIMDLEKRHV
LGRLITVNPIVTEKDSPVNIEAEPPFGDSYIIIGVEPGQLKLNWFKK
DENV2 PM8
   SEQ ID NO:63
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCGTGATCATCATTGGCGTGGAG
CCCGGACAGCTCAAGCTGAACTGGTTCCGGAAG
   SEQ ID NO:64
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSVIIIGVEPGQLKLNWFRK
DENV2 SHP1
   SEQ ID NO:65
      DNA 
ATGAGGTGTATCGGCATGTCCAACAGGGACTTTGTGGAGGGAGAGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCGACGGCGACTCCTACATCATCATTGGCGTGGAG
CCCGGACAGCTCAAGCTGAACTGGAGAAAGAAG
   SEQ ID NO:66
      Protein 
MRCIGMSNRDFVEGESGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPDGDSYIIIGVEPGQLKLNWRKK
DENV2 SHP2
   SEQ ID NO:67
      DNA 
ATGAGGTGTATCGGCATGTCCAACAGGGACTTTGTGGAGGGAGAGAGCGGCGGC
AGCCACGTGGACATTGTGCTGGAGCATGGAAGCTGCGTGACCACGATGGCGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCGACGGCGACTCCTACATCATCATTGGCGTGGAGCC
CGGACAGCTCAAGCTGAACTGGCGGAAGAAG
   SEQ ID NO:68
      Protein 
MRCIGMSNRDFVEGESGGSHVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPDGDSYIIIGVEPGQLKLNWRKK
DENV2 UndPk1
   SEQ ID NO:69
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGGTGCAGATGAGC
TCCGGCAACCTGCTGTTCGCCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:70
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEVQMSSGNLLFAGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 UndPk2
   SEQ ID NO:71
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCTGGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:72
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVWLQMENKAWLVHRQWFLDLPLPWLPGADTQGS
NWIQKETLVTFKNPHAKKQDVVVLGSQEGAMHTALTGATEIQMSSGNLLFTGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 UndPk3
   SEQ ID NO:73
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGATACGATTTCAACGAGATGGTCCTGCTACAG
ATGGAGAACAAGGCCTGGCTCGTGGACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:74
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGYDFNEMVLLQMENKAWLVDRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 UndPk4
   SEQ ID NO:75
      DNA 
ATGAGGTGTATCGGCATGTCCAACAGGGACTTTGTGTTCGCCGTGAGCGGCGGCA
GCTGGGTGGACATTGTGCTGGAGCATGGAAGCTGCGTGACCACGCTGGCGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:76
      Protein 
MRCIGMSNRDFVFAVSGGSWVDIVLEHGSCVTTLAKNKPTLDFELIKTEAKQPATLRKY
CIEAKLTNTTTESRCPTQGEPSLNEEQDKRFVCKHSMVDRGWGNGCGLFGKGGIVTC
AMFRCKKNMEGKVVQPENLEYTIVITPHSGEEHAVGNDTGKHGKEIKITPQSSITEAEL
TGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 UndPk5
   SEQ ID NO:77
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCTACGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:78
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVYEKDSPVNIEAEPPFGDSYIIIGVEPGQLKLNWFKK
DENV2 UndPk6
   SEQ ID NO:79
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTGGCCCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:80
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 UndPk6.1
   SEQ ID NO:81
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCCCCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:82
      Protein 
SMRCIGMSNRDFVEGVSGGSWVDIVLEHGSCVTTMAKNKPTLDFELIKTEAKQPATLR
KYCIEAKLTNTTTESRCPTQGEPSLNEEQDKRFVCKHSMVDRGWGNGCGLFGKGGIV
TCAMFRCKKNMEGKVVQPENLEYTIVITPHSGEEHAVGNDTGKHGKEIKITPQSSITEA
ELTGYGTVTMECSPRTGLDFNEMVLLQMENKAWLVHRQWFLDLPLPWLPGADTQGS
NWIQKETLVTFKNPHAKKQDVVVLGSQEGAMHTALTGATEIQMSSGNLLFPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 UndPk7
   SEQ ID NO:83
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGATCGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACATGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:84
      Protein 
MRCIGMSNRDFVEGVSGGSWVDIVLEHGSCVTTMAKNKPTLDFELIKIEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNMLFTGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 UndPk8
   SEQ ID NO:85
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
TACGAAGCCTGGCTGCACACAGCCCTGACCGGAGCTACCGAGATCCAGATGAGCT
CCGGCAACCTGCTGTTCACCGGCCATCTGAAATGTAGGCTGAGGATGGATAAGCT
GCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:86
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSYEAWLHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 Cm1
   SEQ ID NO:87
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCTGTATGCACACAGCCCTGACCGGAGCTACCGAGATCCAGATGAGCT
CCGGCAACCTGCTGTTCACCGGCCATCTGAAATGTAGGCTGAGGATGGATAAGCT
GCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:88
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGCMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 Cm2
   SEQ ID NO:89
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGATGTTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCTGTGAAACACAGCACGGCACCATCGTGATCAGGGTGCAGTATGAGGGCG
ACGGCTCCCCCTGTAAGATCCCCTTCGAAATCATGGACCTGGAAAAGAGGCACGT
GCTGGGCAGGCTCATCACCGTGAACCCCATTGTCACAGAGAAAGACTCCCCCGTG
AACATCGAGGCCGAGCCTCCCTTTGGCGACTCCTACATCATCATTGGCGTGGAGC
CCGGACAGCTCAAGCTGAACTGGTTCAAGAAG
   SEQ ID NO:90
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGCFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEICETQHGTIVIRVQYEGDGSPCKIPFEIMDLEKRHV
LGRLITVNPIVTEKDSPVNIEAEPPFGDSYIIIGVEPGQLKLNWFKK
DENV2 SC.1 (I2-I8-P4)
   SEQ ID NO:91
      DNA 
ATGAGGTGTATCGGCATGTCCAACAGGGACTTTGTGGAGGGAGTGAGCGGCGGC
AGCTGGGTGGACATTGTGCTGGAGCATGGAAAGTGCGTGACCGTGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:92
      Protein 
MRCIGMSNRDFVEGVSGGSWVDIVLEHGKCVTVMMKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLFTGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.2 (I2-I8-P4-H3)
   SEQ ID NO:93
      DNA 
ATGAGGTGTATCGGCATGTCCAACAGGGACTTTGTGGAGGGAGTGAGCGGCGGC
AGCTGGGTGGACATTGTGCTGGAGCCCGGAAAGTGCGTGACCGTGATGATGAAAA
ACAAGCCCACCCTGGACTTCGAGCTCATCAAGATCGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:94
      Protein 
MRCIGMSNRDFVEGVSGGSWVDIVLEPGKCVTVMMKNKPTLDFELIKIEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLFTGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.3 (I2-I8-U4-U5-U6-P5)
   SEQ ID NO:95
      DNA 
ATGAGGTGTATCGGCATGTCCAACAGGGACTTTGTGTTCGCCGTGAGCGGCGGCA
GCTGGGTGGACATTGTGCTGGAGCATGGAAGCTGCGTGACCACGCTGGCGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGGACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCTGGGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCTACGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:96
      Protein 
MRCIGMSNRDFVFAVSGGSWVDIVLEHGSCVTTLAKNKPTLDFELIKTEAKQPATLRKY
CIEAKLTNTTTESRCPTQGEPSLNEEQDKRFVCKHSMVDRGWGNGCDLFGKGGIVTC
AMFRCKKNMEGKVVQPENLEYTIVITPHSGEEHAVGNDTGKHGKEIKITPQSSITEAEL
TGYGTVTMECSPRTGLDFNEMVLLQMENKAWLVDRQWFLDLPLPWLPGADTQGSN
WIQKETLVTFKNPHAKKQDVVVLGSQEGWMHRALTWATEIQMSSGNLLWPGHLKCR
LRMDKLQLKGMSYSMCTGKFKVVKEIAETQHGTIVIRVQYEGDGSPCKIPFEIMDLEKR
HVLGRLITVNPIVYEKDSPVNIEAEPPFGDSYIIIGVEPGQLKLNWFKK
DENV2 SC.4 (I2-I8-U4-U5-U6-P4-P5-H3)
   SEQ ID NO:97
      DNA 
ATGAGGTGTATCGGCATGTCCAACAGGGACTTTGTGTTCGCCGTGAGCGGCGGCA
GCTGGGTGGACATTGTGCTGGAGCCCGGAAAGTGCGTGACCGTGCTGATGAAAAA
CAAGCCCACCCTGGACTTCGAGCTCATCAAGATCGAGGCTAAACAGCCCGCCACC
CTCAGGAAGTACTGCATCGAGGCCAAGCTGACCAACACAACAACCGAGTCCAGAT
GCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGGACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCTGGGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCTACGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:98
      Protein 
MRCIGMSNRDFVFAVSGGSWVDIVLEPGKCVTVLMKNKPTLDFELIKIEAKQPATLRKY
CIEAKLTNTTTESRCPTQGEPSLNEEQDKRFVCKHSMVDRGWGNGCDLFGKGGIVTC
AMFRCKKNMEGKVVQPENLEYTIVITPHSGEEHAVGNDTGKHGKEIKITPQSSITEAEL
TGYGTVTMECSPRTGLDFNEMVLLQMENKAWLVDRQWFLDLPLPWLPGADTQGSN
WIQKETLVTFKNPHAKKQDVVVLGSQEGWMHRALTWATEIQMSSGNLLWPGHLKCR
LRMDKLQLKGMSYSMCTGKFKVVKEIAETQHGTIVIRVQYEGDGSPCKIPFEIMDLEKR
HVLGRLITVNPIVYEKDSPVNIEAEPPFGDSYIIIGVEPGQLKLNWFKK
DENV2 SC.5 (I2-I8-U4-U6)
   SEQ ID NO:99
      DNA 
ATGAGGTGTATCGGCATGTCCAACAGGGACTTTGTGTTCGCCGTGAGCGGCGGCA
GCTGGGTGGACATTGTGCTGGAGCATGGAAGCTGCGTGACCACGCTGGCGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCGGAGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:100
      Protein 
MRCIGMSNRDFVFAVSGGSWVDIVLEHGSCVTTLAKNKPTLDFELIKTEAKQPATLRKY
CIEAKLTNTTTESRCPTQGEPSLNEEQDKRFVCKHSMVDRGWGNGCDLFGKGGIVTC
AMFRCKKNMEGKVVQPENLEYTIVITPHSGEEHAVGNDTGKHGKEIKITPQSSITEAEL
TGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.6 (I2-I8-U5-P4)
   SEQ ID NO:101
      DNA 
ATGAGGTGTATCGGCATGTCCAACAGGGACTTTGTGGAGGGAGTGAGCGGCGGC
AGCTGGGTGGACATTGTGCTGGAGCATGGAAAGTGCGTGACCGTGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCTACGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:102
      Protein 
MRCIGMSNRDFVEGVSGGSWVDIVLEHGKCVTVMMKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLFTGHLKCRL
RMDKLQLKGMSYSMCTGKFKVVKEIAETQHGTIVIRVQYEGDGSPCKIPFEIMDLEKRH
VLGRLITVNPIVYEKDSPVNIEAEPPFGDSYIIIGVEPGQLKLNWFKK
DENV2 SC.7 (I2-I8-U5-U6)
   SEQ ID NO:103
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGACCTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCTGGATGCACCGGGCCCTGACCGGAGCTACCGAGATCCAGATGAGC
TCCGGCAACCTGCTGTGGCCCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCTACGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:104
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVYEKDSPVNIEAEPPFGDSYIIIGVEPGQLKLNWFKK
DENV2 SC.8 (I2-I8-U5-U6-P4)
   SEQ ID NO:105
      DNA 
ATGAGGTGTATCGGCATGTCCAACAGGGACTTTGTGGAGGGAGTGAGCGGCGGC
AGCTGGGTGGACATTGTGCTGGAGCATGGAAAGTGCGTGACCGTGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCGGAGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCTACGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:106
      Protein 
MRCIGMSNRDFVEGVSGGSWVDIVLEHGKCVTVMMKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVYEKDSPVNIEAEPPFGDSYIIIGVEPGQLKLNWFKK
DENV2 SC.9 (I2-I8-U5-U6-P4-S1)
   SEQ ID NO:107
      DNA 
ATGAGGTGTATCGGCATGTCCAACAGGGACTTTGTGGAGGGAGAGAGCGGCGGC
AGCTGGGTGGACATTGTGCTGGAGCATGGAAAGTGCGTGACCGTGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCGGAGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCTACGAGAAAGACTCCCCCGTGA
ACATCGAGGCCGAGCCTCCCGACGGCGACTCCTACATCATCATTGGCGTGGAGCC
CGGACAGCTCAAGCTGAACTGGAGAAAGAAG
   SEQ ID NO:108
      Protein 
MRCIGMSNRDFVEGESGGSWVDIVLEHGKCVTVMMKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVYEKDSPVNIEAEPPDGDSYIIIGVEPGQLKLNWRKK
DENV2 SC.10 (I2-I8-U6)
   SEQ ID NO:109
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGACCTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCTGGATGCACCGGGCCCTGACCGGAGCTACCGAGATCCAGATGAGC
TCCGGCAACCTGCTGTGGCCCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:110
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.11 (I2-I8-U6-H3)
   SEQ ID NO:111
      DNA 
ATGAGGTGTATCGGCATGTCCAACAGGGACTTTGTGGAGGGAGTGAGCGGCGGC
AGCTGGGTGGACATTGTGCTGGAGCCCGGAAGCTGCGTGACCACGATGGCGAAA
AACAAGCCCACCCTGGACTTCGAGCTCATCAAGATCGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGACCTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCTGGATGCACCGGGCCCTGACCGGAGCTACCGAGATCCAGATGAGC
TCCGGCAACCTGCTGTGGCCCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   Protein
      SEQ ID NO:112 
MRCIGMSNRDFVEGVSGGSWVDIVLEPGSCVTTMAKNKPTLDFELIKIEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.12 (I2-I8-U6-P4)
   DNA
      SEQ ID NO:113 
ATGAGGTGTATCGGCATGTCCAACAGGGACTTTGTGGAGGGAGTGAGCGGCGGC
AGCTGGGTGGACATTGTGCTGGAGCATGGAAAGTGCGTGACCGTGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCGGAGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   SEQ ID NO:114
      Protein 
MRCIGMSNRDFVEGVSGGSWVDIVLEHGKCVTVMMKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.13 (I2-U5-U6-P4)
   DNA
      SEQ ID NO:115 
ATGAGGTGTATCGGCATGTCCAACAGGGACTTTGTGGAGGGAGTGAGCGGCGGC
AGCTGGGTGGACATTGTGCTGGAGCATGGAAAGTGCGTGACCGTGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCGGAGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCTACGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   Protein
      SEQ ID NO:116 
MRCIGMSNRDFVEGVSGGSWVDIVLEHGKCVTVMMKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVYEKDSPVNIEAEPPFGDSYIIIGVEPGQLKLNWFKK
DENV2 SC.14 (I2-U6)
   SEQ ID NO:117
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCTGGATGCACCGGGCCCTGACCGGAGCTACCGAGATCCAGATGAGC
TCCGGCAACCTGCTGTGGCCCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   SEQ ID NO:118
      Protein 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.15 (I8-U6)
   DNA
      SEQ ID NO:119 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGACCTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGCCATGCACACAGCCCTGACCGGAGCTACCGAGATCCAGATGAGC
TCCGGCAACCTGCTGTGGCCCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   Protein
      SEQ ID NO:120 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.16 (I2-U6-H3)
   DNA
      SEQ ID NO:121 
ATGAGGTGTATCGGCATGTCCAACAGGGACTTTGTGGAGGGAGTGAGCGGCGGC
AGCTGGGTGGACATTGTGCTGGAGCCCGGAAGCTGCGTGACCACGATGGCGAAA
AACAAGCCCACCCTGGACTTCGAGCTCATCAAGATCGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCTGGATGCACCGGGCCCTGACCGGAGCTACCGAGATCCAGATGAGC
TCCGGCAACCTGCTGTGGCCCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   Protein
      SEQ ID NO:122 
MRCIGMSNRDFVEGVSGGSWVDIVLEPGSCVTTMAKNKPTLDFELIKIEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.17 (I2-U6-P4)
   DNA SEQ ID NO:123 
ATGAGGTGTATCGGCATGTCCAACAGGGACTTTGTGGAGGGAGTGAGCGGCGGC
AGCTGGGTGGACATTGTGCTGGAGCATGGAAAGTGCGTGACCGTGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCGGAGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   Protein
      SEQ ID NO:124 
MRCIGMSNRDFVEGVSGGSWVDIVLEHGKCVTVMMKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.18 (I2-I8-U4-U6-P4)
   DNA
      SEQ ID NO:125 
ATGAGGTGTATCGGCATGTCCAACAGGGACTTTGTGTTCGCCGTGAGCGGCGGCA
GCTGGGTGGACATTGTGCTGGAGCATGGAAAGTGCGTGACCGTGCTGATGAAAAA
CAAGCCCACCCTGGACTTCGAGCTCATCAAGACAGAGGCTAAACAGCCCGCCACC
CTCAGGAAGTACTGCATCGAGGCCAAGCTGACCAACACAACAACCGAGTCCAGAT
GCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCTGGATGCACCGGGCCCTGACCGGAGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   Protein
      SEQ ID NO:126 
MRCIGMSNRDFVFAVSGGSWVDIVLEHGKCVTVLMKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.19 (I8-U5-U6-P4)
   DNA
      SEQ ID NO:127 
ATGAGGTGTATCGGCATGTCCAACAGGGACTTTGTGGAGGGAGTGAGCGGCGGC
AGCTGGGTGGACATTGTGCTGGAGCATGGAAAGTGCGTGACCGTGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCTACGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   Protein
      SEQ ID NO:128 
MRCIGMSNRDFVEGVSGGSWVDIVLEHGKCVTVMMKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVYEKDSPVNIEAEPPFGDSYIIIGVEPGQLKLNWFKK
DENV2 SC.20 (P4-U4)
   DNA
      SEQ ID NO:129 
ATGAGGTGTATCGGCATGTCCAACAGGGACTTTGTGTTCGCCGTGAGCGGCGGCA
GCTGGGTGGACATTGTGCTGGAGCATGGAAAGTGCGTGACCGTGCTGATGAAAAA
CAAGCCCACCCTGGACTTCGAGCTCATCAAGACAGAGGCTAAACAGCCCGCCACC
CTCAGGAAGTACTGCATCGAGGCCAAGCTGACCAACACAACAACCGAGTCCAGAT
GCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTTCACCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   Protein
      SEQ ID NO:130 
MRCIGMSNRDFVFAVSGGSWVDIVLEHGKCVTVLMKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 SC.21 (U4-U5-U6)
   DNA
      SEQ ID NO:131 
ATGAGGTGTATCGGCATGTCCAACAGGGACTTTGTGTTCGCCGTGAGCGGCGGCA
GCTGGGTGGACATTGTGCTGGAGCATGGAAGCTGCGTGACCACGCTGGCGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCGGAGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCTACGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   Protein
      SEQ ID NO:132 
MRCIGMSNRDFVFAVSGGSWVDIVLEHGSCVTTLAKNKPTLDFELIKTEAKQPATLRKY
CIEAKLTNTTTESRCPTQGEPSLNEEQDKRFVCKHSMVDRGWGNGCGLFGKGGIVTC
AMFRCKKNMEGKVVQPENLEYTIVITPHSGEEHAVGNDTGKHGKEIKITPQSSITEAEL
TGYGTVTMECSPRTGLDFNEMVLLQMENKAWLVHRQWFLDLPLPWLPGADTQGSN
WIQKETLVTFKNPHAKKQDVVVLGSQEGAMHTALTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVYEKDSPVNIEAEPPFGDSYIIIGVEPGQLKLNWFKK
DENV2 SC.22 (U4-U5-U6-P5)
   DNA
      SEQ ID NO:133 
ATGAGGTGTATCGGCATGTCCAACAGGGACTTTGTGTTCGCCGTGAGCGGCGGCA
GCTGGGTGGACATTGTGCTGGAGCATGGAAGCTGCGTGACCACGCTGGCGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGGACTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGGACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGCCATGCACACAGCCCTGACCTGGGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCTACGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   Protein
      SEQ ID NO:134 
MRCIGMSNRDFVFAVSGGSWVDIVLEHGSCVTTLAKNKPTLDFELIKTEAKQPATLRKY
CIEAKLTNTTTESRCPTQGEPSLNEEQDKRFVCKHSMVDRGWGNGCGLFGKGGIVTC
AMFRCKKNMEGKVVQPENLEYTIVITPHSGEEHAVGNDTGKHGKEIKITPQSSITEAEL
TGYGTVTMECSPRTGLDFNEMVLLQMENKAWLVDRQWFLDLPLPWLPGADTQGSN
WIQKETLVTFKNPHAKKQDVVVLGSQEGAMHTALTWATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVYEKDSPVNIEAEPPFGDSYIIIGVEPGQLKLNWFKK
DENV2 SC.23 (12-18)
   DNA
      SEQ ID NO:135 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGACCTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCTGGATGCACCGGGCCCTGACCGGAGCTACCGAGATCCAGATGAGC
TCCGGCAACCTGCTGTTCACCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   Protein
      SEQ ID NO:136 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGWMHRALTGATEIQMSSGNLLFTGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.24 (M2-H3)
   DNA
      SEQ ID NO:137 
ATGAGGTGTATCGGCATGTCCAACAGGGACTTTGTGGAGGGAGTGAGCGGCGGC
AGCTGGGTGGACATTGTGCTGGAGCCCGGAAGCTGCGTGACCACGATGGCGAAA
AACAAGCCCACCCTGGACTTCGAGCTCATCAAGATCGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGAGGCCATGCACACAGCCCTGACCGGAGCTACCGAGATCCAGATGAGCT
CCGGCAACCTGCTGTTCACCGGCCATCTGAAATGTAGGCTGAGGATGGATAAGCT
GCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   Protein
      SEQ ID NO:138 
MRCIGMSNRDFVEGVSGGSWVDIVLEPGSCVTTMAKNKPTLDFELIKIEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEEAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 SC.25 (M2-P4)
   SEQ ID NO:139
      DNA 
CATGAGGTGTATCGGCATGTCCAACAGGGACTTTGTGGAGGGAGTGAGCGGCGG
CAGCTGGGTGGACATTGTGCTGGAGCATGGAAAGTGCGTGACCGTGATGATGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGGACTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGAGGCCATGCACACAGCCCTGACCGGAGCTACCGAGATCCAGATGAGCT
CCGGCAACCTGCTGTTCACCGGCCATCTGAAATGTAGGCTGAGGATGGATAAGCT
GCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   Protein
      SEQ ID NO:140 
MRCIGMSNRDFVEGVSGGSWVDIVLEHGKCVTVMMKNKPTLDFELIKTEAKQPATLRK
YCIEAKLTNTTTESRCPTQGEPSLNEEQDKRFVCKHSMVDRGWGNGCGLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEEAMHTALTGATEIQMSSGNLLFTGHLKCRLR
MDKLQLKGMSYSMCTGKFKVVKEIAETQHGTIVIRVQYEGDGSPCKIPFEIMDLEKRHV
LGRLITVNPIVTEKDSPVNIEAEPPFGDSYIIIGVEPGQLKLNWFKK
DENV2 SC.26 (I3-I8-U6)
   SEQ ID NO:141
      DNA 
ATGAGGTGTATCGGCATGTCCAACAGGGACTTTGTGGAGGGAGTGAGCGGCGGC
AGCTGGGTGGACATTGTGCTGGAGCATGGAAGCTGCGTGACCACGATGGCGAAA
AACAAGCCCACCCTGGACTTCGAGCTCATCAAGACAGAGGCTAAACAGCCCGCCA
CCCTCAGGAAGTACTGCATCGAGGCCAAGCTGACCAACACAACAACCGAGTCCAG
ATGCCCTACACAGGGCGAACCCAGCCTCAACGAAGAGCAGGACAAGAGGTTCGT
GTGCAAACACAGCATGGTGGACAGGGGCTGGGGCAATGGATGCGACCTCTTCGG
AAAAGGCGGCATCGTGACCTGCGCCATGTTCAGGTGTAAAAAGAACATGGAAGGC
AAGGTGGTGCAGCCCGAAAATCTGGAGTATACCATCGTGATTACCCCCCACAGCG
GAGAGGAGCACGCCGTGGGCAATGACACCGGCAAGCACGGCAAAGAGATTAAGA
TCACCCCCCAGTCCTCCATTACCGAAGCTGAACTGACAGGCTACGGCACCGTGAC
AATGGAGTGTAGCCCCAGGACCGGACTGGATTTCAACGAGATGGTCCTGCTACAG
ATGGAGAACAAGGCCTGGCTCGTGCACAGGCAATGGTTTCTGGATCTGCCTCTGC
CTTGGCTGCCTGGCGCCGATACACAGGGCTCCAACTGGATACAGAAAGAGACCCT
CGTCACCTTCAAGAATCCCCATGCTAAGAAGCAGGACGTGGTGGTGCTGGGCAGC
CAAGAAGGCGTGATGCACCGGTGGCTGACCGGAGCTACCGAGATCCAGATGAGC
TCCGGCAACCTGCTGTGGCCCGGCCATCTGAAATGTAGGCTGAGGATGGATAAGC
TGCAACTCAAAGGCATGTCCTACTCCATGTGCACCGGAAAGTTCAAGGTGGTGAAA
GAAATCGCCGAAACACAGCACGGCACCATCGTGATCAGGGTGCAGTATGAGGGC
GACGGCTCCCCCTGTAAGATCCCCTTCGAAATCATGGACCTGGAAAAGAGGCACG
TGCTGGGCAGGCTCATCACCGTGAACCCCATTGTCACAGAGAAAGACTCCCCCGT
GAACATCGAGGCCGAGCCTCCCTTTGGCGACTCCTACATCATCATTGGCGTGGAG
CCCGGACAGCTCAAGCTGAACTGGTTCAAGAAG
   Protein
      SEQ ID NO:142 
MRCIGMSNRDFVEGVSGGSWVDIVLEHGSCVTTMAKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGVMHRWLTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.27 (I3-I8-U6-P4)
   SEQ ID NO:143
      DNA 
ATGAGGTGTATCGGCATGTCCAACAGGGACTTTGTGGAGGGAGTGAGCGGCGGC
AGCTGGGTGGACATTGTGCTGGAGCATGGAAAGTGCGTGACCGTGATGATGAAAA
ACAAGCCCACCCTGGACTTCGAGCTCATCAAGACAGAGGCTAAACAGCCCGCCAC
CCTCAGGAAGTACTGCATCGAGGCCAAGCTGACCAACACAACAACCGAGTCCAGA
TGCCCTACACAGGGCGAACCCAGCCTCAACGAAGAGCAGGACAAGAGGTTCGTGT
GCAAACACAGCATGGTGGACAGGGGCTGGGGCAATGGATGCGACCTCTTCGGAA
AAGGCGGCATCGTGACCTGCGCCATGTTCAGGTGTAAAAAGAACATGGAAGGCAA
GGTGGTGCAGCCCGAAAATCTGGAGTATACCATCGTGATTACCCCCCACAGCGGA
GAGGAGCACGCCGTGGGCAATGACACCGGCAAGCACGGCAAAGAGATTAAGATC
ACCCCCCAGTCCTCCATTACCGAAGCTGAACTGACAGGCTACGGCACCGTGACAA
TGGAGTGTAGCCCCAGGACCGGACTGGATTTCAACGAGATGGTCCTGCTACAGAT
GGAGAACAAGGCCTGGCTCGTGCACAGGCAATGGTTTCTGGATCTGCCTCTGCCT
TGGCTGCCTGGCGCCGATACACAGGGCTCCAACTGGATACAGAAAGAGACCCTCG
TCACCTTCAAGAATCCCCATGCTAAGAAGCAGGACGTGGTGGTGCTGGGCAGCCA
AGAAGGCGTGATGCACCGGTGGCTGACCGGAGCTACCGAGATCCAGATGAGCTC
CGGCAACCTGCTGTGGCCCGGCCATCTGAAATGTAGGCTGAGGATGGATAAGCTG
CAACTCAAAGGCATGTCCTACTCCATGTGCACCGGAAAGTTCAAGGTGGTGAAAG
AAATCGCCGAAACACAGCACGGCACCATCGTGATCAGGGTGCAGTATGAGGGCGA
CGGCTCCCCCTGTAAGATCCCCTTCGAAATCATGGACCTGGAAAAGAGGCACGTG
CTGGGCAGGCTCATCACCGTGAACCCCATTGTCACAGAGAAAGACTCCCCCGTGA
ACATCGAGGCCGAGCCTCCCTTTGGCGACTCCTACATCATCATTGGCGTGGAGCC
CGGACAGCTCAAGCTGAACTGGTTCAAGAAG
   Protein
      SEQ ID NO:144 
MRCIGMSNRDFVEGVSGGSWVDIVLEHGKCVTVMMKNKPTLDFELIKTEAKQPATLRK
YCIEAKLTNTTTESRCPTQGEPSLNEEQDKRFVCKHSMVDRGWGNGCDLFGKGGIVT
CAMFRCKKNMEGKVVQPENLEYTIVITPHSGEEHAVGNDTGKHGKEIKITPQSSITEAE
LTGYGTVTMECSPRTGLDFNEMVLLQMENKAWLVHRQWFLDLPLPWLPGADTQGSN
WIQKETLVTFKNPHAKKQDVVVLGSQEGVMHRWLTGATEIQMSSGNLLWPGHLKCRL
RMDKLQLKGMSYSMCTGKFKVVKEIAETQHGTIVIRVQYEGDGSPCKIPFEIMDLEKRH
VLGRLITVNPIVTEKDSPVNIEAEPPFGDSYIIIGVEPGQLKLNWFKK
DENV2 SC.28 (P4-Cm2)
   DNA
      SEQ ID NO:145 
ATGAGGTGTATCGGCATGTCCAACAGGGACTTTGTGGAGGGAGTGAGCGGCG
GCAGCTGGGTGGACATTGTGCTGGAGCATGGAAAGTGCGTGACCGTGATGATG
AAAAACAAGCCCACCCTGGACTTCGAGCTCATCAAGACAGAGGCTAAACAGCC
CGCCACCCTCAGGAAGTACTGCATCGAGGCCAAGCTGACCAACACAACAACCG
AGTCCAGATGCCCTACACAGGGCGAACCCAGCCTCAACGAAGAGCAGGACAAG
AGGTTCGTGTGCAAACACAGCATGGTGGACAGGGGCTGGGGCAATGGATGCG
GATGTTTCGGAAAAGGCGGCATCGTGACCTGCGCCATGTTCAGGTGTAAAAAG
AACATGGAAGGCAAGGTGGTGCAGCCCGAAAATCTGGAGTATACCATCGTGAT
TACCCCCCACAGCGGAGAGGAGCACGCCGTGGGCAATGACACCGGCAAGCAC
GGCAAAGAGATTAAGATCACCCCCCAGTCCTCCATTACCGAAGCTGAACTGACA
GGCTACGGCACCGTGACAATGGAGTGTAGCCCCAGGACCGGACTGGATTTCAA
CGAGATGGTCCTGCTACAGATGGAGAACAAGGCCTGGCTCGTGCACAGGCAAT
GGTTTCTGGATCTGCCTCTGCCTTGGCTGCCTGGCGCCGATACACAGGGCTCC
AACTGGATACAGAAAGAGACCCTCGTCACCTTCAAGAATCCCCATGCTAAGAAG
CAGGACGTGGTGGTGCTGGGCAGCCAAGAAGGCGCCATGCACACAGCCCTGA
CCGGAGCTACCGAGATCCAGATGAGCTCCGGCAACCTGCTGTTCACCGGCCAT
CTGAAATGTAGGCTGAGGATGGATAAGCTGCAACTCAAAGGCATGTCCTACTCC
ATGTGCACCGGAAAGTTCAAGGTGGTGAAAGAAATCTGTGAAACACAGCACGG
CACCATCGTGATCAGGGTGCAGTATGAGGGCGACGGCTCCCCCTGTAAGATCC
CCTTCGAAATCATGGACCTGGAAAAGAGGCACGTGCTGGGCAGGCTCATCACC
GTGAACCCCATTGTCACAGAGAAAGACTCCCCCGTGAACATCGAGGCCGAGCC
TCCCTTTGGCGACTCCTACATCATCATTGGCGTGGAGCCCGGACAGCTCAAGC
TGAACTGGTTCAAGAAG
   Protein
      SEQ ID NO:146 
MRCIGMSNRDFVEGVSGGSWVDIVLEHGKCVTVMMKNKPTLDFELIKTEAKQPATL
RKYCIEAKLTNTTTESRCPTQGEPSLNEEQDKRFVCKHSMVDRGWGNGCGCFGK
GGIVTCAMFRCKKNMEGKVVQPENLEYTIVITPHSGEEHAVGNDTGKHGKEIKITPQ
SSITEAELTGYGTVTMECSPRTGLDFNEMVLLQMENKAWLVHRQWFLDLPLPWLP
GADTQGSNWIQKETLVTFKNPHAKKQDVVVLGSQEGAMHTALTGATEIQMSSGNL
LFTGHLKCRLRMDKLQLKGMSYSMCTGKFKVVKEICETQHGTIVIRVQYEGDGSPC
KIPFEIMDLEKRHVLGRLITVNPIVTEKDSPVNIEAEPPFGDSYIIIGVEPGQLKLNWFK
K
DENV1 sE WT
   SEQ ID NO: 147 
MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCGLFGKG
SLITCAKFKCVTKLEGKIVQYENLKYSVIVTVHTGDQHQVGNETTEHGTTATITPQAP
TSEIQLTDYGALTLDCSPRTGLDFNEMVLLTMEKKSWLVHKQWFLDLPLPWTSGAS
TSQETWNRQDLLVTFKTAHAKKQEVVVLGSQEGAMHTALTGATEIQTSGTTTIFAG
HLKCRLKMDKLTLKGMSYVMCTGSFKLEKEVAETQHGTVLVQVKYEGTDAPCKIPF
SSQDEKGVTQNGRLITANPIVTDKEKPVNIEAEPPFGESYIVVGAGEKALKLSWFKK
DENV1 sE PM4
   SEQ ID NO:148 
MRCVGIGNRDFVEGLSGATWVDVVLEHGKCVTVMMKDKPTLDIELLKTEVTNPAVL
RKLCIEAKISNTTTDSRCPTQGEATLVEEQDTNFVCRRTFVDRGWGNGCGLFGKG
SLITCAKFKCVTKLEGKIVQYENLKYSVIVTVHTGDQHQVGNETTEHGTTATITPQAP
TSEIQLTDYGALTLDCSPRTGLDFNEMVLLTMEKKSWLVHKQ
WFLDLPLPWTSGASTSQETWNRQDLLVTFKTAHAKKQEVVVLGSQEGAMHTALTG
ATEIQTSGTTTIFAGHLKCRLKMDKLTLKGMSYVMCTGSFKLEKEVAETQHGTVLV
QVKYEGTDAPCKIPFSSQDEKGVTQNGRLITANPIVTDKEKPVNIEAEPPFGESYIVV
GAGEKALKLSWFKK
DENV1 sE IntFc2
   SEQ ID NO:149 
MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCGLFGKG
SLITCAKFKCVTKLEGKIVQYENLKYSVIVTVHTGDQHQVGNETTEHGTTATITPQAP
TSEIQLTDYGALTLDCSPRTGLDFNEMVLLTMEKKSWLVHKQWFLDLPLPWTSGAS
TSQETWNRQDLLVTFKTAHAKKQEVVVLGSQEGWMHRALTGATEIQTSGTTTIFAG
HLKCRLKMDKLTLKGMSYVMCTGSFKLEKEVAETQHGTVLVQVKYEGTDAPCKIPF
SSQDEKGVTQNGRLITANPIVTDKEKPVNIEAEPPFGESYIVVGAGEKALKLSWFKK
DENV1 sE IntFc8
   SEQ ID NO:150 
MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCDLFGKG
SLITCAKFKCVTKLEGKIVQYENLKYSVIVTVHTGDQHQVGNETTEHGTTATITPQAP
TSEIQLTDYGALTLDCSPRTGLDFNEMVLLTMEKKSWLVHKQWFLDLPLPWTSGAS
TSQETWNRQDLLVTFKTAHAKKQEVVVLGSQEGAMHTALTGATEIQTSGTTTIFAG
HLKCRLKMDKLTLKGMSYVMCTGSFKLEKEVAETQHGTVLVQVKYEGTDAPCKIPF
SSQDEKGVTQNGRLITANPIVTDKEKPVNIEAEPPFGESYIVVGAGEKALKLSWFKK
DENV1 sE UndPk6
   SEQ ID NO:151 
MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCGLFGKG
SLITCAKFKCVTKLEGKIVQYENLKYSVIVTVHTGDQHQVGNETTEHGTTATITPQAP
TSEIQLTDYGALTLDCSPRTGLDFNEMVLLTMEKKSWLVHKQWFLDLPLPWTSGAS
TSQETWNRQDLLVTFKTAHAKKQEVVVLGSQEGAMHTALTGATEIQTSGTTTIWPG
HLKCRLKMDKLTLKGMSYVMCTGSFKLEKEVAETQHGTVLVQVKYEGTDAPCKIPF
SSQDEKGVTQNGRLITANPIVTDKEKPVNIEAEPPFGESYIVVGAGEKALKLSWFKK
DENV3 sE WT
   SEQ ID NO:152 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGAMHTALTGATEIQNSGGTSIFAGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
DENV3 sE PM4
   SEQ ID NO:153 
MRCVGVGNRDFVEGLSGATWVDVVLEHGKCVTVMMKNKPTLDIELQKTEATQLAT
LRKLCIEGKITNITTDSRCPTQGEAILPEEQDQNYVCKHTYVDRGWGNGCGLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGAMHTALTGATEIQNSGGTSIFAGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
DENV3 sE IntFc2
   SEQ ID NO:154 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGWMHRALTGATEIQNSGGTSIFAG
HLKCRLKMDKLELKGMSYAMCLNTFVLKKEVSETQHGTILIKVEYKGEDAPCKIPFS
TEDGQGKAHNGRLITANPVVTKKEEPVNIEAEPPFGESNIVIGIGDKALKINWYKK
DENV3 sE IntFc8
   SEQ ID NO:155 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGAMHTALTGATEIQNSGGTSIFAGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
DENV3 sE UndPk6
   SEQ ID NO:156 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGAMHTALTGATEIQNSGGTSIWPG
HLKCRLKMDKLELKGMSYAMCLNTFVLKKEVSETQHGTILIKVEYKGEDAPCKIPFS
TEDGQGKAHNGRLITANPVVTKKEEPVNIEAEPPFGESNIVIGIGDKALKINWYKK
DENV3 sE UndPk6 DV2Hingemut
   SEQ ID NO:157 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWLVHRQWFFDLPLPWTSGATT
ETPTWNRKELLVTFKNAHAKKQEVVVLGSQEGAMHTALTGATEIQNSGGTLIWPG
HLKCRLKMDKLELKGMSYAMCLNTFVLKKEVSETQHGTILIKVEYKGEDAPCKIPFS
TEDGQGKAHNGRLITANPVVTKKEEPVNIEAEPPFGESNIVIGIGDKALKINWYKK
DENV3 sE SC.10 (I2-I8-U6)
   SEQ ID NO:158 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGWMHRALTGATEIQNSGGTSIWPG
HLKCRLKMDKLELKGMSYAMCLNTFVLKKEVSETQHGTILIKVEYKGEDAPCKIPFS
TEDGQGKAHNGRLITANPVVTKKEEPVNIEAEPPFGESNIVIGIGDKALKINWYKK
DENV3 sE SC.14 (I2-U6)
   SEQ ID NO:159 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGWMHRALTGATEIQNSGGTSIWPG
HLKCRLKMDKLELKGMSYAMCLNTFVLKKEVSETQHGTILIKVEYKGEDAPCKIPFS
TEDGQGKAHNGRLITANPVVTKKEEPVNIEAEPPFGESNIVIGIGDKALKINWYKK
DENV3 sE UndPk6.1 (A278P)
   SEQ ID NO:160 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGAMHTALTGATEIQNSGGTSIFPGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
DENV3 sE HCat3
   SEQ ID NO:161 
MRCVGVGNRDFVEGLSGATWVDVVLEPGGCVTTMAKNKPTLDIELQKIEATQLATL
RKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
DENV3 sE SC.12 (I2-I8-U6-P4)
   SEQ ID NO:162 
MRCVGVGNRDFVEGLSGATWVDVVLEHGKCVTVMMKNKPTLDIELQKTEATQLAT
LRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGWMHRALTGATEIQNSGGTSIWPG
HLKCRLKMDKLELKGMSYAMCLNTFVLKKEVSETQHGTILIKVEYKGEDAPCKIPFS
TEDGQGKAHNGRLITANPVVTKKEEPVNIEAEPPFGESNIVIGIGDKALKINWYKK
DENV3 sE SC.29 (I8-U6-P4)
   SEQ ID NO:163 
MRCVGVGNRDFVEGLSGATWVDVVLEHGKCVTVMMKNKPTLDIELQKTEATQLAT
LRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGAMHTALTGATEIQNSGGTSIWPG
HLKCRLKMDKLELKGMSYAMCLNTFVLKKEVSETQHGTILIKVEYKGEDAPCKIPFS
TEDGQGKAHNGRLITANPVVTKKEEPVNIEAEPPFGESNIVIGIGDKALKINWYKK
DENV3 sE SC.16 (I2-U6-H3)
   SEQ ID NO:164 
MRCVGVGNRDFVEGLSGATWVDVVLEPGGCVTTMAKNKPTLDIELQKIEATQLATL
RKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIWPGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
DENV3 sE SC.11 (I2-I8-U6-H3)
   SEQ ID NO:165 
MRCVGVGNRDFVEGLSGATWVDVVLEPGGCVTTMAKNKPTLDIELQKIEATQLATL
RKLCIEGKITNITTDSRCPTQGEAILPEEQDQNYVCKHTYVDRGWGNGCDLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIWPGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
DENV3 sE SC.15 (I8-U6)
   SEQ ID NO:166 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGAMHTALTGATEIQNSGGTSIWPG
HLKCRLKMDKLELKGMSYAMCLNTFVLKKEVSETQHGTILIKVEYKGEDAPCKIPFS
TEDGQGKAHNGRLITANPVVTKKEEPVNIEAEPPFGESNIVIGIGDKALKINWYKK
DENV3 sE SC.23 (12-18)
   SEQ ID NO:167 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGWMHRALTGATEIQNSGGTSIFAG
HLKCRLKMDKLELKGMSYAMCLNTFVLKKEVSETQHGTILIKVEYKGEDAPCKIPFS
TEDGQGKAHNGRLITANPVVTKKEEPVNIEAEPPFGESNIVIGIGDKALKINWYKK
DENV3 sE SC.1 (I2-I8-P4)
   SEQ ID NO:168 
MRCVGVGNRDFVEGLSGATWVDVVLEHGKCVTVMMKNKPTLDIELQKTEATQLAT
LRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQAST
VEAILPEYGTLGLECSPRTGLDFNEMILLTMKNKAWMVHRQWFFDLPLPWTSGATT
ETPTWNRKELLVTFKNAHAKKQEVVVLGSQEGWMHRALTGATEIQNSGGTSIFAG
HLKCRLKMDKLELKGMSYAMCLNTFVLKKEVSETQHGTILIKVEYKGEDAPCKIPFS
TEDGQGKAHNGRLITANPVVTKKEEPVNIEAEPPFGESNIVIGIGDKALKINWYKK
DENV4 sE WT
   SEQ ID NO:169 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGK
GGVVTCAKFSCSGKITGNLVQIENLEYTVVVTVHNGDTHAVGNDTSNHGVTAMITP
RSPSVEVKLPDYGELTLDCEPRSGIDFNEMILMKMKKKTWLVHKQWFLDLPLPWTA
GADTSEVHWNYKERMVTFKVPHAKRQDVTVLGSQEGAMHSALAGATEVDSGDGN
HMFAGHLKCKVRMEKLRIKGMSYTMCSGKFSIDKEMAETQHGTTVVKVKYEGAGA
PCKVPIEIRDVNKEKVVGRIISSTPLAENTNSVTNIELEPPFGDSYIVIGVGNSALTLH
WFRK
DENV4 sE PM4
   SEQ ID NO:170 
MRCVGVGNRDFVEGVSGGAWVDLVLEHGKCVTVMMQGKPTLDFELTKTTAKEVA
LLRTYCIEASISNITTATRCPTQGEPYLKEEQDQQYICRRDVVDRGWGNGCGLFGK
GGVVTCAKFSCSGKITGNLVQIENLEYTVVVTVHNGDTHAVGNDTSNHGVTAMITP
RSPSVEVKLPDYGELTLDCEPRSGIDFNEMILMKMKKKTWLVHKQWFLDLPLPWTA
GADTSEVHWNYKERMVTFKVPHAKRQDVTVLGSQEGAMHSALAGATEVDSGDGN
HMFAGHLKCKVRMEKLRIKGMSYTMCSGKFSIDKEMAETQHGTTVVKVKYEGAGA
PCKVPIEIRDVNKEKVVGRIISSTPLAENTNSVTNIELEPPFGDSYIVIGVGNSALTLH
WFRK
DENV4 sE IntFc2
   SEQ ID NO:171 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGK
GGVVTCAKFSCSGKITGNLVQIENLEYTVVVTVHNGDTHAVGNDTSNHGVTAMITP
RSPSVEVKLPDYGELTLDCEPRSGIDFNEMILMKMKKKTWLVHKQWFLDLPLPWTA
GADTSEVHWNYKERMVTFKVPHAKRQDVTVLGSQEGWMHRALAGATEVDSGDG
NHMFAGHLKCKVRMEKLRIKGMSYTMCSGKFSIDKEMAETQHGTTVVKVKYEGAG
APCKVPIEIRDVNKEKVVGRIISSTPLAENTNSVTNIELEPPFGDSYIVIGVGNSALTL
HWFRK
DENV4 sE IntFc8
   SEQ ID NO:172 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCDLFGK
GGVVTCAKFSCSGKITGNLVQIENLEYTVVVTVHNGDTHAVGNDTSNHGVTAMITP
RSPSVEVKLPDYGELTLDCEPRSGIDFNEMILMKMKKKTWLVHKQWFLDLPLPWTA
GADTSEVHWNYKERMVTFKVPHAKRQDVTVLGSQEGAMHSALAGATEVDSGDGN
HMFAGHLKCKVRMEKLRIKGMSYTMCSGKFSIDKEMAETQHGTTVVKVKYEGAGA
PCKVPIEIRDVNKEKVVGRIISSTPLAENTNSVTNIELEPPFGDSYIVIGVGNSALTLH
WFRK
DENV4 sE UndPk6
   SEQ ID NO:173 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGK
GGVVTCAKFSCSGKITGNLVQIENLEYTVVVTVHNGDTHAVGNDTSNHGVTAMITP
RSPSVEVKLPDYGELTLDCEPRSGIDFNEMILMKMKKKTWLVHKQWFLDLPLPWTA
GADTSEVHWNYKERMVTFKVPHAKRQDVTVLGSQEGAMHSALAGATEVDSGDGN
HMWPAGHLKCKVRMEKLRIKGMSYTMCSGKFSIDKEMAETQHGTTVVKVKYEGA
GAPCKVPIEIRDVNKEKVVGRIISSTPLAENTNSVTNIELEPPFGDSYIVIGVGNSALT
LHWFRK
DENV4 sE SC.12 (I2-I8-U6-P4)
   SEQ ID NO:174 
MRCVGVGNRDFVEGVSGGAWVDLVLEHGKCVTVMMQGKPTLDFELTKTTAKEVA
LLRTYCIEASISNITTATRCPTQGEPYLKEEQDQQYICRRDVVDRGWGNGCDLFGK
GGVVTCAKFSCSGKITGNLVQIENLEYTVVVTVHNGDTHAVGNDTSNHGVTAMITP
RSPSVEVKLPDYGELTLDCEPRSGIDFNEMILMKMKKKTWLVHKQWFLDLPLPWTA
GADTSEVHWNYKERMVTFKVPHAKRQDVTVLGSQEGWMHRALAGATEVDSGDG
NHMWPAGHLKCKVRMEKLRIKGMSYTMCSGKFSIDKEMAETQHGTTVVKVKYEG
AGAPCKVPIEIRDVNKEKVVGRIISSTPLAENTNSVTNIELEPPFGDSYIVIGVGNSAL
TLHWFRK
DENV4 sE UndPk6.1 (A280P)
   SEQ ID NO: 175 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGK
GGVVTCAKFSCSGKITGNLVQIENLEYTVVVTVHNGDTHAVGNDTSNHGVTAMITP
RSPSVEVKLPDYGELTLDCEPRSGIDFNEMILMKMKKKTWLVHKQWFLDLPLPWTA
GADTSEVHWNYKERMVTFKVPHAKRQDVTVLGSQEGAMHSALAGATEVDSGDGN
HMFPGHLKCKVRMEKLRIKGMSYTMCSGKFSIDKEMAETQHGTTVVKVKYEGAGA
PCKVPIEIRDVNKEKVVGRIISSTPLAENTNSVTNIELEPPFGDSYIVIGVGNSALTLH
WFRK
DENV4 sE HCat3
   SEQ ID NO:176 
MRCVGVGNRDFVEGVSGGAWVDLVLEPGGCVTTMAQGKPTLDFELTKITAKEVAL
LRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MFAGHLKCKVRMEKLRIKGMSYTMCSGKFSIDKEMAETQHGTTVVKVKYEGAGAP
CKVPIEIRDVNKEKVVGRIISSTPLAENTNSVTNIELEPPFGDSYIVIGVGNSALTLHW
FRK
DENV4 sE SC.1 (I2-I8-P4)
   SEQ ID NO:177 
MRCVGVGNRDFVEGVSGGAWVDLVLEHGKCVTVMMQGKPTLDFELTKTTAKEVA
LLRTYCIEASISNITTATRCPTQGEPYLKEEQDQQYICRRDVVDRGWGNGCDLFGK
GGVVTCAKFSCSGKITGNLVQIENLEYTVVVTVHNGDTHAVGNDTSNHGVTAMITP
RSPSVEVKLPDYGELTLDCEPRSGIDFNEMILMKMKKKTWLVHKQWFLDLPLPWTA
GADTSEVHWNYKERMVTFKVPHAKRQDVTVLGSQEGWMHRALAGATEVDSGDG
NHMFAGHLKCKVRMEKLRIKGMSYTMCSGKFSIDKEMAETQHGTTVVKVKYEGAG
APCKVPIEIRDVNKEKVVGRIISSTPLAENTNSVTNIELEPPFGDSYIVIGVGNSALTL
HWFRK
ZIKV sE WT
   SEQ ID NO:178 
IRCIGVSNRDFVEGMSGGTWVDIVLEHGGCVTVMAQDKPTVDIELVTTTVSNMAEV
RSYCYEASISDMASDSRCPTQGEAYLDKQSDTQYVCKRTLVDRGWGNGCGLFGK
GSLVTCAKFACSKKMTGKSIQPENLEYRIMLSVHGSQHSGMIVNDTGHETDENRAK
VEITPNSPRAEATLGGFGSLGLDCEPRTGLDFSDLYYLTMNNKHWLVHKEWFHDIP
LPWHAGADTGTPHWNNKEALVEFKDAHAKRQTVVVLGSQEGAVHTALAGALEAE
MDGAKGRLSSGHLKCRLKMDKLRLKGVSYSLCTAAFTFTKIPAETLHGTVTVEVQY
AGTDGPCKVPAQMAVDMQTLTPVGRLITANPVITESTENSKMMLELDPPFGDSYIVI
GVGEKKITHHWHRSG
ZIKV sE PM4
   SEQ ID NO:179 
IRCIGVSNRDFVEGMSGGTWVDIVLEHGKCVTVMMQDKPTVDIELVTTTVSNMAEV
RSYCYEASISDMASDSRCPTQGEAYLDKQSDTQYVCKRTLVDRGWGNGCGLFGK
GSLVTCAKFACSKKMTGKSIQPENLEYRIMLSVHGSQHSGMIVNDTGHETDENRAK
VEITPNSPRAEATLGGFGSLGLDCEPRTGLDFSDLYYLTMNNKHWLVHKEWFHDIP
LPWHAGADTGTPHWNNKEALVEFKDAHAKRQTVVVLGSQEGAVHTALAGALEAE
MDGAKGRLSSGHLKCRLKMDKLRLKGVSYSLCTAAFTFTKIPAETLHGTVTVEVQY
AGTDGPCKVPAQMAVDMQTLTPVGRLITANPVITESTENSKMMLELDPPFGDSYIVI
GVGEKKITHHWHRSG
ZIKV sE IntFc2
   SEQ ID NO:180 
IRCIGVSNRDFVEGMSGGTWVDIVLEHGGCVTVMAQDKPTVDIELVTTTVSNMAEV
RSYCYEASISDMASDSRCPTQGEAYLDKQSDTQYVCKRTLVDRGWGNGCGLFGK
GSLVTCAKFACSKKMTGKSIQPENLEYRIMLSVHGSQHSGMIVNDTGHETDENRAK
VEITPNSPRAEATLGGFGSLGLDCEPRTGLDFSDLYYLTMNNKHWLVHKEWFHDIP
LPWHAGADTGTPHWNNKEALVEFKDAHAKRQTVVVLGSQEGWVHRALAGALEAE
MDGAKGRLSSGHLKCRLKMDKLRLKGVSYSLCTAAFTFTKIPAETLHGTVTVEVQY
AGTDGPCKVPAQMAVDMQTLTPVGRLITANPVITESTENSKMMLELDPPFGDSYIVI
GVGEKKITHHWHRSG
ZIKV sE IntFc8
   SEQ ID NO:181 
IRCIGVSNRDFVEGMSGGTWVDIVLEHGGCVTVMAQDKPTVDIELVTTTVSNMAEV
RSYCYEASISDMASDSRCPTQGEAYLDKQSDTQYVCKRTLVDRGWGNGCDLFGK
GSLVTCAKFACSKKMTGKSIQPENLEYRIMLSVHGSQHSGMIVNDTGHETDENRAK
VEITPNSPRAEATLGGFGSLGLDCEPRTGLDFSDLYYLTMNNKHWLVHKEWFHDIP
LPWHAGADTGTPHWNNKEALVEFKDAHAKRQTVVVLGSQEGAVHTALAGALEAE
MDGAKGRLSSGHLKCRLKMDKLRLKGVSYSLCTAAFTFTKIPAETLHGTVTVEVQY
AGTDGPCKVPAQMAVDMQTLTPVGRLITANPVITESTENSKMMLELDPPFGDSYIVI
GVGEKKITHHWHRSG
Dengue Virus Serotype 2 (DENV2) Strain 16681
NCBI Reference: NC_001474
   (SEQ ID NO:182)
   >NP_056776.2:281-775 polyprotein [Dengue virus 2] 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKKGSSIGQMFETTMRGAKRMAILGDTAWDFGSLGGVFTSIGK
ALHQVFGAIYGAAFSGVSWTMKILIGVIITWIGMNSRSTSLSVTLVLVGIVTLYLGVMVQA
Dengue virus Serotype 1 (DENV1) Strain Western Pacific
GenBank Reference: AY145122.1
   (SEQ ID NO:183)
   >AAN06982.1:281-775 polyprotein precursor [Dengue virus 1] 
MRCVGIGNRDFVEGLSGATWVDVVLEHGSCVTTMAKDKPTLDIELLKTEVTNPAVLRKLCIE
AKISNTTTDSRCPTQGEATLVEEQDTNFVCRRTFVDRGWGNGCGLFGKGSLITCAKFKCVTK
LEGKIVQYENLKYSVIVTVHTGDQHQVGNETTEHGTTATITPQAPTSEIQLTDYGALTLDCS
PRTGLDFNEMVLLTMEKKSWLVHKQWFLDLPLPWTSGASTSQETWNRQDLLVTFKTAHAKKQ
EVVVLGSQEGAMHTALTGATEIQTSGTTTIFAGHLKCRLKMDKLTLKGMSYVMCTGSFKLEK
EVAETQHGTVLVQVKYEGTDAPCKIPFSSQDEKGVTQNGRLITANPIVTDKEKPVNIEAEPP
FGESYIVVGAGEKALKLSWFKKGSSIGKMFEATARGARRMAILGDTAWDFGSIGGVFTSVGK
LIHQIFGTAYGVLFSGVSWTMKIGIGILLTWLGLNSRSTSLSMTCIAVGMVTLYLGVMVQA
Dengue virus Serotype 3 (DENV3) Strain CH53489
GenBank Reference: AAB69126
   (SEQ ID NO:184)
   >AAB69126.2:281-773 polyprotein [Dengue virus 3] 
MRCVGVGNRDFVEGLSGATWVDVVLEHGGCVTTMAKNKPTLDIELQKTEATQLATLRKLCIE
GKITNITTDSRCPTQGEAILPEEQDQNYVCKHTYVDRGWGNGCGLFGKGSLVTCAKFQCLES
IEGKVVQHENLKYTVIITVHTGDQHQVGNETQGVTAEITPQASTVEAILPEYGTLGLECSPR
TGLDFNEMILLTMKNKAWMVHRQWFFDLPLPWTSGATTETPTWNRKELLVTFKNAHAKKQEV
VVLGSQEGAMHTALTGATEIQNSGGTSIFAGHLKCRLKMDKLELKGMSYAMCLNTFVLKKEV
SETQHGTILIKVEYKGEDAPCKIPFSTEDGQGKAHNGRLITANPVVTKKEEPVNIEAEPPFG
ESNIVIGIGDKALKINWYKKGSSIGKMFEATARGARRMAILGDTAWDFGSVGGVLNSLGKMV
HQIFGSAYTALFSGVSWIMKIGIGVLLTWIGLNSKNTSMSFSCIAIGIITLYLGAVVQA
Dengue virus Serotype 4 (DENV4) Strain TVP-376
GenBank Reference: AGS14893.1
   (SEQ ID NO:185)
   >AGS14893.1 polyprotein, partial [Dengue virus 4] 
MAYMIGQTGIQRTVFFVLMMLVAPSYGMRCVGVGNRDFVEGVSGGAWVDLVLEHGGCVTTMA
QGKPTLDFELTKTTAKEVALLRTYCIEASISNITTATRCPTQGEPYLKEEQDQQYICRRDVV
DRGWGNGCGLFGKGGVVTCAKFSCSGKITGNLVQIENLEYTVVVTVHNGDTHAVGNDTSNHG
VTAMITPRSPSVEVKLPDYGELTLDCEPRSGIDFNEMILMKMKKKTWLVHKQWFLDLPLPWT
AGADTSEVHWNYKERMVTFKVPHAKRQDVTVLGSQEGAMHSALAGATEVDSGDGNHMFAGHL
KCKVRMEKLRIKGMSYTMCSGKFSIDKEMAETQHGTTVVKVKYEGAGAPCKVPIEIRDVNKE
KVVGRIISSTPLAENTNSVTNIELEPPFGDSYIVIGVGNSALTLHWFRKGSSIGKMFESTYR
GAKRMAILGETAWDFGSVGGLFTSLGKAVHQVFGSVYTTMFGGVSWMIRILIGFLVLWIGTN
SRNTSMAMTCIAVGGITLFLGFTVQADMGCVVSWSGKELKCGSGIFVVDNVHTWTEQYKFQP
Zika Virus (ZIKV) Strain PF13/251013-18
   (SEQ ID NO:186)
   >ANN44857.1:291-795 polyprotein [Zika virus] 
IRCIGVSNRDFVEGMSGGTWVDVVLEHGGCVTVMAQDKPTVDIELVTTTVSNMAEVRSYCYEASISDMAS
DSRCPTQGEAYLDKQSDTQYVCKRTLVDRGWGNGCGLFGKGSLVTCAKFACSKKMTGKSIQPENLEYRIM
LSVHGSQHSGMIVNDTGHETDENRAKVEITPNSPRAEATLGGFGSLGLDCEPRTGLDFSDLYYLTMNNKH
WLVHKEWFHDIPLPWHAGADTGTPHWNNKEALVEFKDAHAKRQTVVVLGSQEGAVHTALAGALEAEMDGA
KGRLSSGHLKCRLKMDKLRLKGVSYSLCTAAFTFTKIPAETLHGTVTVEVQYAGTDGPCKVPAQMAVDMQ
TLTPVGRLITANPVITESTENSKMMLELDPPFGDSYIVIGVGEKKITHHWHRSGSTIGKAFEATVRGAKR
MAVLGDTAWDFGSVGGALNSLGKGIHQIFGAAFKSLFGGMSWFSQILIGTLLMWLGLNTKNGSISLMCLA
LGGVLIFLSTAVSAD
Yellow Fever Virus. Strain 170
GenBank: X03700.1
   (SEQ ID NO:187)
   >AAR87742.1 protein E, partial [Yellow fever virus] 
HGGTWVSATLEQDKCVTVMAPDKPSLDISLETVAIDRPAEVRKVCYNAVLTHVKINDKCPSTGEAHLAEE
NEGDNACKRTYSDRGWGNGCGLFGKGSIVACAKFTCAKSMSLFEVDQTKIQYVIRAQLHVGAKQENWTTD
IKTLKFDALSGSQEVEFIGYGKATLECQVQTAVDFGNSYIAEMETESWIVDRQWAQDLTLPWQSGSGGVW
REMHHLVEFEPPHAATIRVLALGNQEGSLKTALTGAMRVTKDTNDNNLYKLHGGHVSCRVKLSALTLKGT
SYKICTDKMFFVKNPTDTGHGTVVMQVKVSKGAPCRIPVIVADDLTAAINKGILVTVNPIASTNDDEVLI
EVNPPFGDSYIIVGRGDSRLTYQWHKEGSSIGKLFTQTMKGVERLAVMGDTAWDFSSAGGFFTSVGKGIH
TVFGSAFQGLFGGLNWITKVIMGAVLIWVGINTRNMTMSMSMILVGVIMMFLSLGVGADQGCAINFGKRE
LKCGDGIFIF
Japanese Encephalitis virus. Strain SA14-14-2
GenBank: QCZ42158.1
   (SEQ ID NO:188)
   >QCZ42158.1 polyprotein [Japanese encephalitis virus] 
FNCLGMGNRDFIEGASGATWVDLVLEGDSCLTIMANNKPTLDVRMINIEASQLAEV
RSYCYHASVTDISTVARCPTTGEAHNEKRADSSYVCKQGFTDRGWGNGCGFFGKGSIDTCAKFSCTSKAI
GRTIQPENIKYKVGIFVHGTTTSENHGNYSAQVGASQAAKFTVTPNAPSVALKLGDYGEVTLDCEPRSGL
NTEAFYVMTVGSKSFLVHREWFHDLALPWTSPSSTAWRNRELLMEFEGAHATKQSVVALGSQEGGLHHAL
AGAIVVEYSSSVMLTSGHLKCRLKMDKLALKGTTYGMCTEKFSFAKNPVDTGHGTVVIELSYSGSDGPCK
IPIVSVASLNDMTPVGRLVTVNPFVATSSANSKVLVEMEPPFGDSYIVVGRGAKQINHHWHKAGSTLGKA
FSTTLKGAQRLAALGDTAWDFGSIGGVFNSIGRAVHQVFGGAFRTLFGGMSWITQGLMGALLLWMGVNAR
DRSIALAFLATGGVLVFLATNVHA
West Nile virus. Strain NY99
NCBI Reference: YP_001527880.1
   (SEQ ID NO:189)
   >YP_001527880.1 envelope protein [West Nile virus] 
FNCLGMSNRDFLEGVSGATWVDLVLEGDSCVTIMSKDKPTIDVKMMNMEAANLAEVRSYCYLATVSDLST
KAACPTMGEAHNDKRADPAFVCRQGVVDRGWGNGCGLFGKGSIDTCAKFACSTKAIGRTILKENIKYEVA
IFVHGPTTVESHGNYSTQVGATQAGRLSITPAAPSYTLKLGEYGEVTVDCEPRSGIDTNAYYVMTVGTKT
FLVHREWFMDLNLPWSSAGSTVWRNRETLMEFEEPHATKQSVIALGSQEGALHQALAGAIPVEFSSNTVK
LTSGHLKCRVKMEKLQLKGTTYGVCSKAFKFLGTPADTGHGTVVLELQYTGTDGPCKVPISSVASLNDLT
PVGRLVTVNPFVSVATANAKVLIELEPPFGDSYIVVGRGEQQINHHWHKSGSSIGKAFTTTLKGAQRLAA
LGDTAWDFGSVGGVFTSVGKAVHQVFGGAFRSLFGGMSWITQGLLGALLLWMGINARDRSIALTFLAVGG
VLLFLSVNVHA
Tick-borne encephalitis virus. Strain: Moscow B-4
NCBI Reference: 2022145A
   (SEQ ID NO:189)
   >prf∥2022145A protein E 
SRCTHLENRDFVTGTQGTTRVTLVLELGGCVTIIAEGKTSMDVWLDAIYQENPAKTREYCLHAKLSDTKV
AARCPTMGPATLAEEHQGGTVCKRDQSDRGWGNHCGLFGKGSIVACVKAACEAKKKATGHVYDANKIVYT
VKVEPHTGDYVAANETHSGRKTASFTISSEKTILTMGEYGDVSLLCRVASGVDLAQTVILELDKTVEHLP
TAWQVHRDWFNDLALPWKHEGAQNWNNAERLVEFGAPHAVKMDVYNLGDQTGVLLKALAGVPVAHIEGTK
YHLKSGHVTCEVGLEKLKMKGLTYTMCDKTKFTWKRAPTDSGHDTVVMEVTFSGTKPCRIPVRAVAHGSP
DVNVAMLITPNPTIENNGGGFIEMQLPPGDNIIYVGELSHQWFQKGSSIGRVFQKTKKGIERLTVIGEHA
WDFGSAGGFLSSIGKAVHTVLGGAFNSIFGGVVFLPKLLLGVALAWLGLNMRNPTMSMSFLLAGGLVLAM
TLGVGA
Usutu virus. Strain: MB119/06
European Nucleotide Archive Reference: APT69982.1
   (SEQ ID NO:190)
   >APT69982.1 polyprotein [Usutu virus] 
FNCLGMSNRDFLEGVSGATWVDVVLEGDSCITIMAKDKPTIDIKMMETEATNLAEVR
SYCYLATVSDVSTVSNCPTTGEAHNPKRAEDTYVCKSGVTDRGWGNGCGLFGKGSIDTCANFTCSLKAVG
RMIQPENVKYEVGIFIHGSTSSDTHGNYSSQLGASQAGRFTITPNSPAITVKMGDYGEISVECEPRNGLN
TEAYYIMSVGTKHFLVHREWFNDLALPWTSPASSNWRNREILLEFEEPHATKQSVVALGSQEGALHQALA
GAIPVSFSGSVKLTSGHLKCRVKMEKLTLKGTTYSMCTEKFSFAKNPADTGHGTVVLELQYTGSDGPCKI
PISIVASLSDLTPIGRMVTANPYVASSEANAKVLVEMEPPFGDSYIVVGRGDKQINHHWHKAGSSIGKAF
ITTIKGAQRLAALGDTAWDFGSVGGIFNSVGKAVHQVFGGAFRTLFGGMSWITQGLMGALLLWMGVNARD
RSIALVMLATGGVLLFLATNVHA
Powassan virus. Strain: Lineage I
Genbank Reference: AAL32154
   (SEQ ID NO: 191)
   >AAL32154.1 polyprotein, partial [Powassan virus]

TRCTHLENRDFVTGVQGTTRVSLVLELGGCVTITAEGKPSIDVWLEDIFQESPAETREYCLHAKLSNTKV
EARCPTTGPATLPEEHQADMVCKRDQSDRGWGNHCGFFGKGSIVACAKFECEEAKKAVGHVYDSTKITYV
VKAEPHTGDYQAANETNENRKTAQFTVASEKVILNLGDYGDVSLTCKVASGIDVAQTVVMSLGSSKDHLP
SAWQVHRDWFEDLALPWKHKDNQDWNIVEKLVEFGPPHAVKMDIFNLGDQTAVLLKSLAGVPLASVDNQK
YHLKSGHVTCDVGLEKLKLKGTTYSMCDKTKFKWKRVPVDSGHDTVVMEVSYTGSDKPCRIPVRAVAHGV
PTTNVAMLITPNPTIETSGGGFIEMQLPLGDNIIYVGDLSQQWFQKGSTIGRMFEKTRKGLERLSVVGEH
AWDFGSVGGILSSVGKAIHTVLGGAFNTLFGGVGFIPKMLLGVALVWLGLNARNPTMSMTFLAVGALTLM
MTMGVGA

### Clauses

The following numbered clauses are not claims, but represent preferred aspects and embodiments of the invention:
1. A stabilized recombinant flavivirus E glycoprotein, comprising a flavivirus E glycoprotein backbone; and one or more amino acid substitution selected from the group consisting of 2M, 6L, 8L, 9V, 13F, 14A, 15E, 27P, 29Y, 29K, 32V, 33V, 34L, 35M, 44M, 44L, 481, 106D, 107C, 131I, 144Y, 154L, 154M, 191Y, 198W, 204F, 205L, 206F, 209D, 244Q, 244F, 2461, 246Y, 251F, 255E, 256Y, 258A, 258E, 259V, 259W, 259C, 260L, 261L, 261F, 262H, 262R, 262Y, 263W, 263L, 266W, 270V, 277M, 279W, 280A, 280P, 289W, 299L, 313C, 316M, 330A, 359Y, 373D, 375L, 377V, 3861, 390Q, 392R, 393R, and/or any combination thereof, wherein the numbering is based on the reference amino acid sequence of an E glycoprotein of dengue virus serotype 2 (DENV2) identified as SEQ **ID** NO:6.
2. The stabilized recombinant flavivirus virus E glycoprotein of clause 1, wherein the flavivirus E glycoprotein backbone comprises a backbone of a dengue virus (DENV), Zika virus (ZIKV), yellow fever virus (YFV), Japanese encephalitis virus (JEV), West Nile virus (WNV), Tick-borne Encephalitis virus (TBEV), Powassan virus, Usutu virus, or any combination thereof.
3. The stabilized recombinant flavivirus virus E glycoprotein of clause 2, wherein the flavivirus E glycoprotein backbone comprises a dengue virus backbone.
4. The stabilized recombinant flavivirus virus E glycoprotein of clause 2, wherein the flavivirus E glycoprotein backbone comprises a Zika virus (ZIKV) backbone.
5. The stabilized recombinant flavivirus E glycoprotein of any one of clauses 1-4, wherein the E glycoprotein backbone comprises a soluble E glycoprotein ectodomain of domains DI, DII, and DIII.
6. The stabilized recombinant flavivirus E glycoprotein of any one of clauses 1-5, wherein the E glycoprotein comprises at least one quaternary epitope.
7. The stabilized recombinant flavivirus E glycoprotein of any one of clauses 3, 5, or 6, comprising the amino acid sequence SEQ ID NO:6, wherein said amino acid sequence comprises the following amino acid substitutions: R2M and E44M.
8. The stabilized recombinant flavivirus E glycoprotein of any one of clauses 3 or 5-7, comprising the amino acid sequence: 
MMCIGMSNRDFVEGVSGGSWVDIVLEHGSCVTTMAKNKPTLDFMLIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:10)
9. The stabilized recombinant flavivirus E glycoprotein of any one of clauses 3, 5, or 6, comprising the amino acid sequence SEQ ID NO:6, wherein said amino acid sequence comprises the following amino acid substitutions: H27P and T48I.
10. The stabilized recombinant flavivirus E glycoprotein of any one of clauses 3, 5, 6, or 9, comprising the amino acid sequence: 
MRCIGMSNRDFVEGVSGGSWVDIVLEPGSCVTTMAKNKPTLDFELIKIEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:12)
11. The stabilized recombinant flavivirus E glycoprotein of any one of clauses 3, 5, or 6, comprising the amino acid sequence SEQ ID NO:6, wherein said amino acid sequence comprises the following amino acid substitutions: R2M, E44L, D154L, and K246Y.
12. The stabilized recombinant flavivirus E glycoprotein of any one of clauses 3, 5, 6, or 11, comprising the amino acid sequence: 
MMCIGMSNRDFVEGVSGGSWVDIVLEHGSCVTTMAKNKPTLDFLLIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNLTGKHGKEIKITPQSSITEAELTGYGTVTMECS
PRTGLDFNEMVLLQMENKAWLVHRQWFLDLPLPWLPGADTQGSNWIQKETLVTFKNPHAIKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:14)
13. The stabilized recombinant flavivirus E glycoprotein of any one of clauses 3, 5, or 6, comprising the amino acid sequence SEQ ID NO:6, wherein said amino acid sequence comprises the following amino acid substitutions: K204F and H261L.
14. The stabilized recombinant flavivirus E glycoprotein of any one of clauses 3, 5, 6, or 13, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENFAWLVHRQWFLDLPLPWLPGADTQGSNWIQKETLVTFKNPHAKKQ
DVVVLGSQEGAMLTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:22)
15. The stabilized recombinant flavivirus E glycoprotein of any one of clauses 3, 5, or 6, comprising the amino acid sequence SEQ ID NO:6, wherein said amino acid sequence comprises the following amino acid substitutions: A259V and T262H.
16. The stabilized recombinant flavivirus E glycoprotein of any one of clauses 3, 5, 6, or 15, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGVMHHALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:24)
17. The stabilized recombinant flavivirus E glycoprotein of any one of clauses 3, 5, or 6, comprising the amino acid sequence SEQ ID NO:6, wherein said amino acid sequence comprises the following amino acid substitutions: A259W and T262R.
18. The stabilized recombinant flavivirus E glycoprotein of any one of clauses 3, 5, 6, or 17, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:26)
19. The stabilized recombinant flavivirus E glycoprotein of any one of clauses 3, 5, or 6, comprising the amino acid sequence SEQ ID NO:6, wherein said amino acid sequence comprises the following amino acid substitutions: A259W, T262Y, and A263L.
20. The stabilized recombinant flavivirus E glycoprotein of any one of clauses 3, 5, 6, or 19, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGVMHRWLTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:28)
21. The stabilized recombinant flavivirus E glycoprotein of any one of clauses 3, 5, or 6, comprising the amino acid sequence SEQ ID NO:6, wherein said amino acid sequence comprises the following amino acid substitutions: T262R and A263W .
22. The stabilized recombinant flavivirus E glycoprotein of any one of clauses 3, 5, 6, or 21, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHRWLTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:32)
23. The stabilized recombinant flavivirus E glycoprotein of any one of clauses 3, 5, or 6, comprising the amino acid sequence SEQ ID NO:6, wherein said amino acid sequence comprises the following amino acid substitutions: N8L, S29Y, and H244F.
24. The stabilized recombinant flavivirus E glycoprotein of any one of clauses 3, 5, 6, or 23, comprising the amino acid sequence: 
MRCIGMSLRDFVEGVSGGSWVDIVLEHGYCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPFAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:34)
25. The stabilized recombinant flavivirus E glycoprotein of any one of clauses 3, 5, or 6, comprising the amino acid sequence SEQ ID NO:6, wherein said amino acid sequence comprises the following amino acid substitutions: T262R.
26. The stabilized recombinant flavivirus E glycoprotein of any one of clauses 3, 5, 6, or 25, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:36)
27. The stabilized recombinant flavivirus E glycoprotein of any one of clauses 3, 5, or 6, comprising the amino acid sequence SEQ ID NO:6, wherein said amino acid sequence comprises the following amino acid substitutions: G106D.
28. The stabilized recombinant flavivirus E glycoprotein of any one of clauses 3, 5, 6, or 27, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:38)
29. The stabilized recombinant flavivirus E glycoprotein of any one of clauses 3, 5, or 6, comprising the amino acid sequence SEQ ID NO:6, wherein said amino acid sequence comprises the following amino acid substitutions: M6L and Q316M.
30. The stabilized recombinant flavivirus E glycoprotein of any one of clauses 3, 5, 6, or 29, comprising the amino acid sequence: 
MRCIGLSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETMHGTIVIRVQYEGDGSPCKIPFEIMDLEKRHVLGRLITVNPIVTEKDSPVNIEAEPP
FGDSYIIIGVEPGQLKLNWFKK (SEQ ID NO:44)
31. The stabilized recombinant flavivirus E glycoprotein of any one of clauses 3, 5, or 6, comprising the amino acid sequence SEQ ID NO:6, wherein said amino acid sequence comprises the following amino acid substitutions: Q131I and Y299L.
32. The stabilized recombinant flavivirus E glycoprotein of any one of clauses 3, 5, 6, or 31, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVIPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSLSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:46)
33. The stabilized recombinant flavivirus E glycoprotein of any one of clauses 3, 5, or 6, comprising the amino acid sequence SEQ ID NO:6, wherein said amino acid sequence comprises the following amino acid substitutions: A35M and M289W.
34. The stabilized recombinant flavivirus E glycoprotein of any one of clauses 3, 5, 6, or 33, comprising the amino acid sequence: 
MRCIGMSNRDFVEGVSGGSWVDIVLEHGSCVTTMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRWDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:48)
35. The stabilized recombinant flavivirus E glycoprotein of any one of clauses 3, 5, or 6, comprising the amino acid sequence SEQ ID NO:6, wherein said amino acid sequence comprises the following amino acid substitutions: S29K, T33V, and A35M.
36. The stabilized recombinant flavivirus E glycoprotein of any one of clauses 3, 5, 6, or 34, comprising the amino acid sequence: 
MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:50)
37. The stabilized recombinant flavivirus E glycoprotein of any one of clauses 3, 5, or 6, comprising the amino acid sequence SEQ ID NO:6, wherein said amino acid sequence comprises the following amino acid substitutions: A35M.
38. The stabilized recombinant flavivirus E glycoprotein of any one of clauses 3, 5, 6, or 37, comprising the amino acid sequence: 
MRCIGMSNRDFVEGVSGGSWVDIVLEHGSCVTTMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:52)
39. The stabilized recombinant flavivirus E glycoprotein of any one of clauses 3, 5, or 6, comprising the amino acid sequence SEQ ID NO:6, wherein said amino acid sequence comprises the following amino acid substitutions: T33V and A35M.
40. The stabilized recombinant flavivirus E glycoprotein of any one of clauses 3, 5, 6, or 39, comprising the amino acid sequence: 
MRCIGMSNRDFVEGVSGGSWVDIVLEHGSCVTVMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:54)
41. The stabilized recombinant flavivirus E glycoprotein of any one of clauses 3, 5, or 6, comprising the amino acid sequence SEQ ID NO:6, wherein said amino acid sequence comprises the following amino acid substitutions: S29K.
42. The stabilized recombinant flavivirus E glycoprotein of any one of clauses 3, 5, 6, or 41, comprising the amino acid sequence: 
MRCIGMSNRDFVEGVSGGSWVDIVLEHGKCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:56)
43. The stabilized recombinant flavivirus E glycoprotein of any one of clauses 3, 5, or 6, comprising the amino acid sequence SEQ ID NO:6, wherein said amino acid sequence comprises the following amino acid substitutions: H209D and G266W.
44. The stabilized recombinant flavivirus E glycoprotein of any one of clauses 3, 5, 6, or 43, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVDRQWFLDLPLPWLPGADTQGSNWIQKETLVTFKNPHAKKQ
DVVVLGSQEGAMHTALTWATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:58)
45. The stabilized recombinant flavivirus E glycoprotein of any one of clauses 3, 5, or 6, comprising the amino acid sequence SEQ ID NO:6, wherein said amino acid sequence comprises the following amino acid substitutions: V15E, F373D, and F392R.
46. The stabilized recombinant flavivirus E glycoprotein of any one of clauses 3, 5, 6, or 45, comprising the amino acid sequence: 
MRCIGMSNRDFVEGESGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
DGDSYIIIGVEPGQLKLNWRKK (SEQ ID NO:66)
47. The stabilized recombinant flavivirus E glycoprotein of any one of clauses 3, 5, or 6, comprising the amino acid sequence SEQ ID NO:6, wherein said amino acid sequence comprises the following amino acid substitutions: E13F, G14A, and M34L.
48. The stabilized recombinant flavivirus E glycoprotein of any one of clauses 3, 5, 6, or 47, comprising the amino acid sequence: 
MRCIGMSNRDFVFAVSGGSWVDIVLEHGSCVTTLAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:76)
49. The stabilized recombinant flavivirus E glycoprotein of any one of clauses 3, 5, or 6, comprising the amino acid sequence SEQ ID NO:6, wherein said amino acid sequence comprises the following amino acid substitutions: T359Y.
50. The stabilized recombinant flavivirus E glycoprotein of any one of clauses 3, 5, 6, or 49, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK (SEQ ID NO:78)
51. The stabilized recombinant flavivirus E glycoprotein of any one of clauses 3, 5, or 6, comprising the amino acid sequence SEQ ID NO:6, wherein said amino acid sequence comprises the following amino acid substitutions: F279W and T280P.
52. The stabilized recombinant flavivirus E glycoprotein of any one of clauses 3, 5, 6, or 51, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:80)
53. The stabilized recombinant flavivirus E glycoprotein of any one of clauses 3, 5, or 6, comprising the amino acid sequence SEQ ID NO:6, wherein said amino acid sequence comprises the following amino acid substitutions: T280P.
54. The stabilized recombinant flavivirus E glycoprotein of any one of clauses 3, 5, 6, or 53, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:82)
55. The stabilized recombinant flavivirus E glycoprotein of any one of clauses 3, 5, or 6, comprising the amino acid sequence SEQ ID NO:6, wherein said amino acid sequence comprises the following amino acid substitutions: T481 and L277M.
56. The stabilized recombinant flavivirus E glycoprotein of any one of clauses 3, 5, 6, or 54, comprising the amino acid sequence: 
MRCIGMSNRDFVEGVSGGSWVDIVLEHGSCVTTMAKNKPTLDFELIKIEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNMLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:84)
57. The stabilized recombinant flavivirus E glycoprotein of any one of clauses 3, 5, or 6, comprising the amino acid sequence SEQ ID NO:6, wherein said amino acid sequence comprises the following amino acid substitutions: Q256Y, G258A, A259W, and M260L.
58. The stabilized recombinant flavivirus E glycoprotein of any one of clauses 3, 5, 6, or 57, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSYEAWLHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:86)
59. The stabilized recombinant flavivirus E glycoprotein of any one of clauses 3, 5, or 6, comprising the amino acid sequence **SEQ ID NO:6,** wherein said amino acid sequence comprises the following amino acid substitutions: S29K, T33V, A35M, G106D, A259W, and T262R.
60. The stabilized recombinant flavivirus E glycoprotein of any one of clauses 3, 5, 6, or 59, comprising the amino acid sequence: 
MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:92)
61. The stabilized recombinant flavivirus E glycoprotein of any one of clauses 3, 5, or 6, comprising the amino acid sequence **SEQ ID NO:6,** wherein said amino acid sequence comprises the following amino acid substitutions: H27P, S29K, T33V, A35M, T481, G106D, A259W, and T262R.
62. The stabilized recombinant flavivirus E glycoprotein of any one of clauses 3, 5, 6, or 61, comprising the amino acid sequence: 
MRCIGMSNRDFVEGVSGGSWVDIVLEPGKCVTVMMKNKPTLDFELIKIEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:94)
63. The stabilized recombinant flavivirus E glycoprotein any one of clauses 3, 5, or 6, comprising the amino acid sequence **SEQ ID NO:6,** wherein said amino acid sequence comprises the following amino acid substitutions: S29K, T33V, A35M, G106D, H209D, A259W, T262R, G266W, F279W, F280P, and T359Y.
64. The stabilized recombinant flavivirus E glycoprotein of any one of clauses 3, 5, 6, or 63, comprising the amino acid sequence: 
MRCIGMSNRDFVFAVSGGSWVDIVLEHGSCVTTLAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVDRQWFLDLPLPWLPGADTQGSNWIQKETLVTFKNPHAKKQ
DVVVLGSQEGWMHRALTWATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK (SEQ ID NO:96)
65. The stabilized recombinant flavivirus E glycoprotein of any one of clauses 3, 5, or 6, comprising the amino acid sequence **SEQ ID NO:6,** wherein said amino acid sequence comprises the following amino acid substitutions: E13F, G14A, H27P, S29K, T33V, M34L, A35M, T481, A259W, T262R, F279W, T280P, and T359Y.
66. The stabilized recombinant flavivirus E glycoprotein of any one of clauses 3, 5, 6, or 65, comprising the amino acid sequence: 
MRCIGMSNRDFVFAVSGGSWVDIVLEPGKCVTVLMKNKPTLDFELIKIEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVDRQWFLDLPLPWLPGADTQGSNWIQKETLVTFKNPHAKKQ
DVVVLGSQEGWMHRALTWATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK (SEQ ID NO:98)
67. The stabilized recombinant flavivirus E glycoprotein of any one of clauses 3, 5, or 6, comprising the amino acid sequence **SEQ ID NO:6,** wherein said amino acid sequence comprises the following amino acid substitutions: E13F, G14A, M34L, G106D, A259W, T262R, F279W, T280P.
68. The stabilized recombinant flavivirus E glycoprotein of any one of clauses 3, 5, 6, or 67, comprising the amino acid sequence: 
MRCIGMSNRDFVFAVSGGSWVDIVLEHGSCVTTLAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:100)
69. The stabilized recombinant flavivirus E glycoprotein of any one of clauses 3, 5, or 6, comprising the amino acid sequence **SEQ ID NO:6,** wherein said amino acid sequence comprises the following amino acid substitutions: S29K, T33V, A35M, G106D, A259W, T262R, and T359Y.
70. The stabilized recombinant flavivirus E glycoprotein of any one of clauses 3, 5, 6, or 69, comprising the amino acid sequence: 
MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK (SEQ ID NO:102)
71. The stabilized recombinant flavivirus E glycoprotein of any one of clauses 3, 5, or 6, comprising the amino acid sequence **SEQ ID NO:6,** wherein said amino acid sequence comprises the following amino acid substitutions: G106D, A259W, T262R, F279W, T280P, and T359Y .
72. The stabilized recombinant flavivirus E glycoprotein of any one of clauses 3, 5, 6, or 71, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK (SEQ ID NO:104)
73. The stabilized recombinant flavivirus E glycoprotein of any one of clauses 3, 5, or 6, comprising the amino acid sequence **SEQ ID NO:6,** wherein said amino acid sequence comprises the following amino acid substitutions: S29K, T33V, A35M, G106D, A259W, T262R, F279W, T280P, and T359Y.
74. The stabilized recombinant flavivirus E glycoprotein of any one of clauses 3, 5, 6, or 73, comprising the amino acid sequence: 
MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK (SEQ ID NO:106)
75. The stabilized recombinant flavivirus E glycoprotein of any one of clauses 3, 5, or 6, comprising the amino acid sequence **SEQ ID NO:6,** wherein said amino acid sequence comprises the following amino acid substitutions: V15E, S29K, T33V, A35M, G106D, A259W, T262R, F279W, T280P, T359Y, F373D, and F392R.
76. The stabilized recombinant flavivirus E glycoprotein of any one of clauses 3, 5, 6, or 75, comprising the amino acid sequence: 
MRCIGMSNRDFVEGESGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
DGDSYIIIGVEPGQLKLNWRKK (SEQ ID NO:108)
77. The stabilized recombinant flavivirus E glycoprotein of any one of clauses 3, 5, or 6, comprising the amino acid sequence **SEQ ID NO:6,** wherein said amino acid sequence comprises the following amino acid substitutions: G106D, A259W, T262R, F279W, and T280P.
78. The stabilized recombinant flavivirus E glycoprotein of any one of clauses 3, 5, 6, or 75, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:110)
79. The stabilized recombinant flavivirus E glycoprotein of any one of clauses 3, 5, or 6, comprising the amino acid sequence **SEQ ID NO:6,** wherein said amino acid sequence comprises the following amino acid substitutions: H27P, T481, G106D, A259W, T262R, F279W, and T280P.
80. The stabilized recombinant flavivirus E glycoprotein of any one of clauses 3, 5, 6, or 79, comprising the amino acid sequence: 
MRCIGMSNRDFVEGVSGGSWVDIVLEPGSCVTTMAKNKPTLDFELIKIEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:112)
81. The stabilized recombinant flavivirus E glycoprotein of any one of clauses 3, 5, or 6, comprising the amino acid sequence **SEQ ID NO:6,** wherein said amino acid sequence comprises the following amino acid substitutions: S29K, T33V, A35M, G106D, A259W, T262R, F279W, and T280P.
82. The stabilized recombinant flavivirus E glycoprotein of any one of clauses 3, 5, 6, or 81, comprising the amino acid sequence: 
MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:114)
83. The stabilized recombinant flavivirus E glycoprotein of any one of clauses 3, 5, or 6, comprising the amino acid sequence **SEQ ID NO:6,** wherein said amino acid sequence comprises the following amino acid substitutions: S29K, T33V, A35M, A259W, T262R, F279W, T280P, and T359Y.
84. The stabilized recombinant flavivirus E glycoprotein of any one of clauses 3, 5, 6, or 83, comprising the amino acid sequence: 
MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK (SEQ ID NO:116)
85. The stabilized recombinant flavivirus E glycoprotein of any one of clauses 3, 5, or 6, comprising the amino acid sequence **SEQ ID NO:6,** wherein said amino acid sequence comprises the following amino acid substitutions: A259W, T262R, F279W, and T280P.
86. The stabilized recombinant flavivirus E glycoprotein of any one of clauses 3, 5, 6, or 85, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:118)
87. The stabilized recombinant flavivirus E glycoprotein of any one of clauses 3, 5, or 6, comprising the amino acid sequence **SEQ ID NO:6,** wherein said amino acid sequence comprises the following amino acid substitutions: G106D, F279W, and T280P.
88. The stabilized recombinant flavivirus E glycoprotein of any one of clauses 3, 5, 6, or 87, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:120)
89. The stabilized recombinant flavivirus E glycoprotein of any one of clauses 3, 5, or 6, comprising the amino acid sequence **SEQ ID NO:6,** wherein said amino acid sequence comprises the following amino acid substitutions:H27P, T481, A259W, T262R, F279W, and T280P .
90. The stabilized recombinant flavivirus E glycoprotein of any one of clauses 3, 5, 6, or 89, comprising the amino acid sequence: 
MRCIGMSNRDFVEGVSGGSWVDIVLEPGSCVTTMAKNKPTLDFELIKIEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:122)
91. The stabilized recombinant flavivirus E glycoprotein of any one of clauses 3, 5, or 6, comprising the amino acid sequence **SEQ ID NO:6,** wherein said amino acid sequence comprises the following amino acid substitutions: S29K, T33V, A35M, A259W, T262R, F279W, and T280P.
92. The stabilized recombinant flavivirus E glycoprotein of any one of clauses 3, 5, 6, or 91, comprising the amino acid sequence: 
MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:124)
93. The stabilized recombinant flavivirus E glycoprotein of any one of clauses 3, 5, or 6, comprising the amino acid sequence **SEQ ID NO:6,** wherein said amino acid sequence comprises the following amino acid substitutions: E13F, G14A, S29K, T33V, M34L, A35M, G106D, A259W, T262R, F279W, and T280P.
94. The stabilized recombinant flavivirus E glycoprotein of any one of clauses 3, 5, 6, or 93, comprising the amino acid sequence: 
MRCIGMSNRDFVFAVSGGSWVDIVLEHGKCVTVLMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:126)
95. The stabilized recombinant flavivirus E glycoprotein of any one of clauses 3, 5, or 6, comprising the amino acid sequence **SEQ ID NO:6,** wherein said amino acid sequence comprises the following amino acid substitutions: S29K, T33V, A35M, G106D, F279W, T280P, and T359Y.
96. The stabilized recombinant flavivirus E glycoprotein of any one of clauses 3, 5, 6, or 95, comprising the amino acid sequence: 
MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK (SEQ ID NO:128)
97. The stabilized recombinant flavivirus E glycoprotein of any one of clauses 3, 5, or 6, comprising the amino acid sequence **SEQ ID NO:6,** wherein said amino acid sequence comprises the following amino acid substitutions: E13F, G14A, S29K, T33V, M34L, and A35M.
98. The stabilized recombinant flavivirus E glycoprotein of any one of clauses 3, 5, 6, or 97, comprising the amino acid sequence: 
MRCIGMSNRDFVFAVSGGSWVDIVLEHGKCVTVLMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:130)
99. The stabilized recombinant flavivirus E glycoprotein of any one of clauses 3, 5, or 6, comprising the amino acid sequence (**SEQ ID NO:6**, wherein said amino acid sequence comprises the following amino acid substitutions: E13F, G14A, M34L, F279W, T280P, and T359Y .
100. The stabilized recombinant flavivirus E glycoprotein of any one of clauses 3, 5, 6, or 99, comprising the amino acid sequence: 
MRCIGMSNRDFVFAVSGGSWVDIVLEHGSCVTTLAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK (SEQ ID NO:132)
101. The stabilized recombinant flavivirus E glycoprotein of any one of clauses 3, 5, or 6, comprising the amino acid sequence **SEQ ID NO:6,** wherein said amino acid sequence comprises the following amino acid substitutions: E13F, G14A, M34L, H209D, G266W, F279W, T280P, and T359Y.
102. The stabilized recombinant flavivirus E glycoprotein of any one of clauses 3, 5, 6, or 101, comprising the amino acid sequence: 
MRCIGMSNRDFVFAVSGGSWVDIVLEHGSCVTTLAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVDRQWFLDLPLPWLPGADTQGSNWIQKETLVTFKNPHAKKQ
DVVVLGSQEGAMHTALTWATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVYEKDSPVNIEAEPP
FGDSYIIIGVEPGQLKLNWFKK (SEQ ID NO:134)
103. The stabilized recombinant flavivirus E glycoprotein of any one of clauses 3, 5, or 6, comprising the amino acid sequence **SEQ ID NO:6,** wherein said amino acid sequence comprises the following amino acid substitutions: G106D, A259W, and T262R .
104. The stabilized recombinant flavivirus E glycoprotein of any one of clauses 3, 5, 6, or 103, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHRALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:136)
105. The stabilized recombinant flavivirus E glycoprotein of any one of clauses 3, 5, or 6, comprising the amino acid sequence **SEQ ID NO:6,** wherein said amino acid sequence comprises the following amino acid substitutions: H27P, T481, and G258E.
106. The stabilized recombinant flavivirus E glycoprotein of any one of clauses 3, 5, 6, or 105, comprising the amino acid sequence: 
MRCIGMSNRDFVEGVSGGSWVDIVLEPGSCVTTMAKNKPTLDFELIKIEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEEAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:138)
107. The stabilized recombinant flavivirus E glycoprotein of any one of clauses 3, 5, or 6, comprising the amino acid sequence **SEQ ID NO:6,** wherein said amino acid sequence comprises the following amino acid substitutions: S29K, T33V, A35M, and G258E.
108. The stabilized recombinant flavivirus E glycoprotein of any one of clauses 3, 5, 6, or 107, comprising the amino acid sequence: 
MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEEAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:140)
109. The stabilized recombinant flavivirus E glycoprotein of any one of clauses 3, 5, or 6, comprising the amino acid sequence **SEQ ID NO:6,** wherein said amino acid sequence comprises the following amino acid substitutions: G106D, A259V, T262R, A263W, F279W, and T280P.
110. The stabilized recombinant flavivirus E glycoprotein of any one of clauses 3, 5, 6, or 109, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGVMHRWLTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:142)
111. The stabilized recombinant flavivirus E glycoprotein of any one of clauses 3, 5, or 6, comprising the amino acid sequence **SEQ ID NO:6,** wherein said amino acid sequence comprises the following amino acid substitutions: S29K, T33V, A35M, G106D, A259V, T262R, A263W, F279W, and T280P .
112. The stabilized recombinant flavivirus E glycoprotein of any one of clauses 3, 5, 6, or 111, comprising the amino acid sequence: 
MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCDLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGVMHRWLTGATEIQMSSGNLLWPGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:144)
113. The stabilized recombinant flavivirus E glycoprotein of any one of clauses 3, 5, or 6, comprising the amino acid sequence **SEQ ID NO:6,** wherein said amino acid sequence comprises the following amino acid substitutions: S29K, T33V, A35M, L107C, and A313C.
114. The stabilized recombinant flavivirus E glycoprotein of any one of clauses 3, 5, 6, or 113, comprising the amino acid sequence: 
MRCIGMSNRDFVEGVSGGSWVDIVLEHGKCVTVMMKNKPTLDFELIKTEAKQPATLRKYCIE
AKLTNTTTESRCPTQGEPSLNEEQDKRFVCKHSMVDRGWGNGCGCFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EICETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:146)
115. The stabilized recombinant flavivirus E glycoprotein of any one of clauses 3, 5, or 6, comprising the amino acid sequence **SEQ ID NO:6,** wherein said amino acid sequence comprises the following amino acid substitutions: R9V, T32V, H144Y, and E368I.
116. The stabilized recombinant flavivirus E glycoprotein of any one of clauses 3, 5, 6, or 115, comprising the amino acid sequence: 
MRCIGMSNVDFVEGVSGGSWVDIVLEHGSCVVTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPYSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIIAEPP
FGDSYIIIGVEPGQLKLNWFKK (SEQ ID NO:8)
117. The stabilized recombinant flavivirus E glycoprotein of any one of clauses 3, 5, or 6, comprising the amino acid sequence **SEQ ID NO:6,** wherein said amino acid sequence comprises the following amino acid substitutions: R2M, H27P, E44M, T481, D154M, H244Q, and K246Y.
118. The stabilized recombinant flavivirus E glycoprotein of any one of clauses 3, 5, 6, or 117, comprising the amino acid sequence: 
MMCIGMSNRDFVEGVSGGSWVDIVLEPGSCVTTMAKNKPTLDFMLIKIEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNMTGKHGKEIKITPQSSITEAELTGYGTVTMECS
PRTGLDFNEMVLLQMENKAWLVHRQWFLDLPLPWLPGADTQGSNWIQKETLVTFKNPQAYKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:16)
119. The stabilized recombinant flavivirus E glycoprotein of any one of clauses 3, 5, or 6, comprising the amino acid sequence **SEQ ID NO:6,** wherein said amino acid sequence comprises the following amino acid substitutions: K204F, W206F, and H261L.
120. The stabilized recombinant flavivirus E glycoprotein of any one of clauses 3, 5, 6, or 119, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENFAFLVHRQWFLDLPLPWLPGADTQGSNWIQKETLVTFKNPHAKKQ
DVVVLGSQEGAMLTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:18)
121. The stabilized recombinant flavivirus E glycoprotein of any one of clauses 3, 5, or 6, comprising the amino acid sequence **SEQ ID NO:6,** wherein said amino acid sequence comprises the following amino acid substitutions: K204F, W251F, and H261F.
122. The stabilized recombinant flavivirus E glycoprotein of any one of clauses 3, 5, 6, or 121, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENFAWLVHRQWFLDLPLPWLPGADTQGSNWIQKETLVTFKNPHAKKQ
DVFVLGSQEGAMFTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:20)
123. The stabilized recombinant flavivirus E glycoprotein of any one of clauses 3, 5, or 6, comprising the amino acid sequence **SEQ ID NO:6,** wherein said amino acid sequence comprises the following amino acid substitutions: A259W, T262Y, and A263L.
124. The stabilized recombinant flavivirus E glycoprotein of any one of clauses 3, 5, 6, or 123, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGWMHYLLTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:30)
125. The stabilized recombinant flavivirus E glycoprotein of any one of clauses 3, 5, or 6, comprising the amino acid sequence **SEQ ID NO:6,** wherein said amino acid sequence comprises the following amino acid substitutions: D375L and N390Q.
126. The stabilized recombinant flavivirus E glycoprotein of any one of clauses 3, 5, 6, or 125, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGLSYIIIGVEPGQLKLQWFKK (SEQ ID NO:60)
127. The stabilized recombinant flavivirus E glycoprotein of any one of clauses 3, 5, or 6, comprising the amino acid sequence **SEQ ID NO:6,** wherein said amino acid sequence comprises the following amino acid substitutions: G330A.
128. The stabilized recombinant flavivirus E glycoprotein of any one of clauses 3, 5, 6, or 127, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDASPCKIPFEIMDLEKRHVLGRLITVNPIVTEKDSPVNIEAEPP
FGDSYIIIGVEPGQLKLNWFKK (SEQ ID NO:62)
129. The stabilized recombinant flavivirus E glycoprotein of any one of clauses 3, 5, or 6, comprising the amino acid sequence **SEQ ID NO:6,** wherein said amino acid sequence comprises the following amino acid substitutions: Y377V and K393R.
130. The stabilized recombinant flavivirus E glycoprotein of any one of clauses 3, 5, 6, or 129, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSVIIIGVEPGQLKLNWFRK (SEQ ID NO:64)
131. The stabilized recombinant flavivirus E glycoprotein of any one of clauses 3, 5, or 6, comprising the amino acid sequence **SEQ ID NO:6,** wherein said amino acid sequence comprises the following amino acid substitutions: 1270V and T280A.
132. The stabilized recombinant flavivirus E glycoprotein of any one of clauses 3, 5, 6, or 131, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEVQMSSGNLLFAGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:70)
133. The stabilized recombinant flavivirus E glycoprotein of any one of clauses 3, 5, or 6, comprising the amino acid sequence **SEQ ID NO:6,** wherein said amino acid sequence comprises the following amino acid substitutions: L198W.
134. The stabilized recombinant flavivirus E glycoprotein of any one of clauses 3, 5, 6, or 133, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVWLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:72)
135. The stabilized recombinant flavivirus E glycoprotein of any one of clauses 3, 5, or 6, comprising the amino acid sequence **SEQ ID NO:6,** wherein said amino acid sequence comprises the following amino acid substitutions: L191Y and H209D.
136. The stabilized recombinant flavivirus E glycoprotein of any one of clauses 3, 5, 6, or 135, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGYDFNEMVLLQMENKAWLVDRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:74)
137. The stabilized recombinant flavivirus E glycoprotein of any one of clauses 3, 5, or 6, comprising the amino acid sequence **SEQ ID NO:6,** wherein said amino acid sequence comprises the following amino acid substitutions: V15E, W20H, F373D, and F392R.
138. The stabilized recombinant flavivirus E glycoprotein of any one of clauses 3, 5, 6, or 137, comprising the amino acid sequence: 
MRCIGMSNRDFVEGESGGSHVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
DGDSYIIIGVEPGQLKLNWRKK (SEQ ID NO:68)
139. The stabilized recombinant flavivirus E glycoprotein of any one of clauses 3, 5, or 6, comprising the amino acid sequence **SEQ ID NO:147**, wherein said amino acid sequence comprises the following amino acid substitutions, wherein the numbering is based on the reference amino acid sequence of an E glycoprotein of dengue virus serotype 1 (DENV1) identified as **SEQ ID NO:147:** S29K, T33V, and A35M.
140. The stabilized recombinant flavivirus E glycoprotein of any one of clauses 3, 5, 6, or 139, comprising the amino acid sequence: 
MRCVGIGNRDFVEGLSGATWVDVVLEHGKCVTVMMKDKPTLDIELLKTEVTNPAV
LRKLCIEAKISNTTTDSRCPTQGEATLVEEQDTNFVCRRTFVDRGWGNGCGLFGKGS
LITCAKFKCVTKLEGKIVQYENLKYSVIVTVHTGDQHQVGNETTEHGTTATITPQAPT
SEIQLTDYGALTLDCSPRTGLDFNEMVLLTMEKKSWLVHKQWFLDLPLPWTSGAST
SQETWNRQDLLVTFKTAHAKKQEVVVLGSQEGAMHTALTGATEIQTSGTTTIFAGH
LKCRLKMDKLTLKGMSYVMCTGSFKLEKEVAETQHGTVLVQVKYEGTDAPCKIPFS
SQDEKGVTQNGRLITANPIVTDKEKPVNIEAEPPFGESYIVVGAGEKALKLSWFKK
(SEQ ID NO:148)
141. The stabilized recombinant flavivirus E glycoprotein of any one of clauses 3, 5, or 6, comprising the amino acid sequence **SEQ ID NO:147**, wherein said amino acid sequence comprises the following amino acid substitutions, wherein the numbering is based on the reference amino acid sequence of an E glycoprotein of dengue virus serotype 1 (DENV1) identified as **SEQ ID NO:147**: A259W and S262R.
142. The stabilized recombinant flavivirus E glycoprotein of any one of clauses 3, 5, 6, or 141, comprising the amino acid sequence: 
MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCGLFGKGSLI
TCAKFKCVTKLEGKIVQYENLKYSVIVTVHTGDQHQVGNETTEHGTTATITPQAPTS
EIQLTDYGALTLDCSPRTGLDFNEMVLLTMEKKSWLVHKQWFLDLPLPWTSGASTS
QETWNRQDLLVTFKTAHAKKQEVVVLGSQEGWMHRALTGATEIQTSGTTTIFAGHL
KCRLKMDKLTLKGMSYVMCTGSFKLEKEVAETQHGTVLVQVKYEGTDAPCKIPFSS
QDEKGVTQNGRLITANPIVTDKEKPVNIEAEPPFGESYIVVGAGEKALKLSWFKK
(SEQ ID NO:149)
143. The stabilized recombinant flavivirus E glycoprotein of any one of clauses 3, 5, or 6, comprising the amino acid sequence **SEQ ID NO:147,** wherein said amino acid sequence comprises the following amino acid substitutions, wherein the numbering is based on the reference amino acid sequence of an E glycoprotein of dengue virus serotype 1 (DENV1) identified as **SEQ ID NO:147:** G106D.
144. The stabilized recombinant flavivirus E glycoprotein of any one of clauses 3, 5, 6, or 143, comprising the amino acid sequence: 
MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCDLFGKGSLI
TCAKFKCVTKLEGKIVQYENLKYSVIVTVHTGDQHQVGNETTEHGTTATITPQAPTS
EIQLTDYGALTLDCSPRTGLDFNEMVLLTMEKKSWLVHKQWFLDLPLPWTSGASTS
QETWNRQDLLVTFKTAHAKKQEVVVLGSQEGAMHTALTGATEIQTSGTTTIFAGHL
KCRLKMDKLTLKGMSYVMCTGSFKLEKEVAETQHGTVLVQVKYEGTDAPCKIPFSS
QDEKGVTQNGRLITANPIVTDKEKPVNIEAEPPFGESYIVVGAGEKALKLSWFKK
(SEQ ID NO:150)
145. The stabilized recombinant flavivirus E glycoprotein of any one of clauses 3, 5, or 6, comprising the amino acid sequence **SEQ ID NO:147**, wherein said amino acid sequence comprises the following amino acid substitutions, wherein the numbering is based on the reference amino acid sequence of an E glycoprotein of dengue virus serotype 1 (DENV1) identified as **SEQ ID NO:147**: F279W and A280P.
146. The stabilized recombinant flavivirus E glycoprotein of any one of clauses 3, 5, 6, or 145, comprising the amino acid sequence: 
MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCGLFGKGSLI
TCAKFKCVTKLEGKIVQYENLKYSVIVTVHTGDQHQVGNETTEHGTTATITPQAPTS
EIQLTDYGALTLDCSPRTGLDFNEMVLLTMEKKSWLVHKQWFLDLPLPWTSGASTS
QETWNRQDLLVTFKTAHAKKQEVVVLGSQEGAMHTALTGATEIQTSGTTTIWPGHL
KCRLKMDKLTLKGMSYVMCTGSFKLEKEVAETQHGTVLVQVKYEGTDAPCKIPFSS
QDEKGVTQNGRLITANPIVTDKEKPVNIEAEPPFGESYIVVGAGEKALKLSWFKK
(SEQ ID NO:151)
147. The stabilized recombinant flavivirus E glycoprotein of any one of clauses 3, 5, or 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 1 (DENV1) identified as **SEQ ID NO:152:** G29K, T33V, and A35M.
148. The stabilized recombinant flavivirus E glycoprotein of any one of clauses 3, 5, 6, or 147, comprising the amino acid sequence: 
MRCVGVGNRDFVEGLSGATWVDVVLEHGKCVTVMMKNKPTLDIELQKTEATQLA
TLRKLCIEGKITNITTDSRCPTQGEAILPEEQDQNYVCKHTYVDRGWGNGCGLFGKG
SLVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
(SEQ ID NO:153)
149. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152**: A257W and T260R.
150. The stabilized recombinant flavivirus E glycoprotein of any one of clauses 3, 5, 6, or 149, comprising the amino acid sequence: 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
(SEQ ID NO:154)
151. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152:** G106D.
152. The stabilized recombinant flavivirus E glycoprotein of any one of clauses 3, 5, 6, or 151, comprising the amino acid sequence: 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
(SEQ ID NO:155)
153. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152**: F277W and A278P.
154. The stabilized recombinant flavivirus E glycoprotein of any one of clauses 3, 5, 6, or 153, comprising the amino acid sequence: 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIWPGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
(SEQ ID NO:156)
155. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152**: F277W, A278P, S275L, and M205L.
156. The stabilized recombinant flavivirus E glycoprotein of any one of clauses 3, 5, 6, or 155, comprising the amino acid sequence: 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWLVHRQWFFDLPLPWTSGATTET
PTWNRKELLVTFKNAHAKKQEVVVLGSQEGAMHTALTGATEIQNSGGTLIWPGHLK
CRLKMDKLELKGMSYAMCLNTFVLKKEVSETQHGTILIKVEYKGEDAPCKIPFSTED
GQGKAHNGRLITANPVVTKKEEPVNIEAEPPFGESNIVIGIGDKALKINWYKK
(SEQ ID NO:157)
157. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152:** G106D, A257W, T260R, F277W, and A278P.
158. The stabilized recombinant flavivirus E glycoprotein of any one of clauses 3, 5, 6, or 157, comprising the amino acid sequence: 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIWPGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
(SEQ ID NO:158)
159. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152**: A257W, T260R, F277W, and A278P.
160. The stabilized recombinant flavivirus E glycoprotein of any one of clauses 3, 5, 6, or 159, comprising the amino acid sequence: 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIWPGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
(SEQ ID NO:159)
161. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152**: A278P.
162. The stabilized recombinant flavivirus E glycoprotein of any one of clauses 3, 5, 6, or 161, comprising the amino acid sequence: 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCGLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIFPGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
(SEQ ID NO:160)
163. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152**: H27P and T481.
164. The stabilized recombinant flavivirus E glycoprotein of any one of clauses 3, 5, 6, or 163, comprising the amino acid sequence: 
MRCVGVGNRDFVEGLSGATWVDVVLEPGGCVTTMAKNKPTLDIELQKIEATQLATL
RKLCIEGKITNITTDSRCPTQGEAILPEEQDQNYVCKHTYVDRGWGNGCGLFGKGSL
VTCAKFQCLESIEGKVVQHENLKYTVIITVHTGDQHQVGNETQGVTAEITPQASTVE
AILPEYGTLGLECSPRTGLDFNEMILLTMKNKAWMVHRQWFFDLPLPWTSGATTETP
TWNRKELLVTFKNAHAKKQEVVVLGSQEGAMHTALTGATEIQNSGGTSIFAGHLKC
RLKMDKLELKGMSYAMCLNTFVLKKEVSETQHGTILIKVEYKGEDAPCKIPFSTEDG
QGKAHNGRLITANPVVTKKEEPVNIEAEPPFGESNIVIGIGDKALKINWYKK
(SEQ ID NO:161)
165. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152:** G29K, T33V, A35M, G106D, A257W, T260R, F277W, and A278P.
166. The stabilized recombinant flavivirus E glycoprotein of any one of clauses 3, 5, 6, or 165, comprising the amino acid sequence: 
MRCVGVGNRDFVEGLSGATWVDVVLEHGKCVTVMMKNKPTLDIELQKTEATQLA
TLRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIWPGH
LKCRLKMDKLELKGMSYAMCLNTFVLKKEVSETQHGTILIKVEYKGEDAPCKIPFST
EDGQGKAHNGRLITANPVVTKKEEPVNIEAEPPFGESNIVIGIGDKALKINWYKK
(SEQ ID NO:162)
167. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152:** G29K, T33V, A35M, G106D, F277W, and A278P.
168. The stabilized recombinant flavivirus E glycoprotein of any one of clauses 3, 5, 6, or 167, comprising the amino acid sequence: 
MRCVGVGNRDFVEGLSGATWVDVVLEHGKCVTVMMKNKPTLDIELQKTEATQLA
TLRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIWPGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
(SEQ ID NO:163)
169. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152**: H27P, T481, A257W, T260R, F277W, and A278P.
170. The stabilized recombinant flavivirus E glycoprotein of any one of clauses 3, 5, 6, or 169, comprising the amino acid sequence: 
MRCVGVGNRDFVEGLSGATWVDVVLEPGGCVTTMAKNKPTLDIELQKIEATQLATL
RKLCIEGKITNITTDSRCPTQGEAILPEEQDQNYVCKHTYVDRGWGNGCGLFGKGSL
VTCAKFQCLESIEGKVVQHENLKYTVIITVHTGDQHQVGNETQGVTAEITPQASTVE
AILPEYGTLGLECSPRTGLDFNEMILLTMKNKAWMVHRQWFFDLPLPWTSGATTETP
TWNRKELLVTFKNAHAKKQEVVVLGSQEGWMHRALTGATEIQNSGGTSIWPGHLK
CRLKMDKLELKGMSYAMCLNTFVLKKEVSETQHGTILIKVEYKGEDAPCKIPFSTED
GQGKAHNGRLITANPVVTKKEEPVNIEAEPPFGESNIVIGIGDKALKINWYKK
(SEQ ID NO:164)
171. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152:** H27P, T481, G106D, A257W, T260R, F277W, and A278P.
172. The stabilized recombinant flavivirus E glycoprotein of any one of clauses 3, 5, 6, or 171, comprising the amino acid sequence: 
MRCVGVGNRDFVEGLSGATWVDVVLEPGGCVTTMAKNKPTLDIELQKIEATQLATL
RKLCIEGKITNITTDSRCPTQGEAILPEEQDQNYVCKHTYVDRGWGNGCDLFGKGSL
VTCAKFQCLESIEGKVVQHENLKYTVIITVHTGDQHQVGNETQGVTAEITPQASTVE
AILPEYGTLGLECSPRTGLDFNEMILLTMKNKAWMVHRQWFFDLPLPWTSGATTETP
TWNRKELLVTFKNAHAKKQEVVVLGSQEGWMHRALTGATEIQNSGGTSIWPGHLK
CRLKMDKLELKGMSYAMCLNTFVLKKEVSETQHGTILIKVEYKGEDAPCKIPFSTED
GQGKAHNGRLITANPVVTKKEEPVNIEAEPPFGESNIVIGIGDKALKINWYKK
(SEQ ID NO:165)
173. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152:** G106D, F277W, and A278P.
174. The stabilized recombinant flavivirus E glycoprotein of any one of clauses 3, 5, 6, or 173, comprising the amino acid sequence: 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIWPGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
(SEQ ID NO:166)
175. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152:** G106D, A257W, and T260R.
176. The stabilized recombinant flavivirus E glycoprotein of any one of clauses 3, 5, 6, or 175, comprising the amino acid sequence: 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
(SEQ ID NO:167)
177. The stabilized recombinant flavivirus E glycoprotein of any one of clauses 3, 5, 6, comprising the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152:** G29K, T33V, A35M, G106D, A257W, and T260R.
178. The stabilized recombinant flavivirus E glycoprotein of any one of clauses 3, 5, 6, or 177, comprising the amino acid sequence: 
MRCVGVGNRDFVEGLSGATWVDVVLEHGKCVTVMMKNKPTLDIELQKTEATQLA
TLRKLCIEGKITNITTDSRCPTQGEAILPEEQDQNYVCKHTYVDRGWGNGCDLFGKG
SLVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGWMHRALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK
(SEQ ID NO:168)
179. The stabilized recombinant flavivirus E glycoprotein of any one of clauses 3, 5, 6, comprising the amino acid sequence **SEQ ID NO:169**, wherein said amino acid sequence comprises the following amino acid substitutions, wherein the numbering is based on the reference amino acid sequence of an E glycoprotein of dengue virus serotype 4 (DENV4) identified as **SEQ ID NO:169:** G29K, T33V, and A35M.
180. The stabilized recombinant flavivirus E glycoprotein of any one of clauses 3, 5, 6, or 179, comprising the amino acid sequence: 
MRCVGVGNRDFVEGVSGGAWVDLVLEHGKCVTVMMQGKPTLDFELTKTTAKEVA
LLRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MFAGHLKCKVRMEKLRIKGMSYTMCSGKFSIDKEMAETQHGTTVVKVKYEGAGAP
CKVPIEIRDVNKEKVVGRIISSTPLAENTNSVTNIELEPPFGDSYIVIGVGNSALTLHWF
RK (SEQ ID NO:170)
181. The stabilized recombinant flavivirus E glycoprotein of any one of clauses 3, 5, 6, comprising the amino acid sequence **SEQ ID NO:169**, wherein said amino acid sequence comprises the following amino acid substitutions, wherein the numbering is based on the reference amino acid sequence of an E glycoprotein of dengue virus serotype 4 (DENV4) identified **as SEQ ID NO:169**: A259W and S262R.
182. The stabilized recombinant flavivirus E glycoprotein of any one of clauses 3, 5, 6, or 181, comprising the amino acid sequence: 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGWMHRALAGATEVDSGDGN
HMFAGHLKCKVRMEKLRIKGMSYTMCSGKFSIDKEMAETQHGTTVVKVKYEGAGA
PCKVPIEIRDVNKEKVVGRIISSTPLAENTNSVTNIELEPPFGDSYIVIGVGNSALTLHW
FRK (SEQ ID NO:171)
183. The stabilized recombinant flavivirus E glycoprotein of any one of clauses 3, 5, 6, comprising the amino acid sequence **SEQ ID NO:169,** wherein said amino acid sequence comprises the following amino acid substitutions, wherein the numbering is based on the reference amino acid sequence of an E glycoprotein of dengue virus serotype 4 (DENV4) identified as **SEQ ID NO:169:** G106D.
184. The stabilized recombinant flavivirus E glycoprotein of any one of clauses 3, 5, 6, or 183, comprising the amino acid sequence: 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCDLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MFAGHLKCKVRMEKLRIKGMSYTMCSGKFSIDKEMAETQHGTTVVKVKYEGAGAP
CKVPIEIRDVNKEKVVGRIISSTPLAENTNSVTNIELEPPFGDSYIVIGVGNSALTLHWF
RK (SEQ ID NO:172)
185. The stabilized recombinant flavivirus E glycoprotein of any one of clauses 3, 5, 6, comprising the amino acid sequence **SEQ ID NO:169,** wherein said amino acid sequence comprises the following amino acid substitutions, wherein the numbering is based on the reference amino acid sequence of an E glycoprotein of dengue virus serotype 4 (DENV4) identified as **SEQ ID NO:169**: F279W and A280P.
186. The stabilized recombinant flavivirus E glycoprotein of any one of clauses 3, 5, 6, or 185, comprising the amino acid sequence: 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MWPAGHLKCKVRMEKLRIKGMSYTMCSGKFSIDKEMAETQHGTTVVKVKYEGAG
APCKVPIEIRDVNKEKVVGRIISSTPLAENTNSVTNIELEPPFGDSYIVIGVGNSALTLH
WFRK (SEQ ID NO:173)
187. The stabilized recombinant flavivirus E glycoprotein of any one of clauses 3, 5, 6, comprising the amino acid sequence **SEQ ID NO:169**, wherein said amino acid sequence comprises the following amino acid substitutions, wherein the numbering is based on the reference amino acid sequence of an E glycoprotein of dengue virus serotype 4 (DENV4) identified as **SEQ ID NO:169:** G29K, T33V, A35M, G106D, A259W, S262R, F279W, and A280P.
188. The stabilized recombinant flavivirus E glycoprotein of any one of clauses 3, 5, 6, or 187, comprising the amino acid sequence: 
MRCVGVGNRDFVEGVSGGAWVDLVLEHGKCVTVMMQGKPTLDFELTKTTAKEVA
LLRTYCIEASISNITTATRCPTQGEPYLKEEQDQQYICRRDVVDRGWGNGCDLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGWMHRALAGATEVDSGDGN
HMWPAGHLKCKVRMEKLRIKGMSYTMCSGKFSIDKEMAETQHGTTVVKVKYEGA
GAPCKVPIEIRDVNKEKVVGRIISSTPLAENTNSVTNIELEPPFGDSYIVIGVGNSALTL
HWFRK (SEQ ID NO:174)
189. The stabilized recombinant flavivirus E glycoprotein of any one of clauses 3, 5, 6, comprising the amino acid sequence **SEQ ID NO:169,** wherein said amino acid sequence comprises the following amino acid substitutions, wherein the numbering is based on the reference amino acid sequence of an E glycoprotein of dengue virus serotype 4 (DENV4) identified as **SEQ ID NO:169**: A280P.
190. The stabilized recombinant flavivirus E glycoprotein of any one of clauses 3, 5, 6, or 189, comprising the amino acid sequence: 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MFPGHLKCKVRMEKLRIKGMSYTMCSGKFSIDKEMAETQHGTTVVKVKYEGAGAP
CKVPIEIRDVNKEKVVGRIISSTPLAENTNSVTNIELEPPFGDSYIVIGVGNSALTLHWF
RK (SEQ ID NO:175)
191. The stabilized recombinant flavivirus E glycoprotein of any one of clauses 3, 5, 6, comprising the amino acid sequence **SEQ ID NO:169**, wherein said amino acid sequence comprises the following amino acid substitutions, wherein the numbering is based on the reference amino acid sequence of an E glycoprotein of dengue virus serotype 4 (DENV4) identified as **SEQ ID NO:169**: H27P and T481.
192. The stabilized recombinant flavivirus E glycoprotein of any one of clauses 3, 5, 6, or 191, comprising the amino acid sequence: 
MRCVGVGNRDFVEGVSGGAWVDLVLEPGGCVTTMAQGKPTLDFELTKITAKEVAL
LRTYCIEASISNITTATRCPTQGEPYLKEEQDQQYICRRDVVDRGWGNGCGLFGKGG
VVTCAKFSCSGKITGNLVQIENLEYTVVVTVHNGDTHAVGNDTSNHGVTAMITPRSP
SVEVKLPDYGELTLDCEPRSGIDFNEMILMKMKKKTWLVHKQWFLDLPLPWTAGA
DTSEVHWNYKERMVTFKVPHAKRQDVTVLGSQEGAMHSALAGATEVDSGDGNHM
FAGHLKCKVRMEKLRIKGMSYTMCSGKFSIDKEMAETQHGTTVVKVKYEGAGAPC
KVPIEIRDVNKEKVVGRIISSTPLAENTNSVTNIELEPPFGDSYIVIGVGNSALTLHWFR
K (SEQ ID NO:176)
193. The stabilized recombinant flavivirus E glycoprotein of any one of clauses 3, 5, 6, comprising the amino acid sequence **SEQ ID NO:169**, wherein said amino acid sequence comprises the following amino acid substitutions, wherein the numbering is based on the reference amino acid sequence of an E glycoprotein of dengue virus serotype 4 (DENV4) identified as **SEQ ID NO:169:** G29K, T33V, A35M, G106D, A257W, and S262R.
194. The stabilized recombinant flavivirus E glycoprotein of any one of clauses 3, 5, 6, or 193, comprising the amino acid sequence: 
MRCVGVGNRDFVEGVSGGAWVDLVLEHGKCVTVMMQGKPTLDFELTKTTAKEVA
LLRTYCIEASISNITTATRCPTQGEPYLKEEQDQQYICRRDVVDRGWGNGCDLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGWMHRALAGATEVDSGDGN
HMFAGHLKCKVRMEKLRIKGMSYTMCSGKFSIDKEMAETQHGTTVVKVKYEGAGA
PCKVPIEIRDVNKEKVVGRIISSTPLAENTNSVTNIELEPPFGDSYIVIGVGNSALTLHW
FRK (SEQ ID NO:177)
195. The stabilized recombinant flavivirus E glycoprotein of any one of clauses 4-6, comprising the amino acid sequence **SEQ ID NO:178**, wherein said amino acid sequence comprises the following amino acid substitutions, wherein the numbering is based on the reference amino acid sequence of an E glycoprotein of Zika virus (ZIKV) identified as **SEQ ID NO:178:** S29K, V33, and A35M.
196. The stabilized recombinant flavivirus E glycoprotein of any one of clauses 4-6 or 195, comprising the amino acid sequence: 
IRCIGVSNRDFVEGMSGGTWVDIVLEHGKCVTVMMQDKPTVDIELVTTTVSNMAEV
RSYCYEASISDMASDSRCPTQGEAYLDKQSDTQYVCKRTLVDRGWGNGCGLFGKGS
LVTCAKFACSKKMTGKSIQPENLEYRIMLSVHGSQHSGMIVNDTGHETDENRAKVEI
TPNSPRAEATLGGFGSLGLDCEPRTGLDFSDLYYLTMNNKHWLVHKEWFHDIPLPW
HAGADTGTPHWNNKEALVEFKDAHAKRQTVVVLGSQEGAVHTALAGALEAEMDG
AKGRLSSGHLKCRLKMDKLRLKGVSYSLCTAAFTFTKIPAETLHGTVTVEVQYAGT
DGPCKVPAQMAVDMQTLTPVGRLITANPVITESTENSKMMLELDPPFGDSYIVIGVG
EKKITHHWHRSG (SEQ ID NO:179)
197. The stabilized recombinant flavivirus E glycoprotein of any one of clauses 4-6, comprising the amino acid sequence **SEQ ID NO:178**, wherein said amino acid sequence comprises the following amino acid substitutions, wherein the numbering is based on the reference amino acid sequence of an E glycoprotein of Zika virus (ZIKV) identified as **SEQ ID NO: 178:** A264W and T267R.
198. The stabilized recombinant flavivirus E glycoprotein of any one of clauses 4-6 or 197, comprising the amino acid sequence: 
IRCIGVSNRDFVEGMSGGTWVDIVLEHGGCVTVMAQDKPTVDIELVTTTVSNMAEV
RSYCYEASISDMASDSRCPTQGEAYLDKQSDTQYVCKRTLVDRGWGNGCGLFGKGS
LVTCAKFACSKKMTGKSIQPENLEYRIMLSVHGSQHSGMIVNDTGHETDENRAKVEI
TPNSPRAEATLGGFGSLGLDCEPRTGLDFSDLYYLTMNNKHWLVHKEWFHDIPLPW
HAGADTGTPHWNNKEALVEFKDAHAKRQTVVVLGSQEGWVHRALAGALEAEMD
GAKGRLSSGHLKCRLKMDKLRLKGVSYSLCTAAFTFTKIPAETLHGTVTVEVQYAG
TDGPCKVPAQMAVDMQTLTPVGRLITANPVITESTENSKMMLELDPPFGDSYIVIGV
GEKKITHHWHRSG (SEQ ID NO:180)
199. The stabilized recombinant flavivirus E glycoprotein of any one of clauses 4-6, comprising the amino acid sequence **SEQ ID NO:178**, wherein said amino acid sequence comprises the following amino acid substitutions, wherein the numbering is based on the reference amino acid sequence of an E glycoprotein of Zika virus (ZIKV) identified as **SEQ ID NO: 178:** G106D.
200. The stabilized recombinant flavivirus E glycoprotein of any one of clauses 4-6 or 199, comprising the amino acid sequence: 
IRCIGVSNRDFVEGMSGGTWVDIVLEHGGCVTVMAQDKPTVDIELVTTTVSNMAEV
RSYCYEASISDMASDSRCPTQGEAYLDKQSDTQYVCKRTLVDRGWGNGCDLFGKGS
LVTCAKFACSKKMTGKSIQPENLEYRIMLSVHGSQHSGMIVNDTGHETDENRAKVEI
TPNSPRAEATLGGFGSLGLDCEPRTGLDFSDLYYLTMNNKHWLVHKEWFHDIPLPW
HAGADTGTPHWNNKEALVEFKDAHAKRQTVVVLGSQEGAVHTALAGALEAEMDG
AKGRLSSGHLKCRLKMDKLRLKGVSYSLCTAAFTFTKIPAETLHGTVTVEVQYAGT
DGPCKVPAQMAVDMQTLTPVGRLITANPVITESTENSKMMLELDPPFGDSYIVIGVG
EKKITHHWHRSG (SEQ ID NO:181)
201. The stabilized recombinant flavivirus E glycoprotein of any one of clauses 3, 5, or 6, comprising the amino acid sequence **SEQ ID NO:6,** wherein said amino acid sequence comprises the following amino acid substitutions: S255E.
202. The stabilized recombinant flavivirus E glycoprotein of any one of clauses 3, 5, 6, or 201, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGEQEGAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:40)
203. The stabilized recombinant flavivirus E glycoprotein of any one of clauses 3, 5, or 6, comprising the amino acid sequence **SEQ ID NO:6,** wherein said amino acid sequence comprises the following amino acid substitutions: G258E.
204. The stabilized recombinant flavivirus E glycoprotein of any one of clauses 3, 5, 6, or 203, comprising the amino acid sequence: 
MRCIGMSNRDFVEGVSGGSWVDIVLEHGSCVTTMAKNKPTLDFELIKTEAKQPATLRKYCIE
AKLTNTTTESRCPTQGEPSLNEEQDKRFVCKHSMVDRGWGNGCGLFGKGGIVTCAMFRCKKN
MEGKVVQPENLEYTIVITPHSGEEHAVGNDTGKHGKEIKITPQSSITEAELTGYGTVTMECS
PRTGLDFNEMVLLQMENKAWLVHRQWFLDLPLPWLPGADTQGSNWIQKETLVTFKNPHAKKQ
DVVVLGSQEEAMHTALTGATEIQMSSGNLLFTGHLKCRLRMDKLQLKGMSYSMCTGKFKVVK
EIAETQHGTIVIRVQYEGDGSPCKIPFEIMDLEKRHVLGRLITVNPIVTEKDSPVNIEAEPP
FGDSYIIIGVEPGQLKLNWFKK (SEQ ID NO:42)
205. A stabilized recombinant flavivirus E glycoprotein dimer comprising two flavivirus E glycoproteins of any one of clauses 1-200.
206. The stabilized recombinant flavivirus E glycoprotein dimer of clause 205, wherein the dimer Tm¹ melting point of about 0.5 °C to about 45 °C higher than the Tm¹ melting point of a wildtype flavivirus E glycoprotein of the corresponding backbone.
207. The stabilized recombinant flavivirus E glycoprotein dimer of clause 205 or 206, wherein the dimer has a dimer affinity (K_{d}) of about 18 µM or lower as measured at 37 °C.
208. A flavivirus particle or virus like particle (VLP) comprising the E glycoprotein of any of clauses 1-204.
209. An isolated nucleic acid molecule encoding the E glycoprotein of any of clauses 1-204.
210. An isolated nucleic acid molecule encoding the flavivirus particle or VLP of clause 208.
211. A population of flavivirus particles comprising the flavivirus particle of clause 208.
212. A composition comprising the E glycoprotein of any of clauses 1-204, the E glycoprotein dimer of any one of clauses 205-207, the flavivirus particle or VLP of clause 208, the nucleic acid molecule of clause 209 or 210, and/or the population of clause 211, in a pharmaceutically acceptable carrier.
213. A method of producing a virus like particle (VLP) comprising the E glycoprotein of any of clauses 1-204, wherein the method does not comprise co-expression of pr.
214. A method of producing an immune response to a flavivirus in a subject, comprising administering to the subject an effective amount of the E glycoprotein of any of clauses 1-204, the E glycoprotein dimer of any one of clauses 205-207, the flavivirus particle or VLP of clause 208, the nucleic acid molecule of clause 209 or 210, the population of clause 211, the composition of clause 212 and any combination thereof.
215. A method of treating a flavivirus infection in a subject, comprising administering to the subject an effective amount of the E glycoprotein of any of clauses 1-204, the E glycoprotein dimer of any one of clauses 205-207, the flavivirus particle or VLP of clause 208, the nucleic acid molecule of clause 209 or 210, the population of clause 211, the composition of clause 212 and any combination thereof.
216. A method of preventing a disorder associated with a flavivirus infection in a subject, comprising administering to the subject an effective amount of the E glycoprotein of any of clauses 1-204, the E glycoprotein dimer of any one of clauses 205-207, the flavivirus particle or VLP of clause 208, the nucleic acid molecule of clauses 209 or 210, the population of clause 211, and/or the composition of clause 212 and any combination thereof.
217. A method of protecting a subject from the effects of a flavivirus infection, comprising administering to the subject an effective amount of the E glycoprotein of any of clauses 1-204, the E glycoprotein dimer of any one of clauses 205-207, the flavivirus particle or VLP of clause 208, the nucleic acid molecule of clause 209 or 2010, the population of clause 211, the composition of clause 212 and any combination thereof.
218. A method of identifying the presence of a neutralizing antibody to a flavivirus in a biological sample from a subject, comprising:
   a) administering a composition comprising the E glycoprotein of any of clauses 1-204 or the E glycoprotein dimer of any one of clauses 205-207 to the subject in an amount effective to induce an antibody response to the E glycoprotein;
   b) contacting a biological sample from the subject with flavivirus particles comprising the E glycoprotein of step (a) above under conditions whereby neutralization of the flavivirus particles can be detected; and
   c) detecting neutralization in step (b), thereby identifying the presence of a neutralizing antibody to the flavivirus in the biological sample from the subject.
219. A method of identifying the presence of a neutralizing antibody to a flavivirus in a biological sample from a subject, comprising:
   a) contacting a biological sample from a subject that has been administered a E glycoprotein of any of clauses 1-204 or a E glycoprotein dimer of any one of clauses 205-207 with flavivirus particles comprising the E glycoprotein under conditions whereby neutralization of the flavivirus particles can be detected; and
   b) detecting neutralization in step (a), thereby identifying the presence of a neutralizing antibody to the flavivirus in the biological sample from the subject.
220. A method of identifying an immunogenic composition that induces a neutralizing antibody to a flavivirus in a subject, the method comprising:
   a) administering an immunogenic composition comprising the E glycoprotein of any of clauses 1-204 or the E glycoprotein dimer of any one of clauses 205-207 to a subject in an amount effective to induce an antibody response to the E glycoprotein;
   b) contacting a biological sample from the subject with flavivirus particles comprising the E glycoprotein of step (a) under conditions whereby neutralization of the flavivirus particles can be detected;
   c) determining if the biological sample comprises an antibody that neutralizes flavivirus particles comprising the E glycoprotein of step (a); and
   d) identifying the immunogenic composition as inducing a neutralizing antibody to the flavivirus in the subject if the biological sample comprises an antibody that neutralizes flavivirus particles comprising the E glycoprotein of (a).
221. A method of identifying an immunogenic composition that induces a neutralizing antibody to a flavivirus in a subject, the method comprising:
   a) contacting a biological sample from a subject that has been administered an immunogenic composition comprising the E glycoprotein of any of clauses 1-204 or the E glycoprotein dimer of any one of clauses 205-207 with flavivirus particles comprising the E glycoprotein under conditions whereby neutralization of the flavivirus particles can be detected;
   b) determining if the biological sample comprises an antibody that neutralizes flavivirus particles comprising the E glycoprotein of step (a); and
   c) identifying the immunogenic composition as inducing a neutralizing antibody to the flavivirus in the subject if the biological sample comprises an antibody that neutralizes flavivirus particles comprising the E glycoprotein of (a).
222. A method of detecting an antibody to a flavivirus in a sample, comprising:
   a) contacting the sample with E glycoprotein of any of clauses 1-204 or the E glycoprotein dimer of any one of clauses 205-207 under conditions whereby an antigen/antibody complex can form; and
   b) detecting formation of an antigen/antibody complex, thereby detecting an antibody to the flavivirus in the sample.
223. A method of identifying an antibody to a flavivirus in a biological sample from a subject, comprising:
   a) administering a composition comprising E glycoprotein of any of clauses 1-204 or the E glycoprotein dimer of any one of clauses 205-207 to the subject in an amount effective to induce an antibody response to the E glycoprotein;
   b) contacting a biological sample from the subject with the E glycoprotein of (a) under conditions whereby an antigen/antibody complex can form; and
   c) detecting formation of an antigen/antibody complex, thereby identifying an antibody to the flavivirus in the biological sample from the subject.
224. A method of identifying an antibody to a flavivirus in a biological sample from a subject, comprising:
   a) contacting a biological sample from a subject that has been administered an immunogenic composition comprising E glycoprotein of any of clauses 1-204 or the E glycoprotein dimer of any one of clauses 205-207 with the E glycoprotein under conditions whereby an antigen/antibody complex can form; and
   b) detecting formation of an antigen/antibody complex, thereby identifying an antibody to the flavivirus in the biological sample from the subject.
225. A method of identifying an immunogenic composition that induces an antibody to a flavivirus in a subject, the method comprising:
   a) contacting a biological sample from a subject that has been administered an immunogenic composition comprising the E glycoprotein of any of clauses 1-204 or the E glycoprotein dimer of any one of clauses 205-207 with the E glycoprotein under conditions whereby an antigen/antibody complex can form; and
   b) detecting formation of an antigen/antibody complex, thereby identifying an immunogenic composition that induces an antibody to the flavivirus in the subject.
226. A method of identifying an immunogenic composition that induces a neutralizing antibody to a flavivirus in a subject, comprising:
   a) administering an immunogenic composition comprising the E glycoprotein of any of clauses 1-204 or the E glycoprotein dimer of any one of clauses 205-207 to a subject in an amount effective to induce an antibody response to the E glycoprotein;
   b) contacting a biological sample from the subject with the E glycoprotein of (a) under conditions whereby an antigen/antibody complex can form; and
   c) detecting formation an antigen/antibody complex, thereby identifying an immunogenic composition that induces a neutralizing antibody to the flavivirus in the subj ect.
227. A method of producing a stabilized recombinant E glycoprotein of any one of clauses 1-200, comprising:
   introducing one or more amino acid substitution into a flavivirus E glycoprotein backbone, wherein the one or more amino acid substitution are selected from the group consisting of 2M, 6L, 8L, 9V, 13F, 14A, 15E, 27P, 29Y, 29K, 32V, 33V, 34L, 35M, 44M, 44L, 48I, 106D, 107C, 131I, 144Y, 154L, 154M, 191Y, 198W, 204F, 205L, 206F, 209D, 244Q, 244F, 246I, 246Y, 251F, 255E, 256Y, 258A, 258E, 259V, 259W, 259C, 260L, 261L, 261F, 262H, 262R, 262Y, 263W, 263L, 266W, 270V, 277M, 279W, 280A, 280P, 289W, 299L, 313C, 316M, 330A, 359Y, 373D, 375L, 377V, 386I, 390Q, 392R,393R, and/or any combination thereof, wherein the numbering is based on the reference amino acid sequence of an E glycoprotein of dengue virus serotype 2 (DENV2) identified as SEQ **ID** NO:6,
   thereby stabilizing the flavivirus E glycoprotein into dimer conformation.

## Claims

1. A stabilized recombinant flavivirus E glycoprotein, comprising a flavivirus E glycoprotein backbone; and one or more amino acid substitution selected from the group consisting of 2M, 6L, 8L, 9V, 13F, 14A, 15E, 27P, 29Y, 29K, 32V, 33V, 34L, 35M, 44M, 44L, 48I, 106D, 107C, 131I, 144Y, 154L, 154M, 191Y, 198W, 204F, 205L, 206F, 209D, 244Q, 244F, 246I, 246Y, 251F, 255E, 256Y, 258A, 258E, 259V, 259W, 259C, 260L, 261L, 261F, 262H, 262R, 262Y, 263W, 263L, 266W, 270V, 277M, 279W, 280A, 280P, 289W, 299L, 313C, 316M, 330A, 359Y, 373D, 375L, 377V, 386I, 390Q, 392R, 393R, and/or any combination thereof, wherein the numbering is based on the reference amino acid sequence of an E glycoprotein of dengue virus serotype 2 (DENV2) identified as SEQ ID NO:6, wherein
i) the flavivirus E glycoprotein backbone comprises a dengue virus backbone, and comprises the amino acid sequence **SEQ ID NO:6,** wherein said amino acid sequence comprises the following amino acid substitutions: A259W and T262R; G106D; S29K, T33V, and A35M; or F279W and T280P, wherein the numbering is based on the reference amino acid sequence of an E glycoprotein of dengue virus serotype 2 (DENV2) identified as **SEQ ID NO:6,** or
comprises the amino acid sequence **SEQ ID NO:147**, wherein said amino acid sequence comprises the following amino acid substitutions, wherein the numbering is based on the reference amino acid sequence of an E glycoprotein of dengue virus serotype 1 (DENV1) identified as **SEQ ID NO:147:** S29K, T33V, and A35M; A259W and S262R; G106D; or F279W and A280P, or
comprises the amino acid sequence **SEQ ID NO:152**, wherein said amino acid sequence comprises the following amino acid substitutions, wherein the numbering is based on the reference amino acid sequence of an E glycoprotein of dengue virus serotype 3 (DENV3) identified as **SEQ ID NO:152:** G106D; or G29K, T33V, and A35M, or
comprises the amino acid sequence **SEQ ID NO:169**, wherein said amino acid sequence comprises the following amino acid substitutions, wherein the numbering is based on the reference amino acid sequence of an E glycoprotein of dengue virus serotype 4 (DENV4) identified as **SEQ ID NO:169:** G29K, T33V, and A35M; A259W and S262R; G106D; or F279W and A280P, or
ii) wherein the flavivirus E glycoprotein backbone comprises a Zika virus (ZIKV) backbone, and
comprises the amino acid sequence **SEQ ID NO:178**, wherein said amino acid sequence comprises the following amino acid substitutions, wherein the numbering is based on the reference amino acid sequence of an E glycoprotein of Zika virus (ZIKV) identified as **SEQ ID NO:178:** S29K, V33, and A35M; or G106D.

2. The stabilized recombinant flavivirus E glycoprotein of claim 1, wherein the E glycoprotein backbone comprises a soluble E glycoprotein ectodomain of domains DI, DII, and DIII.

3. The stabilized recombinant flavivirus E glycoprotein of any one of claims 1-2, wherein the E glycoprotein comprises at least one quaternary epitope.

4. The stabilized recombinant flavivirus E glycoprotein of any one of claims 1-3, comprising the amino acid sequence: 
MRCIGMSNRDFVEGVSGGSWVDIVLEHGSCVTTMAKNKPTLDFELIKTEAKQPATL
RKYCIEAKLTNTTTESRCPTQGEPSLNEEQDKRFVCKHSMVDRGWGNGCGLFGKGG
IVTCAMFRCKKNMEGKVVQPENLEYTIVITPHSGEEHAVGNDTGKHGKEIKITPQSSI
TEAELTGYGTVTMECSPRTGLDFNEMVLLQMENKAWLVHRQWFLDLPLPWLPGAD
TQGSNWIQKETLVTFKNPHAKKQDVVVLGSQEGWMHRALTGATEIQMSSGNLLFT
GHLKCRLRMDKLQLKGMSYSMCTGKFKVVKEIAETQHGTIVIRVQYEGDGSPCKIPF
EIMDLEKRHVLGRLITVNPIVTEKDSPVNIEAEPPFGDSYIIIGVEPGQLKLNWFKK
(SEQ ID NO:26),
 or 
MRCIGMSNRDFVEGVSGGSWVDIVLEHGSCVTTMAKNKPTLDFELIKTEAKQPATL
RKYCIEAKLTNTTTESRCPTQGEPSLNEEQDKRFVCKHSMVDRGWGNGCDLFGKGG
IVTCAMFRCKKNMEGKVVQPENLEYTIVITPHSGEEHAVGNDTGKHGKEIKITPQSSI
TEAELTGYGTVTMECSPRTGLDFNEMVLLQMENKAWLVHRQWFLDLPLPWLPGAD
TQGSNWIQKETLVTFKNPHAKKQDVVVLGSQEGAMHTALTGATEIQMSSGNLLFTG
HLKCRLRMDKLQLKGMSYSMCTGKFKVVKEIAETQHGTIVIRVQYEGDGSPCKIPFE
IMDLEKRHVLGRLITVNPIVTEKDSPVNIEAEPPFGDSYIIIGVEPGQLKLNWFKK
(SEQ ID NO:38),
 or 
MRCIGMSNRDFVEGVSGGSWVDIVLEHGKCVTVMMKNKPTLDFELIKTEAKQPATL
RKYCIEAKLTNTTTESRCPTQGEPSLNEEQDKRFVCKHSMVDRGWGNGCGLFGKGG
IVTCAMFRCKKNMEGKVVQPENLEYTIVITPHSGEEHAVGNDTGKHGKEIKITPQSSI
TEAELTGYGTVTMECSPRTGLDFNEMVLLQMENKAWLVHRQWFLDLPLPWLPGAD
 
TQGSNWIQKETLVTFKNPHAKKQDVVVLGSQEGAMHTALTGATEIQMSSGNLLFTG
HLKCRLRMDKLQLKGMSYSMCTGKFKVVKEIAETQHGTIVIRVQYEGDGSPCKIPFE
IMDLEKRHVLGRLITVNPIVTEKDSPVNIEAEPPFGDSYIIIGVEPGQLKLNWFKK
(SEQ ID NO:50),
 or 
MRCIGMSNRDFVEGVSGGSWVDIVLEHGSCVTTMAKNKPTLDFELIKTEAKQPATL
RKYCIEAKLTNTTTESRCPTQGEPSLNEEQDKRFVCKHSMVDRGWGNGCGLFGKGG
IVTCAMFRCKKNMEGKVVQPENLEYTIVITPHSGEEHAVGNDTGKHGKEIKITPQSSI
TEAELTGYGTVTMECSPRTGLDFNEMVLLQMENKAWLVHRQWFLDLPLPWLPGAD
TQGSNWIQKETLVTFKNPHAKKQDVVVLGSQEGAMHTALTGATEIQMSSGNLLWP
GHLKCRLRMDKLQLKGMSYSMCTGKFKVVKEIAETQHGTIVIRVQYEGDGSPCKIPF
EIMDLEKRHVLGRLITVNPIVTEKDSPVNIEAEPPFGDSYIIIGVEPGQLKLNWFKK
(SEQ ID NO:80),
 or 
MRCVGIGNRDFVEGLSGATWVDVVLEHGKCVTVMMKDKPTLDIELLKTEVTNPAV
LRKLCIEAKISNTTTDSRCPTQGEATLVEEQDTNFVCRRTFVDRGWGNGCGLFGKGS
LITCAKFKCVTKLEGKIVQYENLKYSVIVTVHTGDQHQVGNETTEHGTTATITPQAPT
SEIQLTDYGALTLDCSPRTGLDFNEMVLLTMEKKSWLVHKQWFLDLPLPWTSGAST
SQETWNRQDLLVTFKTAHAKKQEVVVLGSQEGAMHTALTGATEIQTSGTTTIFAGH
LKCRLKMDKLTLKGMSYVMCTGSFKLEKEVAETQHGTVLVQVKYEGTDAPCKIPFS
SQDEKGVTQNGRLITANPIVTDKEKPVNIEAEPPFGESYIVVGAGEKALKLSWFKK
(SEQ ID NO:148),
 or 
MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCGLFGKGSLI
TCAKFKCVTKLEGKIVQYENLKYSVIVTVHTGDQHQVGNETTEHGTTATITPQAPTS
EIQLTDYGALTLDCSPRTGLDFNEMVLLTMEKKSWLVHKQWFLDLPLPWTSGASTS
QETWNRQDLLVTFKTAHAKKQEVVVLGSQEGWMHRALTGATEIQTSGTTTIFAGHL
KCRLKMDKLTLKGMSYVMCTGSFKLEKEVAETQHGTVLVQVKYEGTDAPCKIPFSS
QDEKGVTQNGRLITANPIVTDKEKPVNIEAEPPFGESYIVVGAGEKALKLSWFKK(SE
Q ID NO:149),
 or 
MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCDLFGKGSLI
TCAKFKCVTKLEGKIVQYENLKYSVIVTVHTGDQHQVGNETTEHGTTATITPQAPTS
EIQLTDYGALTLDCSPRTGLDFNEMVLLTMEKKSWLVHKQWFLDLPLPWTSGASTS
QETWNRQDLLVTFKTAHAKKQEVVVLGSQEGAMHTALTGATEIQTSGTTTIFAGHL
KCRLKMDKLTLKGMSYVMCTGSFKLEKEVAETQHGTVLVQVKYEGTDAPCKIPFSS
 
QDEKGVTQNGRLITANPIVTDKEKPVNIEAEPPFGESYIVVGAGEKALKLSWFKK
(SEQ ID NO:150),
 or 
MRCVGIGNRDFVEGLSGATWVDVVLEHGSCVTTMAKDKPTLDIELLKTEVTNPAVL
RKLCIEAKISNTTTDSRCPTQGEATLVEEQDTNFVCRRTFVDRGWGNGCGLFGKGSLI
TCAKFKCVTKLEGKIVQYENLKYSVIVTVHTGDQHQVGNETTEHGTTATITPQAPTS
EIQLTDYGALTLDCSPRTGLDFNEMVLLTMEKKSWLVHKQWFLDLPLPWTSGASTS
QETWNRQDLLVTFKTAHAKKQEVVVLGSQEGAMHTALTGATEIQTSGTTTIWPGHL
KCRLKMDKLTLKGMSYVMCTGSFKLEKEVAETQHGTVLVQVKYEGTDAPCKIPFSS
QDEKGVTQNGRLITANPIVTDKEKPVNIEAEPPFGESYIVVGAGEKALKLSWFKK
(SEQ ID NO:151),
 or 
MRCVGVGNRDFVEGLSGATWVDVVLEHGKCVTVMMKNKPTLDIELQKTEATQLA
TLRKLCIEGKITNITTDSRCPTQGEAILPEEQDQNYVCKHTYVDRGWGNGCGLFGKG
SLVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK (SEQ
ID NO:153),
 or 
MRCVGVGNRDFVEGLSGATWVDVVLEHGGCVTTMAKNKPTLDIELQKTEATQLAT
LRKLCIEGKITNITTDSRCPTQGEAILPEEQDQNYVCKHTYVDRGWGNGCDLFGKGS
LVTCAKFQCLESIEGKVVQHENLKYTVIITVHTGDQHQVGNETQGVTAEITPQASTV
EAILPEYGTLGLECSPRTGLDFNEMILLTMKNKAWMVHRQWFFDLPLPWTSGATTE
TPTWNRKELLVTFKNAHAKKQEVVVLGSQEGAMHTALTGATEIQNSGGTSIFAGHL
KCRLKMDKLELKGMSYAMCLNTFVLKKEVSETQHGTILIKVEYKGEDAPCKIPFSTE
DGQGKAHNGRLITANPVVTKKEEPVNIEAEPPFGESNIVIGIGDKALKINWYKK (SEQ
ID NO:155),
 or 
MRCVGVGNRDFVEGVSGGAWVDLVLEHGKCVTVMMQGKPTLDFELTKTTAKEVA
LLRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MFAGHLKCKVRMEKLRIKGMSYTMCSGKFSIDKEMAETQHGTTVVKVKYEGAGAP
CKVPIEIRDVNKEKVVGRIISSTPLAENTNSVTNIELEPPFGDSYIVIGVGNSALTLHWF
RK (SEQ ID NO:170),
 or 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGWMHRALAGATEVDSGDGN
HMFAGHLKCKVRMEKLRIKGMSYTMCSGKFSIDKEMAETQHGTTVVKVKYEGAGA
PCKVPIEIRDVNKEKVVGRIISSTPLAENTNSVTNIELEPPFGDSYIVIGVGNSALTLHW
FRK (SEQ ID NO:171),
 or 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCDLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MFAGHLKCKVRMEKLRIKGMSYTMCSGKFSIDKEMAETQHGTTVVKVKYEGAGAP
CKVPIEIRDVNKEKVVGRIISSTPLAENTNSVTNIELEPPFGDSYIVIGVGNSALTLHWF
RK (SEQ ID NO:172),
 or 
MRCVGVGNRDFVEGVSGGAWVDLVLEHGGCVTTMAQGKPTLDFELTKTTAKEVA
LLRTYCIEASISNITTATRCPTQGEPYLKEEQDQQYICRRDVVDRGWGNGCGLFGKG
GVVTCAKFSCSGKITGNLVQIENLEYTVVVTVHNGDTHAVGNDTSNHGVTAMITPR
SPSVEVKLPDYGELTLDCEPRSGIDFNEMILMKMKKKTWLVHKQWFLDLPLPWTAG
ADTSEVHWNYKERMVTFKVPHAKRQDVTVLGSQEGAMHSALAGATEVDSGDGNH
MWPAGHLKCKVRMEKLRIKGMSYTMCSGKFSIDKEMAETQHGTTVVKVKYEGAG
APCKVPIEIRDVNKEKVVGRIISSTPLAENTNSVTNIELEPPFGDSYIVIGVGNSALTLH
WFRK (SEQ ID NO:173),
 or 
IRCIGVSNRDFVEGMSGGTWVDIVLEHGKCVTVMMQDKPTVDIELVTTTVSNMAEV
RSYCYEASISDMASDSRCPTQGEAYLDKQSDTQYVCKRTLVDRGWGNGCGLFGKGS
LVTCAKFACSKKMTGKSIQPENLEYRIMLSVHGSQHSGMIVNDTGHETDENRAKVEI
TPNSPRAEATLGGFGSLGLDCEPRTGLDFSDLYYLTMNNKHWLVHKEWFHDIPLPW
HAGADTGTPHWNNKEALVEFKDAHAKRQTVVVLGSQEGAVHTALAGALEAEMDG
AKGRLSSGHLKCRLKMDKLRLKGVSYSLCTAAFTFTKIPAETLHGTVTVEVQYAGT
DGPCKVPAQMAVDMQTLTPVGRLITANPVITESTENSKMMLELDPPFGDSYIVIGVG
EKKITHHWHRSG (SEQ ID NO:179),
 or

IRCIGVSNRDFVEGMSGGTWVDIVLEHGGCVTVMAQDKPTVDIELVTTTVSNMAEV
RSYCYEASISDMASDSRCPTQGEAYLDKQSDTQYVCKRTLVDRGWGNGCDLFGKGS
LVTCAKFACSKKMTGKSIQPENLEYRIMLSVHGSQHSGMIVNDTGHETDENRAKVEI
TPNSPRAEATLGGFGSLGLDCEPRTGLDFSDLYYLTMNNKHWLVHKEWFHDIPLPW
HAGADTGTPHWNNKEALVEFKDAHAKRQTVVVLGSQEGAVHTALAGALEAEMDG
AKGRLSSGHLKCRLKMDKLRLKGVSYSLCTAAFTFTKIPAETLHGTVTVEVQYAGT
DGPCKVPAQMAVDMQTLTPVGRLITANPVITESTENSKMMLELDPPFGDSYIVIGVG
EKKITHHWHRSG (SEQ ID NO:181).

5. A stabilized recombinant flavivirus E glycoprotein dimer comprising two flavivirus E glycoproteins of any one of claims 1-4, optionally
wherein the dimer Tm¹ melting point of about 0.5 °C to about 45 °C higher than the Tm¹ melting point of a wildtype flavivirus E glycoprotein of the corresponding backbone, optionally
wherein the dimer has a dimer affinity (K_{d}) of about 18 µM or lower as measured at 37 °C.

6. A flavivirus particle or virus like particle (VLP) comprising the E glycoprotein of any of claims 1-4.

7. An isolated nucleic acid molecule encoding the E glycoprotein of any of claims 1-4.

8. An isolated nucleic acid molecule encoding the flavivirus particle or VLP of claim 6.

9. A population of flavivirus particles comprising the flavivirus particle of claim 6.

10. A composition comprising the E glycoprotein of any of claims 1-4, the E glycoprotein dimer of claim 5, the flavivirus particle or VLP of claim 6, the nucleic acid molecule of claim 7 or 8, and/or the population of claim 9, in a pharmaceutically acceptable carrier.

11. A method of producing a virus like particle (VLP) comprising the E glycoprotein of any of claims 1-4, wherein the method does not comprise co-expression of pr.

12. The E glycoprotein of any of claims 1-4, the E glycoprotein dimer of claim 5, the flavivirus particle or VLP of claim 6, the nucleic acid molecule of claim 7 or 8, the population of claim 9, the composition of claim 10 and any combination thereof for use in a method of producing an immune response to a flavivirus in a subject, or for use in a method of treating a flavivirus infection in a subject, or for use in a method of preventing a disorder associated with a flavivirus infection in a subject, or for use in a method of protecting a subject from the effects of a flavivirus infection, wherein the method comprises administering to the subject an effective amount of the E glycoprotein, the E glycoprotein dimer, the flavivirus particle or VLP, the nucleic acid molecule, the population or the composition and any combination thereof.

13. A method of identifying the presence of a neutralizing antibody to a flavivirus in a biological sample from a subject, comprising:
a) contacting a biological sample from a subject that has been administered a E glycoprotein of any of claims 1-4 or a E glycoprotein dimer of claim 5 with flavivirus particles comprising the E glycoprotein under conditions whereby neutralization of the flavivirus particles can be detected; and
b) detecting neutralization in step (a), thereby identifying the presence of a neutralizing antibody to the flavivirus in the biological sample from the subject.

14. A method of identifying an immunogenic composition that induces a neutralizing antibody to a flavivirus in a subject, the method comprising:
a) contacting a biological sample from a subject that has been administered an immunogenic composition comprising the E glycoprotein of any of claims 1-4 or the E glycoprotein dimer of claim5 with flavivirus particles comprising the E glycoprotein under conditions whereby neutralization of the flavivirus particles can be detected;
b) determining if the biological sample comprises an antibody that neutralizes flavivirus particles comprising the E glycoprotein of step (a); and
c) identifying the immunogenic composition as inducing a neutralizing antibody to the flavivirus in the subject if the biological sample comprises an antibody that neutralizes flavivirus particles comprising the E glycoprotein of (a).

15. A method of detecting an antibody to a flavivirus in a sample, comprising:
a) contacting the sample with E glycoprotein of any of claims 1-4 or the E glycoprotein dimer of claim 5 under conditions whereby an antigen/antibody complex can form; and
b) detecting formation of an antigen/antibody complex, thereby detecting an antibody to the flavivirus in the sample.

16. A method of identifying an antibody to a flavivirus in a biological sample from a subject, comprising:
a) contacting a biological sample from a subject that has been administered an immunogenic composition comprising E glycoprotein of any of claims 1-4 or the E glycoprotein dimer of claim 5 with the E glycoprotein under conditions whereby an antigen/antibody complex can form; and
b) detecting formation of an antigen/antibody complex, thereby identifying an antibody to the flavivirus in the biological sample from the subject.

17. A method of identifying an immunogenic composition that induces an antibody to a flavivirus in a subject, the method comprising:
a) contacting a biological sample from a subject that has been administered an immunogenic composition comprising the E glycoprotein of any of claims 1-4 or the E glycoprotein dimer of claim 5 with the E glycoprotein under conditions whereby an antigen/antibody complex can form; and
b) detecting formation of an antigen/antibody complex, thereby identifying an immunogenic composition that induces an antibody to the flavivirus in the subject.
